# EUROPEAN PATENT APPLICATION

(11) **EP 1 847 531 A1**
(43) Date of publication of application: **24.10.2007**
(21) Application number: 06713827.1
(22) Date of filing: 09.02.2006
(51) Int. Cl.: C07D 231/38, A61K 31/415, A61K 31/4155, A61K 31/497, A61P 3/10, A61P 43/00, C07D 401/12, C07D 403/12, C07D 403/14, C07D 405/12, C07D 405/14

(54) **PYRAZOLE COMPOUND**

(30) Priority: 09.02.2005 JP 2005033356; 22.12.2005 JP 2005370962
(71) Applicant: Takeda Pharmaceutical Company Limited, Osaka-shi, Osaka 541-0045 (JP)
(72) Inventor: TSUKAMOTO, Tetsuya, TAKEDA PHARMA. CO. LTD., Osaka-shi, Osaka 532-8686 (JP); YAMAMOTO, Takeshi, TAKEDA PHARMA. CO. LTD., Osaka-shi, Osaka 532-8686 (JP); TOKUNOH, Ryosuke, TAKEDA PHARMA. CO. LTD., Osaka-shi, Osaka 532-8686 (JP); KAWAMOTO, Tomohiro, TAKEDA PHARMA. CO. LTD., Osaka-shi, Osaka 532-8686 (JP); OKURA, Masahiro, TAKEDA PHARMA. CO. LTD., Osaka-shi, Osaka 532-8686 (JP); KORI, Masakuni, TAKEDA PHARMA. CO. LTD., Osaka-shi, Osaka 532-8686 (JP); MURASE, Katsuhito, TAKEDA PHARMA. CO. LTD., Osaka-shi, Osaka 532-8686 (JP)
(74) Representative: Jones, Nicholas Andrew
(86) International application number: PCT/JP2006/302686
(87) International publication number: WO 2006/085685

(57) **Abstract**

The present invention provides a pyrazole compound represented by the formula (I): 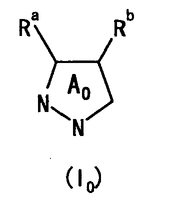 wherein ring A₀ is a pyrazole ring optionally further having 1 or 2 substituents; R^{a} is a substituted carbamoyl group; and R^{b} is an optionally substituted acylamino group, or a salt thereof or a prodrug thereof, which is useful as an agent for the prophylaxis or treatment of GSK-3β related pathology or disease, and a GSK-3β inhibitor including same.

## Description

### Technical Field

The present invention relates to pyrazole compounds having a Glycogen Synthase Kinase 3 (GSK-3) inhibitory activity, which are useful as pharmaceutical agents, and use thereof.

### Background Art

GSK-3 was found to be a kinase that phosphorylates and deactivates glycogen synthase. It has been clarified at present that it is involved in the oxidation and synthesis of fatty acid, or abnormality in insulin signaling pathway via phosphates of various protein groups related to metabolism and signal transduction such as AcylCoA carboxylase, ATP-citrate lyase, Insulin receptor substrate-1 and the like. Moreover, GSK-3 is known to phosphorylate various structural proteins and regulate functions thereof. Particularly, phosphorylation of tau protein has been attracting attention in relation to the onset of Alzheimer's disease. In addition, GSK-3 is involved in phosphorylation of various transcription factors, and particularly, activates activator protein-1, cyclic AMP response element binding protein, nuclear factor of activated T cells, heat shock factor-1, b-catenin, Myc, C/EBP, NF_{κ}-b or the like. Therefore, its inhibitor is expected to be a therapeutic drug for Alzheimer's disease, cerebral apoplexy, manic-depressive illness, schizophrenia, cancer, type II diabetes and obesity.

In insulin signaling pathway, GSK-3 is negatively regulated by phosphorylation via Akt (protein kinase B: also described as PKB). In diabetic patients, increased activity of GSK-3 and synthesis of fatty acids and/or insulin resistance are considered to be synergistically induced by the overlapped occurrence of promoted GSK-3 gene expression and insulin dysfunction. Since GSK-3 positively regulates the process of adipocyte differentiation and/or maturation via phosphorylation of C/EBP, increased GSK-3 activity triggers obesity, which in turn aggravates diabetes. In fact, it has been reported that administration of GSK-3 inhibitor improves insulin resistance of model animals of TYPE II diabetes. We have additionally elucidated as our own findings that GSK-3 inhibitor suppresses adipocyte differentiation and/or maturation, expresses an antiobesity effect, as well as promotes sugar-dependent insulin secretory action of pancreatic β cells. Given these findings in combination, GSK-3 is considered to be additively and/or synergistically involved in the onset of diabetes in the insulin targeting tissues such as liver, skeletal muscle, fat, pancreas and the like, and GSK-3 inhibitor can be an effective therapeutic drug for obesity and/or diabetes because it eliminates these factors.

Activation of GSK-3 in Alzheimer's brain has been reported, and therefore, GSK-3 is considered to be involved in neuritic plaque and neurofibrillary change, which are the two major pathological findings in Alzheimer's disease. In the metabolism of amyloid precursor proteins, GSK-3 is linked to γ secretase to positively regulate the production of β amyloid protein, a main constituent component of neuritic plaque. As for tau protein, which is a main constituent component of neurofibrillary change, GSK-3 is considered to facilitate phosphorylation of the protein, prevent axonal transport, and finally induce neurodegeneration. It is also known that GSK-3 is located downstream of the PI3 kinase - Akt system signal transduction important for the nerve cell survival, and activated during nerve cell death. Accordingly, GSK-3 inhibitor is expected to not only suppress neurodegeneration but also suppress two major pathological findings of Alzheimer's disease. As our own findings, we have clarified that PI3 kinase - Akt system signal transduction plays a key role in neuropoiesis and nerve regeneration and found that inhibition of GSK-3 located downstream thereof can facilitate neuropoiesis. Considering our new findings in combination, there is a possibility that GSK-3 inhibitor suppresses two major pathological findings of Alzheimer's disease and additionally suppresses neurodegeneration, induces neuropoiesis and achieves regeneration of function. It is assumed that GSK-3 inhibitor having the above-mentioned properties can be an ultimate therapeutic drug for Alzheimer's disease, and can also be effective as a therapeutic drug for neurodegenerative diseases such as Parkinson's syndrome and the like, cerebrovascular disorders and the like. Since a report has recently documented that Akt system signal transduction decreases in schizophrenia, GSK-3 inhibitor may become a completely new type of therapeutic drug for schizophrenia.

The following compounds are known as compounds having a GSK-3 inhibitory activity.
(1) Purine, pyrimidine derivatives
   The compounds described in WO200220495 such as 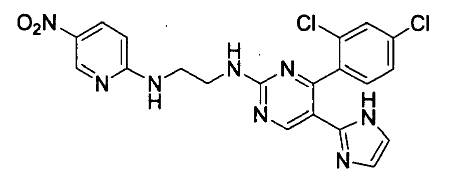 and the like,
   the compounds described in EP 1136099-A1 such as 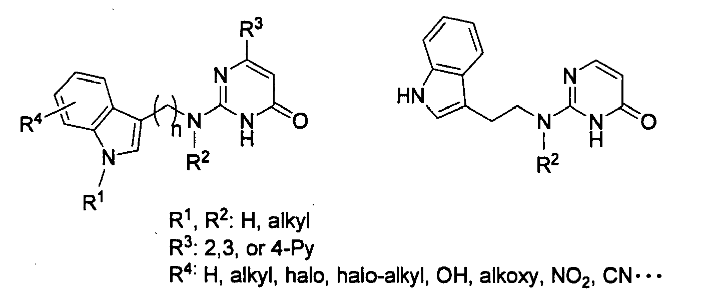 and the like, the compounds described in EP 1136482-A1, EP 1136483, EP 1136486-A1 and W02002/18386-A1 such as 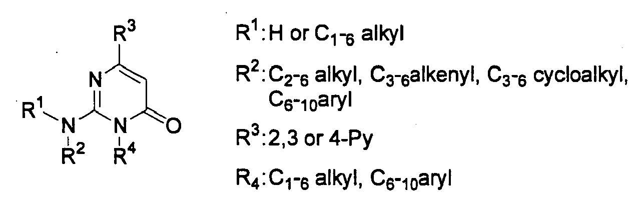 and the like, and the like.
(2) Azole derivatives
   The compounds described in WO2002057259-A2 such as 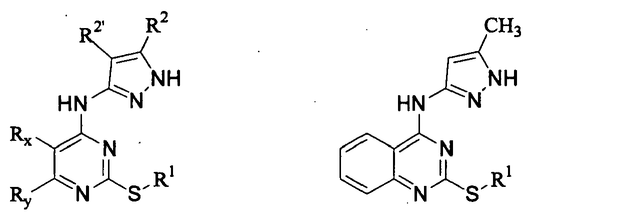 and the like,
   the compounds described in WO2003004478 such as 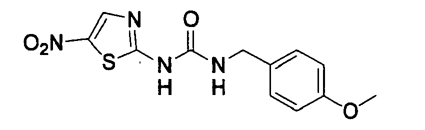 and the like,
   the compounds described in W0200224694-A1 and WO200250073-A1 such as 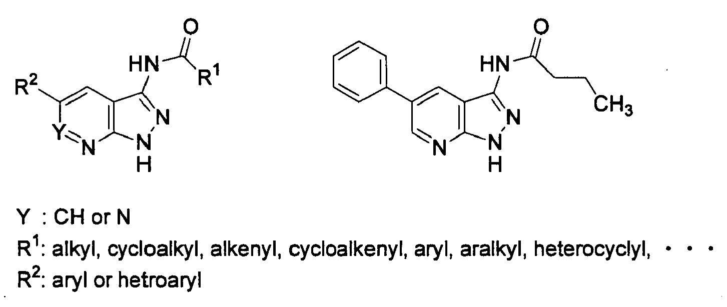 and the like,
   the compounds described in WO 01/56567-A1 such as 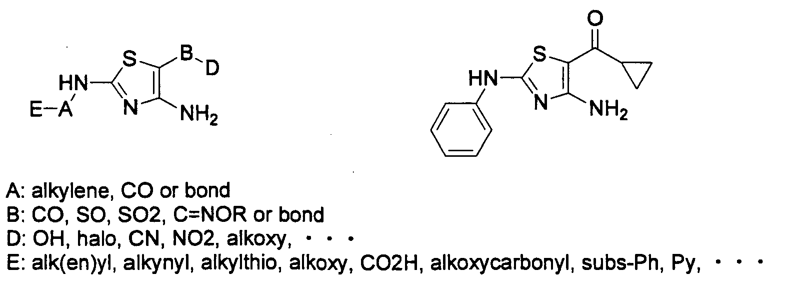 and the like.
(3) Maleimide derivatives
   The compounds described in WO200021927, WO200038675 and WO200174771 such as 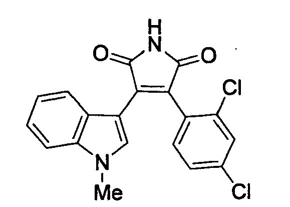 and the like,
   the compounds described in WO2002/10158 such as 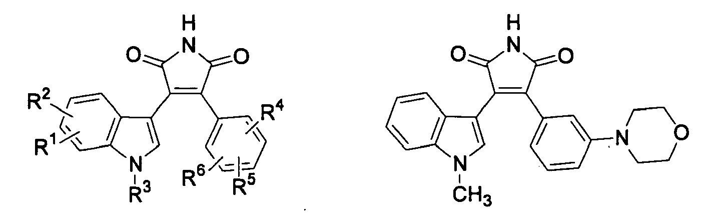 and the like.
(4) Other heterocyclic compounds
   The compounds described in Chemistry & Biology 2000, 7, 51-63 such as 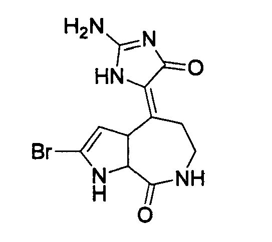 and the like,
   the compounds described in WO 01/60374-A1 such as 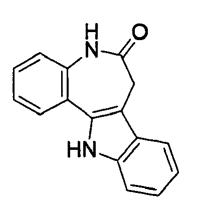 and the like.
(5) As ATP noncompetitive compounds, the compound described in J. Med. Chem., 2002, 45(6), 1292-1299 which is a thiadiazolidinone derivative 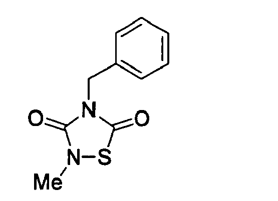 and the like.

On the other hand, the following compounds are known as the pyrazole compounds.
(1) The following compounds have been reported as antibacterial agents or antifungal agents (WO02/094793).
(a) N-methyl-2,5-dimethyl-4-{2-[4-(4-nitrophenoxy)phenyl]acetylamino}-2H-pyrazole-3-carboxamide, and
(b) N-methyl-4-{2-[4-(4-chlorophenoxy)phenyl]acetylamino}-2,5-dimethyl-2H-pyrazole-3-carboxamide.

| | |
|---|---|
| 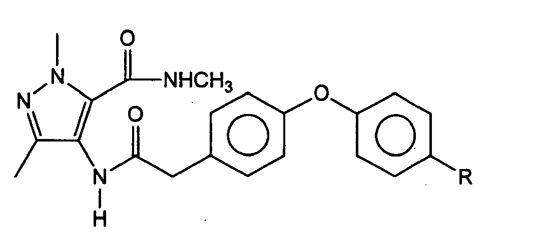 | R = Cl |
| | R = NO₂ |

(2) The following compounds have been reported as phosphodiesterase IV inhibitors (Journal of Computer-Aided Molecular Design, vol. 15, No. 9, pages 767-785, 2001). N-(9-methyl-4-oxo-1-phenyl-3,4,6,7-tetrahydro-[1,4]diazepino[6,7,1-hi]indol-3-yl)-4-acetylamino-2,5-dimethyl-2H-pyrazole-3-carboxamide. 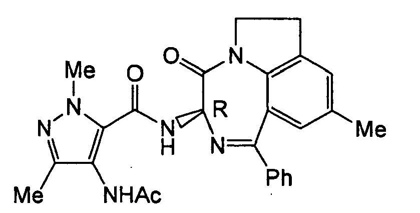
(3) The following compounds have been reported as antibacterial agents (Pamazie, vol. 53, No. 5, pages 346-348, 1998):
(a) N-phenyl-4-benzoylamino-2-ethyl-5-methyl-2H-pyrazole-3-carboxamide,
(b) N-phenyl-2-ethyl-5-methyl-4-(2-nitrobenzoylamino)-2H-pyrazole-3-carboxamide,
(c) N-(4-chlorophenyl)-2-ethyl-5-methyl-4-(4-nitrobenzoylamino)-2H-pyrazole-3-carboxamide, and
(d) N-phenyl-2-ethyl-5-methyl-4-(4-methylbenzoylamino)-2H-pyrazole-3-carboxamide.

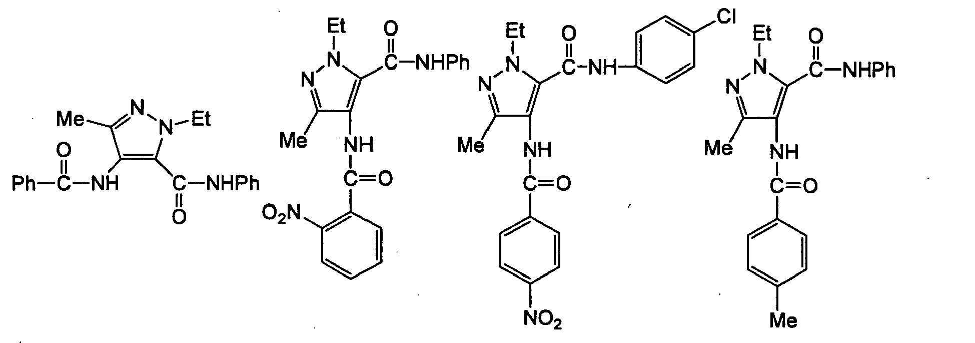

(4) Synthesis of the following compounds has been reported (Journal of Heterocyclic Chemistry, vol. 32, No. 3, pages 835-839, 1995):
(a) N-methyl-N-(2-phenyl-2H-pyrazol-3-yl)-4-acetylamino-2-ethyl-5-methyl-2H-pyrazole-3-carboxamide,
(b) N-methyl-N-(5-methyl-2-phenyl-2H-pyrazol-3-yl)-4-acetylamino-2-ethyl-5-methyl-2H-pyrazole-3-carboxamide,
(c) N-methyl-N-(2-methyl-2H-pyrazol-3-yl)-4-acetylamino-2-ethyl-5-methyl-2H-pyrazole-3-carboxamide, and
(d) N-(2,5-dimethyl-2H-pyrazol-3-yl)-N-methyl-4-acetylamino-2-ethyl-5-methyl-2H-pyrazole-3-carboxamide.

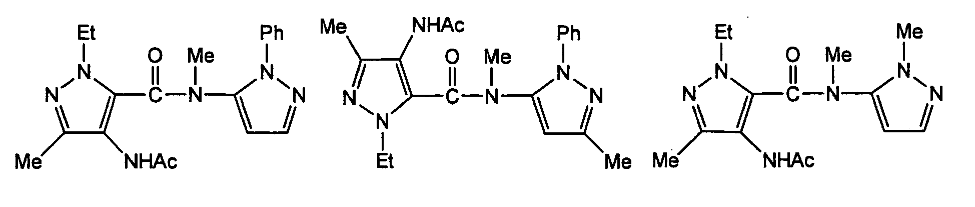

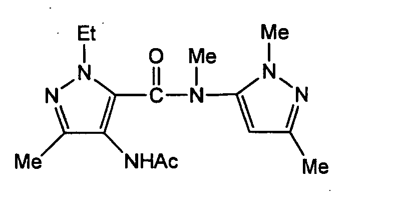

(5) The following compounds have been reported as agrichemicals and insecticides (Journal of Japan Society for Bioscience, Biotechnology and Agrochemistry, vol. 16, No. 4, pages 623-629, 1991 and EP-A-289879):
(a) N-(4-tert-butylbenzyl)-4-acetylamino-2,5-dimethyl-2H-pyrazole-3-carboxamide,
(b) N-(4-tert-butylbenzyl)-4-(2-chloroacetylamino)-2,5-dimethyl-2H-pyrazole-3-carboxamide, and
(c) N-(4-tert-butylbenzyl)-4-benzoylamino-2,5-dimethyl-2H-pyrazole-3-carboxamide.

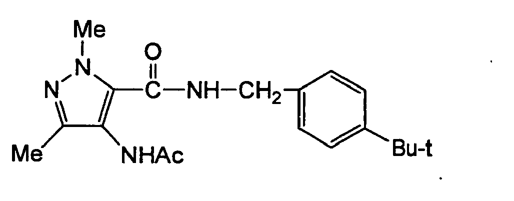

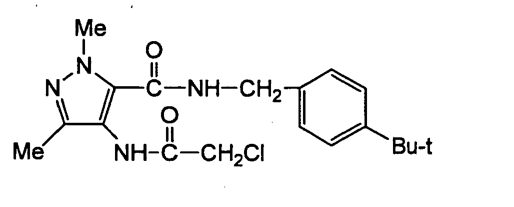

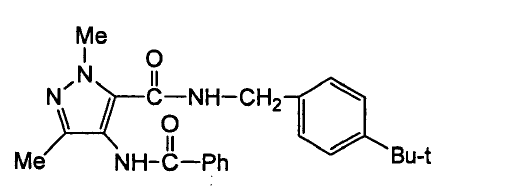

(6) The following compounds have been reported as therapeutic agents for inflammation and cancer, and CXC-chemokine receptor antagonists (WO03/057676 and WO03/031440):
(a) N,N-dimethyl-4-methanesulfonylamino-1-methyl-5-[1-methyl-2,5-dioxo-4-(1-phenylpropylamino)-2,5-dihydro-1H-pyrrol-3-ylamino]-1H-pyrazole-3-carboxamide,
(b) N,N-dimethyl-5-[2,5-dioxo-4-(1-phenylpropylamino)-2,5-dihydro-1H-pyrrol-3-ylamino]-4-methanesulfonylamino-1-methyl-1H-pyrazole-3-carboxamide,
(c) N,N-dimethyl-5-(4-chloro-1-methyl-2,5-dioxo-2,5-dihydro-1H-pyrrol-3-ylamino)-4-methanesulfonylamino-1-methyl-1H-pyrazole-3-carboxamide, and
(d) N,N-dimethyl-5-(4-chloro-1-methyl-2,5-dioxo-2,5-dihydro-1H-pyrrol-3-ylamino)-4-methanesulfonylamino-1-methyl-1H-pyrazole-3-carboxamide.

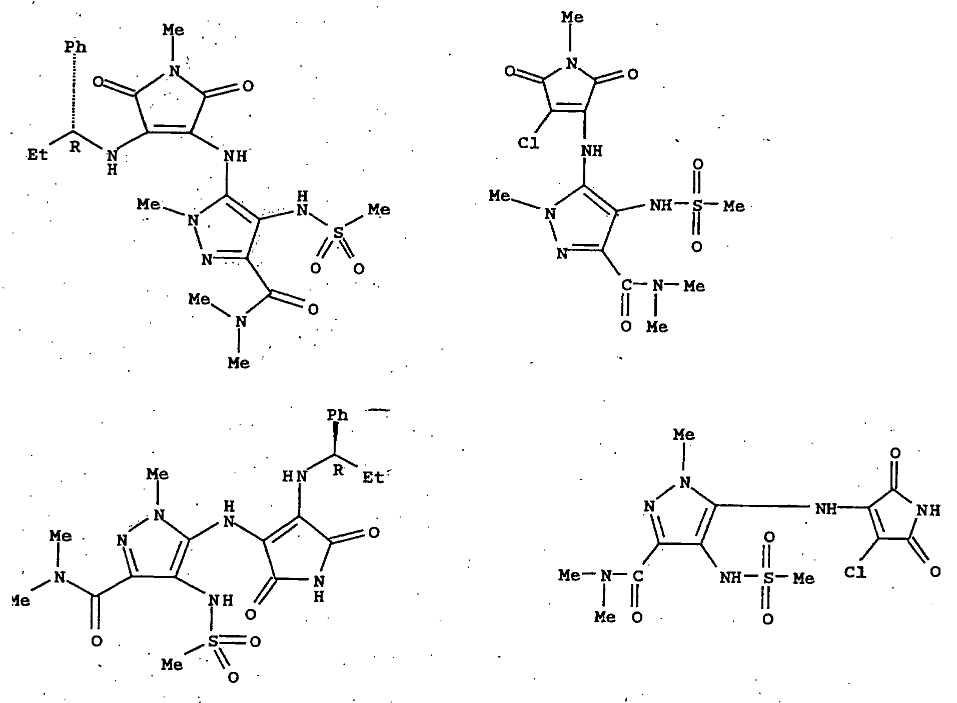

However, it has not been known heretofore that these pyrazole compounds have a GSK-3 inhibitory action.

### Disclosure of the Invention

Conventional compounds having a GSK-3 inhibitory action have some problems to be solved, such as effectiveness (e.g., insufficient GSK-3 inhibitory action, insufficient selectivity to other kinase inhibitory action and the like), and safety (e.g., possible side effects and the like). In addition, since they are not sufficient in the property (stability, solubility and the like), oral absorbability, transferability to target organ and the like, practically satisfactory results as a pharmaceutical agent have not been achieved entirely. Thus, the development of a superior GSK-3 inhibitor effective as a pharmaceutical agent for GSK-3 related pathology or disease has been demanded.

The present invention aims at providing a safe GSK-3 inhibitor useful as an agent for the prophylaxis or treatment of GSK-3 related pathology or disease.

The present inventors have conducted intensive studies and found that the pyrazole compounds represented by the following formulas (I₀) and (I) or salts thereof unexpectedly have a superior GSK-3 specific inhibitory activity based on their specific chemical structures, and further, superior properties of pharmaceutical product such as stability, solubility and the like, and can be safe and useful pharmaceutical agents for the prophylaxis or treatment of GSK-3 related pathology or disease in mammal, which resulted in the completion of the present invention.

Accordingly, the present invention provides the following.
(1) A compound represented by the formula 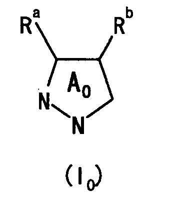 wherein
   ring A₀ is a pyrazole ring optionally further having 1 or 2 substituents;
   R^{a} is a substituted carbamoyl group; and
   R^{b} is an optionally substituted acylamino group]
   or a salt thereof, excluding the following compounds;
   1) N-methyl-2,5-dimethyl-4-{2-[4-(4-nitrophenoxy)phenyl]acetylamino}-2H-pyrazole-3-carboxamide,
   2) N-methyl-4-{2-[4-(4-chlorophenoxy)phenyl]acetylamino}-2,5-dimethyl-2H-pyrazole-3-carboxamide,
   3) N-(9-methyl-4-oxo-1-phenyl-3,4,6,7-tetrahydro-[1,4]diazepino[6,7,1-hi]indol-3-yl)-4-acetylamino-2,5-dimethyl-2H-pyrazole-3-carboxamide,
   4) N-phenyl-4-benzoylamino-2-ethyl-5-methyl-2H-pyrazole-3-carboxamide,
   5) N-phenyl-2-ethyl-5-methyl-4-(2-nitrobenzoylamino)-2H-pyrazole-3-carboxamide,
   6) N-(4-chlorophenyl)-2-ethyl-5-methyl-4-(4-nitrobenzoylamino)-2H-pyrazole-3-carboxamide,
   7) N-phenyl-2-ethyl-5-methyl-4-(4-methylbenzoylamino)-2H-pyrazole-3-carboxamide,
   8) N-methyl-N-(2-phenyl-2H-pyrazol-3-yl)-4-acetylamino-2-ethyl-5-methyl-2H-pyrazole-3-carboxamide,
   9) N-methyl-N-(5-methyl-2-phenyl-2H-pyrazol-3-yl)-4-acetylamino-2-ethyl-5-methyl-2H-pyrazole-3-carboxamide,
   10) N-methyl-N-(2-methyl-2H-pyrazol-3-yl)-4-acetylamino-2-ethyl-5-methyl-2H-pyrazole-3-carboxamide,
   11) N-(2,5-dimethyl-2H-pyrazol-3-yl)-N-methyl-4-acetylamino-2-ethyl-5-methyl-2H-pyrazole-3-carboxamide,
   12) N-(4-tert-butylbenzyl)-4-acetylamino-2,5-dimethyl-2H-pyrazole-3-carboxamide,
   13) N-(4-tert-butylbenzyl)-4-(2-chloroacetylamino)-2,5-dimethyl-2H-pyrazole-3-carboxamide,
   14) N-(4-tert-butylbenzyl)-4-benzoylamino-2,5-dimethyl-2H-pyrazole-3-carboxamide,
   15) N,N-dimethyl-4-methanesulfonylamino-1-methyl-5-[1-methyl-2,5-dioxo-4-(1-phenylpropylamino)-2,5-dihydro-1H-pyrrol-3-ylamino]-1H-pyrazole-3-carboxamide,
   16) N,N-dimethyl-5-[2,5-dioxo-4-(1-phenylpropylamino)-2,5-dihydro-1H-pyrrol-3-ylamino]-4-methanesulfonylamino-1-methyl-1H-pyrazole-3-carboxamide,
   17) N,N-dimethyl-5-(4-chloro-1-methyl-2,5-dioxo-2,5-dihydro-1H-pyrrol-3-ylamino)-4-methanesulfonylamino-1-methyl-1H-pyrazole-3-carboxamide,
   18) N,N-dimethyl-5-(4-chloro-1-methyl-2,5-dioxo-2,5-dihydro-1H-pyrrol-3-ylamino)-4-methanesulfonylamino-1-methyl-1H-pyrazole-3-carboxamide.
(2) The compound of the above-mentioned (1), wherein the compound represented by the formula (I₀) is a compound represented by the formula: 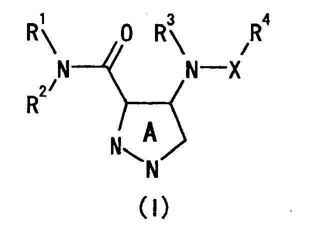 wherein
   R¹ is an optionally substituted hydrocarbon group or an optionally substituted heterocyclic group,
   R² is a hydrogen atom or an optionally substituted hydrocarbon group, or
   R¹ and R² form, together with the adjacent nitrogen atom, an optionally substituted nitrogen-containing heterocycle;
   R³ is a hydrogen atom or an optionally substituted hydrocarbon group;
   R⁴ is an optionally substituted hydrocarbon group, an optionally substituted heterocyclic group, an optionally substituted hydroxy group, or an optionally substituted amino group;
   ring A is a pyrazole ring represented by the formula: 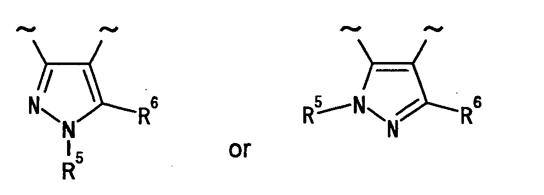 wherein R⁵ is a hydrogen atom, an optionally substituted hydrocarbon group or an optionally substituted acyl group; and R⁶ is a hydrogen atom or an optionally substituted hydrocarbon group; and
   X is a carbonyl group or a sulfonyl group.
(3) The compound of the above-mentioned (2), wherein R² is a hydrogen atom.
(4) The compound of the above-mentioned (2), wherein R¹ is a C₁₋₆ alkyl group, a C₂₋₆ alkenyl group or a C₂₋₆ alkynyl group, which is optionally substituted by 1 to 5 substituents selected from halogen atom, optionally substituted hydroxy group, optionally substituted thio group, optionally substituted sulfinyl group, optionally substituted sulfonyl group, optionally substituted amino group, nitro group, cyano group, and optionally substituted carbonyl group.
(5) The compound of the above-mentioned (2), wherein R¹ is a C₁₋₆ alkyl group, a C₂₋₆ alkenyl group, or a C₂₋₆ alkynyl group optionally having, at substitutable positions, 1 to 5 substituents selected from
   (1) halogen atom;
   (2) hydroxy group;
   (3) amino group;
   (4) nitro group;
   (5) cyano group;
   (7) mono- or di-C₁₋₆ alkyl-amino group;
   (8) mono- or di-C₆₋₁₄ aryl-amino group;
   (9) mono- or di-C₇₋₁₆ aralkyl-amino group;
   (10) N-C₁₋₆ alkyl-N-C₆₋₁₄ aryl-amino group;
   (11) N-C₁₋₆ alkyl-N-(C₁₋₆ alkyl-C₆₋₁₄ aryl)-amino group;
   (12) N-C₁₋₆ alkyl-N-C₇₋₁₆ aralkyl-amino group;
   (14) optionally halogenated C₁₋₆ alkoxy group;
   (15) C₁₋₆ alkylthio group optionally substituted by C₁₋₆ alkoxy group;
   (18) optionally esterified carboxyl group;
   (19) carbamoyl group;
   (20) thiocarbamoyl group;
   (21) mono- or di-C₁₋₆ alkyl-carbamoyl group;
   (22) mono- or di-C₆₋₁₄ aryl-carbamoyl group;
   (23) mono- or di-5- to 7-membered heterocycle-carbamoyl group;
   (24) C₁₋₆ alkyl-carbonylamino group optionally substituted by carboxyl group;
   (25) C₆₋₁₄ aryloxy group optionally substituted by 1 to 3 substituents selected from halogen atom, hydroxy group, amino group, nitro group, cyano group, optionally halogenated C₁₋₆ alkyl group, mono- or di-C₁₋₆ alkyl-amino group, C₆₋₁₄ aryl group, mono- or di-C₆₋₁₄ aryl-amino group, C₃₋₈ cycloalkyl group, C₁₋₆ alkoxy group, C₁₋₆ alkoxy-C₁₋₆ alkoxy group, C₁₋₆ alkylthio group, C₁₋₆ alkylsulfinyl group, C₁₋₆ alkylsulfonyl group, an optionally esterified carboxyl group, carbamoyl group, thiocarbamoyl group, mono- or di-C₁₋₆ alkyl-carbamoyl group, mono- or di-C₆₋₁₄ aryl-carbamoyl group, sulfamoyl group, mono- or di-C₁₋₆ alkyl-sulfamoyl group and mono- or di-C₆₋₁₄ aryl-sulfamoyl group;
   (27) heterocyclic oxy group optionally substituted by 1 to 3 substituents selected from halogen atom, hydroxy group, amino group, nitro group, cyano group, optionally halogenated C₁₋₆ alkyl group, mono- or di-C₁₋₆ alkyl-amino group, C₆₋₁₄ aryl group, mono- or di-C₆₋₁₄ aryl-amino group, C₃₋₈ cycloalkyl group, C₁₋₆ alkoxy group, C₁₋₆ alkoxy-C₁₋₆ alkoxy group, C₁₋₆ alkylthio group, C₁₋₆ alkylsulfinyl group, C₁₋₆ alkylsulfonyl group, an optionally esterified carboxyl group, carbamoyl group, thiocarbamoyl group, mono- or di-C₁₋₆ alkyl-carbamoyl group, mono- or di-C₆₋₁₄ aryl-carbamoyl group, sulfamoyl group, mono- or di-C₁₋₆ alkyl-sulfamoyl group and mono- or di-C₆₋₁₄ aryl-sulfamoyl group;
   (31) a C₇₋₁₆ aralkyloxy group optionally substituted by 1 to 3 substituents selected from halogen atom, hydroxy group, amino group, nitro group, cyano group, optionally halogenated C₁₋₆ alkyl group, mono- or di-C₁₋₆ alkyl-amino group, C₆₋₁₄ aryl group, mono- or di-C₆₋₁₄ aryl-amino group, C₃₋₈ cycloalkyl group, C₁₋₆ alkoxy group, C₁₋₆ alkoxy-C₁₋₆ alkoxy group, C₁₋₆ alkylthio group, C₁₋₆ alkylsulfinyl group, C₁₋₆ alkylsulfonyl group, an optionally esterified carboxyl group, carbamoyl group, thiocarbamoyl group, mono- or di-C₁₋₆ alkyl-carbamoyl group, mono- or di-C₆₋₁₄ aryl-carbamoyl group, sulfamoyl group, mono- or di-C₁₋₆ alkyl-sulfamoyl group and mono- or di-C₆₋₁₄ aryl-sulfamoyl group;
   (32) C₁₋₆ alkylsulfonyloxy group;
   (33) tri-C₁₋₆ alkyl-silyloxy group;
   (34) nitrogen-containing heterocycle-carbonyl group;
   (35) C₆₋₁₄ aryl-carbonyl group;
   (36) C₆₋₁₄ aryl-thio group;
   (37) optionally halogenated C₆₋₁₄ aryl-sulfonyl group;
   (38) nitrogen-containing heterocycle-sulfonyl group; and
   (39) nitrogen-containing heterocycle-amino group optionally substituted by cyano group.
(6) The compound of the above-mentioned (2), wherein R¹ is a C₁₋₆ alkyl group optionally substituted by 1 to 5 substituents selected from halogen atom, optionally substituted hydroxy group, optionally substituted thio group, optionally substituted amino group, nitro group, cyano group, and optionally substituted carbonyl group.
(7) The compound of the above-mentioned (2) wherein R¹ is a C₁₋₆ alkyl group optionally having, at substitutable positions, 1 to 5 substituents selected from
   (1) halogen atom;
   (2) hydroxy group;
   (3) amino group;
   (4) nitro group;
   (5) cyano group;
   (7) mono- or di-C₁₋₆ alkyl-amino group;
   (8) mono- or di-C₆₋₁₄ aryl-amino group;
   (9) mono- or di-C₇₋₁₆ aralkyl-amino group;
   (10) N-C₁₋₆ alkyl-N-C₆₋₁₄ aryl-amino group;
   (11) N-C₁₋₆ alkyl-N-(C₁₋₆ alkyl-C₆₋₁₄ aryl)-amino group;
   (12) N-C₁₋₆ alkyl-N-C₇₋₁₆ aralkyl-amino group;
   (14) optionally halogenated C₁₋₆ alkoxy group;
   (15) C₁₋₆ alkylthio group optionally substituted by C₁₋₆ alkoxy group;
   (18) optionally esterified carboxyl group;
   (19) carbamoyl group;
   (20) thiocarbamoyl group;
   (21) mono- or di-C₁₋₆ alkyl-carbamoyl group;
   (22) mono- or di-C₆₋₁₄ aryl-carbamoyl group;
   (23) mono- or di-5- to 7-membered heterocycle-carbamoyl group;
   (24) C₁₋₆ alkyl-carbonylamino group optionally substituted by carboxyl group;
   (25) C₆₋₁₄ aryloxy group optionally substituted by 1 to 3 substituents selected from halogen atom, hydroxy group, amino group, nitro group, cyano group, optionally halogenated C₁₋₆ alkyl group, mono- or di-C₁₋₆ alkyl-amino group, C₆₋₁₄ aryl group, mono- or di-C₆₋₁₄ aryl-amino group, C₃₋₈ cycloalkyl group, C₁₋₆ alkoxy group, C₁₋₆ alkoxy-C₁₋₆ alkoxy group, C₁₋₆ alkylthio group, C₁₋₆ alkylsulfinyl group, C₁₋₆ alkylsulfonyl group, an optionally esterified carboxyl group, carbamoyl group, thiocarbamoyl group, mono- or di-C₁₋₆ alkyl-carbamoyl group, mono- or di-C₆₋₁₄ aryl-carbamoyl group, sulfamoyl group, mono- or di-C₁₋₆ alkyl-sulfamoyl group and mono- or di-C₆₋₁₄ aryl-sulfamoyl group;
   (27) heterocyclic oxy group optionally substituted by 1 to 3 substituents selected from halogen atom, hydroxy group, amino group, nitro group, cyano group, optionally halogenated C₁₋₆ alkyl group, mono- or di-C₁₋₆ alkyl-amino group, C₆₋₁₄ aryl group, mono- or di-C₆₋₁₄ aryl-amino group, C₃₋₈ cycloalkyl group, C₁₋₆ alkoxy group, C₁₋₆ alkoxy-C₁₋₆ alkoxy group, C₁₋₆ alkylthio group, C₁₋₆ alkylsulfinyl group, C₁₋₆ alkylsulfonyl group, an optionally esterified carboxyl group, carbamoyl group, thiocarbamoyl group, mono- or di-C₁₋₆ alkyl-carbamoyl group, mono- or di-C₆₋₁₄ aryl-carbamoyl group, sulfamoyl group, mono- or di-C₁₋₆ alkyl-sulfamoyl group and mono- or di-C₆₋₁₄ aryl-sulfamoyl group;
   (31) C₇₋₁₆ aralkyloxy group optionally substituted by 1 to 3 substituents selected from halogen atom, hydroxy group, amino group, nitro group, cyano group, optionally halogenated C₁₋₆ alkyl group, mono- or di-C₁₋₆ alkyl-amino group, C₆₋₁₄ aryl group, mono- or di-C₆₋₁₄ aryl-amino group, C₃₋₈ cycloalkyl group, C₁₋₆ alkoxy group, C₁₋₆ alkoxy-C₁₋₆ alkoxy group, C₁₋₆ alkylthio group, C₁₋₆ alkylsulfinyl group, C₁₋₆ alkylsulfonyl group, an optionally esterified carboxyl group, carbamoyl group, thiocarbamoyl group, mono- or di-C₁₋₆ alkyl-carbamoyl group, mono- or di-C₆₋₁₄ aryl-carbamoyl group, sulfamoyl group, mono- or di-C₁₋₆ alkyl-sulfamoyl group and mono- or di-C₆₋₁₄ aryl-sulfamoyl group;
   (32) C₁₋₆ alkylsulfonyloxy group;
   (33) tri-C₁₋₆ alkyl-silyloxy group;
   (34) nitrogen-containing heterocycle-carbonyl group;
   (35) C₆₋₁₄ aryl-carbonyl group;
   (35) C₆₋₁₄ aryl-thio group;
   (37) optionally halogenated C₆₋₁₄ aryl-sulfonyl group;
   (38) nitrogen-containing heterocycle-sulfonyl group; and
   (39) nitrogen-containing heterocycle-amino group optionally substituted by cyano group.
(8) The compound of the above-mentioned (2), wherein R³ is a hydrogen atom.
(9) The compound of the above-mentioned (2), wherein R⁴ is an optionally substituted C₆₋₁₄ aryl group or an optionally substituted heterocyclic group.
(10) The compound of the above-mentioned (2), wherein R⁵ is a hydrogen atom.
(11) The compound of the above-mentioned (2), wherein R⁶ is a hydrogen atom.
(12) The compound of the above-mentioned (2), wherein R⁵ and R⁶ are hydrogen atoms.
(13) The compound of the above-mentioned (2), wherein X is a carbonyl group.
(14) The compound of the above-mentioned (2), which is selected from the following:
   N-(3-(((2-cyanoethyl)amino)carbonyl)-1H-pyrazol-4-yl)pyridine-2-carboxamide;
   N-(3-(((2-cyanoethyl)amino)carbonyl)-1H-pyrazol-4-yl)-6-methylpyridine-2-carboxamide;
   N-(3-(((3,3,3-trifluoropropyl)amino)carbonyl)-1H-pyrazol-4-yl)pyridine-2-carboxamide;
   4-(benzoylamino)-N-(2-cyanoethyl)-1H-pyrazole-3-carboxamide ;
   N-(3-(((3-isopropoxypropyl)amino)carbonyl)-1H-pyrazol-4-yl)-6-methylpyridine-2-carboxamide;
   N-(2-cyanoethyl)-4-(2-furoylamino)-1H-pyrazole-3-carboxamide:
   N-(3-(((3-hydroxypropyl)amino)carbonyl)-1H-pyrazol-4-yl)pyridine-2-carboxamide;
   N-(3-(((2-isopropoxyethyl)amino)carbonyl)-1H-pyrazol-4-yl)-6-methylnicotinamide;
   6-methyl-N-(3-(((2,2,2-trifluoroethyl)amino)carbonyl)-1H-pyrazol-4-yl)pyridine-3-carboxamide; and
   N-(3-(((2-cyanoethyl)amino)carbonyl)-1H-pyrazol-4-yl)-6-methylpyridine-3-carboxamide.
(15) A prodrug of the compound of the above-mentioned (1).
(16) A pharmaceutical agent comprising the compound of the above-mentioned (1) or the prodrug of the above-mentioned (15).
(17) A GSK-3β inhibitor comprising the compound of the above-mentioned (1) or the prodrug of the above-mentioned (15).
(18) A neural stem cell differentiation promoter comprising the GSK-3β inhibitor of the above-mentioned (17).
(19) An agent for the prophylaxis or treatment of neurodegenerative disease or diabetes, which comprises the compound of the above-mentioned (1) or the prodrug of the above-mentioned (15).
(20) A hypoglycemic agent comprising the compound of the above-mentioned (1) or the prodrug of the above-mentioned (15).
(21) A GSK-3β inhibitor comprising a compound represented by the formula: 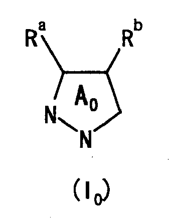 wherein
   ring A₀ is a pyrazole ring optionally further having 1 or 2 substituents;
   R^{a} is substituted carbamoyl; and
   R^{b} is optionally substituted acylamino,
   or a salt thereof or a prodrug thereof.
(22) A neural stem cell differentiation promoter comprising the GSK-3β inhibitor of the above-mentioned (21).
(23) An agent for the prophylaxis or treatment of neurodegenerative disease or diabetes comprising the GSK-3β inhibitor of the above-mentioned (21).
(24) A hypoglycemic agent comprising the GSK-3β inhibitor of the above-mentioned (21).
(25) A method of inhibiting GSK-3β comprising administering a compound represented by the formula: 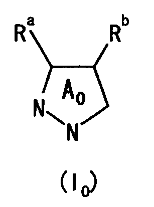 wherein
   ring A₀ is a pyrazole ring optionally further having 1 or 2 substituents;
   R^{a} is substituted carbamoyl; and
   R^{b} is optionally substituted acylamino,
   or a salt thereof or a prodrug thereof to a subject.
(26) A method of promoting neural stem cell differentiation comprising administering a compound represented by the formula: 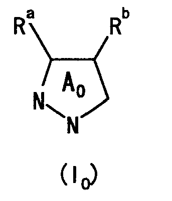 wherein
   ring A₀ is a pyrazole ring optionally further having 1 or 2 substituents;
   R^{a} is substituted carbamoyl; and
   R^{b} is optionally substituted acylamino,
   or a salt thereof or a prodrug thereof to a subject.
(27) A method of preventing or treating a neurodegenerative disease or diabetes comprising administering a compound represented by the formula: 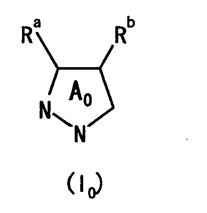 wherein
   ring A₀ is a pyrazole ring optionally further having 1 or 2 substituents;
   R^{a} is substituted carbamoyl; and
   R^{b} is optionally substituted acylamino,
   or a salt thereof or a prodrug thereof to a subject.
(28) A method of lowering blood-glucose comprising administering a compound represented by the formula: 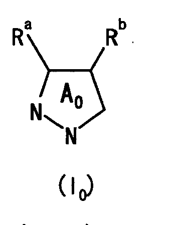 wherein
   ring A₀ is a pyrazole ring optionally further having 1 or 2 substituents;
   R^{a} is substituted carbamoyl; and
   R^{b} is optionally substituted acylamino,
   or a salt thereof or a prodrug thereof to a subject.
(29) Use of a compound represented by the formula: 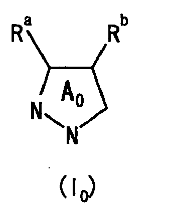 wherein
   ring A₀ is a pyrazole ring optionally further having 1 or 2 substituents;
   R^{a} is substituted carbamoyl; and
   R^{b} is optionally substituted acylamino,
   or a salt thereof or a prodrug thereof for the production of a GSK-3β inhibitor.
(30) Use of a compound represented by the formula: 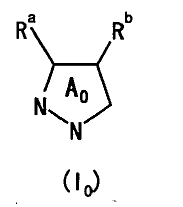 wherein
   ring A₀ is a pyrazole ring optionally further having 1 or 2 substituents;
   R^{a} is substituted carbamoyl; and
   R^{b} is optionally substituted acylamino,
   or a salt thereof or a prodrug thereof for the production of a neural stem cell differentiation promoter.
(31) Use of a compound represented by the formula: 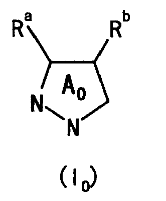 wherein
   ring A₀ is a pyrazole ring optionally further having 1 or 2 substituents;
   R^{a} is substituted carbamoyl; and
   R^{b} is optionally substituted acylamino,
   or a salt thereof or a prodrug thereof for the production of an agent for the prophylaxis or treatment of a neurodegenerative disease or diabetes.
(32) Use of a compound represented by the formula: 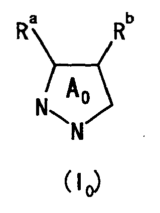 wherein
   ring A₀ is a pyrazole ring optionally further having 1 or 2 substituents;
   R^{a} is substituted carbamoyl; and
   R^{b} is optionally substituted acylamino,
   or a salt thereof or a prodrug thereof for the production of a hypoglycemic agent.

### Best Mode for Embodying the Invention

In the present specification, examples of the "halogen atom" include, unless otherwise specified, fluorine atom, chlorine atom, bromine atom and iodine atom.

In the present specification, examples of the "optionally substituted hydrocarbon group" include, unless otherwise specified, "optionally substituted C₁₋₆ alkyl group", "optionally substituted C₂₋₆ alkenyl group", "optionally substituted C₂₋₆ alkynyl group", "optionally substituted C₃₋₈ cycloalkyl group", "optionally substituted C₆₋₁₄ aryl group", "optionally substituted aralkyl group" and the like.

In the present specification, examples of the "C₁₋₆ alkyl group" include, unless otherwise specified, methyl, ethyl, propyl, isopropyl, butyl, isobutyl, sec-butyl, tert-butyl, pentyl, isopentyl, neopentyl, hexyl and the like.

In the present specification, examples of the "C₂₋₆ alkenyl group" include, unless otherwise specified, vinyl, propenyl, isopropenyl, 2-buten-1-yl, 4-penten-1-yl, 5-hexen-1-yl and the like.

In the present specification, examples of the "C₂₋₆ alkynyl group" include, unless otherwise specified, 2-butyn-1-yl, 4-pentyn-1-yl, 5-hexyn-1-yl and the like.

In the present specification, examples of the "C₃₋₈ cycloalkyl group" include, unless otherwise specified, cyclopropyl, cyclobutyl, cyclopentyl, cyclohexyl, cycloheptyl, cyclooctyl, bicyclo[2.2.1]heptyl, oxobicyclo[2.2.1]heptyl, bicyclo[2.2.2]octyl and the like.

In the present specification, examples of the "C₆₋₁₄ aryl group" include, unless otherwise specified, phenyl, 1-naphthyl, 2-naphthyl, 2-biphenylyl, 3-biphenylyl, 4-biphenylyl, 2-anthryl and the like. The C₆₋₁₄ aryl may be partially saturated, and examples of the partially saturated C₆₋₁₄ aryl include indanyl, tetrahydronaphthyl and the like.

In the present specification, examples of the "C₇₋₁₆ aralkyl group" include, unless otherwise specified, benzyl, phenethyl, diphenylmethyl, 1-naphthylmethyl, 2-naphthylmethyl, 2,2-diphenylethyl, 3-phenylpropyl, 3,3-diphenylpropyl, 4-phenylbutyl, 5-phenylpentyl, 2-biphenylylmethyl, 3-biphenylylmethyl, 4-biphenylylmethyl and the like.

In the present specification, examples of the "optionally substituted hydroxy group" include, unless otherwise specified, "hydroxy group", "optionally substituted C₁₋₁₀ alkoxy group", "optionally substituted heterocyclic oxy group", "optionally substituted C₆₋₁₄ aryloxy group", "optionally substituted C₇₋₁₆ aralkyloxy group", "tri-C₁₋₆ alkyl-silyloxy group", "optionally substituted C₁₋₆ alkylsulfonyloxy group", "optionally substituted heterocyclic sulfonyloxy group" and the like.

In the present specification, examples of the "C₁₋₆ alkoxy group" include, unless otherwise specified, methoxy, ethoxy, propoxy, isopropoxy, butoxy, isobutoxy, tert-butoxy, pentyloxy, hexyloxy and the like.

In the present specification, examples of the "C₁₋₁₀ alkoxy group" include, in addition to the above-mentioned C₁₋₆ alkoxy group, heptyloxy, octyloxy, nonyloxy, decyloxy and the like.

In the present specification, examples of the "C₁₋₆ alkoxy-C₁₋₆ alkoxy group" include, unless otherwise specified, methoxymethoxy, methoxyethoxy, ethoxymethoxy, ethoxyethoxy and the like.

In the present specification, examples of the "heterocyclic oxy group" include hydroxy group substituted by the below-mentioned "heterocyclic group". Preferable examples of the heterocyclic oxy group include tetrahydropyranyloxy, thiazolyloxy, pyridyloxy, pyrazolyloxy, oxazolyloxy, thienyloxy, furyloxy, tetrahydrothiopyranyloxy, 1,1-dioxidotetrahydrothiopyranyloxy and the like.

In the present specification, examples of the "C₆₋₁₄ aryloxy group" include, unless otherwise specified, phenoxy, 1-naphthyloxy, 2-naphthyloxy and the like.

In the present specification, examples of the "C₇₋₁₆ aralkyloxy group" include, unless otherwise specified, benzyloxy, phenethyloxy and the like.

In the present specification, examples of the "tri-C₁₋₆ alkyl-silyloxy group" include, unless otherwise specified, trimethylsilyloxy, tert-butyl(dimethyl)silyloxy and the like.

In the present specification, examples of the "C₁₋₆ alkylsulfonyloxy group" include, unless otherwise specified, methylsulfonyloxy, ethylsulfonyloxy and the like.

In the present specification, examples of the "heterocyclic sulfonyloxy group" include sulfonyloxy group substituted by the below-mentioned "heterocyclic group". Preferable examples of the heterocyclic sulfonyloxy group include thienylsulfonyloxy, furylsulfonyloxy and the like.

In the present specification, examples of the "optionally substituted mercapto group" include, unless otherwise specified, "mercapto group", "optionally substituted C₁₋₁₀ alkylthio group", "optionally substituted heterocyclic thio group", "optionally substituted C₆₋₁₄ arylthio group", "optionally substituted C₇₋₁₆ aralkylthio group" and the like.

In the present specification, examples of the "C₁₋₆ alkylthio group" include, unless otherwise specified, methylthio, ethylthio, propylthio, isopropylthio, butylthio, sec-butylthio, tert-butylthio and the like. In the present specification, examples of the "C₁₋₁₀ alkylthio group" include, in addition to the above-mentioned C₁₋₆ alkylthio group, heptylthio, octylthio, nonylthio, decylthio and the like.

In the present specification, examples of the "heterocyclic thio group" include mercapto group substituted by the below-mentioned "heterocyclic group". Preferable examples of the heterocyclic thio group include tetrahydropyranylthio, thiazolylthio, pyridylthio, pyrazolylthio, oxazolylthio, thienylthio, furylthio, tetrahydrothiopyranylthio, 1,1-dioxidotetrahydrothiopyranylthio and the like.

In the present specification, examples of the "C₆₋₁₄ arylthio group" include, unless otherwise specified, phenylthio, 1-naphthylthio, 2-naphthylthio and the like.

In the present specification, examples of the "C₇₋₁₆ aralkylthio group" include, unless otherwise specified, benzylthio, phenethylthio and the like.

In the present specification, examples of the "heterocyclic group" include, unless otherwise specified, a 5-to 14-membered (monocycle, bicyclic or tricyclic) heterocyclic group, preferably (i) 5- to 14-membered (preferably 5- to 10-membered) aromatic heterocyclic group, (ii) 5- to 10-membered nonaromatic heterocyclic group containing, as a ring constituting atom besides carbon atom, 1 to 4 hetero atoms of 1 or 2 kinds selected from a nitrogen atom, a sulfur atom and an oxygen atom, and the like. Particularly, 5 or 6-membered aromatic heterocyclic group is preferable. Specifically, for example, aromatic heterocyclic groups such as thienyl (e.g., 2-thienyl, 3-thienyl), furyl (e.g., 2-furyl, 3-furyl), pyridyl (e.g., 2-pyridyl, 3-pyridyl, 4-pyridyl), thiazolyl (e.g., 2-thiazolyl, 4-thiazolyl, 5-thiazolyl), oxazolyl (e.g., 2-oxazolyl, 4-oxazolyl, 5-oxazolyl), quinolyl (e.g., 2-quinolyl, 3-quinolyl, 4-quinolyl, 5-quinolyl, 8-quinolyl), isoquinolyl (e.g., 1-isoquinolyl, 3-isoquinolyl, 4-isoquinolyl, 5-isoquinolyl), pyrazinyl, pyrimidinyl (e.g., 2-pyrimidinyl, 4-pyrimidinyl), pyrrolyl (e.g., 1-pyrrolyl, 2-pyrrolyl, 3-pyrrolyl), imidazolyl (e.g., 1-imidazolyl, 2-imidazolyl, 4-imidazolyl), pyrazolyl (e.g., 1-pyrazolyl, 3-pyrazolyl, 4-pyrazolyl), pyridazinyl (e.g., 3-pyridazinyl, 4-pyridazinyl), isothiazolyl,(e.g., 3-isothiazolyl, 4-isothiazolyl, 5-isothiazolyl), isoxazolyl (e.g., 3-isoxazolyl, 4-isoxazolyl, 5-isoxazolyl), indolyl (e.g., 1-indolyl, 2-indolyl, 3-indolyl), 2-benzothiazolyl, 2-benzooxazolyl, benzoimidazolyl (e.g., 1-benzoimidazolyl, 2-benzoimidazolyl), benzo[b]thienyl (e.g., 2-benzo[b]thienyl, 3-benzo[b]thienyl), benzo[b]furanyl (e.g., 2-benzo[b]furanyl, 3-benzo[b]furanyl) and the like; for example, nonaromatic heterocyclic groups such as pyrrolidinyl (e.g., 1-pyrrolidinyl, 2-pyrrolidinyl, 3-pyrrolidinyl), oxazolidinyl (e.g., 2-oxazolidinyl), imidazolinyl (e.g., 1-imidazolinyl, 2-imidazolinyl, 4-imidazolinyl), piperidinyl (e.g., 1-piperidinyl, 2-piperidinyl, 3-piperidinyl, 4-piperidinyl), piperazinyl (e.g., 1-piperazinyl, 2-piperazinyl), morpholinyl (e.g., 2-morpholinyl, 3-morpholinyl, 4-morpholinyl), thiomorpholinyl (e.g., 2-thiomorpholinyl, 3-thiomorpholinyl, 4-thiomorpholinyl), tetrahydropyranyl (e.g., 2-tetrahydropyranyl, 3-tetrahydropyranyl, 4-tetrahydropyranyl), oxetanyl (e.g., 2-oxetanyl, 3-oxetanyl), oxopyrrolidinyl (e.g., 2-oxopyrrolidin-1-yl, 2-oxopyrrolidin-3-yl, 2-oxopyrrolidin-4-yl, 2-oxopyrrolidin-5-yl, 3-oxopyrrolidin-1-yl), dioxopyrrolidinyl (e.g., 2,5-dioxopyrrolidin-1-yl, 2,5-dioxopyrrolidin-3-yl), tetrahydrothiopyranyl (e.g., 2-tetrahydrothiopyranyl, 3-tetrahydrothiopyranyl, 4-tetrahydrothiopyranyl), 1.,1-dioxidotetrahydrothiopyranyl (e.g., 1,1-dioxidotetrahydrothiopyran-2-yl, 1,1-dioxidotetrahydrothiopyran-3-yl, 1,1-dioxidotetrahydrothiopyran-4-yl), dihydrobenzofuranyl (e.g., 2,3-dihydro-1-benzofuran-4-yl, 2,3-dihydro-1-benzofuran-5-yl, 2,3-dihydro-1-benzofuran-6-yl, 2,3-dihydro-1-benzofuran-7-yl) and the like, and the like.

In the present specification, examples of the "optionally substituted sulfinyl group" include, unless otherwise specified, "sulfinyl group", "optionally substituted C₁₋₆ alkylsulfinyl group", "optionally substituted heterocyclic sulfinyl group", "optionally substituted C₆₋₁₄ arylsulfinyl group", "optionally substituted C₇-₁₆ aralkylsulfinyl group" and the like.

In the present specification, examples of the "optionally substituted sulfonyl group" include, unless otherwise specified, "sulfonyl group", "optionally substituted C₁₋₆ alkylsulfonyl group", "optionally substituted heterocyclic sulfonyl group", "optionally substituted C₆₋₁₄ arylsulfonyl group", "optionally substituted C₇₋₁₆ aralkylsulfonyl group" and the like.

In the present specification, examples of the "C₁₋₆ alkylsulfonyl group" include sulfonyl group substituted by the aforementioned "C₁₋₆ alkyl group". Preferable examples of the C₁₋₆ alkylsulfonyl group include methylsulfonyl, ethylsulfonyl and the like.

In the present specification, examples of the "C₁₋₆ alkylsulfinyl group" include sulfinyl group substituted by the aforementioned "C₁₋₆ alkyl group". Preferable examples of the C₁₋₆ alkylsulfinyl group include methylsulfinyl, ethylsulfinyl and the like.

In the present specification, examples of the "heterocyclic sulfonyl group" include sulfonyl group substituted by the aforementioned "heterocyclic group". Preferable examples of the heterocyclic sulfonyl group include thienylsulfonyl, furylsulfonyl and the like.

In the present specification, examples of the "heterocyclic sulfinyl group" include sulfinyl group substituted by the aforementioned "heterocyclic group". Preferable examples of the heterocyclic sulfinyl group include thienylsulfinyl, furylsulfinyl and the like.

In the present specification, examples of the "C₆₋₁₄ arylsulfonyl group" include sulfonyl group substituted by the aforementioned "C₆₋₁₄ aryl group". Preferable examples of the C₆₋₁₄ arylsulfonyl group include phenylsulfonyl, 1-naphthylsulfonyl, 2-naphthylsulfonyl and the like.

In the present specification, examples of the "C₆₋₁₄ arylsulfinyl group" include sulfinyl group substituted by the aforementioned "C₆₋₁₄ aryl group". Preferable examples of the C₆₋₁₄ arylsulfinyl group include phenylsulfinyl, 1-naphthylsulfinyl, 2-naphthylsulfinyl and the like.

In the present specification, examples of the "C₇₋₁₆ aralkylsulfonyl group" include sulfonyl group substituted by the aforementioned "C₇₋₁₆ aralkyl group". Preferable examples of the C₇₋₁₆ aralkylsulfonyl group include benzylsulfonyl, phenethylsulfonyl and the like.

In the present specification, examples of the "C₇₋₁₆ aralkylsulfinyl group" include sulfinyl group substituted by the aforementioned "C₇₋₁₆ aralkyl group". Preferable examples of the C₇₋₁₆ aralkylsulfinyl group include benzylsulfinyl, phenethylsulfinyl and the like.

In the present specification, examples of the "optionally esterified carboxyl group" include, unless otherwise specified, carboxyl group, C₁₋₆ alkoxy-carbonyl group (e.g., methoxycarbonyl, ethoxycarbonyl, propoxycarbonyl, tert-butoxycarbonyl etc.), C₆₋₁₄ aryloxy-carbonyl group (e.g., phenoxycarbonyl etc.), C₇₋₁₆ aralkyloxy-carbonyl group (e.g., benzyloxycarbonyl, phenethyloxycarbonyl etc.) and the like.

In the present specification, examples of the "optionally halogenated C₁₋₆ alkyl group" include, unless otherwise specified, the above-mentioned "C₁₋₆ alkyl group" optionally substituted by 1 to 5 "halogen atoms" mentioned above. Examples thereof include methyl, ethyl, propyl, isopropyl, butyl, tert-butyl, isobutyl, trifluoromethyl and the like.

In the present specification, examples of the "optionally halogenated C₁₋₆ alkoxy group" include, unless otherwise specified, the above-mentioned "C₁₋₆ alkoxy group" optionally substituted by 1 to 5 "halogen atoms" mentioned above. Examples thereof include methoxy, ethoxy, isopropoxy, tert-butoxy, trifluoromethoxy and the like.

In the present specification, examples of the "mono- or di-C₁₋₆ alkyl-amino group" include, unless otherwise specified, amino group mono- or di-substituted by the above-mentioned "C₁₋₆ alkyl group". Examples thereof include methylamino, ethylamino, propylamino, dimethylamino, diethylamino and the like.

In the present specification, examples of the "mono- or di-C₆₋₁₄ aryl-amino group" include, unless otherwise specified, amino group mono- or di-substituted by the above-mentioned "C₆₋₁₄ aryl group". Examples thereof include phenylamino, diphenylamino, 1-naphthylamino, 2-naphthylamino and the like.

In the present specification, examples of the "mono- or di-C₇₋₁₆ aralkyl-amino group" include, unless otherwise specified, amino group mono- or di-substituted by the above-mentioned "C₇₋₁₆ aralkyl group". Examples thereof include benzylamino, phenethylamino and the like.

In the present specification, examples of the "N-C₁₋₆ alkyl-N-C₆₋₁₄ aryl-amino group" include, unless otherwise specified, amino group substituted by the above-mentioned "C₁₋₆ alkyl group" and the above-mentioned "C₆₋₁₄ aryl group". Examples thereof include N-methyl-N-phenylamino, N-ethyl-N-phenylamino and the like.

In the present specification, examples of the "N-C₁₋₆ alkyl-N-C₇₋₁₆ aralkyl-amino group" include, unless otherwise specified, amino group substituted by the above-mentioned "C₁₋₆ alkyl group" and the above-mentioned "C₇₋₁₆ aralkyl group". Examples thereof include N-methyl-N-benzylamino, N-ethyl-N-benzylamino and the like.

In the present specification, examples of the "N-C₁₋₆ alkyl-N-(C₁₋₆ alkyl-carbonyl)-amino group" include, unless otherwise specified, amino group substituted by the aforementioned "C₁₋₆ alkyl group" and C₁₋₆ alkyl-carbonyl group (e.g., acetyl, isobutanoyl, isopentanoyl). Examples thereof include N-acetyl-N-methylamino, N-acetyl-N-ethylamino and the like.

In the present specification, examples of the "mono- or di-C₁₋₆ alkyl-carbamoyl group" include, unless otherwise specified, carbamoyl group mono- or di-substituted by the above-mentioned "C₁₋₆ alkyl group". Examples thereof include methylcarbamoyl, ethylcarbamoyl, dimethylcarbamoyl, diethylcarbamoyl, ethylmethylcarbamoyl and the like.

In the present specification, examples of the "mono- or di-C₆₋₁₄ aryl-carbamoyl group" include, unless otherwise specified, carbamoyl group mono- or di-substituted by the above-mentioned "C₆₋₁₄ aryl group". Examples thereof include phenylcarbamoyl, 1-naphthylcarbamoyl, 2-naphthylcarbamoyl and the like.

In the present specification, examples of the "mono- or di-5- to 7-membered heterocycle-carbamoyl group" include, unless otherwise specified, carbamoyl group mono- or di-substituted by 5- to 7-membered heterocyclic group. Examples of the 5- to 7-membered heterocyclic group include heterocyclic group containing, as a ring constituting atom besides carbon atom, 1 to 4 hetero atoms of 1 or 2 kinds selected from a nitrogen atom, a sulfur atom and an oxygen atom. Preferable examples of the "mono- or di-5- to 7-membered heterocycle-carbamoyl group" include 2-pyridylcarbamoyl, 3-pyridylcarbamoyl, 4-pyridylcarbamoyl, 2-thienylcarbamoyl, 3-thienylcarbamoyl and the like.

In the present specification, examples of the "mono- or di-C₁₋₆ alkyl-sulfamoyl group" include, unless otherwise specified, sulfamoyl group mono- or di-substituted by the above-mentioned "C₁₋₆ alkyl group" and, for example, methylsulfamoyl, ethylsulfamoyl, dimethylsulfamoyl, diethylsulfamoyl and the like can be used.

In the present specification, examples of the "mono- or di-C₆₋₁₄ aryl-sulfamoyl group" include, unless otherwise specified, sulfamoyl group mono- or di-substituted by the above-mentioned "C₆₋₁₄ aryl group" and, for example, phenylsulfamoyl, diphenylsulfamoyl, 1-naphthylsulfamoyl, 2-naphthylsulfamoyl and the like can be used.

In the present specification, examples of the "optionally substituted C₁₋₆ alkyl group", "optionally substituted C₂₋₆ alkenyl group", "optionally substituted C₂₋₆ alkynyl group", "optionally substituted C₁₋₁₀ alkoxy group (including optionally substituted C₁₋₆ alkoxy group)", "optionally substituted C₁₋₆ alkylsulfonyloxy group" and "optionally substituted C₁₋₁₀ alkylthio group (including optionally substituted C₁₋₆ alkylthio group)" include "C₁₋₆ alkyl group", "C₂₋₆ alkenyl group", "C₂₋₆ alkynyl group", "C₁₋₁₀ alkoxy group (including C₁₋₆ alkoxy group)", "C₁₋₆ alkylsulfonyloxy group" and "C₁₋₁₀ alkylthio group (including C₁₋₆ alkylthio group)", each optionally having, at substitutable positions, 1 to 5 substituents selected from (1) halogen atom; (2) hydroxy group; (3) amino group; (4) nitro group; (5) cyano group; (6) heterocyclic group optionally substituted by 1 to 3 substituents selected from halogen atom, hydroxy group, amino group, nitro group, cyano group, optionally halogenated C₁₋₆ alkyl group, mono- or di-C₁₋₆ alkyl-amino group, C₆₋₁₄ aryl group, mono- or di-C₆₋₁₄ aryl-amino group, C₃₋₈ cycloalkyl group, C₁₋₆ alkoxy group, C₁₋₆ alkoxy-C₁₋₆ alkoxy group, C₁₋₆ alkylthio group, C₁₋₆ alkylsulfinyl group, C₁₋₆ alkylsulfonyl group, an optionally esterified carboxyl group, carbamoyl group, thiocarbamoyl group, mono- or di-C₁₋₆ alkyl-carbamoyl group, mono- or di-C₆₋₁₄ aryl-carbamoyl group, sulfamoyl group, mono- or di-C₁₋₆ alkyl-sulfamoyl group and mono- or di-C₆₋₁₄ aryl-sulfamoyl group (preferably furyl, pyridyl, thienyl, pyrazolyl, thiazolyl, oxazolyl, isoxazolyl, isothiazolyl, oxetanyl, morpholinyl, thiomorpholinyl, pyrrolidinyl, oxopyrrolidinyl, dioxopyrrolidinyl, tetrahydropyranyl, tetrahydrothiopyranyl, 1,1-dioxidotetrahydrothiopyranyl); (7) mono- or di-C₁₋₆ alkyl-amino group; (8) mono- or di-C₆₋₁₄ aryl-amino group; (9) mono- or di-C₇₋₁₆ aralkyl-amino group; (10) N-C₁₋₆ alkyl-N-C₆₋₁₄ aryl-amino group; (11) N-C₁₋₆ alkyl-N-(C₁₋₆ alkyl-C₆₋₁₄ aryl)-amino group; (12) N-C₁₋₆ alkyl-N-C₇₋₁₆ aralkyl-amino group; (13) C₃₋₈ cycloalkyl group optionally substituted by C₁₋₆ alkyl group; (14) optionally halogenated C₁₋₆ alkoxy group; (15) C₁₋₆ alkylthio group optionally substituted by C₁₋₆ alkoxy group; (16) C₁₋₆ alkylsulfinyl group optionally substituted by C₁₋₆ alkoxy group; (17) C₁₋₆ alkylsulfonyl group optionally substituted by C₁₋₆ alkoxy group; (18) optionally esterified carboxyl group; (19) carbamoyl group; (20) thiocarbamoyl group; (21) mono- or di-C₁₋₆ alkyl-carbamoyl group; (22) mono- or di-C₆₋₁₄ aryl-carbamoyl group; (23) mono- or di-5- to 7-membered heterocycle-carbamoyl group; (24) C₁₋₆ alkyl-carbonylamino group optionally substituted by carboxyl group (e.g., acetylamino, propionylamino); (25) a C₆₋₁₄ aryloxy group optionally substituted by 1 to 3 substituents selected from halogen atom, hydroxy group, amino group, nitro group, cyano group, optionally halogenated C₁₋₆ alkyl group, mono- or di-C₁₋₆ alkyl-amino group, C₆₋₁₄ aryl group, mono- or di-C₆₋₁₄ aryl-amino group, C₃₋₈ cycloalkyl group, C₁₋₆ alkoxy group, C₁₋₆ alkoxy-C₁₋₆ alkoxy group, C₁₋₆ alkylthio group, C₁₋₆ alkylsulfinyl group, C₁₋₆ alkylsulfonyl group, optionally esterified carboxyl group, carbamoyl group, thiocarbamoyl group, mono- or di-C₁₋₆ alkyl-carbamoyl group, mono- or di-C₆₋₁₄ aryl-carbamoyl group, sulfamoyl group, mono- or di-C₁₋₆ alkyl-sulfamoyl group and mono- or di-C₆₋₁₄ aryl-sulfamoyl group; (26) C₆₋₁₄ aryl group optionally substituted 1 to 3 substituents selected from halogen atom, hydroxy group, amino group, nitro group, cyano group, optionally halogenated C₁₋₆ alkyl group, mono- or di-C₁₋₆ alkyl-amino group, C₆₋₁₄ aryl group, mono- or di-C₆₋₁₄ aryl-amino group, C₃₋₈ cycloalkyl group, C₁₋₆ alkoxy group, C₁₋₆ alkoxy-C₁₋₆ alkoxy group, C₁₋₆ alkylthio group, C₁₋₆ alkylsulfinyl group, C₁₋₆ alkylsulfonyl group, an optionally esterified carboxyl group, carbamoyl group, thiocarbamoyl group, mono- or di-C₁₋₆ alkyl-carbamoyl group, mono- or di-C₆₋₁₄ aryl-carbamoyl group, sulfamoyl group, mono- or di-C₁₋₆ alkyl-sulfamoyl group and mono- or di-C₆₋₁₄ aryl-sulfamoyl group; (27) heterocyclic oxy group optionally substituted by 1 to 3 substituents selected from halogen atom, hydroxy group, amino group, nitro group, cyano group, optionally halogenated C₁₋₆ alkyl group, mono- or di-C₁₋₆ alkyl-amino group, C₆₋₁₄ aryl group, mono- or di-C₆₋₁₄ aryl-amino group, C₃₋₈ cycloalkyl group, C₁₋₆ alkoxy group, C₁₋₆ alkoxy-C₁₋₆ alkoxy group, C₁₋₆ alkylthio group, C₁₋₆ alkylsulfinyl group, C₁₋₆ alkylsulfonyl group, optionally esterified carboxyl group, carbamoyl group, thiocarbamoyl group, mono- or di-C₁₋₆ alkyl-carbamoyl group, mono- or di-C₆₋₁₄ aryl-carbamoyl group, sulfamoyl group, mono- or di-C₁₋₆ alkyl-sulfamoyl group and mono- or di-C₆₋₁₄ aryl-sulfamoyl group; (28) sulfamoyl group; (29) mono- or di-C₁₋₆ alkyl-sulfamoyl group; (30) mono- or di-C₆₋₁₄ aryl-sulfamoyl group; (31) C₇₋₁₆ aralkyloxy group optionally substituted by 1 to 3 substituents selected from halogen atom, hydroxy group, amino group, nitro group, cyano group, optionally halogenated C₁₋₆ alkyl group, mono- or di-C₁₋₆ alkyl-amino group, C₆₋₁₄ aryl group, mono- or di-C₆₋₁₄ aryl-amino group, C₃₋₈ cycloalkyl group, C₁₋₆ alkoxy group, C₁₋₆ alkoxy-C₁₋₆ alkoxy group, C₁₋₆ alkylthio group, C₁₋₆ alkylsulfinyl group, C₁₋₆ alkylsulfonyl group, an optionally esterified carboxyl group, carbamoyl group, thiocarbamoyl group, mono- or di-C₁₋₆ alkyl-carbamoyl group, mono- or di-C₆₋₁₄ aryl-carbamoyl group, sulfamoyl group, mono- or di-C₁₋₆ alkyl-sulfamoyl group and mono- or di-C₆₋₁₄ aryl-sulfamoyl group; (32) C₁₋₆ alkylsulfonyloxy group; (33) tri-C₁₋₆ alkyl-silyloxy group; (34) nitrogen-containing heterocycle-carbonyl group (e.g., pyrrolidinylcarbonyl, piperidinocarbonyl, morpholinocarbonyl, thiomorpholinocarbonyl); (35) C₆₋₁₄ aryl-carbonyl group; (36) C₆-₁₄ aryl-thio group; (37) optionally halogenated C₆₋₁₄ aryl-sulfonyl group; (38) nitrogen-containing heterocycle-sulfonyl group (e.g., pyridylsulfonyl); (39) nitrogen-containing heterocycle-amino group optionally substituted by cyano group (e.g., pyridylamino); (40) nitrogen-containing heterocycle-thio group; (41) nitrogen-containing heterocycle-sulfinyl group; (42) C₇₋₁₆ aralkylsulfinyl group optionally substituted by 1 to 3 substituents selected from halogen atom, hydroxy group, amino group, nitro group, cyano group, optionally halogenated C₁₋₆ alkyl group, mono- or di-C₁₋₆ alkyl-amino group, C₆₋₁₄ aryl group, mono- or di-C₆₋₁₄ aryl-amino group, C₃₋₈ cycloalkyl group, C₁₋₆ alkoxy group, C₁₋₆ alkoxy-C₁₋₆ alkoxy group, C₁₋₆ alkylthio group, C₁₋₆ alkylsulfinyl group, C₁₋₆ alkylsulfonyl group, optionally esterified carboxyl group, carbamoyl group, thiocarbamoyl group, mono- or di-C₁₋₆ alkyl-carbamoyl group, mono- or di-C₆₋₁₄ aryl-carbamoyl group, sulfamoyl group, mono- or di-C₁₋₆ alkyl-sulfamoyl group and mono- or di-C₆₋₁₄ aryl-sulfamoyl group; (43) C₇₋₁₆ aralkylsulfonyl group optionally substituted by 1 to 3 substituents selected from halogen atom, hydroxy group, amino group, nitro group, cyano group, optionally halogenated C₁₋₆ alkyl group, mono- or di-C₁₋₆ alkyl-amino group, C₆₋₁₄ aryl group, mono- or di-C₆₋₁₄ aryl-amino group, C₃₋₈ cycloalkyl group, C₁₋₆ alkoxy group, C₁₋₆ alkoxy-C₁₋₆ alkoxy group, C₁₋₆ alkylthio group, C₁₋₆ alkylsulfinyl group, C₁₋₆ alkylsulfonyl group, an optionally esterified carboxyl group, carbamoyl group, thiocarbamoyl group, mono- or di-C₁₋₆ alkyl-carbamoyl group, mono- or di-C₆₋₁₄ aryl-carbamoyl group, sulfamoyl group, mono- or di-C₁₋₆ alkyl-sulfamoyl group and mono- or di-C₆₋₁₄ aryl-sulfamoyl group and the like.

In the present specification, examples of the "optionally substituted C₃₋₈ cycloalkyl group", "optionally substituted C₆₋₁₄ aryl group", "optionally substituted C₇₋₁₆ aralkyl group", "optionally substituted heterocyclic group", "optionally substituted heterocyclic oxy group", "optionally substituted C₆₋₁₄ aryloxy group", "optionally substituted C₇₋₁₆ aralkyloxy group", "optionally substituted heterocyclic sulfonyloxy group", "optionally substituted heterocyclic thio group", "optionally substituted C₆₋₁₄ arylthio group" and "optionally substituted C₇₋₁₆ aralkylthio group" include "C₃₋₈ cycloalkyl group", "C₆₋₁₄ aryl group", "C₇₋₁₆ aralkyl group", "heterocyclic group", "heterocyclic oxy group", "C₆₋₁₄ aryloxy group", "C₇₋₁₆ aralkyloxy group", "heterocyclic sulfonyloxy group", "heterocyclic thio group", "C₆₋₁₄ arylthio group" and "C₇₋₁₆ aralkylthio group", each optionally having, at substitutable positions, 1 to 5 substituents selected from (1) halogen atom; (2) hydroxy group; (3) amino group; (4) nitro group; (5) cyano group; (6) optionally substituted C₁₋₆ alkyl group; (7) optionally substituted C₂₋₆ alkenyl group; (8) optionally substituted C₂₋₆ alkynyl group; (9) C₆₋₁₄ aryl group optionally substituted by 1 to 3 substituents selected from halogen atom, hydroxy group, amino group, nitro group, cyano group, optionally halogenated C₁₋₆ alkyl group, mono- or di-C₁₋₆ alkyl-amino group, C₆₋₁₄ aryl group, mono- or di-C₆₋₁₄ aryl-amino group, C₃₋₈ cycloalkyl group, C₁₋₆ alkoxy group, C₁₋₆ alkoxy-C₁₋₆ alkoxy group, C₁₋₆ alkylthio group, C₁₋₆ alkylsulfinyl group, C₁₋₆ alkylsulfonyl group, optionally esterified carboxyl group, carbamoyl group, thiocarbamoyl group, mono- or di-C₁₋₆ alkyl-carbamoyl group, mono- or di-C₆₋₁₄ aryl-carbamoyl group, sulfamoyl group, mono- or di-C₁₋₆ alkyl-sulfamoyl group and mono- or di-C₆₋₁₄ aryl-sulfamoyl group; (10) C₆₋₁₄ aryloxy group optionally substituted by 1 to 3 substituents selected from halogen atom, hydroxy group, amino group, nitro group, cyano group, optionally halogenated C₁₋₆ alkyl group, mono- or di-C₁₋₆ alkyl-amino group, C₆₋₁₄ aryl group, mono- or di-C₆₋₁₄ aryl-amino group, C₃₋₈ cycloalkyl group, C₁₋₆ alkoxy group, C₁₋₆ alkoxy-C₁₋₆ alkoxy group, C₁₋₆ alkylthio group, C₁₋₆ alkylsulfinyl group, C₁₋₆ alkylsulfonyl group, an optionally esterified carboxyl group, carbamoyl group, thiocarbamoyl group, mono- or di-C₁₋₆ alkyl-carbamoyl group, mono- or di-C₆₋₁₄ aryl-carbamoyl group, sulfamoyl group, mono- or di-C₁₋₆ alkyl-sulfamoyl group and mono- or di-C₆₋₁₄ aryl-sulfamoyl group; (11) C₇₋₁₆ aralkyloxy group optionally substituted by 1 to 3 substituents selected from halogen atom, hydroxy group, amino group, nitro group, cyano group, optionally halogenated C₁₋₆ alkyl group, mono- or di-C₁₋₆ alkyl-amino group, C₆₋₁₄ aryl group, mono- or di-C₆₋₁₄ aryl-amino group, C₃₋₈ cycloalkyl group, C₁₋₆ alkoxy group, C₁₋₆ alkoxy-C₁₋₆ alkoxy group, C₁₋₆ alkylthio group, C₁₋₆ alkylsulfinyl group, C₁₋₆ alkylsulfonyl group, optionally esterified carboxyl group, carbamoyl group, thiocarbamoyl group, mono- or di-C₁₋₆ alkyl-carbamoyl group, mono- or di-C₆₋₁₄ aryl-carbamoyl group, sulfamoyl group, mono- or di-C₁₋₆ alkyl-sulfamoyl group and mono- or di-C₆₋₁₄ aryl-sulfamoyl group; (12) heterocyclic group optionally substituted 1 to 3 substituents selected from halogen atom, hydroxy group, amino group, nitro group, cyano group, optionally halogenated C₁₋₆ alkyl group, mono- or di-C₁₋₆ alkyl-amino group, C₆₋₁₄ aryl group, mono- or di-C₆₋₁₄ aryl-amino group, C₃₋₈ cycloalkyl group, C₁₋₆ alkoxy group, C₁₋₆ alkoxy-C₁₋₆ alkoxy group, C₁₋₆ alkylthio group, C₁₋₆ alkylsulfinyl group, C₁₋₆ alkylsulfonyl group, optionally esterified carboxyl group, carbamoyl group, thiocarbamoyl group, mono- or di-C₁₋₆ alkyl-carbamoyl group, mono- or di-C₆₋₁₄ aryl-carbamoyl group, sulfamoyl group, mono- or di-C₁₋₆ alkyl-sulfamoyl group and mono- or di-C₆₋₁₄ aryl-sulfamoyl group (preferably furyl, pyridyl, thienyl, pyrazolyl, thiazolyl, oxazolyl, isoxazolyl, isothiazolyl, oxetanyl, morpholinyl, thiomorpholinyl, pyrrolidinyl, oxopyrrolidinyl, dioxopyrrolidinyl, tetrahydropyranyl, tetrahydrothiopyranyl, 1,1-dioxidotetrahydrothiopyranyl); (13) mono- or di-C₁₋₆ alkyl-amino group; (14) mono- or di-C₆₋₁₄ aryl-amino group; (15) mono-or di-C₇₋₁₆ aralkyl-amino group; (16) N-C₁₋₆ alkyl-N-C₆₋₁₄ aryl-amino group; (17) N-C₁₋₆ alkyl-N-C₇₋₁₆ aralkyl-amino group; (18) C₃₋₈ cycloalkyl group; (19) optionally substituted C₁₋₆ alkoxy group; (20) C₁₋₆ alkylthio group optionally substituted by C₁₋₆ alkoxy group; (21) C₁₋₆ alkylsulfinyl group optionally substituted by C₁₋₆ alkoxy group; (22) C₁₋₆ alkylsulfonyl group optionally substituted by C₁₋₆ alkoxy group; (23) optionally esterified carboxyl group; (24) carbamoyl group; (25) thiocarbamoyl group; (26) mono- or di-C₁₋₆ alkyl-carbamoyl group; (27) mono- or di-C₆₋₁₄ aryl-carbamoyl group; (28) mono- or di-5-to 7-membered heterocycle-carbamoyl group; (29) sulfamoyl group; (30) mono- or di-C₁₋₆ alkyl-sulfamoyl group; (31) mono- or di-C₆₋₁₄ aryl-sulfamoyl group; (32) C₁₋₆ alkylsulfonyloxy group; (33) tri-C₁₋₆ alkyl-silyloxy group; (34) nitrogen-containing heterocycle-carbonyl group (e.g., pyrrolidinylcarbonyl, piperidinocarbonyl, morpholinocarbonyl, thiomorpholinocarbonyl); (35) heterocyclic oxy group optionally substituted by 1 to 3 substituents selected from halogen atom, hydroxy group, amino group, nitro group, cyano group, optionally halogenated C₁₋₆ alkyl group, mono- or di-C₁₋₆ alkyl-amino group, C₆₋₁₄ aryl group, mono- or di-C₆₋₁₄ aryl-amino group, C₃₋₈ cycloalkyl group, C₁₋₆ alkoxy group, C₁₋₆ alkoxy-C₁₋₆ alkoxy group, C₁₋₆ alkylthio group, C₁₋₆ alkylsulfinyl group, C₁₋₆ alkylsulfonyl group, optionally esterified carboxyl group, carbamoyl group, thiocarbamoyl group, mono- or di-C₁₋₆ alkyl-carbamoyl group, mono- or di-C₆₋₁₄ aryl-carbamoyl group, sulfamoyl group, mono- or di-C₁₋₆ alkyl-sulfamoyl group and mono- or di-C₆₋₁₄ aryl-sulfamoyl group; (36) optionally substituted C₁₋₄ alkylenedioxy group (e.g., methylenedioxy, ethylenedioxy); (37) C₁₋₆ alkyl-carbonylamino group (e.g., acetylamino); (38) N-C₁₋₆ alkyl-N-(C₁₋₆ alkyl-carbonyl)-amino group (e.g., N-acetyl-N-ethylamino); and the like.

In the present specification, examples of the "optionally substituted amino group" include, unless otherwise specified, amino group optionally substituted by 1 or 2 substituents selected from (1) optionally substituted C₁₋₆ alkyl group; (2) optionally substituted C₂₋₆ alkenyl group; (3) optionally substituted C₂₋₆ alkynyl group; (4) optionally substituted C₃₋₈ cycloalkyl group; (5) optionally substituted C₆₋₁₄ aryl group; (6) optionally substituted C₁₋₆ alkoxy group; (7) optionally substituted acyl group; (8) optionally substituted heterocyclic group (preferably furyl, pyridyl, thienyl, pyrazolyl, thiazolyl, oxazolyl); (9) sulfamoyl group; (10) mono- or di-C₁₋₆ alkyl-sulfamoyl group; (11) mono- or di-C₆₋₁₄ aryl-sulfamoyl group and the like. When the "optionally substituted amino group" is amino group substituted by two substituents, these substituents may form, together with the adjacent nitrogen atom, nitrogen-containing heterocycle. Examples of the "nitrogen-containing heterocycle" include 5- to 7-membered nitrogen-containing heterocycle containing, as a ring constituting atom besides carbon atom, at least one nitrogen atom, and further optionally containing 1 or 2 hetero atoms selected from oxygen atom, sulfur atom and nitrogen atom. Preferable examples of the nitrogen-containing heterocycle include pyrrolidine, imidazolidine, pyrazolidine, piperidine, piperazine, morpholine, thiomorpholine, thiazolidine, oxazolidine and the like.

In the present specification, examples of the "optionally substituted acyl group" include, unless otherwise specified, groups represented by formula: -COR⁷, -CO-OR⁷, -SO₂R⁷, -SOR⁷,-PO(OR⁷) (OR⁸), -CO-NR^{7a}R^{8a} and -CS-NR^{7a}R^{8a} wherein R⁷ and R⁸ are the same or different and each is hydrogen atom, optionally substituted hydrocarbon group or optionally substituted heterocyclic group, R^{7a} and R^{8a} are the same or different and each is hydrogen atom, optionally substituted hydrocarbon group or optionally substituted heterocyclic group, R^{7a} and R^{8a} may form, together with the adjacent nitrogen atom, optionally substituted nitrogen-containing heterocycle, and the like.

Examples of the "nitrogen-containing heterocycle" of the "optionally substituted nitrogen-containing heterocycle" formed by R^{7a} and R^{8a}, together with the adjacent nitrogen atom include 5- to 7-membered nitrogen-containing heterocycle containing, as a ring constituting atom besides carbon atom, at least one nitrogen atom, and further optionally containing 1 or 2 hetero atoms selected from oxygen atom, sulfur atom and nitrogen atom. Preferable examples of the nitrogen-containing heterocycle include pyrrolidine, imidazolidine, pyrazolidine, piperidine, piperazine, morpholine, thiomorpholine, thiazolidine, oxazolidine and the like.

The nitrogen-containing heterocycle optionally has 1 or 2 substituents at substitutable positions. Examples of the substituent include hydroxy group, optionally halogenated C₁₋₆ alkyl group, C₆₋₁₄ aryl group, C₇₋₁₆ aralkyl group and the like.

Preferable examples of the "optionally substituted acyl group" include formyl group; carboxyl group; carbamoyl group; C₁₋₆ alkyl-carbonyl group (e.g., acetyl, isobutanoyl, isopentanoyl) optionally substituted by 1 to 3 halogen atoms; C₁₋₆ alkoxy-carbonyl group (e.g., methoxycarbonyl, ethoxycarbonyl, propoxycarbonyl, tert-butoxycarbonyl) optionally substituted by 1 to 3 halogen atoms; C₃₋₈ cycloalkyl-carbonyl group (e.g., cyclopentylcarbonyl, cyclohexylcarbonyl); C₆₋₁₄ aryl-carbonyl group (e.g., benzoyl, 1-naphthoyl, 2-naphthoyl); C₇₋₁₆ aralkyl-carbonyl group (e.g., phenylacetyl, 2-phenylpropanoyl); C₆₋₁₄ aryloxy-carbonyl group (e.g., phenyloxycarbonyl, naphthyloxycarbonyl); C₇₋₁₆ aralkyloxy-carbonyl group (e.g., benzyloxycarbonyl, phenethyloxycarbonyl); mono- or di-C₁₋₆ alkyl-carbamoyl group; mono- or di-C₆₋₁₄ aryl-carbamoyl group; C₃₋₈ cycloalkyl-carbamoyl group (e.g., cyclopropylcarbamoyl); C₇₋₁₆ aralkyl-carbamoyl group (e.g., benzylcarbamoyl); C₁₋₆ alkylsulfonyl group optionally substituted by 1 to 3 halogen atoms; C₆₋₁₄ arylsulfonyl group (e.g., phenylsulfonyl) optionally substituted by nitro group; nitrogen-containing heterocycle-carbonyl group (e.g., pyrrolidinylcarbonyl, piperidinocarbonyl, morpholinocarbonyl, thiomorpholinocarbonyl); C₁₋₆ alkylsulfinyl group optionally substituted by 1 to 3 halogen atoms; C₆₋₁₄ arylsulfinyl group; thiocarbamoyl group; and the like.

In the present specification, examples of the "C₁₋₄ alkylenedioxy group" of the "optionally substituted C₁₋₄ alkylenedioxy group" include, unless otherwise specified, methylenedioxy, ethylenedioxy, propylenedioxy, tetrafluoroethylenedioxy and the like. The C₁₋₄ alkylenedioxy group optionally has 1 to 3 substituents at substitutable positions. Examples of the substituent include halogen atom, hydroxy group, amino group, mono- or di-C₁₋₆ alkyl-amino group, mono- or di-C₆₋₁₄ aryl-amino group, mono- or di-C₇₋₁₆ aralkyl-amino group, nitro group, cyano group, C₁₋₆ alkoxy group, C₁₋₆ alkylthio group and the like.

Each symbol in the formulas (I₀) and (I) is described in detail in the following.

Ring A₀ in the formula (I₀) is pyrazole ring optionally further having 1 or 2 substituents. The pyrazole ring for ring A₀ optionally further has, besides R^{a} and R^{b}, 1 or 2 substituents at substitutable positions. Examples of the "substituent" include those exemplified as the substituent of the aforementioned "optionally substituted C₃₋₈ cycloalkyl group", with preference given to optionally substituted hydrocarbon group (e.g., C₁₋₆ alkyl group and the like) and optionally substituted acyl group (e.g., mono- or di-C₆₋₁₄ aryl-carbamoyl group and the like). When the pyrazole ring has two substituents, they may be the same or different.

Ring A₀ preferably has no substituent besides R^{a} and R^{b}.

R^{a} in the formula (I₀) is substituted carbamoyl group, preferably carbamoyl group wherein the amino moiety is substituted by 1 or 2 substituents selected from (1) optionally substituted hydrocarbon group, (2) optionally substituted heterocyclic group and the like.

When the amino moiety of carbamoyl group is substituted by 2 substituents, these substituents may form, together with the adjacent nitrogen atom, nitrogen-containing heterocycle. Examples of the "nitrogen-containing heterocycle" include 5- to 7-membered nitrogen-containing heterocycle containing, as a ring constituting atom besides carbon atom, at least one nitrogen atom, and further optionally containing 1 or 2 hetero atoms selected from oxygen atom, sulfur atom and nitrogen atom. Preferable examples of the nitrogen-containing heterocycle include pyrrolidine, piperidine, morpholine, thiomorpholine, azepane, 1,2,3,4-tetrahydroquinoline, octahydroisoquinoline and the like.

Preferable examples of the "substituted carbamoyl group" include group represented by the formula: 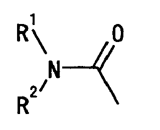 wherein
R¹ is optionally substituted hydrocarbon group or optionally substituted heterocyclic group,
R² is hydrogen atom or optionally substituted hydrocarbon group, or R¹ and R² form, together with the adjacent nitrogen atom, nitrogen-containing heterocycle.

R^{b} in the formula (I₀) is optionally substituted acylamino group. Examples of the "optionally substituted acylamino group" include, unless otherwise specified, the aforementioned "optionally substituted amino group" substituted by 1 or 2 of the aforementioned "optionally substituted acyl groups".

Preferable examples of the "optionally substituted acylamino group" include group represented by the formula: 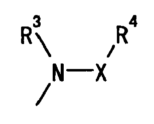 wherein R³ is hydrogen atom or optionally substituted hydrocarbon group;
R⁴ is optionally substituted hydrocarbon group, optionally substituted heterocyclic group, optionally substituted hydroxy group or optionally substituted amino group;
X is carbonyl group or sulfonyl group.

The compound represented by the formula (I₀) is preferably the compound represented by the formula (I).

R¹ in the formula (I) is optionally substituted hydrocarbon group or optionally substituted heterocyclic group. It is preferably (1) optionally substituted C₁₋₆ alkyl group (more preferably, substituted C₁₋₆ alkyl group or unsubstituted C₄₋₆ alkyl group), (2) optionally substituted C₂₋₆ alkenyl group, (3) optionally substituted C₂₋₆ alkynyl group, (4) optionally substituted C₃₋₈ cycloalkyl group, (5) optionally substituted C₆₋₁₄ aryl group (preferably, phenyl, biphenylyl, indanyl), (6) optionally substituted C₇₋₁₆ aralkyl group (preferably, benzyl, phenethyl, diphenylmethyl, 3-phenylpropyl, 3,3-diphenylpropyl), (7) optionally substituted 5- to 14-membered (preferably 5- to 10-membered) aromatic heterocyclic group (preferably, quinolyl), (8) optionally substituted 5- to 10-membered nonaromatic heterocyclic group (preferably, 2-oxo-azepanyl) and the like.

R¹ in the formula (I) is preferably C₁₋₆ alkyl group, C₂₋₆ alkenyl group or C₂₋₆ alkynyl group (particularly C₁₋₆ alkyl group), each of which is optionally substituted by 1 to 5 substituents selected from halogen atom, optionally substituted hydroxy group, optionally substituted thio group, optionally substituted sulfinyl group, optionally substituted sulfonyl group, optionally substituted amino group, nitro group, cyano group, and optionally substituted carbonyl group.

R¹ in the formula (I) is more preferably C₁₋₆ alkyl group, C₂₋₆ alkenyl group or C₂₋₆ alkynyl group (particularly C₁₋₆ alkyl group), each of which optionally has, at substitutable positions, 1 to 5 substituents selected from the followings:
(1) halogen atom;
(2) hydroxy group;
(3) amino group;
(4) nitro group;
(5) cyano group;
(7) mono- or di-C₁₋₆ alkyl-amino group;
(8) mono- or di-C₆₋₁₄ aryl-amino group;
(9) mono- or di-C₇₋₁₆ aralkyl-amino group;
(10) N-C₁₋₆ alkyl-N-C₆₋₁₄ aryl-amino group;
(11) N-C₁₋₆ alkyl-N-(C₁₋₆ alkyl-C₆₋₁₄ aryl)-amino group;
(12) N-C₁₋₆ alkyl-N-C₇₋₁₆ aralkyl-amino group;
(14) optionally halogenated C₁₋₆ alkoxy group;
(15) C₁₋₆ alkylthio group optionally substituted by C₁₋₆ alkoxy group;
(18) optionally esterified carboxyl group;
(19) carbamoyl group;
(20) thiocarbamoyl group;
(21) mono- or di-C₁₋₆ alkyl-carbamoyl group;
(22) mono- or di-C₆₋₁₄ aryl-carbamoyl group;
(23) mono- or di-5- to 7-membered heterocycle-carbamoyl group;
(24) C₁₋₆ alkyl-carbonylamino group optionally substituted by carboxyl group;
(25) C₆₋₁₄ aryloxy group optionally substituted by 1 to 3 substituents selected from halogen atom, hydroxy group, amino group, nitro group, cyano group, optionally halogenated C₁₋₆ alkyl group, mono- or di-C₁₋₆ alkyl-amino group, C₆₋₁₄ aryl group, mono- or di-C₆₋₁₄ aryl-amino group, C₃₋₈ cycloalkyl group, C₁₋₆ alkoxy group, C₁₋₆ alkoxy-C₁₋₆ alkoxy group, C₁₋₆ alkylthio group, C₁₋₆ alkylsulfinyl group, C₁₋₆ alkylsulfonyl group, optionally esterified carboxyl group, carbamoyl group, thiocarbamoyl group, mono- or di-C₁₋₆ alkyl-carbamoyl group, mono- or di-C₆₋₁₄ aryl-carbamoyl group, sulfamoyl group, mono- or di-C₁₋₆ alkyl-sulfamoyl group and mono- or di-C₆₋₁₄ aryl-sulfamoyl group;
(27) heterocyclic oxy group optionally substituted by 1 to 3 substituents selected from halogen atom, hydroxy group, amino group, nitro group, cyano group, optionally halogenated C₁₋₆ alkyl group, mono- or di-C₁₋₆ alkyl-amino group, C₆₋₁₄ aryl group, mono- or di-C₆₋₁₄ aryl-amino group, C₃₋₈ cycloalkyl group, C₁₋₆ alkoxy group, C₁₋₆ alkoxy-C₁₋₆ alkoxy group, C₁₋₆ alkylthio group, C₁₋₆ alkylsulfinyl group, C₁₋₆ alkylsulfonyl group, optionally esterified carboxyl group, carbamoyl group, thiocarbamoyl group, mono- or di-C₁₋₆ alkyl-carbamoyl group, mono- or di-C₆₋₁₄ aryl-carbamoyl group, sulfamoyl group, mono- or di-C₁₋₆ alkyl-sulfamoyl group and mono- or di-C₆₋₁₄ aryl-sulfamoyl group;
(31) C₇₋₁₆ aralkyloxy group optionally substituted by 1 to 3 substituents selected from halogen atom, hydroxy group, amino group, nitro group, cyano group, optionally halogenated C₁₋₆ alkyl group, mono- or di-C₁₋₆ alkyl-amino group, C₆₋₁₄ aryl group, mono- or di-C₆₋₁₄ aryl-amino group, C₃₋₈ cycloalkyl group, C₁₋₆ alkoxy group, C₁₋₆ alkoxy-C₁₋₆ alkoxy group, C₁₋₆ alkylthio group, C₁₋₆ alkylsulfinyl group, C₁₋₆ alkylsulfonyl group, optionally esterified carboxyl group, carbamoyl group, thiocarbamoyl group, mono- or di-C₁₋₆ alkyl-carbamoyl group, mono- or di-C₆₋₁₄ aryl-carbamoyl group, sulfamoyl group, mono- or di-C₁₋₆ alkyl-sulfamoyl group and mono- or di-C₆₋₁₄ aryl-sulfamoyl group;
(32) C₁₋₆ alkylsulfonyloxy group;
(33) tri-C₁₋₆ alkyl-silyloxy group;
(34) nitrogen-containing heterocycle-carbonyl group;
(35) C₆₋₁₄ aryl-carbonyl group;
(36) C₆₋₁₄ aryl-thio group;
(37) optionally halogenated C₆₋₁₄ aryl-sulfonyl group;
(38) nitrogen-containing heterocycle-sulfonyl group; and
(39) nitrogen-containing heterocycle-amino group optionally substituted by cyano group.

When the compound of the formula (I) is used as an agent for the prophylaxis or treatment of GSK-3 related pathology or disease, R¹ is more preferably C₁₋₆ alkyl group substituted by halogen atom (particularly, fluorine atom), hydroxy group or cyano group. Particularly, when the GSK-3 related pathology or disease is neurodegenerative disease, R¹ is particularly preferably C₁₋₆ alkyl group substituted by fluorine atom (particularly, three fluorine atoms).

R² in the formula (I) is hydrogen atom or optionally substituted hydrocarbon group, preferably hydrogen atom, optionally substituted C₁₋₆ alkyl group and the like, more preferably hydrogen atom.

R¹ and R² in the formula (I) optionally form, together with the adjacent nitrogen atom, optionally substituted nitrogen-containing heterocycle. Preferable examples of the nitrogen-containing heterocycle include 5- to 7-membered nitrogen-containing heterocycle (preferably, pyrrolidine, piperidine, morpholine, thiomorpholine, azepane, 1,2,3,4-tetrahydroquinoline, octahydroisoquinoline) and the like, containing at least one nitrogen atom, and further optionally containing 1 or 2 hetero atoms selected from oxygen atom, sulfur atom and nitrogen atom. The nitrogen-containing heterocycle optionally has 1 or 2 substituents at substitutable positions. Examples of the substituent include those exemplified as the substituent of the aforementioned "optionally substituted C₃₋₈ cycloalkyl group".

R³ in the formula (I) is hydrogen atom or optionally substituted hydrocarbon group, preferably hydrogen atom.

R⁴ in the formula (I) is optionally substituted hydrocarbon group, optionally substituted heterocyclic group, optionally substituted hydroxy group or optionally substituted amino group. It is preferably (1) optionally substituted C₁₋₆ alkyl group, (2) optionally substituted C₂₋₆ alkenyl group, (3) optionally substituted C₃₋₈ cycloalkyl group, (4) optionally substituted C₆₋₁₄ aryl group (preferably, phenyl, biphenylyl), (5) optionally substituted C₇₋₁₆ aralkyl group (preferably, benzyl, phenethyl, diphenylmethyl, biphenylmethyl), (6) optionally substituted 5-to 14-membered (preferably 5- to 10-membered) aromatic heterocyclic group (preferably, 1H-indolyl, pyridyl, pyrazinyl, quinolyl), (7) optionally substituted amino group [preferably amino group optionally substituted by 1 or 2 substituents selected from C₁₋₆ alkyl group and C₆₋₁₄ aryl group (e.g., phenyl)].

Ring A in the formula (I) is pyrazole ring represented by the formula:

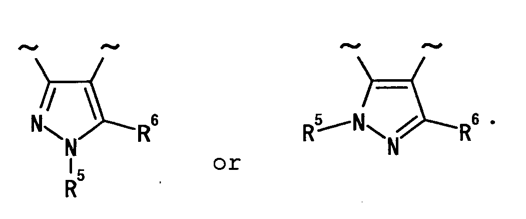

R⁵ in the formula is hydrogen atom, optionally substituted hydrocarbon group or optionally substituted acyl group. It is preferably (1) hydrogen atom, (2) optionally substituted C₁₋₆ alkyl group, (3) mono- or di-C₆₋₁₄ aryl-carbamoyl group (preferably, phenylcarbamoyl group) and the like, more preferably hydrogen atom.

R⁶ in the formula is hydrogen atom or optionally substituted hydrocarbon group. It is preferably (1) hydrogen atom, or (2) optionally substituted C₁₋₆ alkyl group, more preferably hydrogen atom.

X in the formula (I) is carbonyl group or sulfonyl group, preferably carbonyl group.

Preferable examples of the compound represented by the formula (I) include the following compounds.

### [Compound IA]

A compound wherein
R¹ is
   (1) C₁₋₆ alkyl group optionally substituted by 1 to 3 substituents selected from (a) cyano group, (b) C₃₋₈ cycloalkyl group, (c) 5- to 14-membered (preferably 5- to 10-membered) aromatic heterocyclic group (e.g., furanyl, pyridyl, 1H-indolyl), (d) 5- to 10-membered nonaromatic heterocyclic group (e.g., tetrahydrofuryl, 2-oxo-pyrrolyl), (e) C₁₋₆ alkoxy group, and (f) C₁₋₆ alkylthio group;
   (2) C₃₋₈ cycloalkyl group;
   (3) C₆₋₁₄ aryl group (e.g., phenyl, biphenylyl, indanyl) optionally substituted by 1 to 3 substituents selected from (a) C₁₋₆ alkoxy group optionally substituted by halogen atom(s), (b) C₇₋₁₆ aralkyl group (e.g., benzyl) and (c) C₁₋₄ alkylenedioxy group (e.g., ethylenedioxy);
   (4) C₇₋₁₆ aralkyl group (e.g., benzyl, phenethyl, diphenylmethyl, 3-phenylpropyl, 3,3-diphenylpropyl) optionally substituted by 1 to 3 substituents selected from (a) C₁₋₆ alkyl group optionally substituted by halogen atom(s), (b) C₁₋₆ alkoxy group, (c) carboxyl group and (d) C₁₋₄ alkylenedioxy group (e.g., methylenedioxy);
   (5) 5- to 14-membered (preferably 5- to 10-membered) aromatic heterocyclic group (e.g., quinolyl); or
   (6) 5- to 10-membered nonaromatic heterocyclic group (e.g., 2-oxo-azepanyl);
R² is hydrogen atom, or C₁₋₆ alkyl group optionally substituted by C₁₋₆ alkoxy group(s); or
R¹ and R² form, together with the adjacent nitrogen atom, 5- to 7-membered nitrogen-containing heterocycle (e.g., pyrrolidine, piperidine, morpholine, thiomorpholine, azepane, 1,2,3,4-tetrahydroquinoline, octahydroisoquinoline) containing at least one nitrogen atom, and further optionally containing 1 or 2 hetero atoms selected from oxygen atom, sulfur atom and nitrogen atom and optionally substituted by 1 to 3 substituents selected from (a) hydroxy group, (b) C₇₋₁₆ aralkyl group (e.g., benzyl), (c) C₁₋₆ alkyl group optionally substituted by 1 to 3 substituents selected from mono- or di-C₆₋₁₄ aryl-amino group (e.g., 2,6-dimethylphenylamino) optionally substituted by C₁₋₆ alkyl group(s) and hydroxy group, (d) C₆₋₁₄ aryl group (e.g., phenyl)optionally substituted by 1 to 3 substituents selected from halogen atom, C₁₋₆ alkyl group optionally substituted by halogen atom(s), C₁₋₆ alkyl-carbonyl group and C₁₋₆ alkoxy group, (e) carbamoyl group, (f) C₁₋₆ alkyl-carbonylamino group (e.g., acetylamino), and (g) N-C₁₋₆ alkyl-N-(C₁₋₆ alkyl-carbonyl)-amino group (e.g., acetylethylamino);
R³ is hydrogen atom;
R⁴ is
   (1) C₁₋₆ alkyl group optionally substituted by 1 to 3 substituents selected from (a) C₁₋₆ alkoxy group, (b) C₃₋₈ cycloalkyl group optionally substituted by C₁₋₆ alkyl group(s) (e.g., 7,7-dimethyl-2-oxo-bicyclo[2.2.1]heptyl), (c) C₆₋₁₄ aryloxy group (e.g., phenoxy), (d) 5- to 14-membered aromatic heterocycle (preferably 5- to 10-membered) (e.g., 1H-indole), and (e) mono- or di-C₁₋₆ alkyl-amino group;
   (2) C₂₋₆ alkenyl group optionally substituted by C₆₋₁₄ aryl group(s);
   (3) C₃₋₈ cycloalkyl group;
   (4) C₆₋₁₄ aryl group (e.g., phenyl, biphenylyl) optionally substituted by 1 to 3 substituents selected from (a) halogen atom, (b) nitro group, (c) cyano group, (d) C₁₋₆ alkyl group optionally substituted by halogen atom(s), (e) C₂₋₆ alkenyl group, (f) C₁₋₆ alkoxy group optionally substituted by halogen atom(s), (g) C₆₋₁₄ aryloxy group (e.g., phenoxy), (h) mono- or di-C₁₋₆ alkyl-sulfamoyl group, and (i) C₁₋₆ alkyl-carbonylamino group (e.g., acetylamino);
   (5) C₇₋₁₆ aralkyl group (e.g., benzyl, phenethyl, diphenylmethyl, biphenylylmethyl) optionally substituted by 1 to 3 substituents selected from (a) halogen atom, and (b) C₁₋₆ alkoxy group;
   (6) 5- to 14-membered (preferably 5- to 10-membered) aromatic heterocyclic group (e.g., 1H-indolyl, pyridyl, pyrazinyl, quinolyl) optionally substituted by halogen atom(s); or
   (7) amino group optionally substituted by 1 or 2 substituents selected from C₁₋₆ alkyl group and C₆₋₁₄ aryl group (e.g., phenyl);

Ring A is pyrazole ring represented by the formula: 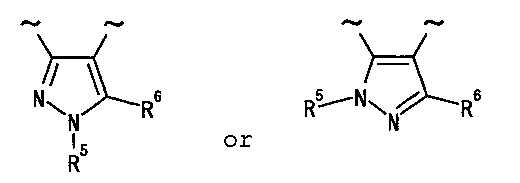 wherein
R⁵ is hydrogen atom, C₁₋₆ alkyl group, or mono- or di-C₆₋₁₄ aryl-carbamoyl group (e.g., phenylcarbamoyl group); or
R⁶ is hydrogen atom, or C₁₋₆ alkyl group; and
   X is carbonyl group or sulfonyl group.

Of the compounds represented by the above-mentioned formulas (I₀) and (I), the following compounds are known, but a GSK-3 inhibitory action of these compounds has not been known heretofore.
1) N-methyl-2,5-dimethyl-4-{2-[4-(4-nitrophenoxy)phenyl]acetylamino}-2H-pyrazole-3-carboxamide,
2) N-methyl-4-{2-[4-(4-chlorophenoxy)phenyl]acetylamino}-2,5-dimethyl-2H-pyrazole-3-carboxamide,
3) N-(9-methyl-4-oxo-1-phenyl-3,4,6,7-tetrahydro-[1,4]diazepino[6,7,1-hi]indol-3-yl)-4-acetylamino-2,5-dimethyl-2H-pyrazole-3-carboxamide,
4) N-phenyl-4-benzoylamino-2-ethyl-5-methyl-2H-pyrazole-3-carboxamide,
5) N-phenyl-2-ethyl-5-methyl-4-(2-nitrobenzoylamino)-2H-pyrazole-3-carboxamide,
6) N-(4-chlorophenyl)-2-ethyl-5-methyl-4-(4-nitrobenzoylamino)-2H-pyrazole-3-carboxamide,
7) N-phenyl-2-ethyl-5-methyl-4-(4-methylbenzoylamino)-2H-pyrazole-3-carboxamide,
8) N-methyl-N-(2-phenyl-2H-pyrazol-3-yl)-4-acetylamino-2-ethyl-5-methyl-2H-pyrazole-3-carboxamide,
9) N-methyl-N-(5-methyl-2-phenyl-2H-pyrazol-3-yl)-4-acetylamino-2-ethyl-5-methyl-2H-pyrazole-3-carboxamide,
10) N-methyl-N-(2-methyl-2H-pyrazol-3-yl)-4-acetylamino-2-ethyl-5-methyl-2H-pyrazole-3-carboxamide,
11) N-(2,5-dimethyl-2H-pyrazol-3-yl)-N-methyl-4-acetylamino-2-ethyl-5-methyl-2H-pyrazole-3-carboxamide,
12) N-(4-tert-butylbenzyl)-4-acetylamino-2,5-dimethyl-2H-pyrazole-3-carboxamide,
13) N-(4-tert-butylbenzyl)-4-(2-chloroacetylamino)-2,5-dimethyl-2H-pyrazole-3-carboxamide,
14) N-(4-tert-butylbenzyl)-4-benzoylamino-2,5-dimethyl-2H-pyrazole-3-carboxamide,
15) N,N-dimethyl-4-methanesulfonylamino-1-methyl-5-[1-methyl-2,5-dioxo-4-(1-phenylpropylamino)-2,5-dihydro-1H-pyrrol-3-ylamino]-1H-pyrazole-3-carboxamide,
16) N,N-dimethyl-5-[2,5-dioxo-4-(1-phenylpropylamino)-2,5-dihydro-1H-pyrrol-3-ylamino]-4-methanesulfonylamino-1-methyl-1H-pyrazole-3-carboxamide,
17) N,N-dimethyl-5-(4-chloro-1-methyl-2,5-dioxo-2,5-dihydro-1H-pyrrol-3-ylamino)-4-methanesulfonylamino-1-methyl-1H-pyrazole-3-carboxamide, and
18) N,N-dimethyl-5-(4-chloro-1-methyl-2,5-dioxo-2,5-dihydro-1H-pyrrol-3-ylamino)-4-methanesulfonylamino-1-methyl-1H-pyrazole-3-carboxamide.

Examples of salts of compound (I₀) and compound (I) of the present invention (unless otherwise specified, these are collectively referred to as compound A) include metal salt, ammonium salt, salt with organic base, salt with inorganic acid, salt with organic acid, salt with basic or acidic amino acid and the like.

Here, preferable examples of the metal salt include alkali metal salt such as sodium salt, potassium salt and the like; alkaline earth metal salts such as calcium salt, magnesium salt, barium salt and the like, and the like. Preferable examples of the salt with organic base include salts with trimethylamine, triethylamine, pyridine, picoline, 2,6-lutidine, ethanolamine, diethanolamine, triethanolamine, cyclohexylamine, dicyclohexylamine, N,N'-dibenzylethylenediamine and the like. Preferable examples of the salt with inorganic acid include salts with hydrochloric acid, hydrobromic acid, nitric acid, sulfuric acid, phosphoric acid and the like. Preferable examples of the salt with organic acid include salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid and the like. Preferable examples of the salt with basic amino acid include salts with arginine, lysine, ornithine and the like, and preferable examples of the salt with acidic amino acid include salts with aspartic acid, glutamic acid and the like.

Of these salts, a pharmaceutically acceptable salt is preferable. For example, when compound A has an acidic functional group, metal salts such as alkali metal salt, alkaline earth metal salt and the like; ammonium salt and the like, and when compound A has a basic functional group, for example, a salt with inorganic acid or organic acid is preferable.

A prodrug of the compound A or a salt thereof means a compound which is converted to the compound A with a reaction due to an enzyme, an gastric acid, etc. under the physiological condition in the living body, that is, a compound which is converted to the compound A by enzymatic oxidation, reduction, hydrolysis, etc.; a compound which is converted to the compound A by hydrolysis etc. due to gastric acid, etc.

A prodrug of compound A may be a compound obtained by subjecting an amino group in compound A to an acylation, alkylation or phosphorylation (e.g., a compound obtained by subjecting an amino group in compound A to an eicosanoylation, alanylation, pentylaminocarbonylation, (5-methyl-2-oxo-1,3-dioxolen-4-yl)methoxycarbonylation, tetrahydrofuranylation, pyrrolidylmethylation, pivaloyloxymethylation and tert-butylation, etc.); a compound obtained by subjecting a hydroxy group in compound A to an acylation, alkylation, phosphorylation or boration (e.g., a compound obtained by subjecting a hydroxy group in compound A to an acetylation, palmitoylation, propanoylation, pivaloylation, succinylation, fumarylation, alanylation, dimethylaminomethylcarbonylation, etc.); a compound obtained by subjecting a carboxyl group in compound A to an esterification or amidation (e.g., a compound obtained by subjecting a carboxy group in compound A to an C₁₋₆ alkyl esterification, phenyl esterification, carboxymethyl esterification, dimethylaminomethyl esterification, pivaloyloxymethyl esterification, ethoxycarbonyloxyethyl esterification, phthalidyl esterification, (5-methyl-2-oxo-1,3-dioxolen-4-yl)methyl esterification, cyclohexyloxycarbonylethyl esterification and methylamidation, etc.) and the like. Of these, a compound wherein the carboxy group in the compound A is esterified with a C₁₋₆ alkyl group such as methyl, ethyl, tert-butyl and the like is preferably used. Any of these compounds can be produced from compound A by a method known *per se.*

A prodrug of the compound A may be a compound that converts to the compound A under physiological conditions as described in Development of Pharmaceutical Products, vol. 7, Molecule Design, 163-198, Hirokawa Shoten (1990).

Compound A and a salt thereof can be produced by the following method and the like.

For convenience, ring A is defined as 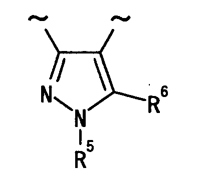 in the explanation. As those of ordinary skill in the art will readily understand, compound A wherein ring A is 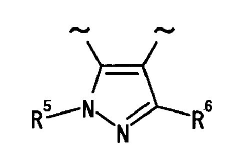 can be produced in the same manner.

In addition, compound A other than compound (I) can also be produced by a method analogous thereto.

Unless otherwise specified, each symbol of the compounds in the following reaction schemes means the same as mentioned above. The compounds in the reaction schemes include salts and examples of the salts are those defined for compound (I) and the like. The production methods of compound (I) are now explained.

Compound (I) can be produced by reacting compound (2) with compound (3) in, where desired, the presence of a base or acid, according to the method described in the following reaction scheme 1.

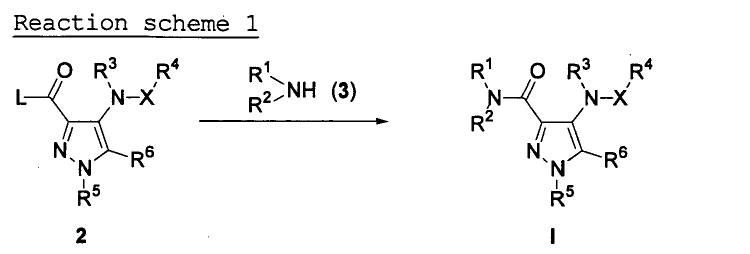

In the reaction scheme 1, L is a leaving group, and other symbols are as defined above.

Examples of the "leaving group" for L include hydroxy, optionally substituted alkoxy group, optionally substituted aryloxy group (e.g., phenyloxy, pyridyloxy, pyrazinyloxy, N-methylpyridinium oxy), halogen atom (e.g., fluorine, chlorine, bromine, iodine etc.), optionally halogenated C₁₋₅ alkylsulfonyloxy (e.g., methanesulfonyloxy, ethanesulfonyloxy, trichloromethanesulfonyloxy etc.), optionally substituted C₆₋₁₀ arylsulfonyloxy and the like. Examples of the "optionally substituted C₆₋₁₀ arylsulfonyloxy" include C₆₋₁₀ arylsulfonyloxy (e.g., phenylsulfonyloxy, naphthylsulfonyloxy etc.) optionally having 1 to 3 substituents selected from C₁₋₆ alkyl (e.g., methyl, ethyl, propyl, isopropyl, butyl, isobutyl, sec-butyl, tert-butyl, pentyl, hexyl etc.), C₁₋₆ alkoxy (e.g., methoxy, ethoxy, propoxy, isopropoxy, butoxy, isobutoxy, sec-butoxy, pentyloxy, hexyloxy etc.) and nitro and the like. Specific examples thereof include benzenesulfonyloxy, m-nitrobenzenesulfonyloxy, p-toluenesulfonyloxy and the like.

Commercially available products of compound (2) and compound (3) can be easily obtained, and these compounds can also be produced by a method known per se or a method according thereto.

The amount of compound (3) to be used is about 1.0 to 10 mol, preferably about 1.0 to 2.0 mol, relative to 1 mol of compound (2).

As the "base", for example, basic salts such as sodium carbonate, potassium carbonate, cesium carbonate, sodium hydrogencarbonate and the like, aromatic amines such as pyridine, lutidine and the like, tertiary amines such as triethylamine, tripropylamine, tributylamine, cyclohexyldimethylamine, 4-dimethylaminopyridine, N-methylpiperidine, N-methylpyrrolidine, N-methylmorpholine and the like, alkali metals such as metal sodium and the like, alkali metal hydrides such as sodium hydride, potassium hydride and the like, metal amides such as sodium amide, lithium diisopropylamide, lithium hexamethyldisilazide and the like, metal alkoxides such as sodium methoxide, sodium ethoxide, potassium tert-butoxide and the like, and the like can be used.

As the "acid", for example, methanesulfonic acid, p-toluenesulfonic acid, camphorsulfonic acid and the like can be used.

The amount of the "base" to be use is about 0.1 to 10 equivalent amount, preferably 0.8 to 2 equivalent amount, relative to compound (2).

The amount of the "acid" to be use is about 0.1 to 10 equivalent amount, preferably 0.8 to 3 equivalent amount, relative to compound (2).

This reaction is advantageously carried out without solvent or using a solvent inert to the reaction. Such solvent is not particularly limited as long as the reaction proceeds and, for example, solvents including ethers such as diethyl ether, tetrahydrofuran, dioxane, 1,2-dimethoxyethane and the like, hydrocarbons such as benzene, toluene, cyclohexane, hexane and the like, amides such as N,N-dimethylformamide, N,N-dimethylacetamide and the like, halogenated hydrocarbons such as dichloromethane, chloroform, carbon tetrachloride, 1,2-dichloroethane and the like, nitriles such as acetonitrile, propionitrile and the like, sulfoxides such as dimethyl sulfoxide and the like, nitrogen-containing aromatic hydrocarbons such as pyridine, lutidine, quinoline and the like, and the like, and a mixed solvent thereof and the like are preferable. The reaction temperature is about -40°C to 150°C, preferably 0°C to 100°C. The reaction time is generally 5 min to 24 hr, preferably 10 min to 5 hr.

The resultant product (I) obtained as mentioned above can be used for the next reaction in the form of a reaction mixture or a crude product. It can also be isolated from the reaction mixture according to a conventional method, and easily purified by a separation means such as recrystallization, distillation, chromatography and the like.

As an alternative method replacing the aforementioned reaction, compound (2) and compound (3) may be reacted in the presence of a suitable condensing agent.

The amount of compound (3) to be used is about 0.8 to about 10.0 mol, preferably about 0.8 to about 2.0 mol, relative to 1 mol of compound (2).

As the "condensing agent", for example, N,N'-carbodiimides such as N,N'-dicyclohexylcarbodiimide, 1-ethyl-3-(3-dimethylaminopropyl)carbodiimide (EDC) hydrochloride and the like, azolites such as N,N'-carbonylimidazole and the like, dehydrating agents such as N-ethoxycarbonyl-2-ethoxy-1,2-dihydroquinoline, diethyl cyanophosphate, phosphorus oxychloride, acetic anhydride and the like, 2-halogenopyridinium salt such as 2-chloromethylpyridinium iodide, 2-fluoro-1-chloromethylpyridinium iodide and the like, and the like are used.

The amount of the condensing agent to be used is about 0.8 to about 5.0 mol, preferably about 1.0 to about 2.0 mol, relative to 1 mol of compound (2).

Where desired, the reaction may be carried out in the co-presence of a base together with the condensing agent. As the "base", for example, basic salts such as potassium acetate, sodium acetate and the like, tertiary amines such as triethylamine, tripropylamine, tributylamine, cyclohexyldimethylamine, 4-dimethylaminopyridine, N-methylpiperidine, N-methylpyrrolidine, N-methylmorpholine and the like, 1-hydroxy-1H-benzotriazole (HOBt)·monohydrate and the like can be used.

The amount of the base to be used is about 0.5 to about 5.0 mol, preferably about 1.0 to about 3.0 mol, relative to 1 mol of compound (2).

This reaction is advantageously carried out using a solvent inert to the reaction. As such solvent, for example, solvents including alcohols such as methanol, ethanol, propanol and the like, hydrocarbons such as hexane, cyclohexane, benzene, toluene, xylene and the like, ethers such as diethyl ether, diisopropyl ether, tetrahydrofuran, dioxane, 1,2-dimethoxyethane and the like, amides such as N,N-dimethylformamide, N,N-dimethylacetamide, hexamethylphosphoric triamide and the like, sulfoxides such as dimethylsulfoxide and the like, halogenated hydrocarbons such as dichloromethane, chloroform, carbon tetrachloride, 1,2-dichloroethane and the like, nitriles such as acetonitrile, propionitrile and the like, acid anhydrides such as acetic anhydride and the like, and the like and a mixed solvent thereof and the like are preferable.

The reaction time is generally about 10 min to about 72 hr, preferably about 30 min to about 24 hr. The reaction temperature is generally about -20°C to about 150°C, preferably about 0°C to about 100°C.

The resultant product can be isolated from the reaction mixture according to a conventional method, and can be easily purified by a general separation means (e.g., recrystallization, distillation, chromatography etc.).

Compound (Ia) encompassed in compound (I) can also be produced by the method described in the following reaction scheme 2.

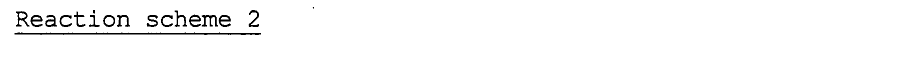

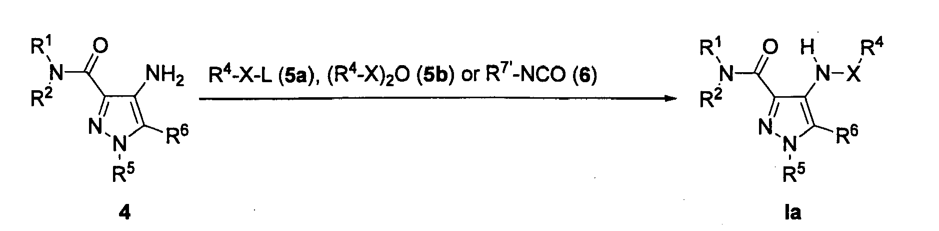

In the reaction scheme 2, L is a leaving group, R^{7'} is a group after removing >NH group from the optionally substituted amino group for R⁴ (i.e., substituent of the "optionally substituted amino group" for R⁴), and other symbols are as defined above.

Compound (Ia) can be produced by reacting compound (4) with compound (5a), compound (5b) or compound (6) in, where desired, the presence of a base or acid.

Commercially available products of compound (5a), compound (5b) and compound (6) can be easily obtained, and these compounds can also be produced by a method known per se or a method according thereto.

The amount of compound (5a), compound (5b) or compound (6) to be used is about 0.8 to 10 mol, preferably about 0.8 to 2.0 mol, more preferably about 0.9 to 1.2 mol, relative to 1 mol of compound (4).

As the "base", for example, basic salts such as sodium carbonate, potassium carbonate, cesium carbonate, sodium hydrogencarbonate and the like, aromatic amines such as pyridine, lutidine and the like, tertiary amines such as triethylamine, tripropylamine, tributylamine, cyclohexyldimethylamine, 4-dimethylaminopyridine, N-methylpiperidine, N-methylpyrrolidine, N-methylmorpholine and the like, alkali metal hydrides such as sodium hydride, potassium hydride and the like, metal amides such as sodium amide, lithium diisopropylamide, lithium hexamethyldisilazide and the like, metal alkoxides such as sodium methoxide, sodium ethoxide, potassium tert-butoxide and the like and the like can be mentioned.

As the "acid", for example, methanesulfonic acid, p-toluenesulfonic acid, camphorsulfonic acid and the like can be used.

The amount of the "base" to be use is about 0.1 to 10 equivalent amount, preferably 0.8 to 2 equivalent amount, relative to compound (4).

The amount of the "acid" to be use is about 0.1 to 10 equivalent amount, preferably 0.8 to 3 equivalent amount, relative to compound (4).

This reaction is advantageously carried out without solvent or using a solvent inert to the reaction. Such solvent is not particularly limited as long as the reaction proceeds and, for example, solvents including ethers such as diethyl ether, tetrahydrofuran, dioxane, 1,2-dimethoxyethane and the like, hydrocarbons such as benzene, toluene, cyclohexane, hexane and the like, amides such as N,N-dimethylformamide, N,N-dimethylacetamide and the like, halogenated hydrocarbons such as dichloromethane, chloroform, carbon tetrachloride, 1,2-dichloroethane and the like, nitriles such as acetonitrile, propionitrile and the like, sulfoxides such as dimethyl sulfoxide and the like, nitrogen-containing aromatic hydrocarbons such as pyridine, lutidine, quinoline and the like, and the like, and a mixed solvent thereof and the like are preferable. The reaction temperature is about -40°C to 150°C, preferably 0°C to 100°C. The reaction time is generally 5 min to 24 hr, preferably 10 min to 5 hr.

The resultant product (I) obtained as mentioned above can be used for the next reaction in the form of a reaction mixture or a crude product. It can also be isolated from the reaction mixture according to a conventional method, and easily purified by a separation means such as recrystallization, distillation, chromatography and the like.

As an alternative method replacing the aforementioned reaction, compound (4) and compound (5a) may be reacted in the presence of a suitable condensing agent.

The amount of compound (5a) to be used is about 0.8 to about 10.0 mol, preferably about 0.8 to about 2.0 mol, relative to 1 mol of compound (4).

As the "condensing agent", for example, N,N'-carbodiimides such as N,N'-dicyclohexylcarbodiimide, 1-ethyl-3-(3-dimethylaminopropyl)carbodiimide (EDC) hydrochloride and the like, azolites such as N,N'-carbonylimidazole and the like, dehydrating agents such as N-ethoxycarbonyl-2-ethoxy-1,2-dihydroquinoline, diethyl cyanophosphate, phosphorus oxychloride, acetic anhydride and the like, 2-halogenopyridinium salt such as 2-chloromethylpyridinium iodide, 2-fluoro-1-chloromethylpyridinium iodide and the like, and the like are used.

The amount of the condensing agent to be used is about 0.8 to about 5.0 mol, preferably about 1.0 to about 3.0 mol, relative to 1 mol of compound (4).

Where desired, the reaction may be carried out in the co-presence of a base together with the condensing agent. As the "base", for example, basic salts such as potassium acetate, sodium acetate and the like, tertiary amines such as triethylamine, tripropylamine, tributylamine, cyclohexyldimethylamine, 4-dimethylaminopyridine, N-methylpiperidine, N-methylpyrrolidine, N-methylmorpholine and the like, 1-hydroxy-1H-benzotriazole (HOBt)·monohydrate and the like can be used. The amount of the base to be used is about 0.5 to about 5.0 mol, preferably about 2.0 to about 3.0 mol, relative to 1 mol of compound (4).

This reaction is advantageously carried out using a solvent inert to the reaction. As such solvent, for example, solvents including alcohols such as methanol, ethanol, propanol and the like, hydrocarbons such as hexane, cyclohexane, benzene, toluene, xylene and the like, ethers such as diethyl ether, diisopropyl ether, tetrahydrofuran, dioxane, 1,2-dimethoxyethane and the like, amides such as N,N-dimethylformamide, N,N-dimethylacetamide, hexamethylphosphoric triamide and the like, sulfoxides such as dimethylsulfoxide and the like, halogenated hydrocarbons such as dichloromethane, chloroform, carbon tetrachloride, 1,2-dichloroethane and the like, nitriles such as acetonitrile, propionitrile and the like, acid anhydrides such as acetic anhydride and the like, and the like and a mixed solvent thereof and the like are preferable.

The reaction time is generally about 10 min to about 48 hr, preferably about 30 min to about 24 hr. The reaction temperature is generally about -20°C to about 150°C, preferably about 0°C to about 100°C.

The resultant product can be isolated from the reaction mixture according to a conventional method, and can be easily purified by a general separation means (e.g., recrystallization, distillation, chromatography etc.).

When R³ is an optionally substituted alkyl group, compound (I) can be produced from compound (Ia) by the method described in the following reaction scheme 3.

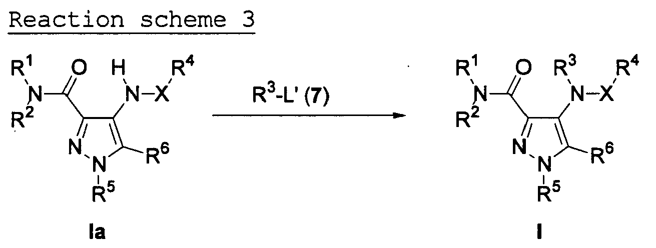

In the reaction scheme 3, L' is a leaving group, and other symbols are as defined above.

As the "leaving group" for L', for example, halogen atom (e.g., fluorine, chlorine, bromine, iodine and the like), optionally halogenated C₁₋₅ alkylsulfonyloxy (e.g., methanesulfonyloxy, ethanesulfonyloxy, trichloromethanesulfonyloxy etc.), optionally substituted C₆₋₁₀ arylsulfonyloxy, optionally substituted phenyl group, optionally substituted 2-thiobenzothiazole and the like can be used.

Compound (I) can be produced by reacting compound (Ia) with compound (7) in, where desired, the presence of a base.

The amount of compound (7) to be used is about 1.0 to about 10.0 mol, preferably about 1.0 to about 2.0 mol, relative to 1 mol of compound (Ia).

As the "base", for example, basic salts such as sodium carbonate, potassium carbonate, cesium carbonate, sodium hydrogencarbonate and the like, aromatic amines such as pyridine, lutidine and the like, tertiary amines such as triethylamine, tripropylamine, tributylamine, cyclohexyldimethylamine, 4-dimethylaminopyridine, N,N-dimethylaniline, N-methylpiperidine, N-methylpyrrolidine, N-methylmorpholine and the like, alkali metal hydrides such as sodium hydride, potassium hydride and the like, metal amides such as sodium amide, lithium diisopropylamide, lithium hexamethyldisilazide and the like, metal alkoxides such as sodium methoxide, sodium ethoxide, potassium tert-butoxide and the like, and the like can be used.

The amount of the base to be used is about 1.0 to about 10.0 mol, preferably about 1.0 to about 2.0 mol, relative to 1 mol of compound (Ia).

This reaction is advantageously carried out using a solvent inert to the reaction. Such solvent is not particularly limited as long as the reaction proceeds and, for example, solvent including alcohols such as methanol, ethanol, propanol and the like, ethers such as diethyl ether, tetrahydrofuran, dioxane, 1,2-dimethoxyethane and the like, hydrocarbons such as benzene, toluene, cyclohexane, hexane and the like, amides such as N,N-dimethylformamide, N,N-dimethylacetamide and the like, halogenated hydrocarbons such as dichloromethane, chloroform, carbon tetrachloride, 1,2-dichloroethane and the like, nitriles such as acetonitrile, propionitrile and the like, sulfoxides such as dimethylsulfoxide and the like, and the like and a mixed solvent thereof and the like are preferable.

The reaction time is generally about 30 min to about 48 hr, preferably about 1 hr to about 24 hr. The reaction temperature is generally about -20°C to about 200°C, preferably about 0°C to about 150°C.

The resultant product can be isolated from the reaction mixture according to a conventional method, and can be easily purified by a general separation means (e.g., recrystallization, distillation, chromatography etc.).

In addition, compound (I) can also be produced from compound (Ib) encompassed in compound (I), by the method described in the following reaction scheme 4.

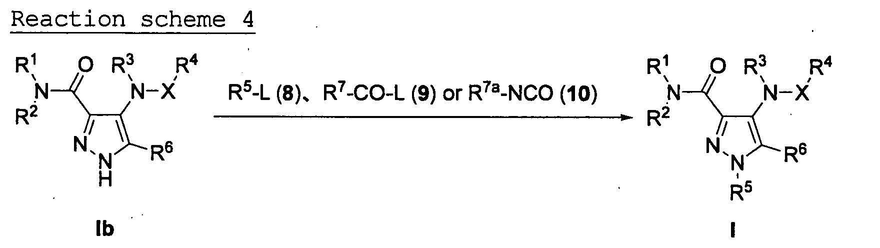

In the reaction scheme 4, the symbols are as defined above.

When R⁵ is an optionally substituted alkyl group, compound (I) can be produced by reacting compound (Ib) with compound (8) in, where desired, the presence of a base.

The amount of compound (8) to be used is about 1.0 to about 10.0 mol, preferably about 1.0 to about 2.0 mol, relative to 1 mol of compound (Ib).

As the "base", for example, basic salts such as sodium carbonate, potassium carbonate, cesium carbonate, sodium hydrogencarbonate and the like, aromatic amines such as pyridine, lutidine and the like, tertiary amines such as triethylamine, tripropylamine, tributylamine, cyclohexyldimethylamine, 4-dimethylaminopyridine, N,N-dimethylaniline, N-methylpiperidine, N-methylpyrrolidine, N-methylmorpholine and the like, alkali metal hydrides such as sodium hydride, potassium hydride and the like, metal amides such as sodium amide, lithium diisopropylamide, lithium hexamethyldisilazide and the like, metal alkoxides such as sodium methoxide, sodium ethoxide, potassium tert-butoxide and the like and the like can be used.

The amount of the base to be used is about 1.0 to about 10.0 mol, preferably about 1.0 to about 2.0 mol, relative to 1 mol of compound (Ib).

This reaction is advantageously carried out using a solvent inert to the reaction. Such solvent is not particularly limited as long as the reaction proceeds and, for example, solvents including alcohols such as methanol, ethanol, propanol and the like, ethers such as diethyl ether, tetrahydrofuran, dioxane, 1,2-dimethoxyethane and the like, hydrocarbons such as benzene, toluene, cyclohexane, hexane and the like, amides such as N,N-dimethylformamide, N,N-dimethylacetamide and the like, halogenated hydrocarbons such as dichloromethane, chloroform, carbon tetrachloride, 1,2-dichloroethane and the like, nitriles such as acetonitrile, propionitrile and the like, sulfoxides such as dimethylsulfoxide and the like, and the like and a mixed solvent thereof and the like are preferable.

The reaction time is generally about 30 min to about 48 hr, preferably about 1 hr to about 24 hr. The reaction temperature is generally about -20°C to about 200°C, preferably about 0°C to about 150°C.

The resultant product can be used for the next reaction in the form of a reaction mixture or a crude product. It can also be isolated from the reaction mixture according to a conventional method, and can be easily purified by a general separation means (e.g., recrystallization, distillation, chromatography etc.).

When R⁵ is an optionally substituted acyl group, compound (I) can be produced by reacting compound (Ib) with compound (9) or compound (10) in, where desired, the presence of a base or acid.

The amount of compound (9) or compound (10) to be used is about 1.0 to 5 mol, preferably about 1.0 to 2.0 mol, relative to 1 mol of compound (Ib).

As the "base", for example, basic salts such as sodium carbonate, potassium carbonate, cesium carbonate, sodium hydrogencarbonate and the like, aromatic amines such as pyridine, lutidine and the like, tertiary amines such as triethylamine, tripropylamine, tributylamine, cyclohexyldimethylamine, 4-dimethylaminopyridine, N-methylpiperidine, N-methylpyrrolidine, N-methylmorpholine and the like, alkali metals such as metal sodium and the like, alkali metal hydrides such as sodium hydride, potassium hydride and the like, metal amides such as sodium amide, lithium diisopropylamide, lithium hexamethyldisilazide and the like, metal alkoxides such as sodium methoxide, sodium ethoxide, potassium tert-butoxide and the like, and the like can be used.

As the "acid", for example, methanesulfonic acid, p-toluenesulfonic acid, camphorsulfonic acid and the like can be used.

The amount of the "base" to be use is about 0.1 to 10 equivalent amount, preferably 0.8 to 2 equivalent amount, relative to compound (Ib).

The amount of the "acid" to be use is about 0.1 to 10 equivalent amount, preferably 0.8 to 3 equivalent amount, relative to compound (Ib).

This reaction is advantageously carried out without solvent or using a solvent inert to the reaction. Such solvent is not particularly limited as long as the reaction proceeds and, for example, solvents including ethers such as diethyl ether, tetrahydrofuran, dioxane, 1,2-dimethoxyethane and the like, hydrocarbons such as benzene, toluene, cyclohexane, hexane and the like, amides such as N,N-dimethylformamide, N,N-dimethylacetamide and the like, halogenated hydrocarbons such as dichloromethane, chloroform, carbon tetrachloride, 1,2-dichloroethane and the like, nitriles such as acetonitrile, propionitrile and the like, sulfoxides such as dimethyl sulfoxide and the like, nitrogen-containing aromatic hydrocarbons such as pyridine, lutidine, quinoline and the like, and the like and a mixed solvent thereof and the like are preferable. The reaction temperature is about -40 to 150°C, preferably 0 to 100°C. The reaction time is generally 5 min to 24 hr, preferably 10 min to 5 hr.

The resultant product (I) obtained as mentioned above can be used for the next reaction in the form of a reaction mixture or a crude product. It can also be isolated from the reaction mixture according to a conventional method, and easily purified by a separation means such as recrystallization, distillation, chromatography and the like.

As an alternative method replacing the aforementioned reaction, compound (Ib) and compound (9) may be reacted in the presence of a suitable condensing agent.

The amount of compound (9) to be used is about 0.8 to about 10.0 mol, preferably about 0.8 to about 2.0 mol, relative to 1 mol of compound (Ib).

As the "condensing agent", for example, N,N'-dicarboimides such as N,N'-dicyclohexylcarbodiimide, 1-ethyl-3-(3-dimethylaminopropyl)carbodiimide (EDC) hydrochloride and the like, azolites such as N,N'-carbonylimidazole and the like, dehydrating agents such as N-ethoxycarbonyl-2-ethoxy-1,2-dihydroquinoline, diethyl cyanophosphate, phosphorus oxychloride, acetic anhydride and the like, 2-halogenopyridinium salt such as 2-chloromethylpyridinium iodide, 2-fluoro-1-chloromethylpyridinium iodide and the like, and the like can be used.

The amount of the condensing agent to be used is about 0.8 to about 5.0 mol, preferably about 1.0 to about 2.0 mol, relative to 1 mol of compound (Ib).

Where desired, the reaction may be carried out in the co-presence of a base together with the condensing agent. As the "base", for example, basic salts such as potassium acetate, sodium acetate and the like, tertiary amines such as triethylamine, tripropylamine, tributylamine, cyclohexyldimethylamine, 4-dimethylaminopyridine, N-methylpiperidine, N-methylpyrrolidine, N-methylmorpholine and the like, 1-hydroxy-1H-benzotriazole (HOBt)·monohydrate and the like can be used.

The amount of the base to be used is about 0.5- to about 5.0 mol, preferably about 1.0 to about 3.0 mol, relative to 1 mol of compound (Ib).

This reaction is advantageously carried out using a solvent inert to the reaction. As such solvent, for example, solvents including alcohols such as methanol, ethanol, propanol and the like, hydrocarbons such as hexane, cyclohexane, benzene, toluene, xylene and the like, ethers such as diethyl ether, diisopropyl ether, tetrahydrofuran, dioxane, 1,2-dimethoxyethane and the like, amides such as N,N-dimethylformamide, N,N-dimethylacetamide, hexamethylphosphoric triamide and the like, sulfoxides such as dimethylsulfoxide and the like, halogenated hydrocarbons such as dichloromethane, chloroform, carbon tetrachloride, 1,2-dichloroethane and the like, nitriles such as acetonitrile, propionitrile and the like, acid anhydrides such as acetic anhydride and the like, and the like and a mixed solvent thereof and the like are preferable.

The reaction time is generally about 10 min to about 72 hr, preferably about 30 min to about 24 hr. The reaction temperature is generally about -20°C to about 150°C, preferably about 0°C to about 100°C.

The resultant product can be used for the next reaction in the form of a reaction mixture or a crude product. It can also be isolated from the reaction mixture according to a conventional method, and can be easily purified by a general separation means (e.g., recrystallization, distillation, chromatography etc.).

The above-mentioned compound (4) can be produced by a method known per se, for example, the method described in J. Med. Chem., 35, 917-912 (1992) or the method described in reaction scheme 5.

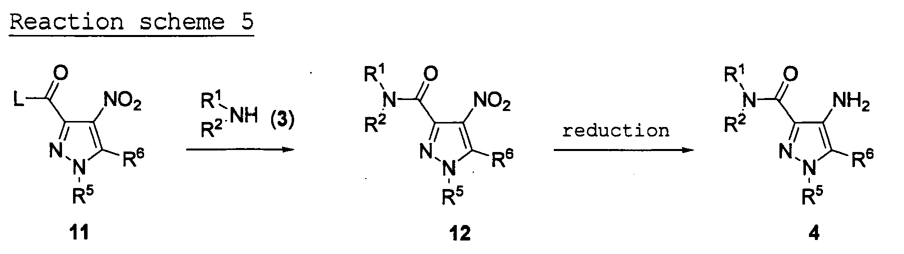

Compound (11) may be a commercially available product, or can be produced by a method known per se, for example, the method described in Chemistry of Heterocyclic Compounds, 19, 1326-1330 (1983) or Russian Chemical Bulletin (English Translation), 42, 1861-1864 (1993) or a method according thereto. Compound (12) can be produced by reacting compound (11) with compound (3) by the method described in reaction scheme 1, from which compound (4) can be produced by a reduction reaction.

According to the catalytic reduction method, compound (4) can be produced by reacting compound (12) with a metal catalyst under a hydrogen atmosphere. Where desired, a suitable acid catalyst may be added.

As the "metal catalyst", Raney-nickel, platinum oxide, metal palladium, palladium-activated carbon and the like are used. The amount of the "metal catalyst" to be used is generally about 0.1 to about 1000 wt%, preferably about 1 to about 20 wt%, relative to compound (12).

As the "acid catalyst", organic acids such as formic acid, acetic acid, trifluoroacetic acid, p-toluenesulfonic acid and the like, mineral acids such as sulfuric acid, hydrochloric acid, hydrobromic acid and the like, and the like can be used. The amount of the "acid catalyst" to be used is about 0.01 to excess amount, relative to 1 mol of compound (12).

This reaction is advantageously carried out using a solvent inert to the reaction. Such solvent is not particularly limited as long as the reaction proceeds and, for example, solvents including alcohols such as methanol, ethanol, propanol and the like; ethers such as diethyl ether, tetrahydrofuran, dioxane, 1,2-dimethoxyethane and the like; hydrocarbons such as benzene, toluene, cyclohexane, hexane and the like; amides such as N,N-dimethylformamide, N,N-dimethylacetamide and the like; organic acids such as acetic acid and the like; water etc., and the like or a mixed solvent thereof and the like are preferable. The hydrogen pressure is generally about 1 to about 100 atm, preferably about 1 to about 5 atm. The reaction time is generally about 30 min to about 48 hr, preferably about 1 to 24 hr. The reaction temperature is generally about 0°C to about 120°C, preferably about 20°C to about 80°C.

After removing the catalyst, the resultant product can also be isolated from the reaction mixture according to a conventional method, and can be easily purified by a general separation means (e.g., recrystallization, distillation, chromatography etc.).

According to the method using an organic silyl reagent (alkylsilane reagent), compound (4) can be produced by reacting compound (12) with an alkylsilane reagent and an acid.

As the alkylsilane reagent, for example, triethylsilane, phenyldimethylsilane and the like can be used. The amount of the "alkylsilane reagent" to be used is about 0.8 to about 20 mol, preferably about 1 to about 5 mol, relative to 1 mol of compound (12).

As the acid, for example, organic acid such as trifluoroacetic acid and the like can be used. The amount of the acid is about 0.1 to excess amount, relative to 1 mol of compound (12).

This reaction is advantageously carried out without solvent or using a solvent inert to the reaction. Such solvent is not particularly limited as long as the reaction proceeds and, for example, solvents including ethers such as diethyl ether, tetrahydrofuran, dioxane, 1,2-dimethoxyethane and the like, hydrocarbons such as benzene, toluene, cyclohexane, hexane and the like; halogenated hydrocarbons such as dichloromethane, chloroform, carbon tetrachloride, 1,2-dichloroethane and the like; organic acids such as acetic acid, trifluoroacetic acid and the like, and the like, or a mixed solvent thereof and the like are preferable.

The above-mentioned compound (2) can be produced by a method known per se, for example, a method according to the description of Indian J. Chem. Sect. B, 28, 782-785 (1989) or Bull. Chem. Soc. Jpn., 52, 208-211 (1979) or the method described in reaction scheme 6.

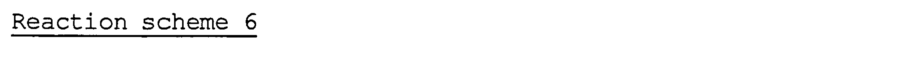

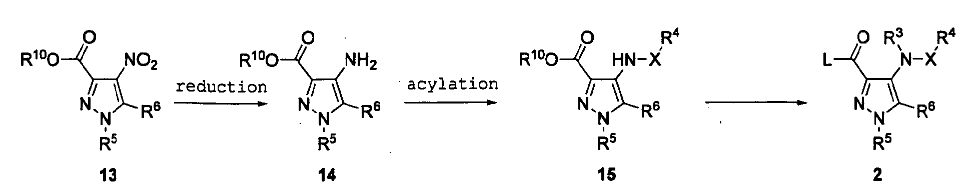

In the reaction scheme 6, R¹⁰ is an optionally substituted alkyl group, and other symbols are as defined above.

Compound (13) may be a commercially available product, or can be produced by a method known per se, for example, a method according to the description of Indian J. Chem. Sect. B, 28, 56-60 (1989) or Tetrahedron, 3, 209-224 (1958).

Compound (14) can be produced by reducing compound (13) by the method described in reaction scheme 5.

Compound (15) can be produced by acylating compound (14) by the methods described in reaction schemes 2 and 3.

Compound (2) can be produced by converting the ester group of compound (15) to a carboxyl group under basic or acidic conditions or by a method generally used. Where necessary, for example, compound (2) can be produced by converting to active ester, acid anhydride, acid halide and the like by a method according to the description of Shin Jikken Kagaku Koza (New Experimental Chemistry Course), vol. 14 edited by the Chemical Society of Japan and the like.

The starting compound of the aforementioned compound (I) may form a salt and is not particularly limited as long as the reaction can be conducted. For example, a salt similar to the salt formed by the aforementioned compound (I) and the like, and the like can be used.

When isomerization occurs, the configuration isomers (E, Z forms) of compound (I) can be isolated or purified by, for example, conventional separation means such as extraction, recrystallization, distillation, chromatography and the like to give a pure compound. It is also possible to progress isomerization of a double bond by heating, acid catalyst, transition metal complex, metal catalyst, radical species catalyst, photoirradiation or strong base catalyst and the like according to the method described in Shin Jikken Kagaku Koza (New Experimental Chemistry Course) 14 (edited by the Chemical Society of Japan), pages 251-253, Shin Jikken Kagaku Koza (New Experimental Chemistry Course) 19, 4th Ed. (edited by the Chemical Society of Japan), pages 273-274 and a method according thereto to give the corresponding pure isomer.

While a stereoisomer occurs depending on the kind of the substituent of compound (I), both the isomers alone and mixtures of each isomers are encompassed in the present invention.

Compound (I) may be a hydrate or a non-hydrate.

In any case, where desired, compound (I) can be synthesized by further using deprotection reaction, acylation reaction, alkylation reaction, hydrogenation reaction, oxidation reaction, reduction reaction, carbon chain extension reaction and substituent exchange reaction alone or in a combination of two or more thereof.

When the object product is obtained in a free form by the above-mentioned reactions, the product may be converted to a salt by a conventional method, and when it is obtained as a salt, the product may be converted to a free form or a different salt by a conventional, method. The compound (I) thus obtained can be isolated and purified from a reaction mixture by a known means, such as, phase transfer, concentration, solvent extraction, fractionation, crystallization, recrystallization, chromatography and the like.

When compound (I) is present as a configurational isomer, diastereomer, conformer or the like, each can be isolated by the above separation and purification methods on demand. In addition, when compound (I) is in the form of racemates, they can be separated into d- and 1-forms by any conventional optical resolution.

When, in each of the aforementioned reactions, a functional group such as amino group, hydroxy group, carboxy group and the like is present, the compound may be subjected to the reaction after introducing a protecting group generally used in peptide chemistry etc. and the object compound can be obtained by removing the protecting group as necessary after the reaction.

As the protecting group, for example, formyl or C₁₋₆ alkyl-carbonyl (e.g., acetyl, propionyl etc.), phenylcarbonyl, C₁₋₆ alkoxy-carbonyl (e.g., methoxycarbonyl, ethoxycarbonyl etc.), phenyloxycarbonyl, C₇₋₁₀ aralkyloxy-carbonyl (e.g., benzyloxycarbonyl etc.), trityl, phthaloyl and the like, each of which is optionally substituted, can be used. As these substituents, halogen atom (e.g., fluorine, chlorine, bromine, iodine etc.), C₁₋₆ alkyl-carbonyl (e.g., acetyl, propionyl, valeryl etc.), nitro and the like are used. The number of the substituents is, for example, about 1 to 3.

As a method for removing a protecting group, a method known *per se* or a thereof analogous thereto is used. For example, a method of treating with acid, base, ultraviolet rays, hydrazine, phenylhydrazine, sodium N-methyldithiocarbamate, tetrabutylammonium fluoride, palladium acetate and the like, or reduction reaction is used.

Compound A obtained in this way, other reaction intermediate and a starting compound thereof can be isolated and purified from a reaction mixture by a method known per se, for example, extraction, concentration, neutralization, filtration, distillation, recrystallization, column chromatography, thin layer chromatography, preparative high performance liquid chromatography (preparative HPLC), moderate-pressure preparative liquid chromatography (moderate-pressure preparative LC) and the like.

A salt of compound A can be produced by a method known per se. For example, when compound A is a basic compound, it can be produced by adding an inorganic acid or organic acid, or when compound A is an acidic compound, by adding an organic base or inorganic base.

When compound A has an optical isomer, both the individual optical isomers alone and mixtures thereof are naturally encompassed in the present invention. Where desired, these isomers can be optically resolved by a method known per se, or individually produced.

When compound A is present as a configurational isomer, diastereomer, conformer or the like, each can be isolated by the above separation and purification methods on demand. In addition, when compound A is in the form of racemates, they can be separated into S- and R-forms by any conventional optical resolution method.

When compound A includes stereoisomers, both the isomers alone and mixtures of each isomer are encompassed in the present invention.

Compound A may be a hydrate, and both hydrate and non-hydrate are encompassed in the scope of the present invention. Compound A may be labeled with an isotope (e.g., ³H, ¹⁴C, ³⁵ S, ¹²⁵I and the like) or the like.

Since the GSK-3 inhibitor of the present invention selectively inhibits GSK-3 and shows low toxicity and a fewer side effects, it is useful as a safe pharmaceutical product. The GSK-3 inhibitor of the present invention shows a superior GSK-3 selective inhibitory action for a mammal (e.g., mouse, rat, hamster, rabbit, cat, dog, bovine, sheep, monkey, human etc.) and is superior in (oral) absorbability, (metabolic) stability and the like. Therefore, it can be used as an agent for the prophylaxis or treatment of GSK-3 related pathology or disease, for example, metabolic diseases (e.g., diabetes (type 1 diabetes, type 2 diabetes, gestational diabetes etc.), impaired glucose tolerance, obesity, diabetic neuropathy, diabetic retinopathy, diabetic nephropathy, lipid metabolism abnormalities (hypertriglyceridemia, hypercholesterolemia, hypoHDL-emia, postprandial hyperlipemia etc.) and the like), circulatory diseases (e.g., hypertension, cardiac hypertrophy, angina pectoris, arteriosclerosis and the like), inflammatory diseases (e.g., allergy, asthma, rheumatism and the like), cirrhosis, alcoholic hepatitis and osteoporosis or an agent for preventing the progress from impaired glucose tolerance to diabetes.

In the area of nervous diseases, the GSK-3 inhibitor has a neural stem cell differentiation-stimulated action. Accordingly, the GSK-3 inhibitor can be used as an agent for the prophylaxis or treatment of neurodegenerative diseases such as Alzheimer's disease, mild cognitive impairment (MCI), Huntington's chorea, Parkinson's syndrome, epilepsy, amyotrophic lateral sclerosis (ALS), multiple sclerosis, cerebellum spinal cord denaturation, Pick disease, peripheral nerve disorders and the like and mental diseases such as schizophrenia, depression, anxiety, manic depression, PTSD (posttraumatic stress disorder; hereinafter sometimes to be abbreviated to PTSD) and the like. Based on cell protection action and/or function regeneration action, it can be used as an agent for the prophylaxis or treatment of ischemic diseases such as cerebral infarction, myocardial infarction and the like. Particularly preferred is an agent for the prophylaxis or treatment of diabetes or neurodegenerative disease.

For diagnostic criteria of diabetes, Japan Diabetes Society reported new diagnostic criteria in 1999.

According to this report, diabetes is a condition showing any of a fasting blood glucose level (glucose concentration of intravenous plasma) of not less than 126 mg/dl, a 75 g oral glucose tolerance test (75 g OGTT) 2 h level (glucose concentration of intravenous plasma) of not less than 200 mg/dl, and a non-fasting blood glucose level (glucose concentration of intravenous plasma) of not less than 200 mg/dl. A condition not falling under the above-mentioned diabetes and different from "a condition showing a fasting blood glucose level (glucose concentration of intravenous plasma) of less than 110 mg/dl or a 75 g oral glucose tolerance test (75 g OGTT) 2 h level (glucose concentration of intravenous plasma) of less than 140 mg/dl" (normal type) is called a "borderline type".

In addition, ADA (American Diabetes Association) reported new diagnostic criteria of diabetes in 1997 and WHO in 1998.

According to these reports, diabetes is a condition showing a fasting blood glucose level (glucose concentration of intravenous plasma) of not less than 126 mg/dl and a 75 g oral glucose tolerance test 2 h level (glucose concentration of intravenous plasma) of not less than 200 mg/dl.

According to the above-mentioned reports, impaired glucose tolerance is a condition showing a fasting blood glucose level (glucose concentration of intravenous plasma) of less than 126 mg/dl and a 75 g oral glucose tolerance test 2 h level (glucose concentration of intravenous plasma) of not less than 140 mg/dl and less than 200 mg/dl. According to the report of ADA, a condition showing a fasting blood glucose level (glucose concentration of intravenous plasma) of not less than 110 mg/dl and less than 126 mg/dl is called IFG (Impaired Fasting Glucose). According to the report of WHO, among the IFG (Impaired Fasting Glucose), a condition showing a 75 g oral glucose tolerance test 2 h level (glucose concentration of intravenous plasma) of less than 140 mg/dl is called IFG (Impaired Fasting Glycemia).

Compound A of the present invention can be also used as an agent for the prophylaxis or treatment of diabetes, borderline type, impaired glucose tolerance, IFG (Impaired Fasting Glucose) and IFG (Impaired Fasting Glycemia), as determined according to the above-mentioned new diagnostic criteria. Moreover, the compound of the present invention can prevent progress of borderline type, impaired glucose tolerance, IFG (Impaired Fasting Glucose) or IFG (Impaired Fasting Glycemia) into diabetes.

When the compound A of the present invention is applied to each of the above-mentioned diseases, it can be used in an appropriate combination with a pharmaceutical agent or a treatment method generally employed for the disease. For example, acetylcholine esterase inhibitors (e.g., donepezil, rivastigmine, galanthamine, zanapezil (TAK-147) etc.), antidementia agents (memantine etc.), inhibitors of β amyloid protein production, secretion, accumulation, coagulation and/or deposition, β secretase inhibitors (e.g., 6-(4-biphenylyl)methoxy-2-[2-(N,N-dimethylamino)ethyl]tetralin, 6-(4-biphenylyl)methoxy-2-(N,N-dimethylamino)methyltetralin, 6-(4-biphenylyl)methoxy-2-(N,N-dipropylamino)methyltetralin, 2-(N,N-dimethylamino)methyl-6-(4'-methoxybiphenyl-4-yl)methoxytetralin, 6-(4-biphenylyl)methoxy-2-[2-(N,N-diethylamino)ethyl]tetralin, 2-[2-(N,N-dimethylamino)ethyl]-6-(4'-methylbiphenyl-4-yl)methoxytetralin, 2-[2-(N,N-dimethylamino)ethyl]-6-(4'-methoxybiphenyl-4-yl)methoxytetralin, 6-(2',4'-dimethoxybiphenyl-4-yl)methoxy-2-[2-(N,N-dimethylamino)ethyl]tetralin, 6-[4-(1,3-benzodioxol-5-yl)phenyl]methoxy-2-[2-(N,N-dimethylamino)ethyl]tetralin, 6-(3',4'-dimethoxybiphenyl-4-yl)methoxy-2-[2-(N,N-dimethylamino)ethyl]tetralin, an optically active form thereof, a salt thereof and a hydrate thereof, OM99-2 (WO01/00663)), γ secretase inhibitory agent, β amyloid protein coagulation inhibitory agent (e.g., PTI-00703, ALZHEMED (NC-531), PPI-368 (JP-A-11-514333), PPI-558 (JP-A-2001-500852), SKF-74652 (Biochem. J. (1999), 340(1), 283-289)), β amyloid vaccine, β amyloid degrading enzyme and the like, cerebral function activators (e.g., aniracetam, nicergoline etc.), other therapeutic drug for Parkinson's disease [(e.g., dopamine receptor agonists (L-DOPA, bromocriptine, pergolide, talipexole, pramipexole, Cabergoline, adamantadine etc.), monoamine oxidase (MAO) inhibitors (deprenyl, Selgiline (selegiline), remacemide, riluzole etc.), anticholinergic agents (e.g., trihexyphenidyl, biperiden etc.), COMT inhibitors (e.g., entacapone etc.)), therapeutic drug for amyotropic lateral sclerosis (e.g., riluzole etc., neurotrophic factor etc.), therapeutic drug for abnormal behavior, wandering and the like due to the progress of dementia (e.g., sedative drug, antianxiety drug etc.), apoptosis inhibitors (e.g., CPI-1189, IDN-6556, CEP-1347 etc.), neuronal differentiation or regeneration promoters (e.g., leteprinim, xaliproden (SR-57746-A), SB-216763, Y-128, VX-853, prosaptide, 5,6-dimethoxy-2-[2,2,4,6,7-pentamethyl-3-(4-methylphenyl)-2,3-dihydro-1-benzofuran-5-yl]isoindoline, 5,6-dimethoxy-2-[3-(4-isopropylphenyl)-2,2,4,6,7-pentamethyl-2,3-dihydro-1-benzofuran-5-yl]isoindoline, 6-[3-(4-isopropylphenyl)-2,2,4,6,7-pentamethyl-2,3-dihydro-1-benzofuran-5-yl]-6,7-dihydro-5H-[1,3]dioxolo[4,5-f]isoindole and optically active forms, salts and hydrates, etc. thereof), antidepressants (e.g., desipramine, amitriptyline, imipramine, tramadol etc.), anticonvulsants (e.g., lamotrigine etc.), antianxiety drugs (e.g., benzodiazepine etc.), non-steroidal anti-inflammatory drugs (e.g., meloxicam, tenoxicam, indomethacin, ibuprofen, celecoxib, rofecoxib, aspirin, indomethacin etc.), disease-modifying anti-rheumatic drugs (DMARDs), anti-cytokine drugs (TNF inhibitor, MAP kinase inhibitor and the like), steroidal drugs (e.g., dexamethasone, hexestrol, cortisone acetate etc.), therapeutic agents for incontinence or frequent urination (e.g., flavoxate hydrochloride, oxybutynin hydrochloride, propiverine hydrochloride etc.), phosphodiesterase inhibitors (e.g., sildenafil (citrate) etc.), dopamine agonists (e.g., apomorphine etc.), antiarrhythmics (e.g., mexiletine etc.), sex hormones or derivatives thereof (e.g., progesterone, estradiol, estradiol benzoate etc.), therapeutic agents for osteoporosis (e.g., alfacalcidol, calcitriol, elcatonin, calcitonin salmon, estriol, ipriflavone, disodium pamidronate, sodium alendronate hydrate, disodium incadronate etc.), parathyroid hormone (PTH), calcium receptor antagonists and the like can be mentioned. Particularly, a combined use with a β secretase inhibitory agent such as 6-(4-biphenylyl)methoxy-2-[2-(N,N-dimethylamino)ethyl]tetralin hydrochloride·monohydrate etc., and the like is preferable.

In addition, a combined use with a transplantation method of neural stem cell or neural precursor cell, or fetal neural tissue prepared from embryonic stem cell or nervous tissue, and a combined use with a pharmaceutical agent such as an immunosuppressant after the transplantation and the like can be mentioned.

Examples of therapeutic agent for diabetes include insulin preparations (e.g., animal insulin preparation extracted from the pancreas of bovine, swine; human insulin preparation genetically synthesized using Escherichia coli, yeast; zinc insulin; protamine zinc insulin; insulin fragment or derivatives (e.g., INS-1 etc.) and the like), insulin sensitizers [e.g., pioglitazone hydrochloride, troglitazone, rosiglitazone or maleate thereof, GI-262570, JTT-501, MCC-555, YM-440, KRP-297, CS-011, FK-614, compounds described in WO99/58510 (e.g., (E)-4-[4-(5-methyl-2-phenyl-4-oxazolylmethoxy)benzyloxyimino]-4-phenylbutyric acid), NN-622, AZ-242, BMS-298585, ONO-5816, LM-4156, BM-13-1258, MBX-102, GW-1536 etc.], α-glucosidase inhibitors (e.g., voglibose, acarbose, miglitol, emiglitate etc.), biguanides (e.g., phenformin, metformin, buformin etc.), insulin secretagogues [sulfonylurea (e.g., tolbutamide, glibenclamide, gliclazide, chlorpropamide, tolazamide, acetohexamide, glyclopyramide, glimepiride, glipizide, glybuzole etc.), repaglinide, nateglinide, mitiglinide or calcium salt hydrate thereof, GLP-1 etc.], dipeptidyl-peptidase IV inhibitors (e.g., NVP-DPP-278, PT-100, NVP-DPP-728, LAF237 etc.), β3 agonists (e.g., CL-316243, SR-58611-A, UL-TG-307, SB-226552, AJ-9677, BMS-196085, AZ-40140 etc.), amylin agonists (e.g., pramlintide etc.), phosphotyrosine phosphatase inhibitors (e.g., vanadic acid etc.), gluconeogenesis inhibitors (e.g., glycogen phosphorylase inhibitor, glucose-6-phosphatase inhibitors, glucagon antagonist etc.), SGLUT (sodium-glucose cotransporter) inhibitors (e.g., T-1095 etc.) and the like.

Examples of the therapeutic agents for diabetic complications include aldose reductase inhibitors (e.g., tolrestat, epalrestat, zenarestat, zopolrestat, minalrestat, fidarestat (SNK-860), CT-112 etc.), neurotrophic factors (e.g., NGF, NT-3, BDNF etc.), neurotrophic factor production-secretion promoters [e.g., neurotrophin production-secretion promoters described in WO01/14372 (e.g., 4-(4-chlorophenyl)-2-(2-methyl-1-imidazolyl)-5-[3-(2-methylphenoxy)propyl]oxazole and the like)], PKC inhibitors (e.g., LY-333531 etc.), AGE inhibitors (e.g., ALT946, pimagedine, pyratoxanthine, N-phenacylthiazolium bromide (ALT766), EXO-226 etc.), active oxygen scavengers (e.g., thioctic acid etc.), cerebral vasodilators (e.g., tiapuride, mexiletine etc.).

Examples of the therapeutic agents for hyperlipidemia include HMG-CoA reductase inhibitors (e.g., pravastatin, simvastatin, lovastatin, atorvastatin, fluvastatin, lipantil, cerivastatin, itavastatin, ZD-4522 or a salt thereof (e.g., sodium salt etc.) and the like), fibrate compounds (e.g., bezafibrate, beclobrate, binifibrate, ciprofibrate, clinofibrate, clofibrate, clofibric acid, etofibrate, fenofibrate, gemfibrozil, nicofibrate, pirifibrate, ronifibrate, simfibrate, theofibrate and the like), squalene synthase inhibitors (e.g., compounds described in WO97/10224, for example, N-[[(3R,5S)-1-(3-acetoxy-2,2-dimethylpropyl)-7-chloro-5-(2,3-dimethoxyphenyl)-2-oxo-1,2,3,5-tetrahydro-4,1-benzooxazepin-3-yl]acetyl]piperidine-4-acetic acid and the like), ACAT inhibitors (e.g., avasimibe, eflucimibe and the like), anion exchange resins (e.g., colestyramine and the like), probucol, nicotinic acid drugs (e.g., nicomol, niceritrol and the like), ethyl icosapentate, phytosterol (e.g., soysterol, γ oryzanol and the like) and the like.

Examples of the antihypertensive agent include angiotensin converting enzyme inhibitors (e.g., captopril, enalapril, delapril etc.), angiotensin II antagonist (e.g., candesartan cilexetil, losartan, eprosartan, valsartan, telmisartan, irbesartan, tasosartan etc.), calcium antagonists (e.g., manidipine, nifedipine, nicardipine, amlodipine, efonidipine etc.), potassium channel openers (e.g., levcromakalim, L-27152, AL 0671, NIP-121 and the like), clonidine and the like. Examples of the antiobesity agents include antiobesity agents acting on the central nervous system (e.g., dexfenfluramine, fenfluramine, phentermine, sibutramine, anfepramone, dexamphetamine, mazindol, phenylpropanolamine, clobenzorex etc.), pancreatic lipase inhibitors (e.g., orlistat etc.), β3 agonists (e.g., CL-316243, SR-58611-A, UL-TG-307, SB-226552, AJ-9677, BMS-196085, AZ-40140 etc.), anorectic peptides (e.g., leptin, CNTF (ciliary neurotrophic factor) etc.), cholecystokinin agonists (e.g., lintitript, FPL-15849 etc.) and the like.

Examples of the diuretics include xanthine derivatives (e.g., sodium salicylate and theobromine, calcium salicylate and theobromine etc.), thiazide preparations (e.g., ethiazide, cyclopenthiazide, trichloromethiazide, hydrochlorothiazide, hydroflumethiazide, benzylhydrochlorothiazide, penflutizide, polythiazide, methyclothiazide etc.), antialdosterone preparations (e.g., spironolactone, triamterene etc.); carbonate dehydratase inhibitors (e.g., acetazolamide and the like), chlorobenzenesulfonamide preparations (e.g., chlortalidone, mefruside, indapamide etc.), azosemide, isosorbide, etacrynic acid, piretanide, bumetanide, furosemide and the like.

Examples of the chemotherapeutic agents include alkylating agents (e.g., cyclophosphamide, ifosfamide etc.), metabolic antagonists (e.g., methotrexate, 5-fluorouracil or derivative thereof etc.), antitumor antibiotics (e.g., mitomycin, adriamycin etc.), plant-derived antitumor agents (e.g., vincristine, vindesine, Taxol etc.), cisplatin, carboplatin, etopoxide and the like. Of these, Furtulon and NeoFurtulon, which are 5-fluorouracil derivatives, and the like are preferable.

Examples of the immunotherapeutic agents include microorganism,or bacterial components (e.g., muramyl dipeptide derivative, Picibanil etc.), polysaccharides having immunity potentiating activity (e.g., lentinan, schizophyllan, krestin etc.), cytokines obtained by genetic engineering techniques (e.g., interferon, interleukin (IL) etc.), colony stimulating factors (e.g., granulocyte colony stimulating factor, erythropoietin etc.) and the like, with preference given to interleukins such as IL-1, IL-2, IL-12 and the like.

Examples of the antithrombotic agents include heparin (e.g., heparin sodium, heparin calcium, dalteparin sodium etc.), warfarin (e.g., warfarin potassium etc.), anti-thrombin drugs (e.g., aragatroban etc.), thrombolytic agents (e.g., urokinase, tisokinase, alteplase, nateplase, monteplase, pamiteplase etc.), platelet aggregation inhibitors (e.g., ticlopidine hydrochloride, cilostazol, ethyl icosapentate, beraprost sodium, sarpogrelate hydrochloride etc.) and the like.

Examples of the cachexia improvement pharmaceutical agent include cyclooxygenase inhibitors (e.g., indomethacin etc.) [Cancer Research, Vol. 49, pages 5935-5939, 1989], progesterone derivatives (e.g., megestrol acetate) [Journal of Clinical Oncology, Vol. 12, pages 213-225, 1994], glucosteroids (e.g., dexamethasone etc.), metoclopramide agents, tetrahydrocannabinol agents (publications are all as mentioned above), fat metabolism improving agents (e.g., eicosapentanoic acid etc.) [British Journal of Cancer, Vol. 68, pages 314-318, 1993], growth hormones, IGF-1, or antibodies to a cachexia-inducing factor such as TNF-α, LIF, IL-6, oncostatin M and the like.

It is also possible to apply compound A of the present invention to each of the above-mentioned diseases in combination with a biologic (e.g., antibody, vaccine preparation and the like), or as a combination therapy in combination with gene therapy method and the like. Examples of the antibody and vaccine preparation include vaccine preparation to angiotensin II, vaccine preparation to CETP, CETP antibody, TNFα antibody and antibody to other cytokine, amyloid β vaccine preparation, type 1 diabetes vaccine (DIAPEP-277 manufactured by Peptor Ltd. and the like), anti-HIV antibody, HIV vaccine preparation and the like, antibody or vaccine preparation to cytokine, renin-angiotensin enzyme and a product thereof, antibody or vaccine preparation to enzyme or protein involved in blood lipid metabolism, antibody or vaccine to enzyme or protein involved in blood coagulation or fibrinolytic system, antibody or vaccine preparation to protein involved in saccharometabolism or insulin resistance and the like. In addition, a combined use with a biological preparation involved in a growth factor such as GH, IGF and the like is possible. Examples of the gene therapy method include a treatment method using a gene relating to cytokine, renin-angiotensin enzyme and a product thereof, G protein, G protein conjugated receptor and its phosphorylation enzyme, a treatment method using a DNA decoy such as NF_{κ}B decoy and the like, a treatment method using an antisense, a treatment method using a gene relating to an enzyme or protein involved in blood lipid metabolism (e.g., gene relating to metabolism, excretion or absorption of cholesterol or triglyceride or HDL-cholesterol or blood phospholipid and the like), a treatment method using a gene relating to an enzyme or protein involved in angiogenesis therapy targeting obstruction of peripheral vessel and the like (e.g., growth factors such as HGF, VEGF etc., and the like), a treatment method using a gene relating to a protein involved in saccharometabolism or insulin resistance, an antisense to cytokine such as TNF and the like, and the like can be mentioned. In addition, it is possible to use compound A in combination with various organ regeneration methods utilizing heart regeneration, kidney regeneration, pancreas regeneration, blood vessel regeneration and the like or cell transplantation therapy utilizing bone marrow cell (myelomonocytic cell, myeloid stem cell and the like) or an artificial organ utilizing tissue engineering (artificial blood vessel and cardiac muscle cell sheet).

Compound A of the present invention or a salt thereof can be administered orally or parenterally as it is or after mixing with a pharmacologically acceptable carrier. As pharmacologically acceptable carriers, various organic or inorganic carrier substances conventionally used as preparation materials can be used, and added as excipient, lubricant, binder and disintegrant for solid preparations; or solvent, solubilizing agents, suspending agent, isotonicity agent, buffer, soothing agent and the like for liquid preparation. Where necessary, preparation additive such as preservative, antioxidant, colorant, sweetening agent and the like can be used.

For the pharmaceutical agent of the present invention containing compound A or a salt thereof, the dosage form for oral administration is, for example, tablet (including sugar-coated tablet, film-coated tablet), pill, granule, powder, capsule (including soft capsule, microcapsule), syrup, emulsion, suspension and the like, and the dosage form for parenteral administration is, for example, injection, injecting agent, drip infusion, suppository and the like. In addition, it is effective to make a sustained release preparation by combining with a suitable base (e.g., polymer of butyric acid, polymer of glycolic acid, copolymer of butyric acid-glycolic acid, a mixture of polymer of butyric acid and polymer of glycolic acid, polyglycerol fatty acid ester etc.).

While the content of compound A or a salt thereof in the pharmaceutical agent of the present invention varies depending on the form of the pharmaceutical preparation, it is generally about 2 to 85 wt%, preferably about 5 to 70 wt%, relative to the whole preparation.

As a method for forming compound A or a salt thereof in the above-mentioned dosage form, a known production method generally used in the pertinent field can be applied. When the above-mentioned dosage form is produced, various preparation additives such as carrier (e.g., excipient, binder, disintegrant, lubricant and the like), sweetening agent, surfactant, suspending agent, emulsifier and the like generally used in the field of preparation are appropriately added in suitable amounts as necessary for production.

When the compound A or a salt thereof is prepared in to a tablet, for example, it can be produced by adding an excipient, a binder, a disintegrant, a lubricant and the like, and when a pill and a granule are to be prepared, they can be produced by adding an excipient, a binder, a disintegrant and the like. When a powder and a capsule are to be prepared, they can be produced by adding an excipient and the like, and when a syrup is to be prepared, it can be produced by adding a sweetener and the like, and when an emulsion or a suspension is to be prepared, it can be produced by adding a suspending agent, a surfactant, an emulsifier and the like.

Examples of the excipient include lactose, sucrose, glucose, starch, sucrose, microcrystalline cellulose, powdered glycyrrhiza, mannitol, sodium hydrogencarbonate, calcium phosphate, calcium sulfate and the like.

Examples of the binder include 5 - 10 wt% starch liquid paste, 10 - 20 wt% gum arabic solution or gelatin solution, 1 - 5 wt% tragacanth solution, carboxymethyl cellulose solution, sodium alginate solution, glycerin and the like.

Examples of the disintegrant include starch, calcium carbonate and the like.

Examples of the lubricant include magnesium stearate, stearic acid, calcium stearate, purified talc and the like.

Examples of the sweetener include glucose, fructose, invert sugar, sorbitol, xylitol, glycerin, simple syrup and the like.

Examples of the surfactant include sodium lauryl sulfate, polysorbate 80, sorbitan monofatty acid ester, polyoxyl 40 stearate and the like.

Examples of the suspending agent include gum arabic, sodium alginate, sodium carboxymethyl cellulose, methyl cellulose, bentonite and the like.

Examples of the emulsifier include gum arabic, tragacanth, gelatin, polysorbate 80 and the like.

Furthermore, when the compound A or a salt thereof is produced in the above-mentioned dosage form, a suitable amount of a coloring agent, a preservative, an aromatic, a corrigent, a stabilizer, viscous agents and the like typically used in the field of preparation can be added on demand.

The pharmaceutical agent of the present invention containing compound A or a salt thereof is stable and low toxic, and can be used safely. The daily dose varies depending on the condition and body weight of patients, the kind of compound, administration route and the like. For example, in the case of oral administration to patients with diabetes, neurodegenerative disease and the like, the daily dose to an adult (body weight about 60 kg) is about 1 to 1000 mg, preferably about 3 to 300 mg, more preferably about 10 to 200 mg, as an active ingredient (the compound A or a salt thereof), which can be given in a single administration or administered in 2 or 3 portions a day.

When the compound A of the present invention or a salt thereof is administered parenterally, it is generally administered in the form of a liquid (e.g., injection). While the dose varies depending on the subject of administration, target organ, symptom, administration method and the like, it is, for example, about 0.01 mg - about 100 mg, preferably about 0.01 - about 50 mg, more preferably about 0.01 - about 20 mg, in the form of an injection, relative to 1 kg of body weight, which is preferably given by intravenous injection. As the injection, intravenous injection as well as subcutaneous injection, intracutaneous injection, intramuscular injection, instillation and the like are mentioned, and as a sustained release preparation, iontophoresis transdermal agent and the like are mentioned. Such injections are prepared by methods known *per se*, or by dissolving, suspending or emulsifying the compound A of the present invention or a salt thereof in a sterilized aqueous solution or oily liquid. As an aqueous solution for injection, physiological saline, isotonic solutions containing glucose or other auxiliary drugs (e.g., D-sorbitol, D-mannitol, sodium chloride and the like) and the like can be mentioned, and they can be used in combination with suitable dissolution aids, such as alcohols (e.g., ethanol), polyalcohols (e.g., propylene glycol, polyethylene glycol), nonionic surfactants (e.g., polysorbate 80, HCO-50) and the like. As an oily liquid, sesame oil, soybean oil and the like can be mentioned, which may be used in combination with dissolution aids (e.g., benzyl benzoate, benzyl alcohol and the like) and the like. In addition, buffers (e.g., phosphate buffer, sodium acetate buffer), soothing agents (e.g., benzalkonium chloride, procaine hydrochloride and the like), stabilizers (e.g., human serum albumin, polyethylene glycol and the like), preservatives (e.g., benzyl alcohol, phenol and the like) and the like may be added. A prepared injection is generally filled in an ampoule.

When the compound of the present invention is used in combination with other pharmaceutical agent, the administration mode of the compound of the present invention and a combination drug is not particularly limited, and the compound of the present invention and a combination drug only need to be combined on administration. Examples of such administration mode include the following:
(1) administration of a single preparation obtained by simultaneously processing the compound of the present invention and the concomitant drug, (2) simultaneous administration of two kinds of preparations of the compound of the present invention and the concomitant drug, which have been separately produced, by the same administration route,
(3) administration of two kinds of preparations of the compound of the present invention and the concomitant drug, which have been separately produced, by the same administration route in a staggered manner, (4) simultaneous administration of two kinds of preparations of the compound of the present invention and the concomitant drug, which have been separately produced, by different administration routes,
(5) administration of two kinds of preparations of the compound of the present invention and the concomitant drug, which have been separately produced, by different administration routes in a staggered manner (for example, administration in the order of the compound of the present invention and the concomitant drug, or in the reverse order) and the like. The dose of the concomitant drug can be appropriately determined based on the dose employed in clinical situations. The mixing ratio of the compound of the present invention and a concomitant drug can be appropriately determined depending on the administration subject, administration route, target disease, symptom, combination and the like. When the subject of administration is human, for example, a concomitant drug can be used in 0.01 - 100 parts by weight relative to 1 part by weight of the compound of the present invention.

### Examples

The present invention is explained in detail in the following by referring to Reference Examples, Examples, Formulation Examples and Experimental Examples. However, the examples are mere exemplifications and do not limit the present invention. The present invention may be modified without departing from the scope of the invention.

The term "room temperature" in the following Reference Examples and Examples indicates the range of generally from about 10°C to about 35°C. As for "%", the yield is in mol/mol%, the solvent used for chromatography is in % by volume and other "%" is in % by weight. OH proton, NH proton etc. that could not be confirmed due to broad peak by proton NMR spectrum are not included in the data.

The other symbols used herein mean the following:
s: singlet
d: doublet
dd: doublet of doublets
dt: doublet of triplets
t: triplet
q: quartet
septet: septet
m: multiplet
br: broad
J: coupling constant
Hz: Hertz
CDCl₃: deuterated chloroform
DMSO-d₆: deuterated dimethyl sulfoxide
¹H-NMR: proton nuclear magnetic resonance
HPLC: high performance liquid chromatography
THF: tetrahydrofuran
DMF: dimethylformamide
HOBt: 1-hydroxybenzotriazole
WSC: 1-ethyl-3-(3-dimethylaminopropyl)carbodiimide hydrochloride
LC-MS: liquid chromatography-mass spectrometry spectrum
ESI: electrospray ionization method

### Reference Example 1

### Methyl 4-nitro-1H-pyrazole-3-carboxylate

Acetyl chloride (9 mL) was added dropwise to methanol (90 mL), 4-nitro-1H-pyrazole-3-carboxylic acid (9.00 g, 57.3 mmol) was added to the mixture. The mixture was stirred at room temperature for 16 hr, and concentrated under reduced pressure. Methanol was added to the residue, and the mixture was concentrated under reduced pressure, the operation was twice repeated. The residue was diluted with methanol and ethyl acetate. 5% sodium hydrogencarbonate aqueous solution was added, and the pH was adjusted to 8 - 9. The mixture was extracted with ethyl acetate. The extract was washed with water, brine, dried over anhydrous magnesium sulfate, and the solvent was evaporated under reduced pressure to give the title compound (9.83 g, yield 100%).
¹H-NMR (DMSO-d₆, 200 MHz):δ 3.98(3H, s), 8.28(1H, s).

### Reference Example 2

### Methyl 4-amino-1H-pyrazole-3-carboxylate

A mixture of methyl 4-nitro-1H-pyrazole-3-carboxylate obtained in Reference Example 1 (9.63 g, 56.3 mmol), 10% palladium/carbon (1.00 g) and methanol (150 mL) was stirred under hydrogen atmosphere at room temperature for 16 hr. The catalyst was filtrated, and the solvent was evaporated under reduced pressure to give the title compound (7.87 g, yield 99%).
¹H-NMR (CDCl₃):δ 3.93(3H, s), 4.08(2H, brs), 7.25(1H, s).

### Reference Example 3

### N-isopropyl-4-nitro-1H-pyrazole-3-carboxamide

To a mixture of 4-nitro-1H-pyrazole-3-carboxylic acid (6.50 g, 41.4 mmol) and N,N-dimethylformamide (100 mL), isopropylamine (4.7 mL, 55 mmol), HOBt (6.71 g, 50 mmol) and (3-dimethylaminopropyl)ethylcarbodiimide hydrochloride (9.52 g, 50 mmol) were added, and the mixture was stirred at room temperature for 16 hr. 5% Aqueous solution of citric acid was added to the reaction mixture, and the mixture was extracted with ethyl acetate. The extract was washed with 5% aqueous solution of citric acid, 5% sodium hydrogencarbonate aqueous solution, brine, and dried over anhydrous magnesium sulfate. The solvent was evaporated under reduced pressure to give the title compound (5.72 g, yield 70%).
¹H-NMR (DMSO-d₆, 200 MHz) :δ 1.14(6H,d, J=6.6 Hz), 3.91-4.17(1H, m), 8.45-8.65(1H, brm), 8.76(1H, brs).

### Reference Example 4

### 4-nitro-3-(pyrrolidin-1-ylcarbonyl)-1H-pyrazole

In the same manner as in Reference Example 3, the title compound was obtained using pyrrolidine. yield 74%.
¹H-NMR (DMSO-d₆, 200 MHz):δ 1.79-1.91(4H, m), 3.19(2H, t, J=6.3 Hz), 3.49(2H,t, J=6.8 Hz), 8.90(1H, brs).

### Reference Example 5

### N-cyclopentyl-4-nitro-1H-pyrazole-3-carboxamide

In the same manner as in Reference Example 3, the title compound was obtained using cyclopentylamine. yield 71%.
¹H-NMR (DMSO-d₆):δ 1.45-1.67(6H, m), 1.82-1.89(2H, m), 4.12-4.23(1H, m), 8.50-8.90(2H, brm).

### Reference Example 6

### 4-nitro-N-phenyl-1H-pyrazole-3-carboxamide

In the same manner as in Reference Example 3, the title compound was obtained using aniline. yield 88%.
¹H-NMR (DMSO-d₆, 200 MHz):δ 7.14(1H, t, J=7.3 Hz), 7.37-7.41(2H, m), 7.69(2H, d, J=7.2 Hz), 8.90(1H, brs), 10.70(1H, brs).

### Reference Example 7

### N-benzyl-4-nitro-1H-pyrazole-3-carboxamide

In the same manner as in Reference Example 3, the title compound was obtained using benzylamine. yield 82%.
¹H-NMR (DMSO-d₆) :δ 4.47(2H, d, J=6.0 Hz), 7.22-7.35(5H, m), 8.70-8.90(1H, brm), 9.17(1H, brs).

### Reference Example 8

### 4-nitro-N-(2-phenylethyl)-1H-pyrazole-3-carboxamide

In the same manner as in Reference Example 3, the title compound was obtained using 2-phenylethylamine. yield 81%.
¹H-NMR (DMSO-d₆, 200 MHz) :δ 2.83(2H, t, J=7.3 Hz), 3.35-3.52(2H, m), 7.18-7.35(5H, m), 8.65-8.90(2H, brm).

### Reference Example 9

### N-(2-furylmethyl)-4-nitro-1H-pyrazole-3-carboxamide

In the same manner as in Reference Example 3, the title compound was obtained using furfurylamine. yield 78%.
¹H-NMR (DMSO-d₆, 200 MHz) :δ 4.46(2H, d, J=6.0 Hz), 6.32-6.34 (1H, m), 6.41-6.44(1H, m), 7.61-7.62(1H, m), 8.70-8.90(1H, brm), 9.15(1H, brs).

### Reference Example 10

### 4-amino-N-methyl-1H-pyrazole-3-carboxamide

A mixture of methyl 4-amino-1H-pyrazole-3-carboxylate obtained in Reference Example 2 (5.39 g, 38.2 mmol) and 40% methylamine/methanol solution (25 mL) was stirred at room temperature for 2 days. The mixture was concentrated under reduced pressure. Then methanol was added to the residue, and the mixture was concentrated under reduced pressure, the operation was twice repeated. Methanol and activated carbon (4.5 g) were added to the residue. After filtration, the solvent was evaporated under reduced pressure to give the title compound (5.31 g, yield 99%).
¹H-NMR (DMSO-d₆, 200 MHz) :δ 2.71(3H, d, J=4.8 Hz), 4.58(2H, brs), 7.09(1H, s), 7.72-7.86(1H, brm).

### Reference Example 11

### 4-amino-N-isopropyl-1H-pyrazole-3-carboxamide

A mixture of N-isopropyl-4-nitro-1H-pyrazole-3-carboxamide obtained in Reference Example 3 (5.42 g, 32.2 mmol), 10% palladium carbon (1.0 g) and methanol (200 mL) was stirred under hydrogen atmosphere at room temperature for 6 hr. The catalyst was removed by filtration, and the solvent was evaporated under reduced pressure to give the title compound (4.48 g, yield 83%).
¹H-NMR (DMSO-d₆) :δ 1.13(6H, d, J=6.6 Hz), 3.96-4.11 (1H, m), 4.56(2H, brs), 7.08(1H, s), 7.46(1H, brd, J=8.1 Hz).

### Reference Example 12

### 3-(pyrrolidin-1-ylcarbonyl)-1H-pyrazole-4-amine

In the same manner as in Reference Example 11, the title compound was obtained using 4-nitro-3-(pyrrolidin-1-ylcarbonyl)-1H-pyrazole obtained in Reference Example 4. yield 92%.
¹H-NMR (DMSO-d₆, 200 MHz) :δ 1.72-7. 97 (4H, m), 3.42 (2H, t, J=7.7 Hz), 3.86(2H, t, J=6.4 Hz), 4.70(2H, brs), 7.11(1H, s).

### Reference Example 13

### 4-amino-N-cyclopentyl-1H-pyrazole-3-carboxamide

In the same manner as in Reference Example 11, the title compound was obtained using N-cyclopentyl-4-nitro-1H-pyrazole-3-carboxamide obtained in Reference Example 5: yield 96%.
¹H-NMR (DMSO-d₆) :δ 1.40-1.95(8H, m), 4.10-4.23(1H, m), 4.57(2H, brs), 7.08(1H, s), 7.55(1H, brd, J=7.8 Hz).

### Reference Example 14

### 4-amino-N-phenyl-1H-pyrazole-3-carboxamide

In the same manner as in Reference Example 11, the title compound was obtained using 4-nitro-N-phenyl-1H-pyrazole-3-carboxamide obtained in Reference Example 6. yield 93%.
¹H-NMR (DMSO-d₆):δ 4.70(2H, brs), 7.03 (1H, t, J=7.4 Hz), 7.18 (1H, s), 7.26-7.31(2H, m), 7.79(2H, d, J=8.4 Hz), 9.74 (1H, brs).

### Reference Example 15

### 4-amino-N-benzyl-1H-pyrazole-3-carboxamide

In the same manner as in Reference Example 11, the title compound was obtained using N-benzyl-4-nitro-1H-pyrazole-3-carboxamide obtained in Reference Example 7. yield 98%.
¹H-NMR (DMSO-d₆, 200 MHz):δ 4.38(2H, d, J=6.2 Hz), 4.59(2H, brs), 7.11(1H, s), 7.20-7.31(5H, m), 8.43(1H, brt, J=6.2 Hz).

### Reference Example 16

### 4-amino-N-(2-phenylethyl)-1H-pyrazole-3-carboxamide

In the same manner as in Reference Example 11, the title compound was obtained using 4-nitro-N-(2-phenylethyl)-1H-pyrazole-3-carboxamide obtained in Reference Example 8. yield 99%.
¹H-NMR (DMSO-d₆, 300 MHz):δ 2. 80 (2H, t, J=7.4 Hz), 3. 40-3. 47 (2H, m), 4.59(2H, brs), 7.08(1H, s), 7.16-7.32(5H, m), 7.80-7.90(1H, brm).

### Reference Example 17

### 4-amino-N-(2-furylmethyl)-1H-pyrazole-3-carboxamide

In the same manner as in Reference Example 11, the title compound was obtained using N-(2-furylmethyl)-4-nitro-1H-pyrazole-3-carboxamide obtained in Reference Example 9. yield 68%.
¹H-NMR (DMSO-d₆):δ 4.37(2H, d, J=6.2 Hz), 4.58(2H, brs), 6.19(1H, d, J=3.3 Hz), 6.36-6.37(1H, m), 7.10(1H, s), 7.53(1H, d, J=0.9 Hz), 8.22 (1H, brt, J=6.2 Hz).

### Reference Example 18

### Methyl 4-nitro-1H-pyrazole-3-carboxylate

To a solution of 4-nitro-1H-pyrazole-3-carboxylic acid (10.0 g, 63.7 mmol) in methanol (100 mL), thionyl chloride (5.1 mL, 70.0 mmol) was added dropwise at 0°C, and the mixture was stirred at room temperature for 2 hr. The solvent was evaporated under reduced pressure, and the obtained residue was dissolved in ethyl acetate. This solution was washed with saturated aqueous sodium hydrogencarbonate solution, and dried over anhydrous sodium sulfate. The solvent was evaporated under reduced pressure to give the title compound (9.9 g, yield 91%). ¹H-NMR (CDCl₃) :δ 4.06(3H, s), 8.51(1H, s).

### Reference Example 19

### Methyl 1-methyl-4-nitro-1H-pyrazole-3-carboxylate

### Reference Example 20

### Methyl 1-methyl-4-nitro-1H-pyrazole-5-carboxylate

A mixture of methyl 4-nitro-1H-pyrazole-3-carboxylate obtained in Reference Example 18 (2.0 g, 11.7 mmol), methyl iodide (2.0 g, 14.0 mmol), potassium carbonate (1.62 g, 11.7 mmol) and acetone (60 mL) was stirred at 30°C for 5 hr. The mixture was diluted with water, and extracted with chloroform. The extract was dried over anhydrous sodium sulfate, and the solvent was evaporated under reduced pressure. The obtained residue was purified by silica gel column chromatography (hexane-ethyl acetate 4:1 - 1:1) to give the title compound, methyl 1-methyl-4-nitro-1-pyrazole-3-carboxylate (Rf value 0.43) (0.95 g, yield 44%) and the title compound, methyl 1-methyl-4-nitro-1H-pyrazole-5-carboxylate (Rf value 0.71) (0.61 g, yield 28%).
Methyl 1-methyl-4-nitro-1H-pyrazole-3-carboxylate
¹H-NMR (CDCl₃):δ 3.99(3H, s), 4.01(3H, s), 8.13(1H, s).
Methyl 1-methyl-4-nitro-1H-pyrazole-5-carboxylate
¹H-NMR (CDCl₃):δ 4.02(3H, s), 4.03(3H, s), 8.01 (1H, s).

### Reference Example 21

### 1-methyl-4-nitro-1H-pyrazole-3-carboxylic acid

To a mixture of methyl -1-methyl-4-nitro-1H-pyrazole-3-carboxylate obtained in Reference Example 19 (0.95 g, 5.1 mmol) and THF (25 mL)-methanol (5 mL), 1N aqueous sodium hydroxide solution (8.0 mL) was added, and the mixture was stirred at room temperature for 2 hr. The mixture was acidified with hydrochloric acid, and extracted with ethyl acetate. The extract was dried over anhydrous sodium sulfate, and the solvent was evaporated under reduced pressure to give the title compound (0.69 g, yield 79%).
¹H-NMR (CDCl₃) :δ 4.08(3H, s), 8.28 (1H, s).

### Reference Example 22

### N-cyclopentyl-1-methyl-4-nitro-1H-pyrazole-3-carboxamide

A solution of 1-methyl-4-nitro-1H-pyrazole-3-carboxylic acid obtained in Reference Example 21 (0.56 g, 3.3 mmol), cyclopentylamine (0.31 g, 3.6 mmol), HOBt (0.53 g, 3.9 mmol) and WSC (0.75 g, 3.9 mmol) in DMF (10 mL) was stirred at room temperature for 4 hr. The mixture was diluted with water, and extracted with ethyl acetate. The extract was washed with saturated aqueous sodium hydrogencarbonate solution, and dried over anhydrous sodium sulfate. The solvent was evaporated under reduced pressure, and the obtained residue was purified by silica gel column chromatography (hexane-ethyl acetate 1:1) to give the title compound (0.71 g, yield 91%). melting point 176-177°C (ethyl acetate-hexane).
¹H-NMR (CDCl₃): δ 1.56-1.75(6H, m), 2.04-2.08(2H, m), 4.00(3H, s), 4.42-4.48(1H, m), 8.22(1H, s).

### Reference Example 23

### 4-amino-N-cyclopentyl-1-methyl-1H-pyrazole-3-carboxamide

A mixture of N-cyclopentyl-1-methyl-4-nitro-1H-pyrazole-3-carboxamide obtained in Reference Example 22 (0.61 g, 2.6 mmol), ammonium formate (0.65 g, 10.2 mmol), 10% palladium carbon (200 mg) and ethanol (20 mL) was stirred under argon atmosphere at 65°C for 4 hr. The catalyst was removed by filtration, and the filtrate was concentrated under reduced pressure. The obtained residue was purified by silica gel column chromatography (ethyl acetate) to give the title compound (0.44 g, yield 71%).
¹H-NMR (CDCl₃):δ 1.47-1.75(6H, m), 1.98-2.09(2H, m), 3.78(3H, s), 4.30-4.37(1H, m), 6.91(1H, s).

### Reference Example 24

### 1-methyl-4-nitro-1H-pyrazole-5-carboxylic acid

In the same manner as in Reference Example 21, the title compound was obtained using methyl 1-methyl-4-nitro-1H-pyrazole-5-carboxylate obtained in Reference Example 20. yield 95%.
¹H-NMR (CDCl₃) :δ 4.29(3H, s), 8.21 (1H, s).

### Reference Example 25

### N-cyclopentyl-1-methyl-4-nitro-1H-pyrazole-5-carboxamide

In the same manner as in Reference Example 22, the title compound was obtained using 1-methyl-4-nitro-1H-pyrazole-5-carboxylic acid obtained in Reference Example 24 and cyclopentylamine. yield 47%.
melting point 137-138°C (ethyl acetate-hexane).
¹H-NMR (CDCl₃) :δ 1.55-1.81(6H, m), 2.02-2.13(2H, m), 4.18(3H, s), 4.36-4.45(1H, m), 8.18(1H, s).

### Reference Example 26

### 4-amino-N-cyclopentyl-1-methyl-1H-pyrazole-5-carboxamide

In the same manner as in Reference Example 23, the title compound was obtained using N-cyclopentyl-1-methyl-4-nitro-1H-pyrazole-5-carboxamide obtained in Reference Example 25. yield 82%.
¹H-NMR (CDCl₃):δ 1.46-1.75(6H, m), 1.98-2.09(2H, m), 4.14(3H, s), 4.31-4.38(1H, m), 7.20(1H, s).

### Reference Example 27

### Methyl 4-nitro-1-trityl-1H-pyrazole-3-carboxylate

To a solution of methyl 4-nitro-1H-pyrazole-3-carboxylate obtained in Reference Example 18 (5.0 g, 29.0 mmol) and triethylamine (4.5 mL, 32.0 mmol) in DMF (50 mL), trityl chloride (8.9 g, 32.0 mmol) was added at 0°C, and the mixture was stirred at room temperature for 4 hr. The mixture was diluted with water, and extracted with ethyl acetate. The extract was washed with saturated aqueous sodium hydrogencarbonate solution, and dried over anhydrous sodium sulfate. The solvent was evaporated under reduced pressure, and the obtained residue was purified by silica gel column chromatography (hexane-ethyl acetate 9:1) to give the title compound (7.3 g, yield 61%).
¹H-NMR (CDCl₃):δ 3.28(3H, s), 7.14-7.19(5H, m), 7.30-7.35(10H, m), 8.11(1H, s).

### Reference Example 28

### Methyl 4-amino-1-trityl-1H-pyrazole-3-carboxylate

A mixture of methyl 4-nitro-1-trityl-1H-pyrazole-3-carboxylate obtained in Reference Example 27 (7.3 g, 17.7 mmol), ammonium formate (4.45 g, 70.8 mmol), 10% palladium carbon (1.8 g) and ethanol (220 mL) was stirred under argon atmosphere at 65°C for 2 hr. The catalyst was removed by filtration, and the solvent of the filtrate was evaporated under reduced pressure to give the title compound (6.8 g, yield 95%).
¹H-NMR (CDCl₃) :δ 3.84(3H, s), 6.82(1H, s), 7.10-7.16(5H, m), 7.24-7.31(10H, m).

### Reference Example 29

### Methyl 4-((pyrazin-2-ylcarbonyl)amino)-1-trityl-1H-pyrazole-3-carboxylate

A solution of methyl 4-amino-1-trityl-1H-pyrazole-3-carboxylate obtained in Reference Example 28 (6.8 g, 17.7 mmol), pyrazinecarboxylic acid (2.4 g, 19.4 mmol), HOBt (2.9 g, 21.2 mmol) and WSC (4.1 g, 21.2 mmol) in DMF (115 mL) was stirred at room temperature for 2 hr. The mixture was diluted with water, and extracted with ethyl acetate. The extract was washed with saturated aqueous sodium hydrogencarbonate solution, and dried over anhydrous sodium sulfate. The solvent was evaporated under reduced pressure, and the obtained residue was washed with diethyl ether to give the title compound (7.5 g, yield 87%).
¹H-NMR (CDCl₃):δ 3.89(3H, s), 7.10-7.13 (5H, m), 7.41-7.46(10H, m), 8.23(1H, s), 8.84(1H, m), 8.96(1H, d, J=2.4 Hz), 9.25(1H, s), 11.0(1H, s).

### Reference Example 30

### Methyl 4-(methyl(pyrazin-2-ylcarbonyl)amino)-1-trityl-1H-pyrazole-3-carboxylate

To a solution of methyl 4-((pyrazin-2-ylcarbonyl)amino)-1-trityl-1H-pyrazole-3-carboxylate obtained in Reference Example 29 (2.0 g, 4.1 mmol) in DMF (75 mL), sodium hydride (60% liquid paraffin dispersion, 0.18 g, 4.5 mmol) was added at 0°C, and the mixture was stirred at the same temperature for 20 min, and at room temperature for 15 min. This solution was cooled to 0°C. Methyl iodide (0.28 mL, 4.5 mmol) was added, and the mixture was stirred at 60°C for one day. The reaction mixture was poured into saturated aqueous ammonium chloride solution, and the mixture was extracted with ethyl acetate. The extract was washed with water, saturated aqueous sodium chloride solution, and dried over anhydrous sodium sulfate. The solvent was evaporated under reduced pressure, and the obtained residue was purified by silica gel column chromatography (hexane-ethyl acetate 1:1) to give the title compound (0.91 g, yield 44%).
¹H-NMR (CDCl₃) :δ 3.40(3H, s), 3.82(3H, s), 6.94-6.97 (5H, m), 7.23-7.30(10H, m), 8.30(1H, dd, J=1.5 Hz and J=2.4 Hz), 8.49(1H, d, J=2. 4 Hz), 8.78(1H, d, J=1.5 Hz).

### Reference Example 31

### 4-(methyl(pyrazin-2-ylcarbonyl)amino)-1-trityl-1H-pyrazole-3-carboxylic acid

In the same manner as in Reference Example 21, the title compound was obtained using methyl 4-(methyl(pyrazin-2-ylcarbonyl)amino)-1-trityl-1H-pyrazole-3-carboxylate obtained in Reference Example 30. yield 95%.
¹H-NMR (CDCl₃) :δ 3.42(3H, s), 6.96-6.99(5H, m), 7.26-7.34(11H, m), 8.32(1H, d, J=1.2 Hz), 8.50(1H, d, J=2.4 Hz), 8.88(1H, s).

### Reference Example 32

### 4-amino-1-trityl-1H-pyrazole-3-carboxylic acid

In the same manner as in Reference Example 21, the title compound was obtained using methyl 4-nitro-1-trityl-1H-pyrazole-3-carboxylate obtained in Reference Example 27. yield 95%.
¹H-NMR (CDCl₃):δ 7.14-7.19(5H, m), 7.30-7.35(10H, m), 8.09(1H, s).

### Reference Example 33

### N-cyclopentyl-4-nitro-1-trityl-1H-pyrazole-3-carboxamide

In the same manner as in Reference Example 22, the title compound was obtained using 4-amino-1-trityl-1H-pyrazole-3-carboxylic acid obtained in Reference Example 32 and cyclopentylamine. yield 93%.
¹H-NMR (CDCl₃):δ 1.44-1.72(6H, m), 1.98-2.07(2H, m), 4.33-4.40(1H, m), 7.09-7.13(6H, m), 7.32-7.41(10H, m), 8.10(1H, d, J=1.2 Hz).

### Reference Example 34

### N-cyclopentyl-N-methyl-4-nitro-1-trityl-1H-pyrazole-3-carboxamide

In the same manner as in Reference Example 30, the title compound was obtained using N-cyclopentyl-4-nitro-1-trityl-1H-pyrazole-3-carboxamide obtained in Reference Example 33 and methyl iodide. yield 85%.
¹H-NMR (CDCl₃) :δ 1.35-1.70(8H, m), 2.96(3H, s), 3.64-3.75(1H, m), 7.12-7.18(5H, m), 7.31-7.39(10H, m), 8.17(1H, s).

### Reference Example 35

### 4-amino-N-cyclopentyl-N-methyl-1-trityl-1H-pyrazole-3-carboxamide

In the same manner as in Reference Example 23, the title compound was obtained using N-cyclopentyl-N-methyl-4-nitro-1-trityl-1H-pyrazole-3-carboxamide obtained in Reference Example 34. yield 95%.
¹H-NMR (CDCl₃) :δ 1.25-1.74(8H, m), 2.92(3H, s), 3.64-3.75(1H, m), 7.13-7.18(5H, m), 7.26-7.33(10H, m), 6.86(1H, s).

### Reference Example 36

### Methyl 4-((pyridin-2-ylcarbonyl)amino)-1H-pyrazole-3-carboxylate

A solution of methyl 4-amino-1H-pyrazole-3-carboxylate obtained in Reference Example 2 (3.00 g, 21.3 mmol), 2-pyridinecarboxylic acid (2.88 g, 23.4 mmol), HOBt (3.46 g, 25.6 mmol) and WSC (4.91 g, 25.6 mmol) in DMF (75 mL) was stirred at room temperature overnight. The mixture was diluted with water, and the resulting white precipitate was collected by filtration. This was dried under vacuum to give the title compound (3.03 g, yield 58%).
¹H-NMR (DMSO-d₆) : δ 3.92(3H, s), 7.65-7.77(1H, m), 8.09(1H, dt, J=1.3 Hz and 7.7 Hz), 8.18(1H, d, J=7.7 Hz), 8.45(1H, s), 8.76(1H, d, J=4.8 Hz), 11.20(1H, s), 13.60(1H, s).

### Reference Example 37

### 4-((pyridin-2-ylcarbonyl)amino)-1H-pyrazole-3-carboxylic acid

To a solution of methyl 4-amino-1H-pyrazole-3-carboxylate obtained in Reference Example 36 (3.00 g, 12.2 mmol) in THF (150 mL)/methanol (30 mL), 1N aqueous sodium hydroxide solution (18 mL) was added, and the mixture was stirred at 50°C overnight. The reaction solution was concentrated under reduced pressure, and the pH was adjusted to 6-7 with 1N aqueous hydrochloric acid solution. The resulting white solid was collected by filtration, and this was dried under vacuum to give the title compound (1.31 g, yield 46%).
¹H-NMR (DMSO-d₆):δ 7.67-7.72(1H, m), 8.09(1H, dt, J=1.3 Hz and 7.6 Hz), 8.18(1H, d, J=7.6 Hz), 8.41(1H, s), 8.74(1H, d, J=4.3 Hz), 11.13(1H, s), 13.52(2H, br).

### Reference Example 38

### 6-((2-aminoethyl)amino)nicotinonitrile

6-Chloronicotinonitrile (12.5 g, 90.6 mmol) was slowly added to ethylenediamine (182 mL) over 15 min, and the mixture was stirred at room temperature for 2 hr. Potassium carbonate (50.1 g, 362 mmol) was added, and the mixture was stirred for additional 30 min. The insoluble material was removed by filtration, and the filtrate was concentrated under reduced pressure. DMF (100 mL) was added to the residue, and the mixture was further concentrated under reduced pressure. The obtained white solid was washed with ethyl acetate, and dried under vacuum to give the title compound (9.57 g, yield 65%).
¹H-NMR (CDCl₃) :δ 2.97(2H, d, J=6.0 Hz), 3.43 (2H, d, J=6.0 Hz), 6.41(1H, d, J=8.7 Hz), 7.55(1H, dd, J=2.4 Hz and 8.7 Hz), 8.36(1H, d, J=2.4 Hz).

### Reference Example 39

### Methyl 4-nitro-1-(tetrahydro-2H-pyran-2-yl)-1H-pyrazole-3-carboxylate

To a solution (275 mL) of methyl 4-nitro-1H-pyrazole-3-carboxylate obtained in Reference Example 1 (27.4 g, 160 mmol) and p-toluenesulfonic acid monohydrate (3.04 g, 16 mmol) in chloroform, 2,3-dihydropyran (19.0 mL, 330 mmol) was added at 0°C, and the mixture was warmed to room temperature, and stirred for 3 hr. The reaction mixture was washed with saturated aqueous sodium hydrogencarbonate solution, and dried over anhydrous sodium sulfate. The solvent was evaporated under reduced pressure. The residue was purified by silica gel column chromatography (hexane-ethyl acetate 2:1) to give the title compound (34.8 g, yield 85%) as pale-yellow oily substance.
¹H-NMR (CDCl₃) : δ 1.62-1.77(3H, m), 1.83-2.04(2H, m), 2.15-2.27 (1H, m), 3.68-3.76(1H, m), 3.99(3H, s), 4.03-4.13(1H, m), 5.43(1H, dd, J=2.7 Hz, 9.1 Hz), 8.38(1H, s).

### Reference Example 40

### Methyl 4-amino-1-(tetrahydro-2H-pyran-2-yl)-1H-pyrazole-3-carboxylate

A mixture of methyl 4-nitro-1-(tetrahydro-2H-pyran-2-yl)-1H-pyrazole-3-carboxylate obtained in Reference Example 39 (12.8 g, 50 mmol), 10% palladium carbon (containing 50% water, 1.28 g) and ethanol (125 mL) was stirred under hydrogen atmosphere at room temperature for 24 hr. The catalyst was removed by filtration, and the filtrate was concentrated under reduced pressure to give the title compound as an oil.
¹H-NMR (CDCl₃):δ 1.51-1.66(3H, m), 1.91-2.01(3H, m), 3.59-3.68(1H, m), 3.88(3H, s), 4.00-4.04 (1H, m), 5.29 (1H, d, J=5.7 Hz), 7.16 (1H, s).

### Reference Example 41

### Methyl 4-((3-(acetylamino)benzoyl)amino)-1-(tetrahydro-2H-pyran-2-yl)-1H-pyrazole-3-carboxylate

In the same manner as in Reference Example 36, the title compound was obtained using methyl 4-amino-1-(tetrahydro-2H-pyran-2-yl)-1H-pyrazole-3-carboxylate obtained in Reference Example 40 and 3-acetamidobenzoic acid. yield 57%.

### Reference Example 42

### 4-((3-(acetylamino)benzoyl)amino)-1-(tetrahydro-2H-pyran-2-yl)-1H-pyrazole-3-carboxylic acid

To a solution of methyl 4-((3-(acetylamino)benzoyl)amino)-1-(tetrahydro-2H-pyran-2-yl)-1H-pyrazole-3-carboxylate obtained in Reference Example 41 (10.82 g, 28.0 mmol) in THF (70 mL), 2N aqueous sodium hydroxide solution (77 mL) was added, and the mixture was stirred at room temperature for 1 hr. The reaction mixture was neutralized with 6N hydrochloric acid, and extracted with ethyl acetate. The extract was washed with saturated brine, and dried over anhydrous sodium sulfate. The solvent was evaporated. The residue was crystallized from methanol-ethyl acetate to give the title compound (8.07 g, yield 77%).
¹H-NMR (DMSO-d₆):δ 1.49-1.79(3H, m), 1.88-2.17(3H, m), 2.08(3H, s), 3.59-3.75(1H, m), 3.87-4.01(1H, m), 5.54(1H, dd, J=2.3, 9.6 Hz), 7.42-7.56(2H, m), 7.75-7.89(1H, m), 8.17(1H, s), 8.45(1H, s), 10.00(1H, s), 10.22(1H, s).

### Reference Example 43

### 4-((3-(acetylamino)benzoyl)amino)-1H-pyrazole-3-carboxylic acid p-toluenesulfonic acid

To a mixture of 4-((3-(acetylamino)benzoyl)amino)-1-(tetrahydro-2H-pyran-2-yl)-1H-pyrazole-3-carboxylic acid obtained in Reference Example 42 (7.45 g, 20 mmol) and ethanol (50 mL), p-toluenesulfonic acid monohydrate (7.61 g, 40 mmol) was added, and the mixture was stirred at 60°C for 3 hr. The crystals were collected by filtration, and washed with ethanol and ethyl acetate to give the title compound (8.05 g, yield 60%). ¹H-NMR (DMSO-d₆) : δ 2.08(3H, s), 2.29(3H, s), 7.12 (2H, d, J=8.1 Hz), 7.44-7.55(4H, m), 7.75-7.90(1H, m), 8.18(1H, s), 8.29(1H, s), 9.92(1H, s), 10.21(1H, s).

### Reference Example 44

### N-cyclopentyl-5-methyl-4-nitro-1H-pyrazine-3-carboxamide

To a solution of 5-methyl-4-nitro-1H-pyrazole-3-carboxylic acid (0.4 g, 2.34 mmol) and cyclopentylamine (0.3 mL, 3.04 mmol) in DMF (10 mL), WSC (0.60 g, 3.51 mmol) and HOBt (0.54 g, 3.51 mmol) were added, and the mixture was stirred at room temperature for 15 hr. The reaction mixture was diluted with water, and extracted with ethyl acetate. The extract was washed with saturated aqueous sodium hydrogen carbonate, dried over anhydrous magnesium sulfate, and evaporated under reduced pressure. The residue was purified by silica gel column chromatography (hexane-ethyl acetate 1:1) to give the title compound 156 m g (yield 28%) as crystals. melting point 163-165°C.
¹H-NMR (CDCl₃) :δ 1.50-1.85(6H, m), 2.20-2.20(2H, m), 2.60(3H, s), 4.35-4.50(1H, m), 9.08(1H, brs), 11.55(1H, brs).

### Reference Example 45

### N-cyclopentyl-5-ethyl-4-nitro-1H-pyrazole-3-carboxamide

To a solution of 5-ethyl-4-nitro-1H-pyrazole-3-carboxylic acid (0.25 g, 1.35 mmol) and cyclopentylamine (0.17 mL, 1.76 mmol) in DMF (10 mL), WSC (0.39 g, 2.03 mmol) and HOBt (0.31 g, 2.03 mmol) were added, and the mixture was stirred at room temperature for 6 hr. The reaction mixture was diluted with water, and extracted with ethyl acetate. The extract was washed with saturated aqueous sodium hydrogen carbonate, dried over anhydrous magnesium sulfate, and evaporated under reduced pressure. The residue was purified by silica gel column chromatography (hexane-ethyl acetate 1:1) to give the title compound 96.9 mg (yield 28%) as crystals. melting point 156-157°C.
¹H-NMR (CDCl₃) : δ 1.31(3H, t, J=7.5 Hz), 1.50-1.90(6H, m), 2.00-2.20(2H, m), 3.02(2H, q, J=7.5 Hz), 4.35-4.50(1H, m), 9.08(1H, brs), 11.65(1H, brs).

### Reference Example 46

### Methyl 4-(((6-methylpyridin-2-yl)carbonyl)amino)-1-(tetrahydro-2H-pyran-2-yl)-1H-pyrazole-3-carboxylate

To a mixture of methyl 4-nitro-1-(tetrahydro-2H-pyran-2-yl)-1H-pyrazole-3-carboxylate obtained in Reference Example 39 (9.30 g, 36.5 mmol), ammonium formate (10 g) and methanol (150 mL), 10% palladium carbon (containing 50% water, 0.8 g) was added, and the mixture was stirred for 24 hr. The catalyst was removed by filtration, and the filtrate was evaporated under reduced pressure to give methyl 4-amino-1-(tetrahydro-2H-pyran-2-yl)-1H-pyrazole-3-carboxylate as an oil. To a solution of this oily substance and 6-methylpicolinic acid (5.0 g, 36.5 mmol) in DMF (100 mL), WSC (6.88 g, 40 mmol) and HOBt (5.41 g, 40 mmol) were added, and the mixture was stirred at room temperature for 14 hr. Saturated aqueous sodium hydrogen carbonate solution was added to the reaction mixture, and the mixture was extracted with ethyl acetate. The extract was washed with water, dried over anhydrous magnesium sulfate, and evaporated under reduced pressure. The residue was purified by silica gel column chromatography (ethyl acetate) to give the title compound (9.02 g, yield 72%) as crystals. melting point 100-102°C.
¹H-NMR (d₆-DMSO):δ 1.50-1.80(3H, m), 1.95-2.20(3H, m), 2.62(3H, s), 3.60-3.80(1H, m), 3.95(3H, s), 3.90-4.00(1H, m), 5.57(1H, d, J=9.6 Hz), 7.55-7.65(1H, m), 7.95-8.05(2H, m), 8.56(1H, s), 11.20(1H, s).

### Reference Example 47

### 4-(((6-methylpyridin-2-yl)carbonyl)amino)-1-(tetrahydro-2H-pyran-2-yl)-1H-pyrazole-3-carboxylic acid

To a solution of methyl 4-(((6-methylpyridin-2-yl)carbonyl)amino)-1-(tetrahydro-2H-pyran-2-yl)-1H-pyrazole-3-carboxylate obtained in Reference Example 46 (8.8 g, 25.6 mmol) in THF-methanol (80-40 mL), 2N aqueous sodium hydroxide solution (25.6 mL) was added, and the mixture was stirred at room temperature for 4 hr. The reaction mixture was neutralized with 6N hydrochloric acid (8.53 mL), diluted with water, and extracted with ethyl acetate. The extract was washed with water, dried over anhydrous magnesium sulfate, and concentrated under reduced pressure. The precipitated crystals were collected by filtration to give the title compound (7.75 g, yield 92%). melting point 305-310°C (decomposition).
¹H-NMR (d₆-DMSO):δ 1.50-1.80(3H, m), 1.85-2.15(3H, m), 3.60-3.75(1H, m), 3.90-4.00(1H, m), 5.53(1H, d, J=7.2 Hz), 7.53(1H, t, J=4.4 Hz), 7.90-8.00(2H, m), 8.51(1H, s), 11.31 (1H, s), 13.47(1H, s).

### Reference Example 48

### Methyl 4-(benzoylamino)-1-(tetrahydro-2H-pyran-2-yl)-1H-pyrazole-3-carboxylate

To a mixture of methyl 4-nitro-1-(tetrahydro-2H-pyran-2-yl)-1H-pyrazole-3-carboxylate obtained in Reference Example 39 (7.66 g, 30 mmol) and ammonium formate (10 g) in methanol (100 mL), 10% palladium carbon (containing 50% water, 0.77 g) was added, and the mixture was stirred for 14 hr. The catalyst was removed by filtration, and the filtrate was evaporated under reduced pressure to give methyl 4-amino-1-(tetrahydro-2H-pyran-2-yl)-1H-pyrazole-3-carboxylate as an oil. To a solution of this oily substance and benzoic acid (3.66 g, 30 mmol) in DMF (70 mL), WSC (5.67 g, 33 mmol) and HOBt (4.46 g, 33 mmol) were added, and the mixture was stirred at room temperature for 4 hr. Water was added to the reaction mixture, and the mixture was extracted with ethyl acetate. The extract was washed with saturated aqueous sodium hydrogen carbonate solution and water, dried over anhydrous magnesium sulfate, and evaporated under reduced pressure. The residue was purified by silica gel column chromatography (hexane-ethyl acetate 1:1) to give the title compound (6.69 g, yield 67%) as crystals. melting point 124-125°C.
¹H-NMR (d₆-DMSO) :δ 1.55-1.80(3H, m), 2.00-3.20(3H, m), 3.60-3.80(1H, m), 4.00(3H, s), 4.02-4.15(1H, m), 5.40-5.50(1H, m), 7.45-7.60(3H, m), 7.95(2H, d, J=8.1 Hz), 8.59(1H, s), 9.95(1H, s).

### Reference Example 49

### 4-(benzoylamino)-1-(tetrahydro-2H-pyran-2-yl)-1H-pyrazole-3-carboxylic acid

To a solution of methyl 4-(benzoylamino)-1-(tetrahydro-2H-pyran-2-yl)-1H-pyrazole-3-carboxylate obtained in Reference Example 48 (6.52 g, 19.8 mmol) in THF-methanol (20-50 mL), 2N aqueous sodium hydroxide solution (19.8 mL) was added, and the mixture was stirred at room temperature for 2.5 hr. The reaction mixture was neutralized with 6N hydrochloric acid (6.67 mL), diluted with water, and extracted with ethyl acetate. The extract was washed with water, dried over anhydrous magnesium sulfate, and concentrated under reduced pressure. Diethyl ether was added to the precipitated crystals, and the crystals were collected by filtration to give the title compound (4.90 g, yield 78%). melting point 291-292°C (decomposition).
¹H-NMR (d₆-DMSO): δ 1.50-1.80(3H, m), 1.90-2.00(2H, m), 2.00-2.20 (1H, m), 3.60-3.80 (1H, m), 3.90-4.00 (1H, m), 5.54 (1H, d, J=9.0 Hz), 7.55-7.70(3H, m), 7.88(2H, d, J=8.1 Hz), 8.45 (1H, s), 10.01 (1H, s), 13.50 (1H, s).

### Reference Example 50

### 4-nitro-1-(tetrahydro-2H-pyran-2-yl)-1H-pyrazole-3-carboxylic acid

To a solution of methyl 4-nitro-1-(tetrahydro-2H-pyran-2-yl)-1H-pyrazole-3-carboxylate obtained in Reference Example 39 (25.5 g, 100 mmol) in THF (200 mL), 2N sodium hydroxide (100 mL) was added at 0°C, and the mixture was warmed to room temperature. The reaction mixture was stirred at room temperature for 4 hr, cooled to 0°C, and weakly-acidified with 2N hydrochloric acid. The mixture was extracted with ethyl acetate, and the extract was washed with saturated brine, and dried over anhydrous sodium sulfate. The solvent was evaporated under reduced pressure, and the residue was crystallized from ether-hexane to give the title compound (14.5 g, yield 60%).
¹H-NMR (DMSO-d₆) :δ 1.50-1.77(3H, m), 1.85-2.17(3H, m), 3.62-3.69 (1H, m), 3.91-4.00(1H, m), 5.53(1H dd, J=2.6 Hz, 9.5 Hz), 9.09(1H, s).

### Reference Example 51

### N-(2-cyanoethyl)-4-nitro-1-(tetrahydro-2H-pyran-2-yl)-1H-pyrazole-3-carboxamide

To a solution of 4-nitro-1-(tetrahydro-2H-pyran-2-yl)-1H-pyrazole-3-carboxylic acid obtained in Reference Example 50 (14.5 g, 60 mmol) and 2-cyanoethylamine (4.87 mL, 66 mmol) in DMF (145 mL), WSC (13.8 g, 72 mmol) and HOBt (9.73 g, 72 mmol) were added, and the mixture was stirred at room temperature for 4 hr. The reaction mixture was diluted with water, and extracted with ethyl acetate. The extract was washed with saturated aqueous sodium hydrogen carbonate, dried over anhydrous sodium sulfate, and evaporated under reduced pressure. The residue was purified by silica gel column chromatography (ethyl acetate), and crystallized from ethyl acetate-hexane to give the title compound (14.1 g, yield 80%).
¹H-NMR (CDCl₃) :δ 1. 63-1.79 (3H, m), 1.82-2.05(2H, m), 2.20-2.29 (1H, m), 2.78(2H, t, J=6.3 Hz), 3.70-3.79(3H, m), 4.07-4.17(1H, m), 5.44(1H, dd, J=2.7, 9.1 Hz), 8.04(1H, br), 8.47(1H, s).

### Reference Example 52

### 4-amino-N-(2-cyanoethyl)-1-(tetrahydro-2H-pyran-2-yl)-1H-pyrazole-3-carboxamide

A solution of N-(2-cyanoethyl)-4-nitro-1-(tetrahydro-2H-pyran-2-yl)-1H-pyrazole-3-carboxamide obtained in Reference Example 51 (7.33 g, 25 mmol) and 10% palladium carbon (containing 50% water, 0.37 g) in ethanol (80 mL) was stirred under hydrogen atmosphere at room temperature. The reaction mixture was stirred for 2 hr, and the catalyst was removed by filtration. The filtrate was concentrated under reduced pressure. The residue was dissolved in ethyl acetate, and the mixture was washed with saturated brine, dried over anhydrous sodium sulfate, and evaporated under reduced pressure. The residue was purified by silica gel column chromatography (ethyl acetate), and crystallized from ethyl acetate-hexane to give the title compound (4.98 g, yield 76%).
¹H-NMR (CDCl₃) : δ 1.57-1.77 (3H, m), 1.93-2.16 (3H, m), 2.69(2H, t, J=6.6 Hz), 3.61-3.72(3H, m), 3.98-4.07(1H, m), 4.13(2H, br), 5.22(1H, dd, J=2.6 Hz and 8.9 Hz), 7.11 (1H, br), 7.14(1H, s).

### Reference Example 53

### Methyl 4-(((pyridin-2-yl)carbonyl)amino)-1-(tetrahydro-2H-pyran-2-yl)-1H-pyrazole-3-carboxylate

To a mixture of methyl 4-nitro-1-(tetrahydro-2H-pyran-2-yl)-1H-pyrazole-3-carboxylate obtained in Reference Example 39 (10.2 g, 40 mmol), ammonium formate (10 g) and methanol (150 ml), 10% palladium carbon (containing 50% water, 1.0 g) was added, and the mixture was stirred for 19 hr. The catalyst was removed by filtration, and the filtrate was evaporated under reduced pressure to give methyl 4-amino-1-(tetrahydro-2H-pyran-2-yl)-1H-pyrazole-3-carboxylate as an oil. To a solution of this oily substance and pyridine-2-carboxylic acid (4.92 g, 40 mmol) in DMF (100 ml), WSC (6.88 g, 40 mmol), HOBt (5.41 g, 40 mmol) and triethylamine (5.58 ml, 40 mmol) were added, and the mixture was stirred at room temperature for 6 hr. Saturated aqueous sodium hydrogen carbonate solution was added to the reaction mixture, and the mixture was extracted with ethyl acetate. The extract was washed with water, dried over anhydrous magnesium sulfate, evaporated under reduced pressure, passed through a small amount of silica gel and evaporated under reduced pressure to give the title compound (9.48 g, yield 72%) as crystals. melting point 131-132°C.
¹H-NMR (CDCl₃) :δ 1.60-1.80(3H, m), 2.00-2.20(3H, m), 3.65-3.80(1H, m), 4.03(3H, s), 4.05-4.20(1H, m), 5.46(1H, t, J=6.2 Hz), 7.20-7.50(1H, m), 7.85-7.95(1H, m), 8.24(1H, d, J=9.0 Hz), 8.63(1H, s), 8.70-8.80(1H, m), 11.35(1H, s).

### Reference Example 54

### 4-(((pyridin-2-yl)carbonyl)amino)-1-(tetrahydro-2H-pyran-2-yl)-1H-pyrazole-3-carboxylic acid

To a mixture of methyl 4-(((pyridin-2-yl)carbonyl)amino)-1-(tetrahydro-2H-pyran-2-yl)-1H-pyrazole-3-carboxylate obtained in Reference Example 53 (9.0 g, 27.2 mmol), tetrahydrofuran (100 ml) and methanol (50 ml), 2N aqueous sodium hydroxide solution (27.2 ml) was added, and the mixture was stirred at room temperature for 8 hr. The reaction mixture was neutralized with 6N hydrochloric acid (9.1 ml), diluted with water, and extracted with ethyl acetate. The extract was washed with water, dried over anhydrous magnesium sulfate, and evaporated under reduced pressure. The precipitated crystals were collected by filtration to give the title compound (6.51 g, yield 76%). melting point 300-301°C (decomposition).
¹H-NMR (d₆-DMSO) :δ 1.50-1.80(3H, m), 1.85-2.20(2H, m), 3.60-3.80(1H, m), 3.94(1H d, J=12.2 Hz), 5.55(1H, d, J=9.3 Hz), 7.65-7.80(1H, m), 8.08(1H, t, J=7.2 Hz), 8.17(1H, d, J=7.2 Hz), 8.56(1H, s), 8.70-8.80(1H, m), 11.18(1H, s), 13.25(1H, s).

### Reference Example 55

### Methyl 4-(((6-methylpyridin-3-yl)carbonyl)amino)-1-(tetrahydro-2H-pyran-2-yl)-1H-pyrazole-3-carboxylate

To a mixture of methyl 4-nitro-1-(tetrahydro-2H-pyran-2-yl)-1H-pyrazole-3-carboxylate obtained in Reference Example 39 (8.71 g, 34.1 mmol), ammonium formate (9 g) and methanol (150 ml), 10% palladium carbon (containing 50% water, 0.8 g) was added, and the mixture was stirred for 29 hr. The catalyst was removed by filtration, and the filtrate was evaporated under reduced pressure. The residue was diluted with ethyl acetate, washed with saturated aqueous sodium hydrogen carbonate solution, and evaporated under reduced pressure to give methyl 4-amino-1-(tetrahydro-2H-pyran-2-yl)-1H-pyrazole-3-carboxylate as an oil. To a solution of this oily substance and 6-methyl-3-pyridinecarboxylic acid (4.69 g, 34.1 mmol) in DMF (100 ml), WSC (7.04 g, 41.0 mmol) and HOBt (5.54 g, 41.0 mmol) were added, and the mixture was stirred at room temperature for 24 hr. Saturated aqueous sodium hydrogen carbonate solution was added to the reaction mixture, and the mixture was extracted with ethyl acetate. The extract was washed with water,'dried over anhydrous magnesium sulfate, passed through a small amount of silica gel, and evaporated under reduced pressure. The residue was crystallized from diethyl ether to give the title compound (7.67 g, yield 65%) as crystals. melting point 142-143°C.
¹H-NMR (CDCl₃):δ 1.50-1.80(3H, m), 2.00-2.20(3H, m), 2.65(3H, s), 3.65-3.80(1H, m), 4.00(3H, s), 4.08(1H, d, J=10.5 Hz), 5.45(1H, t, J=6.3 Hz), 7.28 (1H, d, J=8.1 Hz), 8.09(1H, dd, J=8.1, 2.4 Hz), 8.56 (1H, s), 9.08 (1H, d, J=1.6 Hz), 9.93(1H, s).

### Reference Example 56

### 4-(((6-methylpyridin-3-yl)carbonyl)amino)-1-(tetrahydro-2H-pyran-2-yl)-1H-pyrazole-3-carboxylic acid

To a mixture of methyl 4-(((6-methylpyridin-3-yl)carbonyl) amino)-1-(tetrahydro-2H-pyran-2-yl)-1H-pyrazole-3-carboxylate obtained in Reference Example 55 (7.37 g, 21.4 mmol), tetrahydrofuran (100 ml) and methanol (100 ml), 2N aqueous sodium hydroxide solution (21.4 ml) was added, and the mixture was stirred at room temperature for 3 hr. The reaction mixture was neutralized with 6N hydrochloric acid (7.13 ml), and diluted with water. The precipitated crystals were collected by filtration to give the title compound (5.76 g, yield 81%). melting point 304-307°C (decomposition).
¹H-NMR (d₆-DMSO) :δ 1.50-1.80(3H, m), 1.90-2.00(2H, m), 2.00-2.20(1H, m), 2.56(3H, s), 3.60-3.75(1H, m), 3.90-4.00(1H, m), 5.53(1H, d, J=6.9 Hz), 7.45(1H, d, J=8.4 Hz), 8.11(1H, dd, J=8.4, 1.8 Hz), 8.42(1H, s), 8.92(1H, d, J=1.8 Hz), 10.01(1H, s), 13.45(1H, s).

### Reference Example 57

### 3-amino-2,2-dimethylpropanenitrile

### 1) 3-hydroxy-2,2-dimethylpropanenitrile

To a mixture of ethyl 2-cyano-2-methylpropionate (5.0 g, 35.4 mmol), tetrahydrofuran (40 ml) and water (100 ml), sodium borohydride (4.47 g, 106 mmol) was slowly added, and the mixture was stirred at room temperature for 3 hr. 6N hydrochloric acid was added to the reaction mixture to quench the reaction, and the mixture was extracted with ethyl acetate. The extract was washed with water, dried over anhydrous magnesium sulfate, passed through a small amount of silica gel and concentrated under reduced pressure to give the title compound (3.4 g, yield 97%) as an oil.

### ¹H-NMR (CDCl₃) :δ 1.36 (6H, s), 3.58 (2H, s).

### 2) 2-cyano-2-methylpropyl methanesulfonate

To a solution of 3-hydroxy-2,2-dimethylpropanenitrile obtained in 1) (3.3 g, 33.3 mmol) in ethyl acetate (50 ml), triethylamine (5.10 ml, 36.6 mmol) and methanesulfonyl chloride (2.71 ml, 35.0 mmol) were added, and the mixture was stirred at room temperature for 2 hr. The reaction mixture was washed with water, dried over anhydrous magnesium sulfate, passed through a small amount of silica gel and concentrated under reduced pressure to give the title compound 5.14 g(87%) as an oil.
¹H-NMR (CDCl₃) :δ 1.45 (6H, s), 3.13 (3H, s), 4.12 (2H, s).

### 3) 3-azido-2,2-dimethylpropanenitrile

To a solution of 2-cyano-2-methylpropyl methanesulfonate obtained in 2) (2.82 g, 15.8 mmol) in DMF (20 ml), sodium azide (2.06 g, 31.6 mmol) was added, and the mixture was stirred at 120°C for 23 hr. The reaction mixture was diluted with water, and extracted with ethyl acetate. The extract was washed with brine, dried over anhydrous magnesium sulfate, and evaporated under reduced pressure to give the title compound (1.62 g, yield 83%) as an oil.
¹H-NMR (CDCl₃) :δ 1.40(6H, s), 3.40(2H, s).

### 4) 3-amino-2,2-dimethylpropanenitrile

To a solution of 3-azido-2,2-dimethylpropanenitrile obtained in 3) (1.6 g, 12.9 mmol) in methanol (20 ml), 10% palladium carbon (containing 50% water, 0.4 g) was added, and the mixture was stirred under hydrogen atmosphere for 3 days. The catalyst was removed by filtration, and the filtrate was evaporated under reduced pressure to give the title compound (1.2 g, yield 95%) as an oil.
¹H-NMR (CDCl₃) :δ 1.33(6H, s), 2.75 (2H, m).

### Reference Example 58

### Methyl 4-(((4-chloropyridin-2-yl)carbonyl)amino)-1-(tetrahydro-2H-pyran-2-yl)-1H-pyrazole-3-carboxylate

To a mixture of methyl 4-nitro-1-(tetrahydro-2H-pyran-2-yl)-1H-pyrazole-3-carboxylate obtained in Reference Example 39 (7.5 g, 29.4 mmol), ammonium formate (8 g) and methanol (150 ml), 10% palladium carbon (containing 50% water, 0.8 g) was added, and the mixture was stirred for 24 hr. The catalyst was removed by filtration, the filtrate was evaporated under reduced pressure to give methyl 4-amino-1-(tetrahydro-2H-pyran-2-yl)-1H-pyrazole-3-carboxylate as an oil. To a solution of this oily substance and 4-chloropicolinic acid (4.63 g, 29.4 mmol) in DMF (100 ml), WSC (6.06 g, 35.3 mmol) and HOBt (4.76 g, 35.3 mmol) were added, and the mixture was stirred at room temperature for 24 hr. Saturated aqueous sodium hydrogen carbonate solution was added to the reaction mixture, and the mixture was extracted with ethyl acetate. The extract was washed with water, dried over anhydrous magnesium sulfate, and evaporated under reduced pressure, passed through a small amount of silica gel and evaporated under reduced pressure to give the title compound (7.44 g, yield 69%) as crystals. melting point 201-203°C.
¹H-NMR (d₆-DMSO):δ 1.60-1.80(3H, m), 2.00-2.20(3H, m), 3.65-3.80 (1H, m), 4.02(3H, s), 4.05-4.20 (1H, m), 5.46 (1H, t, J=6.2 Hz), 7.45-7.55(1H, m), 8.24(1H, s), 8.60-8.65(1H, m), 11.29(1H, s).

### Reference Example 59

### 4-(((4-chloropyridin-2-yl)carbonyl)amino)-1-(tetrahydro-2H-pyran-2-yl)-1H-pyrazole-3-carboxylic acid

To a mixture of methyl 4-(((4-chloropyridin-2-yl)carbonyl)amino)-1-(tetrahydro-2H-pyran-2-yl)-1H-pyrazole-3-carboxylate obtained in Reference Example 58 (7.1 g, 19.5 mmol), tetrahydrofuran (100 ml) and methanol (100 ml), 2N aqueous sodium hydroxide solution (38.9 ml) was added, and the mixture was stirred at room temperature for 3 hr. The reaction mixture was neutralized with 6N hydrochloric acid (12.9 ml), diluted with water, and extracted with ethyl acetate. The extract was washed with water, dried over anhydrous magnesium sulfate, and evaporated under reduced pressure. The precipitated crystals were collected by filtration to give the title compound (4.95 g, yield 72%). melting point 310-312°C (decomposition).
¹H-NMR (d₆-DMSO):δ 1.50-1.80(3H, m), 1.90-2.00(2H, m), 2.00-2.20(1H, m), 3.60-3.75(1H, m), 3.90-4.05(1H, m), 5.56(1H, d, J=12.0 Hz), 7.80-7.90(1H, m), 8.16(1H, s), 8.55(1H, s), 8.72(1H, d, J=5.4 Hz), 11.15(1H, s), 13.28(1H, s).

In the following Examples, liquid chromatography-mass spectrometry spectrum (LC-MS), preparative liquid chromatography and nuclear magnetic resonance spectrum (NMR) were measured under the following conditions.

LC-MS measurement device: Waters Corporation LC-MS system (HPLC part: Agilent Technologies HP1100, MS part: Micromass ZMD), column: CAPCELL PAK C18UG120, S-3 µm, 1.5x35 mm (Shiseido Co., Ltd.), solvent: SOLUTION A; 0.05% trifluoroacetic acid-containing water, SOLUTION B; 0.04% trifluoroacetic acid-containing acetonitrile, gradient cycle: 0.00 min (SOLUTION A/SOLUTION B=90/10), 2.00 min (SOLUTION A/SOLUTION B=5/95), 2.75 min (SOLUTION A/SOLUTION B=5/95), 2.76 min (SOLUTION A/SOLUTION B=90/10), 3.60 min (SOLUTION A/SOLUTION B 90/10), injection volume: 2 µL, flow rate: 0.5 mL/min, detection method: UV 220 nm, MS conditions ionization method: ESI
preparative liquid chromatography (preparative HPLC) instrument: Gilson high throughput purification system, column:
YMC CombiPrep ODS-A S-5 µm, 50×20 mm
solvent: SOLUTION A; 0.1% trifluoroacetic acid-containing water, SOLUTION B; 0.1% trifluoroacetic acid-containing acetonitrile, gradient cycle: 0.00 min (SOLUTION A/SOLUTION B=95/5), 1.00 min (SOLUTION A/SOLUTION B=95/5), 5.20 min (SOLUTION A/SOLUTION B=5/95), 6.40 min (SOLUTION A/SOLUTION B=5/95), 6.50 min (SOLUTION A/SOLUTION B=95/5), 6.60 min (SOLUTION A/SOLUTION B=95/5)
flow rate: 20 mL/min, detection method: UV 220 nm

NMR measurement device: Varian, Inc. Varian gemini 300 (300 MHz), Bruker BioSpin K.K. AVANCE 300.

### Example 1

### N-isopropyl-4-((phenoxyacetyl)amino)-1H-pyrazole-3-carboxamide

To a solution of 4-amino-N-isopropyl-1H-pyrazole-3-carboxamide obtained in Reference Example 11 (10.0 mg, 0.06 mmol) in dichloromethane (0.5 mL)-DMF (0.5 mL), phenoxyacetic acid (10.9 mg, 0.072 mmol), diisopropylcarbodiimide (11.3 µL, 0.072 mmol) and HOBt (11.0 mg, 0.072 mmol) were added at room temperature. After 12 hr, the solvent was evaporated under reduced pressure, and the residue was dissolved in dimethylsulfoxide (1 mL). The mixture was purified by preparative HPLC to give the title compound 11.1 mg (yield 61%) as amorphous.
LC-MS analysis: purity 97% (retention time: 1.86 min).
MS(ESI+):303(M+H).

### Examples 2 - 162

Amine derivative moieties having 8 kinds of pyrazole skeleton (B-1 - B-8) indicated in Table 2, synthesized in Reference Example 10 to Reference Example 17 were reacted with 23 kinds of commercially available carboxylic acids (R⁴-CO₂H) shown in Table 1 in the same manner as in Example 1, which was followed by work-up, to give the title compound shown in Table 3-1 to Table 3-11.

**Table 1**

| |
|---|
| R⁴-COOH |

| |
|---|
| 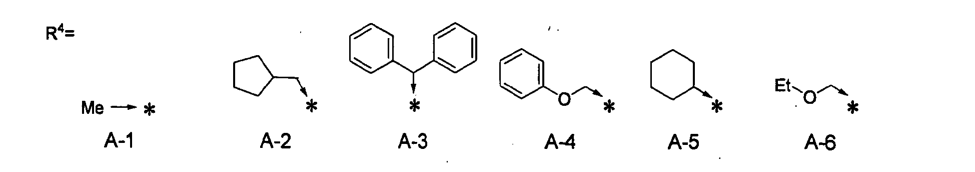 |
| 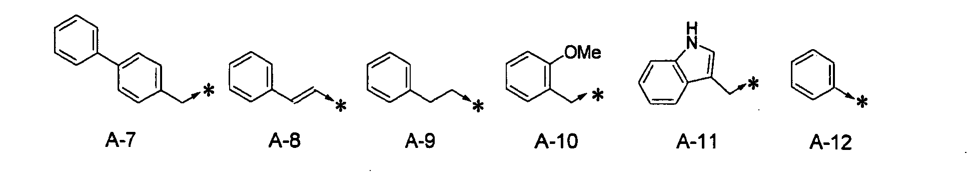 |
| 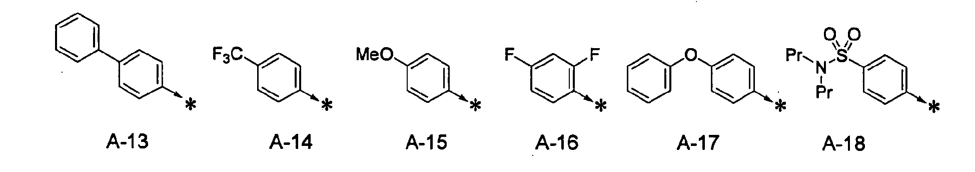 |
| 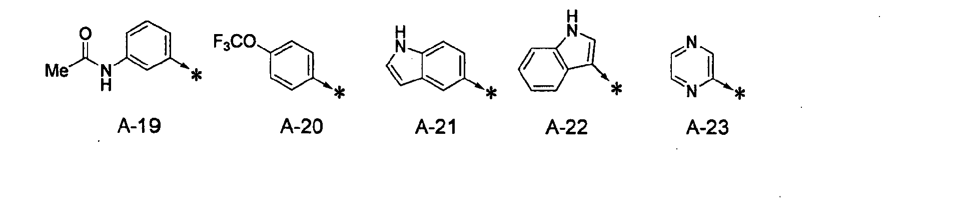 |

**Table 2**

| | | | |
|---|---|---|---|
| 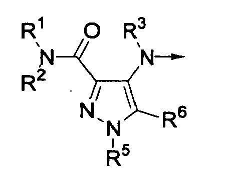 | | | |
| 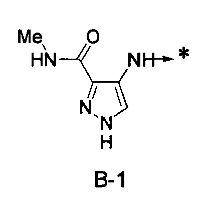 | 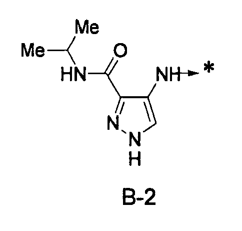 | 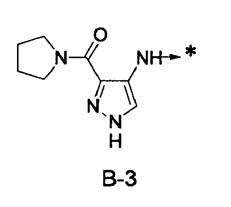 | 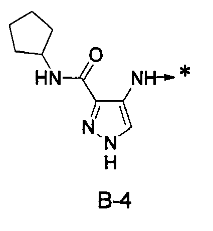 |
| 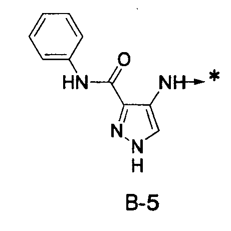 | 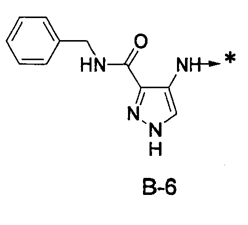 | 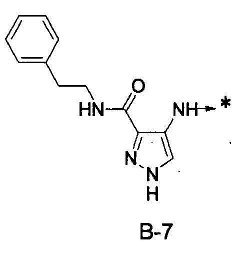 | 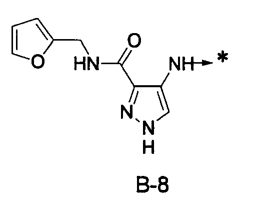 |

**Table 3-1**

| Ex. No. | 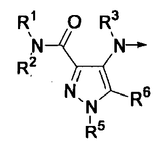 | R⁴ | compound name | MS (M⁺+1) | yield (mg) | yield (%) | retention time (min) | purity |
|---|---|---|---|---|---|---|---|---|
| 2 | B1 | A2 | 4-[(cyclopentylacetyl)amino]-N-methyl-1H-pyrazole-3-carboxamide | 251 | 5.4 | 36.0 | 1.7 | 96% |
| 3 | B1 | A3 | 4-[(diphenylacetyl)amino]-N-methyl-1H-pyrazole-3-carboxamide | 335 | 10.3 | 51.4 | 1.9 | 96% |
| 4 | B1 | A4 | N-methyl-4-[(phenoxyacetyl)amino]-1H-pyrazole-3-carboxamide | 275 | 2.2 | 13.4 | 1.6 | 84% |
| 5 | B1 | A5 | 4-[(cyclohexylcarbonyl)amino]-N-methyl-1H-pyrazole-3-carboxamide | 251 | 1.2 | 8.0 | 1.7 | 84% |
| 6 | B1 | A6 | 4-[(ethoxyacetyl)amino]-N-methyl-1H-pyrazole-3-carboxamide | 227 | 7.0 | 51.6 | 1.2 | 96% |
| 7 | B1 | A7 | 4-[(biphenyl-4-ylacetyl)amino]-N-methyl-1H-pyrazole-3-carboxamide | 335 | 10.3 | 51.4 | 2.0 | 95% |
| 8 | B1 | A8 | N-methyl-4-{[(2E)-3-phenylprop-2-enoyl]amino}-1H-pyrazole-3-carboxamide | 271 | 3.1 | 19.1 | 1.8 | 80% |
| 9 | B1 | A9 | N-methyl-4-[(3-phenylpropanoyl)amino]-1H-pyrazole-3-carboxamide | 273 | 9.5 | 58.2 | 1.7 | 99% |
| 10 | B1 | A14 | N-methyl-4-{[4-(trifluoromethyl)benzoyl]amino}-1H-pyrazole-3-carboxamide | 313 | 7.3 | 39.0 | 1.9 | 99% |
| 11 | B1 | A15 | 4-[(4-methoxybenzoyl)amino]-N-methyl-1H-pyrazole-3-carboxamide | 275 | 10.0 | 60.8 | 1.6 | 81% |
| 12 | B1 | A17 | N-methyl-4-[(4-phenoxybenzoyl)amino]-1H-pyrazole-3-carboxamide | 337 | 3.6 | 17.9 | 2.0 | 98% |
| 13 | B1 | A18 | 4-({4-[(dipropylamino)sulfonyl]benzoyl}amino)-N-methyl-1H-pyrazole-3-carboxamide | 408 | 20.1 | 82.3 | 1.6 | 99% |
| 14 | B1 | A19 | 4-{[3-(acetylamino)benzoyl]amino}-N-methyl-1H-pyrazole-3-carboxamide | 302 | 7.2 | 39.9 | 1.5 | 91% |
| 15 | B1 | A20 | N-methyl-4-{[4-(trifluoromethoxy)benzoyl]amino}-1H-pyrazole-3-carboxamide | 329 | 9.2 | 46.7 | 1.9 | 97% |
| 16 | B1 | A23 | N-{3-[(methylamino)carbonyl]-1H-pyrazol-4-yl}pyrazine-2-carboxamide | 247 | 7.6 | 51.5 | 1.4 | 85% |

**Table 3-2**

| Ex. No. | 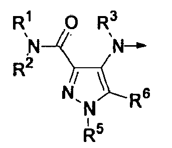 | R⁴ | compound name | MS (M⁺+1) | yield (mg) | yield (%) | retention time (min) | purity |
|---|---|---|---|---|---|---|---|---|
| 17 | B2 | A1 | 4-acetylamino-N-isopropyl-1H-pyrazole-3-carboxamide | 211 | 7.8 | 61.9 | 1.3 | 99% |
| 18 | B2 | A2 | 4-[(cyclopentylacetyl)amino]-N-isopropyl-1H-pyrazole-3-carboxamide | 279 | 7.2 | 43.1 | 1.9 | 99% |
| 19 | B2 | A3 | 4-[(diphenylacetyl)amino]-N-isopropyl-1H-pyrazole-3-carboxamide | 363 | 8.7 | 40.0 | 2.1 | 89% |
| 20 | B2 | A5 | 4-[(cyclohexylcarbonyl)amino]-N-isopropyl-1H-pyrazole-3-carboxamide | 279 | 4.1 | 24.6 | 1.8 | 81% |
| 21 | B2 | A6 | 4-[(ethoxyacetyl)amino]-N-isopropyl-1H-pyrazole-3-carboxamide | 255 | 8.3 | 54.4 | 1.6 | 99% |
| 22 | B2 | A7 | 4-[(biphenyl-4-ylacetyl)amino]-N-isopropyl-1H-pyrazole-3-carboxamide | 363 | 7.3 | 33.6 | 2.1 | 94% |
| 23 | B2 | A8 | N-isopropyl-4-{[(2E)-3-phenylprop-2-enoyl]amino}-1H-pyrazole-3-carboxamide | 299 | 5.5 | 30.7 | 2.0 | 98% |
| 24 | B2 | A9 | N-isopropyl-4-[(3-phenylpropanoyl)amino]-1H-pyrazole-3-carboxamide | 301 | 7.5 | 41.6 | 1.9 | 99% |
| 25 | B2 | A10 | N-isopropyl-4-{[(2-methoxyphenyl)acetyl]amino}-1H-pyrazole-3-carboxamide | 317 | 6.0 | 31.6 | 1.9 | 98% |
| 26 | B2 | A11 | 4-[(1H-indol-3-ylacetyl)amino]-N-isopropyl-1H-pyrazole-3-carboxamide | 326 | 1.8 | 9.2 | 1.8 | 93% |
| 27 | B2 | A12 | 4-(benzoylamino)-N-isopropyl-1H-pyrazole-3-carboxamide | 273 | 7.0 | 42.9 | 1.8 | 99% |
| 28 | B2 | A13 | 4-[(biphenyl-4-ylcarbonyl)amino]-N-isopropyl-1H-pyrazole-3-carboxamide | 349 | 3.1 | 14.8 | 2.2 | 89% |
| 29 | B2 | A14 | N-isopropyl-4-{[4-(trifluoromethyl)benzoyl]-amino}-1H-pyrazole-3-carboxamide | 341 | 10.7 | 52.4 | 2.1 | 80% |
| 30 | B2 | A15 | N-isopropyl-4-[(4-methoxybenzoyl)amino]-1H-pyrazole-3-carboxamide | 303 | 10.8 | 59.6 | 1.9 | 99% |
| 31 | B2 | A16 | 4-[(2,4-difluorobenzoyl)amino]-N-isopropyl-1H-pyrazole-3-carboxamide | 309 | 10.6 | 57.3 | 1.9 | 80% |

**Table 3-3**

| Ex. No. | 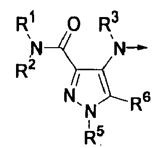 | R⁴ | compound name | MS (M⁺+1) | yield (mg) | yield (%) | retention time (min) | purity |
|---|---|---|---|---|---|---|---|---|
| 32 | B2 | A17 | N-isopropyl-4-[(4-phenoxybenzoyl)amino]-1H-pyrazole-3-carboxamide | 365 | 4.3 | 19.7 | 2.2 | 99% |
| 33 | B2 | A18 | 4-({4-[(dipropylamino)sulfonyl]-benzoyl}amino)-N-isopropyl-1H-pyrazole-3-carboxamide | 436 | 14.6 | 55.9 | 2.2 | 97% |
| 34 | B2 | A19 | 4-{[3-(acetylamino)benzoyl]amino}-N-isopropyl-1H-pyrazole-3-carboxamide | 330 | 10.2 | 51.6 | 1.6 | 84% |
| 35 | B2 | A20 | N-isopropyl-4-{[4-(trifluoromethoxy)benzoyl]-amino}-1H-pyrazole-3-carboxamide | 357 | 12.1 | 56.6 | 2.1 | 98% |
| 36 | B2 | A21 | N-{3-[(isopropylamino)carbonyl]-1H-pyrazol-4-yl}-1H-indole-5-carboxamide | 312 | 12.6 | 67.5 | 1.8 | 98% |
| 37 | B2 | A23 | N-{3-[(isopropylamino)carbonyl]-1H-pyrazol-4-yl}pyrazine-2-carboxamide | 275 | 11.3 | 68.7 | 1.6 | 99% |
| 38 | B3 | A1 | N-[3-(pyrrolidin-1-ylcarbonyl)-1H-pyrazol-4-yl]acetamide | 223 | 7.7 | 57.8 | 1.1 | 96% |
| 39 | B3 | A2 | 2-cyclopentyl-N-[3-(pyrrolidin-1-ylcarbonyl)-1H-pyrazol-4-yl]acetamide | 291 | 7.2 | 41.4 | 1.9 | 98% |
| 40 | B3 | A3 | 2,2-diphenyl-N-[3-(pyrrolidin-1-ylcarbonyl)-1H-pyrazol-4-yl]acetamide | 375 | 12.0 | 53.5 | 2.1 | 99% |
| 41 | B3 | A4 | 2-phenoxy-N-[3-(pyrrolidin-1-ylcarbonyl)-1H-pyrazol-4-yl] acetamide | 315 | 10.5 | 55.7 | 1.9 | 94% |
| 42 | B3 | A5 | N-[3-(pyrrolidin-1-ylcarbonyl)-1H-pyrazol-4-yl]cyclohexanecarboxamide | 291 | 7.2 | 41.4 | 1.9 | 99% |
| 43 | B3 | A6 | 2-ethoxy-N-[3-(pyrrolidin-1-ylcarbonyl)-1H-pyrazol-4-yl]acetamide | 267 | 7.4 | 46.3 | 1.6 | 99% |
| 44 | B3 | A7 | 2-biphenyl-4-yl-N-[3-(pyrrolidin-1-ylcarbonyl)-1H-pyrazol-4-yl]acetamide | 375 | 10.2 | 45.4 | 2.1 | 98% |
| 45 | B3 | A8 | (2E)-3-phenyl-N-[3-(pyrrolidin-l-ylcarbonyl)-1H-pyrazol-4-yl]acrylamide | 311 | 8.8 | 47.3 | 1.9 | 93% |
| 46 | B3 | A9 | 3-phenyl-N-[3-(pyrolidin-1-ylcarbonyl)-1H-pyrazol-4-yl]propanamide | 313 | 7.4 | 39.5 | 1.8 | 99% |

**Table 3-4**

| Ex. No. | 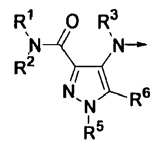 | R⁴ | compound name | MS (M⁺+1) | yield (mg) | yield (%) | retention time (min) | purity |
|---|---|---|---|---|---|---|---|---|
| 47 | B3 | A10 | 2-(2-methoxyphenyl)-N-[3-(pyrrolidin-1-ylcarbonyl)-1H-pyrazol-4-yl]acetamide | 329 | 9.2 | 46.7 | 1.8 | 99% |
| 48 | B3 | A11 | 2-(1H-indol-3-yl)-N-[3-(pyrrolidin-1-ylcarbonyl)-1H-pyrazol-4-yl]acetamide | 338 | 8.8 | 43.5 | 1.7 | 99% |
| 49 | B3 | A12 | N-[3-(pyrrolidin-1-ylcarbonyl)-1H-pyrazol-4-yl]benzamide | 285 | 6.4 | 37.5 | 1.8 | 99% |
| 50 | B3 | A14 | N-[3-(pyrrolidin-1-ylcarbonyl)-1H-pyrazol-4-yl]-4-(trifluoromethyl)benzamide | 353 | 0.6 | 2.8 | 2.1 | 94% |
| 51 | B3 | A15 | 4-methoxy-N-[3-(pyrrolidin-1-ylcarbonyl)-1H-pyrazol-4-yl]benzamide | 315 | 8.2 | 43.5 | 1.8 | 99% |
| 52 | B3 | A16 | 2,4-difluoro-N-[3-(pyrrolidin-1-ylcarbonyl)-1H-pyrazol-4-yl]benzamide | 321 | 10.8 | 56.2 | 1.9 | 99% |
| 53 | B3 | A17 | 4-phenoxy-N-[3-(pyrrolidin-1-ylcarbonyl)-1H-pyrazol-4-yl]benzamide | 377 | 1.8 | 8.0 | 2.2 | 99% |
| 54 | B3 | A19 | 3-(acetylamino)-N-[3-(pyrrolidin-1-ylcarbonyl)-1H-pyrazol-4-yl] benzamide | 342 | 10.4 | 50.8 | 1.6 | 97% |
| 55 | B3 | A20 | N-[3-(pyrrolidin-1-ylcarbonyl)-1H-pyrazol-4-yl]-4-(trifluoromethoxy)benzamide | 369 | 7.3 | 33.1 | 2.1 | 98% |
| 56 | B3 | A21 | N-[3-(pyrrolidin-1-ylcarbonyl)-1H-pyrazol-4-yl]-1H-indole-5-carboxamide | 324 | 9.2 | 47.5 | 1.8 | 99% |
| 57 | B3 | A22 | N-[3-(pyrrolidin-1-ylcarbonyl)-1H-pyrazol-4-yl]-1H-indole-3-carboxamide | 324 | 1.7 | 8.8 | 1.8 | 89% |
| 58 | B3 | A23 | N-[3-(pyrrolidin-1-ylcarbonyl)-1H-pyrazol-4-yl]pyrazine-2-carboxamide | 287 | 7.0 | 40.8 | 1.5 | 99% |
| 59 | B4 | A1 | 4-acetylamino-N-cyclopentyl-1H-pyrazole-3-carboxamide | 237 | 8.0 | 56.5 | 1.4 | 97% |
| 60 | B4 | A2 | 4-[(cyclopentylacetyl)amino]-N-cyclopentyl-1H-pyrazole-3-carboxamide | 305 | 3.8 | 20.8 | 2.1 | 98% |
| 61 | B4 | A3 | N-cyclopentyl-4-[(diphenylacetyl)amino]-1H-pyrazole-3-carboxamide | 389 | 9.1 | 39.1 | 2.2 | 98% |

**Table 3-5**

| Ex. No. | 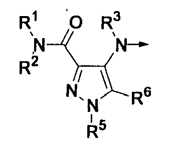 | R⁴ | compound name | MS (M⁺+1) | yield (mg) | yield (%) | retention time (min) | purity |
|---|---|---|---|---|---|---|---|---|
| 62 | B4 | A4 | N-cyclopentyl-4-[(phenoxyacetyl)amino]-1H-pyrazole-3-carboxamide | 329 | 11.6 | 58.9 | 2.0 | 99% |
| 63 | B4 | A5 | 4-[(cyclohexylcarbonyl)amino]-N-cyclopentyl-1H-pyrazole-3-carboxamide | 305 | 6.3 | 34.5 | 2.1 | 99% |
| 64 | B4 | A6 | N-cyclopentyl-4-[(ethoxyacetyl)amino]-1H-pyrazole-3-carboxamide | 281 | 8.8 | 52.4 | 1.8 | 99% |
| 65 | B4 | A7 | 4-[(biphenyl-4-ylacetyl)amino]-N-cyclopentyl-1H-pyrazole-3-carboxamide | 389 | 10.0 | 42.9 | 2.2 | 99% |
| 66 | B4 | A8 | N-cyclopentyl-4-{[(2E)-3-phenylprop-2-enoyl]amino}-1H-pyrazole-3-carboxamide | 325 | 10.5 | 54.0 | 2.1 | 94% |
| 67 | B4 | A9 | N-cyclopentyl-4-[(3-phenylpropanoyl)amino]-1H-pyrazole-3-carboxamide | 327 | 9.5 | 48.5 | 2.0 | 99% |
| 68 | B4 | A10 | N-cyclopentyl-4-{[(2-methoxyphenyl)acetyl]-amino}-1H-pyrazole-3-carboxamide | 343 | 8.4 | 40.9 | 2.0 | 99% |
| 69 | B4 | A11 | 4-[(1H-indol-3-ylacetyl)amino]-N-cyclopentyl-1H-pyrazole-3-carboxamide | 352 | 11.6 | 55.1 | 1.9 | 90% |
| 70 | B4 | A12 | 4-(benzoylamino)-N-cyclopentyl-1H-pyrazole-3-carboxamide | 299 | 10.6 | 59.3 | 2.0 | 99% |
| 71 | B4 | A14 | N-cyclopentyl-4-{[4-(trifluoromethyl)-benzoyl]amino}-1H-pyrazole-3-carboxamide | 367 | 4.2 | 19.1 | 2.2 | 99% |
| 72 | B4 | A15 | N-cyclopentyl-4-[(4-methoxybenzoyl)amino]-1H-pyrazole-3-carboxamide | 329 | 10.9 | 55.4 | 2.0 | 81% |
| 73 | B4 | A16 | N-cyclopentyl-4-[(2,4-difluorobenzoyl)amino]-1H-pyrazole-3-carboxamide | 335 | 12.2 | 60.9 | 2.0 | 97% |
| 74 | B4 | A18 | N-cyclopentyl-4-({4-[(dipropylamino)-sulfonyl]benzoyl}amino)-1H-pyrazole-3-carboxamide | 462 | 14.4 | 52.0 | 2.0 | 83% |
| 75 | B4 | A19 | 4-{[3-(acetylamino)benzoyl]amino}-N-cyclopentyl-1H-pyrazole-3-carboxamide | 356 | 12.5 | 58.7 | 1.8 | 99% |
| 76 | B4 | A20 | N-cyclopentyl-4-{[4-(trifluoromethoxy)-benzoyl]amino}-1H-pyrazole-3-carboxamide | 383 | 3.6 | 15.7 | 2.2 | 99% |

**Table 3-6**

| Ex. No. | 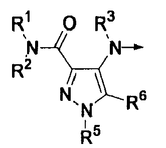 | R⁴ | compound name | MS(M⁺+1) | yield (mg) | yield (%) | retention time (min) | purity |
|---|---|---|---|---|---|---|---|---|
| 77 | B4 | A21 | N-{3-[(cyclopentylamino)carbonyl]-1H-pyrazol-4-yl}-1H-indole-5-carboxamide | 338 | 10.5 | 51.9 | 1.9 | 93% |
| 78 | B4 | A22 | N-{3-[(cyclopentylamino)carbonyl]-1H-pyrazol-4-yl}-1H-indole-3-carboxamide | 338 | 3.6 | 17.8 | 1.9 | 99% |
| 79 | B4 | A23 | N-{3-[(cyclopentylamino)carbonyl]-1H-pyrazol-4-yl}pyrazine-2-carboxamide | 301 | 12.3 | 68.3 | 1.7 | 96% |
| 80 | B5 | A1 | 4-acetylamino-N-phenyl-1H-pyrazole-3-carboxamide | 245 | 6.9 | 47.1 | 1.6 | 98% |
| 81 | B5 | A2 | 4-[(cyclopentylacetyl)amino]-N-phenyl-1H-pyrazole-3-carboxamide | 313 | 5.8 | 31.0 | 2.2 | 92% |
| 82 | B5 | A3 | 4-[(diphenylacetyl)amino]-N-phenyl-1H-pyrazole-3-carboxamide | 397 | 4.9 | 20.6 | 2.2 | 99% |
| 83 | B5 | A5 | N-phenyl-4-[(phenoxyacetyl)amino]-1H-pyrazole-3-carboxamide | 337 | 11.6 | 57.5 | 2.1 | 98% |
| 84 | B5 | A6 | 4-[(cyclohexylcarbonyl)amino]-N-phenyl-1H-pyrazole-3-carboxamide | 313 | 8.2 | 43.8 | 2.2 | 99% |
| 85 | B5 | A7 | 4-[(ethoxyacetyl)amino]-N-phenyl-1H-pyrazole-3-carboxamide | 289 | 1.2 | 6.9 | 1.9 | 95% |
| 86 | B5 | A9 | N-phenyl-4-{[(2E)-3-phenylprop-2-enoyl]amino}-1H--pyrazole-3-carboxamide | 333 | 7.1 | 35.6 | 2.1 | 96% |
| 87 | B5 | A10 | N-phenyl-4-[(3-phenylpropanoyl)amino]-1H-pyrazole-3-carboxamide | 335 | 7.2 | 35.9 | 2.1 | 95% |
| 88 | B5 | A11 | 4-{[(2-methoxyphenyl)acetyl] amino}-N-phenyl-1H-pyrazole-3-carboxamide | 351 | 8.5 | 40.5 | 2.1 | 99% |
| 89 | B5 | A12 | 4-[(1H-indol-3-ylacetyl)amino]-N-phenyl-1H-pyrazole-3-carboxamide | 360 | 1.0 | 4.6 | 2.1 | 85% |
| 90 | B5 | A13 | 4-(benzoylamino)-N-phenyl-1H-pyrazole-3-carboxamide | 307 | 10.1 | 55.0 | 2.1 | 98% |
| 91 | B5 | A15 | N-phenyl-4-{[4-(trifluoromethyl)benzoyl]-amino}-1H-pyrazole-3-carboxamide | 375 | 9.5 | 42.3 | 2.0 | 83% |

**Table 3-7**

| Ex. No. | 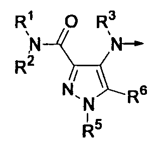 | R⁴ | compound name | Ms (M⁺+1) | yield (mg) | yield (%) | retention time (min) | purity |
|---|---|---|---|---|---|---|---|---|
| 92 | B5 | A16 | 4-[(4-methoxybenzoyl)amino]-N-phenyl-1H-pyrazole-3-carboxamide | 337 | 10.6 | 52.6 | 2.1 | 93% |
| 93 | B5 | A17 | 4-[(2,4-difluorobenzoyl)amino]-N-phenyl-1H-pyrazole-3-carboxamide | 343 | 11.4 | 55.5 | 2.1 | 99% |
| 94 | B5 | A19 | 4-({4-[(dipropylamino)sulfonyl]benzoyl}-amino)-N-phenyl-1H-pyrazole-3-carboxamide | 470 | 14.6 | 51.9 | 2.3 | 80% |
| 95 | B5 | A20 | 4-{[3-(acetylamino)benzoyl]amino}-N-phenyl-1H-pyrazole-3-carboxamide | 364 | 12.6 | 57.8 | 1.9 | 99% |
| 96 | B5 | A21 | N-phenyl-4-{[4-(trifluoromethoxy)benzoyl]-amino}-1H-pyrazole-3-carboxamide | 391 | 11.4 | 48.7 | 2.3 | 80% |
| 97 | B5 | A23 | N-{3-[(phenylamino)carbonyl]-1H-pyrazol-4-yl}-1H-indole-3-carboxamide | 346 | 2.1 | 10.1 | 2.0 | 96% |
| 98 | B5 | A24 | N-{3-[(phenylamino)carbonyl]-1H-pyrazol-4-yl}pyrazine-2-carboxamide | 309 | 10.7 | 57.9 | 1.8 | 98% |
| 99 | B6 | A2 | N-benzyl-4-[(cyclopentylacetyl)amino]-1H-pyrazole-3-carboxamide | 327 | 11.0 | 56.2 | 2.1 | 97% |
| 100 | B6 | A3 | N-benzyl-4-[(diphenylacetyl)amino]-1H-pyrazole-3-carboxamide | 411 | 17.0 | 69.1 | 2.2 | 99% |
| 101 | B6 | A5 | N-benzyl-4-[(phenoxyacetyl)amino]-1H-pyrazole-3-carboxamide | 351 | 8.4 | 40.0 | 2.1 | 80% |
| 102 | B6 | A6 | N-benzyl-4-[(cyclohexylcarbonyl)amino]-1H-pyrazole-3-carboxamide | 327 | 7.9 | 40.4 | 2.1 | 98% |
| 103 | B6 | A7 | N-benzyl-4-[(ettioxyacetyl)amino]-1H-pyrazole-3-carboxamide | 303 | 10.9 | 60.1 | 1.8 | 99% |
| 104 | B6 | A8 | N-benzyl-4-[(biphenyl-4-ylacetyl)amino]-1H-pyrazole-3-carboxamide | 411 | 10.2 | 41.4 | 2.2 | 97% |
| 105 | B6 | A9 | N-benzyl-4-{[(2E)-3-phenylprop-2-enoyl]amino}-1H-pyrazole-3-carboxamide | 347 | 5.3 | 25.5 | 2.1 | 83% |
| 106 | B6 | A10 | N-benzyl-4-[(3-phenylpropanoyl)amino]-1H-pyrazole-3-carboxamide | 349 | 9.8 | 46.9 | 2.1 | 99% |

**Table 3-8**

| Ex. No. | 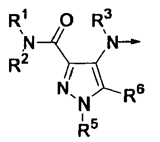 | R⁴ | compound name | MS(M⁺+1) | yield (mg) | yield (%) | retention time (min) | purity |
|---|---|---|---|---|---|---|---|---|
| 107 | B6 | A11 | N-benzyl-4-{[(2-methoxyphenyl)acetyl]amino}-1H-pyrazole-3-carboxamide | 365 | 9.0 | 41.2 | 2.0 | 99% |
| 108 | B6 | A12 | N-benzyl-4-[(1H-indol-3-ylacetyl)amino]-1H-pyrazole-3-carboxamide | 374 | 3.9 | 17.4 | 1.9 | 87% |
| 109 | B6 | A13 | 4-(benzoylamino)-N-benzyl-1H-pyrazole-3-carboxamide | 321 | 9.8 | 51.0 | 2.0 | 99% |
| 110 | B6 | A14 | N-benzyl-4-[(biphenyl-4-ylcarbonyl)amino]-1H-pyrazole-3-carboxamide | 397 | 12.9 | 54.3 | 2.3 | 96% |
| 111 | B6 | A15 | N-benzyl-4-{[4-(trifluoromethyl])benzoyl]-amino}-1H-pyrazole-3-carboxamide | 389 | 10.8 | 46.4 | 2.2 | 97% |
| 112 | B6 | A16 | N-benzyl-4-[(4-methoxybenzoyl)amino]-1H-pyrazole-3-carboxamide | 351 | 9.5 | 45.2 | 2.0 | 87% |
| 113 | B6 | A17 | N-benzyl-4-[(2,4-difluorobenzoyl)amino]-1H-pyrazole-3-carboxamide | 357 | 9.4 | 44.0 | 2.1 | 98% |
| 114 | B6 | A18 | N-benzyl-4-[(4-phenoxybenzoyl)amino]-1H-pyrazole-3-carboxamide | 413 | 13.4 | 54.2 | 2.3 | 99% |
| 115 | B6 | A19 | N-benzyl-4-{(4-[(dipropylamino)sulfonyl]-benzoyl}amino)-1H-pyrazole-3-carboxamide | 484 | 16.6 | 57.3 | 2.3 | 85% |
| 116 | B6 | A20 | 4-{[3-(acetylamino)benzoyl]amino}-N-benzyl-1H-pyrazole-3-carboxamide | 378 | 15.8 | 69.8 | 1.8 | 98% |
| 117 | B6 | A21 | N-benzyl-4-{[4-(trifluoromethoxy)benzoyl]-amino}-1H-pyrazole-3-carboxamide | 405 | 12.5 | 51.6 | 2.2 | 98% |
| 118 | B6 | A22 | N-{3-[(benzylamino)carbonyl]-1H-pyrazol-4-yl}-1H-indole-5-carboxamide | 360 | 0.2 | 0.9 | 1.9 | 83% |
| 119 | B6 | A24 | N-{3-[(benzylamino)carbonyl]-1H-pyrazol-4-yl}pyrazine-2-carboxamide | 323 | 7.3 | 37.8 | 1.8 | 99% |
| 120 | B7 | A1 | 9-acetylamino-N-(2-phenylethyl)-1H-pyrazole-3-carboxamide | 273 | 6.7 | 41.0 | 1.7 | 95% |
| 121 | B7 | A2 | 9-[(cyclopentylacetyl)amino]-N-(2-phenylethyl)-1H-pyrazole-3-carboxamide | 341 | 15.1 | 74.0 | 2.1 | 99% |

**Table 3-9**

| Ex. No. | 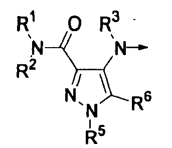 | R⁴ | compound name | MS (M⁺+1) | yield (mg) | yield (%) | retention time (min) | purity |
|---|---|---|---|---|---|---|---|---|
| 122 | B7 | A3 | 4-[(diphenylacetyl)amino]-N-(2-phenylethyl)-1H-pyrazole-3-carboxamide | 425 | 18.8 | 73.9 | 2.2 | 88% |
| 123 | B7 | A5 | 4-[(phenoxyacetyl)amino]-N-(2-phenylethyl)-1H-pyrazole-3-carboxamide | 365 | 13.5 | 61.8 | 2.1 | 99% |
| 124 | B7 | A6 | 4-[(cyclohexylcarbonyl)amino]-N-(2-phenylethyl)-1H-pyrazole-3-carboxamide | 341 | 8.3 | 40.7 | 2.1 | 97% |
| 125 | B7 | A7 | 4-[(ethoxyacetyl)amino]-N-(2-phenylethyl)-1H-pyrazole-3-carboxamide | 317 | 13.3 | 70.1 | 1.9 | 99% |
| 126 | B7 | A8 | 4-[(biphenyl-4-ylacetyl)amino]-N-(2-phenylethyl)-1H-pyrazole-3-carboxamide | 425 | 13.2 | 51.9 | 2.3 | 83% |
| 127 | B7 | A9 | N-(2-phenylethyl)-4-{[(2E)-3-phenylprop-2-enoyl]amino}-1H-pyrazole-3-carboxamide | 361 | 12.5 | 57.8 | 2.2 | 96% |
| 128 | B7 | A10 | N-(2-phenylethyl)-4-[(3-phenylpropanoyl)amino]-1H-pyrazole-3-carboxamide | 363 | 13.3 | 61.2 | 2.1 | 99% |
| 129 | B7 | A11 | 4-{[(2-methoxyphenyl)acetyl]amino}-N-(2-phenylethyl)-1H-pyrazole-3-carboxamide | 379 | 12.2 | 53.8 | 2.1 | 99% |
| 130 | B7 | A12 | 4-[(1H-indol-3-ylacetyl)amino]-N-(2-phenylethyl)-1H-pyrazole-3-carboxamide | 388 | 9.5 | 40.9 | 2.0 | 89% |
| 131 | B7 | A13 | 4-benzoylamino-N-(2-phenylethyl)-1H-pyrazole-3-carboxamide | 335 | 6.7 | 33.4 | 2.1 | 99% |
| 132 | B7 | A15 | N-(2-phenylethyl)-4-{[4-(trifluoromethyl)benzoyl]-amino}-1H-pyrazole-3-carboxamide | 403 | 9.1 | 37.7 | 2.3 | 99% |
| 133 | B7 | A16 | 4-[(4-methoxybenzoyl)amino]-N-(2-phenylethyl)-1H-pyrazole-3-carboxamide | 365 | 5.9 | 27.0 | 2.1 | 93% |
| 134 | B7 | A17 | 4-[(2,4-difluorobenzoyl)amino]-N-(2-phenylethyl)-1H-pyrazole-3-carboxamide | 371 | 5.2 | 23.4 | 2.1 | 96% |
| 135 | B7 | A18 | 4-[(4-phenoxybenzoyl)amino]-N-(2-phenylethyl)-1H-pyrazole-3-carboxamide | 427 | 8.4 | 32.9 | 2.3 | 84% |
| 136 | B7 | A19 | 4-({4-[(dipropylamino)sulfonyl]benzoyl}amino)-N-(2-phenylethyl)-1H-pyrazole-3-carboxamide | 498 | 12.4 | 41.6 | 2.3 | 80% |

**Table 3-10**

| Ex. No. | 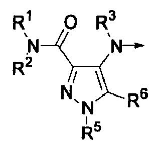 | R⁴ | compound name | MS (M⁺+1) | yield (mg) | yield (%) | retention time (min) | purity |
|---|---|---|---|---|---|---|---|---|
| 137 | B7 | A20 | 4-{[3-(acetylamino)benzoyl]amino}-N-(2-phenylethyl)-1H-pyrazole-3-carboxamide | 392 | 7.0 | 29.8 | 1.9 | 94% |
| 138 | B7 | A21 | N-(2-phenylethyl)-4-{[4-(trifluoromethoxy)-benzoyl]amino}-1H-pyrazole-3-carboxamide | 419 | 7.2 | 28.7 | 2.3 | 86% |
| 139 | B7 | A22 | N-(3-{[(2-phenylethyl)amino]carbonyl}-1H-pyrazol-4-yl)-1H-indole-5-carboxamide | 374 | 4.6 | 20.5 | 2.0 | 99% |
| 140 | B7 | A23 | N-(3-{[(2-phenylethyl)amino]carbonyl}-1H-pyrazol-4= yl)-1H-indole-3-carboxamid | 374 | 2.4 | 10.7 | 2.0 | 99% |
| 141 | B7 | A24 | N-(3-{[(2-phenylethyl)amino]carbonyl}-1H-pyrazol-4-yl)pyrazine-2-carboxamide | 337 | 6.8 | 33.7 | 1.9 | 99% |
| 142 | B8 | A1 | 4-acetylamino-N-(2-furylmethyl)-1H-pyrazole-3-carboxamide | 249 | 9.0 | 60.5 | 1.4 | 99% |
| 143 | B8 | A2 | 4-[(cyclopentylacetyl)amino]-N-(2-furylmethyl)-1H-pyrazole-3-carboxamide | 317 | 13.1 | 69.1 | 2.0 | 99% |
| 144 | B8 | A3 | 4-[(diphenylacetyl)amino]-N-(2-furylmethyl)-1H-pyrazole-3-carboxamide | 401 | 14.6 | 60.8 | 2.1 | 98% |
| 145 | B8 | A4 | N-(2-furylmethyl)-4-[(phenoxyacetyl)amino]-1H-pyrazole-3-carboxamide | 341 | 10.9 | 53.4 | 1.9 | 87% |
| 146 | B8 | A5 | 4-[(cyclohexylcarbonyl)amino]-N-(2-furylmethyl)-1H-pyrazole-3-carboxamide | 317 | 6.6 | 34.8 | 1.9 | 96% |
| 147 | B8 | A6 | 4-[(ethoxyacetyl)amino]-N-(2-furylmethyl)-1H-pyrazole-3-carboxamide | 293 | 11.3 | 64.5 | 1.7 | 99% |
| 148 | B8 | A8 | N-(2-furylmethyl)-4-{[(2E)-3-phenylprop-2-enoyl]amino}-1H-pyrazole-3-carboxamide | 337 | 10.8 | 53.6 | 2.0 | 99% |
| 149 | B8 | A9 | N-(2-furylmethyl)-4-[(3-phenylpropanoyl)amino]-1H-pyrazole-3-carboxamide | 339 | 11.9 | 58.7 | 2.0 | 99% |
| 150 | B8 | A10 | N-(2-furylmethyl)-4-{[(2-methoxyphenyl)acetyl]-amino}-1H-pyrazole-3-carboxamide | 355 | 10.1 | 47.5 | 1.9 | 95% |
| 151 | B8 | A11 | N-(2-furylmethyl)-4-[(1H-indol-3-ylacetyl)amino]-1H-pyrazole-3-carboxamide | 364 | 11.5 | 52.8 | 1.8 | 98% |

**Table 3-11**

| Ex. No. | 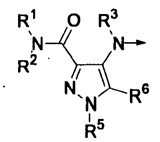 | R⁴ | compound name | MS (M⁺+1) | yield (mg) | yield (%) | retention time (min) | purity |
|---|---|---|---|---|---|---|---|---|
| 152 | B8 | A12 | 4-benzoylamino-N-(2-furylmethyl)-1H-pyrazole-3-carboxamide | 311 | 6.3 | 33.9 | 1.9 | 98% |
| 153 | B8 | A13 | 4-[(biphenyl-4-ylcarbonyl)amino]-N-(2-furylmethyl)-1H-pyrazole-3-carboxamide | 387 | 5.6 | 24.2 | 2.2 | 98% |
| 154 | B8 | A14 | N-(2-furylmethyl)-4-{[4-(trifluoromethyl)-benzoyl]amino}-1H-pyrazole-3-carboxamide | 379 | 11.1 | 48.9 | 2.1 | 99% |
| 155 | B8 | A15 | N-(2-furylmethyl)-4-[(4-methoxybenzoyl)amino]-1H-pyrazole-3-carboxamide | 341 | 12.2 | 59.8 | 1.9 | 87% |
| 156 | B8 | A16 | 4-[(2,4-difluorobenzoyl)amino]-N-(2-furylmethyl)-1H-pyrazole-3-carboxamid | 347 | 12.2 | 58.8 | 2.0 | 99% |
| 157 | B8 | A17 | N-(2-furylmethyl)-4-[(4-phenoxybenzoyl)amino]-1H-pyrazole-3-carboxamide | 403 | 12.9 | 53.5 | 2.2 | 80% |
| 158 | B8 | A18 | 4-({4-[(dipropylamino)sulfonyl]benzoyl}amino)-N-(2-furylmethyl)-1H-pyrazole-3-carboxamide | 474 | 17.7 | 62.3 | 2.2 | 99% |
| 159 | B8 | A19 | 4-{[3-(acetylamino)benzoyl]amino}-N-(2-furylmethyl)-1H-pyrazole-3-carboxamide | 368 | 11.2 | 50.8 | 1.7 | 97% |
| 160 | B8 | A20 | N-(2-furylmethyl)-4-{[4-(trifluoromethoxy)-benzoyl]amino}-1H-pyrazole-3-carboxamide | 395 | 12.3 | 52.0 | 2.1 | 99% |
| 161 | B8 | A21 | N-(3-{[(2-furylmethyl)amino]carbonyl}-1H-pyrazol-4-yl)-1H-indole-5-carboxamide | 350 | 8.9 | 42.5 | 1.8 | 80% |
| 162 | B8 | A23 | N-(3-{[(2-furylmethyl)amino]carbonyl}-1H-pyrazol-4-yl)pyrazine-2-carboxamide | 313 | 4.3 | 23.0 | 1.7 | 99% |

### Example 163

### N-(3-((cyclopentylamino)carbonyl)-1-trityl-1H-pyrazol-4-yl)-N-methylpyrazine-2-carboxamide

In the same manner as in Reference Example 22, the title compound was obtained using 4-(methyl(pyrazin-2-ylcarbonyl)amino)-1-trityl-1H-pyrazole-3-carboxylic acid obtained in Reference Example 31 and cyclopentylamine. yield 81%.
¹H-NMR (CDCl₃) :δ 1.26-1. 39 (2H, m), 1.57-1.65(4H, m), 1.92-2.00(2H, m), 3.43(1H, s), 4.08-4.26(1H, m), 6.49(1H, d, J=7.8 Hz), 6.91-6.94(5H, m), 7.16(1H, s), 7.24-7.37(10H, m), 8.31(1H, dd, J=1.8 Hz and J=2.4 Hz), 8.49(1H, d, J=2.4 Hz), 8.80(1H, d, J=1.8 Hz).

### Example 164

### N-(3-((cyclopentyl(methyl)amino)carbonyl)-1-trityl-1H-pyrazol-4-yl)pyrazine-2-carboxamide

In the same manner as in Reference Example 22, the title compound was obtained using 4-amino-N-cyclopentyl-N-methyl-1-trityl-1H-pyrazole-3-carboxamide obtained in Reference Example 35 and pyrazinecarboxylic acid. yield 77%.
¹H-NMR (CDCl₃):δ 1.44-1.71(6H, m), 1.89-1.94(2H, m), 2.96(3H, s), 5.21-5.31(1H, m), 7.14-7.19(5H, m), 7.26-7.33(10H, m), 8.40(1H, d, J=2.7 Hz), 8.68-8.73(1H, m), 9.37(1H d, J=1.2 Hz), 11.8(1H, s).

### Example 165

### 4-((biphenyl-4-sulfonyl)amino)-N-methyl-1H-pyrazole-3-carboxamide

To a solution of 4-amino-N-methyl-1H-pyrazole-3-carboxamide obtained in Reference Example 10 (8.4 mg, 0.06 mmol) in dichloromethane (0.5 ml)-DMF (0.5 ml), triethylamine (0.0125 mL, 0.09 mmol) and biphenyl-4-sulfonylchloride (18.2 mg, 0.072 mmol) were added at room temperature. After stirring for 12 hr, saturated aqueous sodium hydrogencarbonate solution (2.5 mL) was added, and the mixture was extracted with dichloromethane (2 mL). The extract was washed with saturated brine (2.5 mL), fractionated by PhaseSep tube (manufactured by Whatman), and concentrated under reduced pressure. The residue was dissolved in dimethylsulfoxide (1 mL), and the mixture was purified by preparative HPLC to give the title compound 6.4 mg (yield 30%) as amorphous.
LC-MS analysis :purity 96% (retention time :1.69 min).
MS(ESI+):357(M+H).
¹H-NMR (CDCl₃) : δ 2.90(3H, s), 6.70 (1H, brs), 7.28-7.90(9H, m), 7.79 (1H, s), 8.86 (1H, brs).

### Examples 166 - 336

Amine derivative moieties having 8 kinds of pyrazole skeletons (B-1 - B-8) shown in Table 2, synthesized in Reference Example 10 to Reference Example 17, were reacted with 23 kinds of commercially available sulfonyl chlorides (R⁴-SO₂Cl) shown in Table 4 in the same manner as in Example 165, which was followed by work-up, to give the title compound shown in Table 5-1 to Table 5-12.

**Table 4**

| |
|---|
| R⁴-SO₂Cl |
| 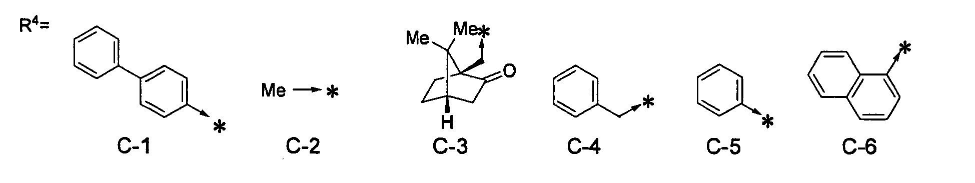 |
| 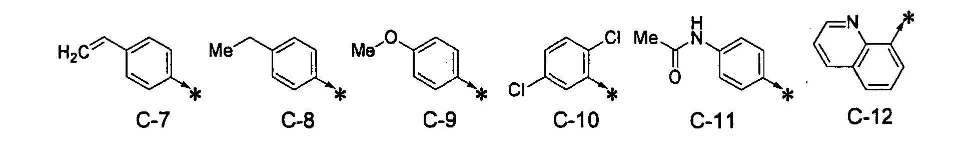 |
| 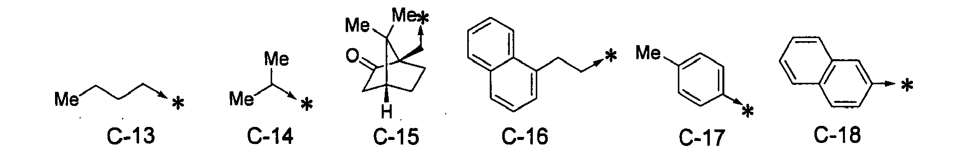 |
| 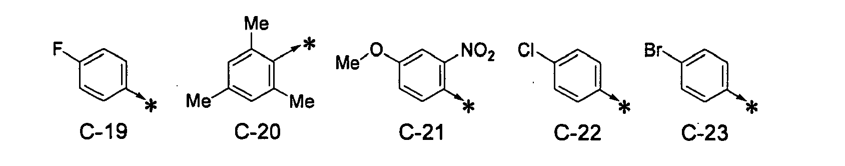 |

**Table 5-1**

| Ex. No. | 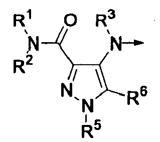 | R⁴ | compound name | MS (M⁺+1) | yield (mg) | yield (%) | retention time (min) | purity |
|---|---|---|---|---|---|---|---|---|
| 166 | B1 | C3 | N-methyl-4-[{(1R)-2-oxo-bornane-10-sulfonyl}amino]-1H-pyrazole-3-carboxamide | 355 | 4.4 | 20.7 | 1.4 | 93% |
| 167 | B1 | C4 | 4-[(benzylsulfonyl)amino]-N-methyl-1H-pyrazole-3-carboxamide | 295 | 2.3 | 13.0 | 1.3 | 91% |
| 168 | B1 | C5 | N-methyl-4-[(phenylsulfonyl)ami.no]-1H-pyrazole-3-carboxamide | 281 | 1.8 | 10.7 | 1.2 | 86% |
| 169 | B1 | C6 | N-methyl-4-[(1-naphthylsulfonyl)amino]-1H-pyrazole-3-carboxamide | 331 | 2.3 | 11.6 | 1.5 | 96% |
| 170 | B1 | C7 | N-methyl-4-{[(4-vinylphenyl)sulfonyl]amino}-1H-pyrazole-3-carboxamide | 307 | 1.6 | 8.7 | 1.4 | 98% |
| 171 | B1 | C8 | 4-{[(4-ethylphenyl)sulfonyl]amino}-N-methyl-1H-pyrazole-3-carboxamide | 309 | 6.3 | 34.1 | 1.5 | 99% |
| 172 | B1 | C9 | 4-{[(4-methoxyphenyl)sulfonyl]amino}-N-methyl-1H-pyrazole-3-carboxamide | 311 | 2.4 | 12.9 | 1.3 | 95% |
| 173 | B1 | C10 | 4-{[(2,5-dichlorophenyl)sulfonyl]amino}-N-methyl-1H-pyrazole-3-carboxamide | 349 | 5.8 | 27.8 | 1.5 | 93% |
| 174 | B1 | C12 | N-methyl-4-[(quinolin-8-ylsulfonyl)amino]-1H-pyrazole-3-carboxamide | 332 | 6.3 | 31.7 | 1.2 | 88% |
| 175 | B1 | C13 | 4-[(butylsulfonyl)amino]-N-methyl-1H-pyrazole-3-carboxamide | 261 | 5.6 | 35.9 | 1.2 | 90% |
| 176 | B1 | C15 | N-methyl-4-[{(1S)-2-oxo-bornane-10-sulfonyl}amino]-1H-pyrazole-3-carboxamide | 355 | 2.8 | 13.2 | 1.4 | 96% |
| 177 | B1 | C16 | N-methyl-4-({[2-(1-naphthyl)ethyl]sulfonyl}-amino)-1H-pyrazole-3-carboxamide | 359 | 1.1 | 5.1 | 1.7 | 99% |
| 178 | B1 | C17 | N-methyl-4-{[(4-methylphenyl)sulfonyl]amino}-1H-pyrazole-3-carboxamide | 295 | 0.1 | 0.6 | 1.4 | 93% |
| 179 | B1 | C18 | N-methyl-4-[(2-naphthylsulfonyl)amino]-1H-pyrazole-3-carboxamide | 331 | 1.4 | 7.1 | 1.5 | 97% |

**Table 5-2**

| Ex. No. | 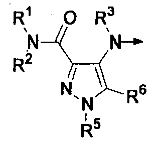 | R⁴ | compound name | MS (M⁺+1) | yield (mg) | yield (%) | retention time (min) | purity |
|---|---|---|---|---|---|---|---|---|
| 180 | B1 | C19 | 4-{[(4-fluorophenyl)sulfonyl]amino}-N-methyl-1H-pyrazole-3-carboxamide | 299 | 8.8 | 49.2 | 1.3 | 80% |
| 181 | B1 | C20 | 4-[(mesitylsulfonyl)amino]-N-methyl-1H-pyrazole-3-carboxamide | 323 | 8.1 | 41.9 | 1.6 | 99% |
| 182 | B1 | C21 | 4-{[(4-methoxy-2-nitrophenyl)sulfonyl]amino}-N-methyl-1H-pyrazole-3-carboxamide | 356 | 3.5 | 16.4 | 1.4 | 84% |
| 183 | B1 | C22 | 4-{[(4-chlorophenyl)sulfonyl]amino}-N-methyl-1H-pyrazole-3-carboxamide | 315 | 1.7 | 9.0 | 1.4 | 84% |
| 184 | B2 | C1 | 4-[(biphenyl-4-ylsulfonyl)amino]-N-isopropyl-1H-pyrazole-3-carboxamide | 385 | 6.5 | 28.2 | 1.8 | 98% |
| 185 | B2 | C2 | N-isopropyl-4-(methanesulfonyl)amino-1H-pyrazole-3-carboxamide | 247 | 2.4 | 16.3 | 1.6 | 85% |
| 186 | B2 | C4 | 4-[(benzylsulfonyl)amino]-N-isopropyl-1H-pyrazole-3-carboxamide | 323 | 2.9 | 15.0 | 1.6 | 97% |
| 187 | B2 | C5 | N-isopropyl-4-[(phenylsulfonyl)amino]-1H-pyrazole-3-carboxamide | 309 | 4.7 | 25.4 | 1.5 | 99% |
| 188 | B2 | C6 | N-isopropyl-4-[(1-naphthylsulfonyl)amino]-1H-pyrazole-3-carboxamide | 359 | 2.8 | 13.0 | 1.7 | 99% |
| 189 | B2 | C7 | N-isopropyl-4-{[(4-vinylphenyl)sulfonyl]amino}-1H-pyrazole-3-carboxamide | 335 | 1.7 | 8.5 | 1.6 | 97% |
| 190 | B2 | C8 | 4-[(4-ethylphenylsulfonyl)amino]-N-isopropyl-1H-pyrazole-3-carboxamide | 337 | 0.7 | 3.5 | 1.7 | 99% |
| 191 | B2 | C9 | N-isopropyl-4-{[(4-methoxyphenyl)sulfonyl]-amino}-1H-pyrazole-3-carboxamide | 339 | 8.4 | 41.4 | 1.5 | 96% |
| 192 | B2 | C10 | 4-{[(2,5-dichlorophenyl)sulfonyl]amino}-N-isopropyl--1H-pyrazole-3-carboxamide | 377 | 4.2 | 18.6 | 1.7 | 98% |
| 193 | B2 | C11 | 4-{[(4-acetamidophenyl)sulfonyl]amino}-N-isopropyl--1H-pyrazole-3-carboxamide | 366 | 8.9 | 40.6 | 1.3 | 96% |
| 194 | B2 | C12 | N-isopropyl-4-[(quinolin-8-ylsulfonyl)amino]-1H-pyrazole-3-carboxamide | 360 | 3.9 | 18.1 | 1.5 | 99% |

**Table 5-3**

| Ex. No. | 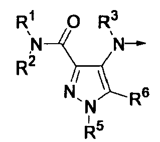 | R⁴ | compound name | MS (M⁺+1) | yield (mg) | yield (%) | retention time (min) | purity |
|---|---|---|---|---|---|---|---|---|
| 195 | B2 | C13 | 4-[(butylsulfonyl)amino]-N-isopropyl-1H-pyrazole-3-carboxamide | 289 | 3.9 | 22.6 | 1.5 | 98% |
| 196 | B2 | C14 | N-isopropyl-4-[(isopropylsulfonyl)amino]-1H-pyrazole-3-carboxamide | 275 | 0.7 | 4.3 | 1.3 | 96% |
| 197 | B2 | C15 | N-isopropyl-4-[{(1S)-2-oxo-bornane-10-sulfonyl}amino]-1H-pyrazole-3-carboxamide | 383 | 1.6 | 7.0 | 1.6 | 99% |
| 198 | B2 | C16 | N-isopropyl-4-({[2-(1-naphthyl)ethyl]sulfonyl}-amino)-1H-pyrazole-3-carboxamide | 387 | 1.3 | 5.6 | 1.8 | 97% |
| 199 | B2 | C17 | N-isopropyl-4-{[(4-methylphenyl)sulfonyl]amino}-1H-pyrazole-3-carboxamide | 323 | 5.4 | 27.9 | 1.6 | 98% |
| 200 | B2 | C18 | N-isopropyl-4-[(2-naphthylsulfonyl)amino]-1H-pyrazole-3-carboxamide | 359 | 2.7 | 12.6 | 1.7 | 99% |
| 201 | B2 | C19 | 4-[(4-fluorobenzenesulfonyl)amino]-N-isopropyl-1H-pyrazole-3-carboxamide | 327 | 8.0 | 40.9 | 1.5 | 95% |
| 202 | B2 | C20 | N-isopropyl-4-[(mesitylsulfonyl)amino]-1H-pyrazole-3-carboxamide | 351 | 4.2 | 20.0 | 1.8 | 97% |
| 203 | B3 | C1 | N-[3-(pyrrolidin-1-ylcarbonyl)-1H-pyrazol-4-yl]biphenyl-4-sulfonamide | 397 | 1.3 | 5.5 | 1.7 | 93% |
| 204 | B3 | C2 | N-[3-(pyrrolidin-1-ylcarbonyl)-1H-pyrazol-4-yl]methanesulfonamide | 259 | 3.6 | 23.2 | 1.6 | 89% |
| 205 | B3 | C3 | 1-{(1R)-2-oxo-bornan-10-yl}-N-[3-(pyrrolidin-1-ylcarbonyl)-1H-pyrazol-4-yl]sulfonamide | 395 | 6.9 | 29.2 | 1.6 | 98% |
| 206 | B3 | C4 | 1-phenyl-N-[3-(pyrrolidin-1-ylcarbonyl)-1H-pyrazol-4-yl]methanesulfonamide | 335 | 4.0 | 20.0 | 1.5 | 98% |
| 207 | B3 | C5 | N-[3-(pyrrolidin-1-yl-carbonyl)-1H-pyrazol-4-yl]benzenesulfonamide | 321 | 7.8 | 40.6 | 1.4 | 98% |
| 208 | B3 | C6 | N-[3-(pyrrolidin-1-ylcarbonyl)-1H-pyrazol-5-yl]naphthalene-1-sulfonamide | 371 | 7.3 | 32.9 | 1.6 | 99% |
| 209 | B3 | C7 | N-[3-(pyrrolidin-1-ylcarbonyl)-1H-pyrazol-5-yl]-4-vinylbenzenesulfonamide | 347 | 6.4 | 30.8 | 1.6 | 96% |

**Table 5-4**

| Ex. No. | 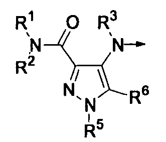 | R⁴ | compound name | MS (M⁺+1) | yield (mg) | yield (%) | retention time (min) | purity |
|---|---|---|---|---|---|---|---|---|
| 210 | B3 | C8 | 4-ethyl-N-[3-(pyrrolidin-1-ylcarbonyl)-1H-pyrazol-5-yl]benzenesulfonamide | 349 | 9.5 | 45.5 | 1.6 | 99% |
| 211 | B3 | C9 | 4-methoxy-N-[3-(pyrrolidin-1-ylcarbonyl)-1H-pyrazol-4-yl]benzenesulfonamide | 351 | 3.7 | 17.6 | 1.4 | 99% |
| 212 | B3 | C10 | 2,5-dichloro-N-[3-(pyrrolidin-1-ylcarbonyl)-1H-pyrazol-4-yl]benzenesulfonamide | 389 | 4.9 | 21.0 | 1.6 | 96% |
| 213 | B3 | C11 | 4-acetamido-N-[3-(pyrrolidin-1-ylcarbonyl)-1H-pyrazol-4-yl]benzenesulfonamide | 378 | 4.6 | 20.3 | 1.2 | 97% |
| 214 | B3 | C12 | N-[3-(pyrrolidin-1-ylcarbonyl)-1H-pyrazol-4-yl]quinoline-8-sulfonamide | 372 | 4.5 | 20.2 | 1.4 | 99% |
| 215 | B3 | C13 | N-[3-(pyrrolidin-1-ylcarbonyl)-1H-pyrazol-4-yl]butane-1-sulfonamide | 301 | 7.3 | 40.5 | 1.4 | 98% |
| 216 | B3 | C14 | N-[3-(pyrrolidin-1-ylcarbonyl)-1H-pyrazol-4-yl]propane-2-sulfonamide | 287 | 6.1 | 35.5 | 1.2 | 89% |
| 217 | B3 | C15 | 1-((1S)-2-oxo-bornan-10-yl)-N-[3-(pyrrolidin-1-ylcarbonyl)-1H-pyrazol-4-yl]sulfonamide | 395 | 7.2 | 30.4 | 1.6 | 96% |
| 218 | B3 | C16 | 2-(1-naphthyl)-N-[3-(pyrrolidin-1-ylcarbonyl)-1H-pyrazol-4-yl]ethanesulfonamide | 399 | 4.2 | 17.6 | 1.8 | 99% |
| 219 | B3 | C17 | 4-methyl-N-[3-(pyrrolidin-1-ylcarbonyl)-1H-pyrazol-4-yl]benzenesulfonamide | 335 | 3.7 | 18.5 | 1.5 | 98% |
| 220 | B3 | C18 | N-[3-(pyrrolidin-1-ylcarbonyl)-1H-pyrazol-4-yl]naphthalene-2-sulfonamide | 371 | 3.3 | 14.9 | 1.6 | 99% |
| 221 | B3 | C19 | 4-fluoro-N-[3-(pyrrolidin-1-ylcarbonyl)-1H-pyrazol-4-yl]benzenesulfonamide | 339 | 6.3 | 31.1 | 1.5 | 97% |
| 222 | B3 | C20 | N-[3-(pyrrolidin-1-ylcarbonyl)-1H-pyrazol-4-yl]-2,4,6-trimethyl-benzenesulfonamide | 363 | 1.3 | 6.0 | 1.7 | 84% |
| 223 | B3 | C21 | 4-methoxy-2-nitro-N-[3-(pyrrolidin-1-ylcarbonyl)-1H-pyrazol-4-yl]benzenesulfonamide | 396 | 4.2 | 17.7 | 1.5 | 97% |
| 224 | B3 | C22 | 4-chloro-N-[3-(pyrrolidin-1-ylcarbonyl)-1H-pyrazol-4-yl]benzenesulfonamide | 355 | 2.0 | 9.4 | 1.6 | 99% |

**Table 5-5**

| Ex. No. | 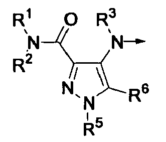 | R⁴ | compound name | MS (M⁺+1) | yield (mg) | yield (%) | retention time (min) | purity |
|---|---|---|---|---|---|---|---|---|
| 225 | B3 | C23 | 4-bromo-N-[3-(pyrrolidin-1-ylcarbonyl)-1H-pyrazol-4-yl]benzenesulfonamide | 399 | 4.1 | 17.2 | 1.6 | 99% |
| 226 | B4 | C1 | 4-[(biphenyl-4-ylsulfonyl)amino]-N-cyclopentyl-1H-pyrazole-3-carboxamide | 411 | 5.8 | 23.6 | 1.9 | 99% |
| 227 | B4 | C2 | N-cyclopentyl-4-methanesulfonylamino-1H-pyrazole-3-carboxamide | 273 | 1.4 | 8.6 | 1.6 | 90% |
| 228 | B4 | C3 | N-cyclopentyl-4-[{(1R)-2-oxo-bornane-10-sulfonyl}amino]-1H-pyrazole-3-carboxamide | 409 | 5.9 | 24.1 | 1.7 | 95% |
| 229 | B4 | C4 | 4-[(benzylsulfonyl)amino]-N-cyclopentyl-1H-pyrazole-3-carboxamide | 349 | 6.0 | 28.7 | 1.7 | 99% |
| 230 | B4 | C5 | N-cyclopentyl-4-[(phenylsulfonyl)amino]-1H-pyrazole-3-carboxamide | 335 | 6.8 | 33.9 | 1.6 | 98% |
| 231 | B4 | C6 | N-cyclopentyl-4-{[(1-naphthyl)sulfonyl]amino}-1H-pyrazole-3-carboxamide | 385 | 5.3 | 23.0 | 1.8 | 99% |
| 232 | B4 | C7 | N-cyclopentyl-4-{[(4-vinylphenyl)sulfonyl]-amino}-1H-pyrazole-3-carboxamide | 361 | 4.8 | 22.2 | 1.7 | 90% |
| 233 | B4 | C8 | N-cyclopentyl-4-{[(4-ethylphenyl)sulfonyl]-amino}-1H-pyrazole-3-carboxamide | 363 | 7.3 | 33.6 | 1.8 | 99% |
| 234 | B4 | C9 | N-cyclopentyl-4-{[(4-methoxylphenyl)-sulfonyl]amino}-1H-pyrazole-3-carboxamide | 365 | 0.9 | 4.1 | 1.6 | 99% |
| 235 | B4 | C10 | N-cyclopentyl-4-{[(2,5-dichlorophenyl)-sulfonyl]amino}-1H-pyrazole-3-carboxamide | 403 | 4.3 | 17.8 | 1.8 | 98% |
| 236 | B4 | C11 | 4-({[4-(acetylamino)phenyl]sulfonyl}amino)-N-cyclopentyl-1H-pyrazole-3-carboxamide | 392 | 5.9 | 25.1 | 1.4 | 96% |
| 237 | B4 | C12 | N-cyclopentyl-4-[(quinolin-8-ylsulfonyl)amino]-1H-pyrazole-3-carboxamide | 386 | 4.4 | 19.0 | 1.6 | 99% |
| 238 | B4 | C13 | 4-[(butylsulfonyl)amino]-N-cyclopentyl-1H-pyrazole-3-carboxamide | 315 | 5.8 | 30.8 | 1.7 | 99% |
| 239 | B4 | C14 | N-cyclopentyl-4-[(isopropylsulfonyl)amino]-1H-pyrazole-3-carboxamide | 301 | 4.6 | 25.5 | 1.5 | 88% |

**Table 5-6**

| Ex. No. | 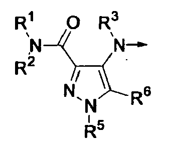 | R⁴ | compound name | MS (M⁺+1) | yield (mg) | yield (%) | retention time (min) | purity |
|---|---|---|---|---|---|---|---|---|
| 240 | B4 | C15 | N-cyclopentyl-4-[{(1S)-2-oxo-bornane-10-sulfonyl}amino]-1H-pyrazole-3-carboxamide | 409 | 4.5 | 18.4 | 1.7 | 94% |
| 241 | B4 | C16 | N-cyclopentyl-4-({[2-(1-naphthyl)ethyl]-sulfonyl}amino)-1H-pyrazole-3-carboxamide | 413 | 1.2 | 4.9 | 1.9 | 99% |
| 242 | B4 | C17 | N-cyclopentyl-4-{[(4-methylphenyl)sulfonyl]-amino}-1H-pyrazole-3-carboxamide | 349 | 5.6 | 26.8 | 1.7 | 97% |
| 243 | B4 | C18 | N-cyclopentyl-4-[(2-naphthylsulfonyl)amino]-1H-pyrazole-3-carboxamide | 385 | 7.6 | 33.0 | 1.8 | 99% |
| 244 | B4 | C19 | N-cyclopentyl-4-{[(4-fluorophenyl)sulfonyl]-amino}-1H-pyrazole-3-carboxamide | 353 | 5.7 | 27.0 | 1.7 | 95% |
| 245 | B4 | C20 | N-cyclopentyl-4-{[(2,4,6-trimethylphenyl)-sulfonyl]amino}-1H-pyrazole-3-carboxamide | 377 | 3.1 | 13.7 | 1.9 | 97% |
| 246 | B4 | C21 | N-cyclopentyl-4-{[(4-methoxy-2-nitrophenyl)-sulfonyl]amino}-1H-pyrazole-3-carboxamide | 410 | 7.0 | 28.5 | 1.7 | 94% |
| 247 | B4 | C22 | N-cyclopentyl-4-{[(4-chlorophenyl)sulfonyl]-amino}-1H-pyrazole-3-carboxamide | 369 | 5.8 | 26.3 | 1.8 | 98% |
| 248 | B4 | C23 | N-cyclopentyl-4-{[(4-bromophenyl)sulfonyl]-amino}-1H-pyrazole-3-carboxamide | 413 | 5.8 | 23.5 | 1.8 | 98% |
| 249 | B5 | C1 | 4-[(biphenyl-4-ylsulfonyl)amino]-N-phenyl-1H-pyrazole-3-carboxamide | 419 | 6.1 | 24.3 | 1.9 | 99% |
| 250 | B5 | C2 | 4-[(methanesulfonyl)amino]-N-phenyl-1H-pyrazole-3-carboxamide | 281 | 2.4 | 14.3 | 1.6 | 82% |
| 251 | B5 | C3 | 4-[{(1R)-2-oxo-bornane-10-sulfonyl}amino]-N-phenyl-1H-pyrazole-3-carboxamide | 417 | 4.9 | 19.6 | 1.8 | 94% |
| 252 | B5 | C5 | 4-[(benzenesulfonyl)amino]-N-phenyl-1H-pyrazole-3-carboxamide | 343 | 3.3 | 16.1 | 1.7 | 98% |
| 253 | B5 | C6 | 4-[(1-naphthylsulfonyl)amino]-N-phenyl-1H-pyrazole-3-carboxamide | 393 | 4.7 | 20.0 | 1.9 | 99% |
| 254 | B5 | C7 | 4-[(4-vinylbenzenesulfonyl)amino]-N-phenyl-1H-pyrazole-3-carboxamide | 369 | 4.0 | 18.1 | 1.8 | 94% |

**Table 5-7**

| Ex. No. | 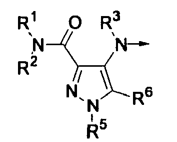 | R⁴ | compound name | MS (M⁺+1) | yield (mg) | yield (%) | retention time (min) | purity |
|---|---|---|---|---|---|---|---|---|
| 255 | B5 | C8 | 4-[(4-ethylbenzenesulfonyl)amino]-N-phenyl-1H-pyrazole-3-carboxamide | 371 | 6.0 | 27.0 | 1.9 | 99% |
| 256 | B5 | C9 | 4-[(4-methoxybenzenesulfonyl)amino]-N-phenyl-1H-pyrazole-3-carboxamide | 373 | 4.6 | 20.6 | 1.7 | 99% |
| 257 | B5 | C10 | 4-[(2,5-dichlorobenzenesulfonyl)amino]-N-phenyl-1H-pyrazole-3-carboxamide | 411 | 2.6 | 10.6 | 1.9 | 99% |
| 258 | B5 | C11 | 4-({[4-(acetylamino)phenyl]sulfonyl}amino)-N-phenyl-1H-pyrazole-3-carboxamide | 400 | 6.9 | 28.8 | 1.5 | 96% |
| 259 | B5 | C12 | N-phenyl-4-[(quinolin-8-ylsulfonyl)amino]-1H-pyrazole-3-carboxamide | 394 | - 4.5 | 19.1 | 1.7 | 98% |
| 260 | B5 | C13 | 4-[(butylsulfonyl)amino]-N-phenyl-1H-pyrazole-3-carboxamide | 323 | 6.3 | 32.6 | 1.7 | 99% |
| 261 | B5 | C14 | 4-[(isopropylsulfonyl)amino]-N-phenyl-1H-pyrazole-3-carboxamide | 309 | 2.8 | 15.1 | 1.6 | 99% |
| 262 | B5 | C16 | 4-({[2-(1-naphthyl)ethyl]sulfonyl}amino)-N-phenyl-1H-pyrazole-3-carboxamide | 421 | 3.7 | 14.7 | 1.9 | 99% |
| 263 | B5 | C17 | 4-{[(4-methylphenyl)sulfonyl]amino}-N-phenyl-1H-pyrazole-3-carboxamide | 357 | 3.0 | 14.0 | 1.8 | 98% |
| 264 | B5 | C18 | 4-[(2-naphthylsulfonyl)amino]-N-phenyl-1H-pyrazole-3-carboxamide | 393 | 3.1 | 13.2 | 1.8 | 99% |
| 265 | B5 | C19 | 4-{[(4-fluorophenyl)sulfonyl]amino}-N-phenyl-1H-pyrazole-3-carboxamide | 361 | 4.8 | 22.2 | 1.7 | 96% |
| 266 | B5 | C20 | 4-[(mesitylsulfonyl)amino]-N-phenyl-1H-pyrazole-3-carboxamide | 385 | 1.5 | 6.5 | 1.9 | 99% |
| 267 | B5 | C22 | 4-{[(4-chlorophenyl)sulfonyl]amino}-N-phenyl-1H-pyrazole-3-carboxamide | 377 | 3.9 | 17.3 | 1.8 | 99% |
| 268 | B5 | C23 | 4-{[(4-bromophenyl)sulfonyl]amino}-N-phenyl-1H-pyrazole-3-carboxamide | 421 | 3.9 | 15.5 | 1.8 | 99% |
| 269 | B6 | C1 | N-benzyl-4-[(biphenyl-4-ylsulfonyl)amino]-1H-pyrazole-3-carboxamide | 433 | 4.7 | 18.1 | 1.9 | 99% |

**Table 5-8**

| Ex. No. | 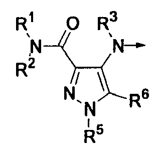 | R⁴ | compound name | MS (M⁺+1) | yield (mg) | yield (%) | retention time (min) | purity |
|---|---|---|---|---|---|---|---|---|
| 270 | B6 | C2 | N-benzyl-4-[(methylsulfonyl)amino]-1H-pyrazole-3-carboxamide | 295 | 1.4 | 7.9 | 1.4 | 98% |
| 271 | B6 | C3 | N-benzyl-4-[{(1R)-2-oxo-bornane-10-sulfonyl}amino]-1H-pyrazole-3-carboxamide | 431 | 5.2 | 20.1 | 1.8 | 99% |
| 272 | B6 | C4 | N-benzyl-4-[(benzylsulfonyl)amino]-1H-pyrazole-3-carboxamide | 371 | 5.5 | 24.8 | 1.7 | 85% |
| 273 | B6 | C5 | N-benzyl-4-[(phenylsulfonyl)amino]-1H-pyrazole-3-carboxamide | 357 | 5.1 | 23.9 | 1.7 | 99% |
| 274 | B6 | C6 | N-benzyl-4-[(1-naphthylsulfonyl)amino]-1H-pyrazole-3-carboxamide | 407 | 4.7 | 19.3 | 1.8 | 99% |
| 275 | B6 | C7 | N-benzyl-4-{[(4-vinylphenyl)sulfonyl]amino}-1H-pyrazole-3-carboxamide | 383 | 3.4 | 14.8 | 1.8 | 97% |
| 276 | B6 | C8 | N-benzyl-4-{[(4-ethylphenyl)sulfonyl]amino}-1H-pyrazole-3-carboxamide | 385 | 2.7 | 11.7 | 1.8 | 99% |
| 277 | B6 | C9 | N-benzyl-4-{[(4-methoxyphenyl)sulfonyl]amino}-1H-pyrazole-3-carboxamide | 387 | 5.9 | 25.5 | 1.7 | 99% |
| 278 | B6 | C10 | N-benzyl-4-{[(2,5-dichlorophenyl)sulfonyl]-amino}-1H-pyrazole-3-carboxamide | 425 | 4.0 | 15.7 | 1.9 | 86% |
| 279 | B6 | C11 | 4-({[4-(acetylamino)phenyl]sulfonyl}amino)-N-benzyl-1H-pyrazole-3-carboxamide | 414 | 3.8 | 15.3 | 1.5 | 98% |
| 280 | B6 | C12 | N-benzyl-4-[(quinolin-8-ylsulfonyl)amino]-1H-pyrazole-3-carboxamide | 408 | 7.5 | 30.7 | 1.6 | 99% |
| 281 | B6 | C13 | N-benzyl-4-[(butylsulfonyl)amino]-1H-pyrazole-3-carboxamide | 337 | 3.9 | 19.3 | 1.7 | 99% |
| 282 | B6 | C14 | N-benzyl-4-[(isopropylsulfonyl)amino]-1H-pyrazole-3-carboxamide | 323 | 5.6 | 29.0 | 1.6 | 96% |
| 283 | B6 | C15 | N-benzyl-4-[{(1S)-2-oxo-bornane-10-sulfonyl}amino]-1H-pyrazole-3-carboxamide | 431 | 4.1 | 15.9 | 1.8 | 99% |
| 284 | B6 | C16 | N-benzyl-4-{[(2-(1-naphthyl)ethyl)sulfonyl]-amino}-1H-pyrazole-3-carboxamide | 435 | 5.2 | 20.0 | 1.9 | 99% |

**Table 5-9**

| Ex. No. | 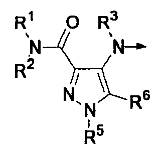 | R⁴ | compound name | MS (M⁺+1) | yield (mg) | yield (%) | retention time (min) | purity |
|---|---|---|---|---|---|---|---|---|
| 285 | B6 | C17 | N-benzyl-4-{[(4-methylphenyl)sulfonyl]amino}-1H-pyrazole-3-carboxamide | 371 | 5.9 | 26.6 | 1.7 | 98% |
| 286 | B6 | C18 | N-benzyl-4-[(2-naphthylsulfonyl)amino]-1H-pyrazole-3-carboxamide | 407 | 3.1 | 12.7 | 1.8 | 99% |
| 287 | B6 | C19 | N-benzyl-4-{[(4-fluorophenyl)sulfonyl]amino}-1H-pyrazole-3-carboxamide | 375 | 3.4 | 15.1 | 1.7 | 99% |
| 288 | B6 | C20 | N-benzyl-4-[(mesitylsulfonyl)amino]-1H-pyrazole-3-carboxamide | 399 | 3.8 | 15.9 | 1.9 | 99% |
| 289 | B6 | C21 | N-benzyl-4-{[(4-methoxy-2-nitrophenyl)sulfonyl]-amino}-1H-pyrazole-3-carboxamide | 432 | 4.4 | 17.0 | 1.8 | 98% |
| 290 | B6 | C22 | N-benzyl-4-{[(4-chlorophenyl)sulfonyl]amino}-1H-pyrazole-3-carboxamide | 391 | 2.9 | 12.4 | 1.8 | 99% |
| 291 | B6 | C23 | N-benzyl-4-{[(4-bromophenyl)sulfonyl]amino}-1H-pyrazole-3-carboxamide | 435 | 4.1 | 15.7 | 1.8 | 99% |
| 292 | B7 | C1 | 4-[(biphenyl-4-ylsulfonyl)amino]-N-(2-phenylethyl)-1H-pyrazole-3-carboxamide | 447 | 4.8 | 17.9 | 1.9 | 99% |
| 293 | B7 | C2 | 4-[(methylsulfonyl)amino]-N-(2-phenylethyl)-1H-pyrazole-3-carboxamide | 309 | 3.3 | 17.9 | 1.5 | 95% |
| 294 | B7 | C3 | 4-[{(1R)-2-oxo-bornane-10-sulfonyl}amino]-N-(2-phenylethyl)-1H-pyrazole-3-carboxamide | 445 | 4.2 | 15.8 | 1.8 | 93% |
| 295 | B7 | C4 | 4-[(benzylsulfonyl)amino]-N-(2-phenylethyl)-1H-pyrazole-3-carboxamide | 385 | 1.8 | 7.8 | 1.8 | 97% |
| 296 | B7 | C5 | N-(2-phenylethyl)-4-[(phenylsulfonyl)amino]-1H-pyrazole-3-carboxamide | 371 | 4.5 | 20.3 | 1.7 | 99% |
| 297 | B7 | C6 | 4-[(1-naphthylsulfonyl)amino]-N-(2-phenylethyl)-1H-pyrazole-3-carboxamide | 421 | 6.0 | 23.8 | 1.9 | 98% |
| 298 | B7 | C7 | N-(2-phenylethyl)-4-{[(4-vinylphenyl)sulfonyl]-amino}-1H-pyrazole-3-carboxamide | 397 | 3.8 | 16.0 | 1.8 | 97% |
| 299 | B7 | C8 | 4-{[(4-ethylphenyl)sulfonyl]amino}-N-(2-phenylethyl)-1H-pyrazole-3-carboxamide | 399 | 5.6 | 23.4 | 1.9 | 99% |

**Table 5-10**

| Ex. No. | 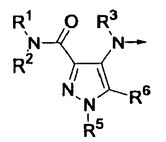 | R⁴ | compound name | MS (M⁺+1) | yield (mg) | yield (%) | retention time (min) | purity |
|---|---|---|---|---|---|---|---|---|
| 300 | B7 | C9 | 4-{[(4-methoxyphenyl)sulfonyl]amino}-N-(2-phenylethyl)-1H-pyrazole-3-carboxamide | 401 | 2.1 | 8.7 | 1.7 | 82% |
| 301 | B7 | C10 | 4-{[(2,5-dichlorophenyl)sulfonyl]amino}-N-(2-phenylethyl)-1H-pyrazole-3-carboxamide | 439 | 0.9 | 3.4 | 1.9 | 86% |
| 302 | B7 | C11 | 4-({[4-(acetylamino)phenyl]sulfonyl}amino)-N-(2-phenylethyl)-1H-pyrazole-3-carboxamide | 428 | 2.4 | 9.4 | 1.6 | 99% |
| 303 | B7 | C12 | N-(2-phenylethyl)-4-[(quinolin-8-ylsulfonyl)amino]-1H-pyrazole-3-carboxamide | 422 | 1.9 | 7.5 | 1.7 | 99% |
| 304 | B7 | C13 | 4-[(butylsulfonyl)amino]-N-(2-phenylethyl)-1H-pyrazole-3-carboxamide | 351 | 4.1 | 19.5 | 1.8 | 97% |
| 305 | B7 | C15 | 4-[{(1S)-2-oxo-bornane-10-sulfonyl}amino]-N-(2-phenylethyl)-1H-pyrazole-3-carboxamide | 445 | 0.8 | 3.0 | 1.8 | 97% |
| 306 | B7 | C16 | N-(2-phenylethyl)-4-{[(2-phenylethyl)sulfonyl]-amino}-1H-pyrazole-3-carboxamide | 449 | 1.0 | 3.7 | 2.0 | 99% |
| 307 | B7 | C17 | 4-{[(4-methylphenyl)sulfonyl]amino}-N-(2-phenylethyl)-1H-pyrazole-3-carboxamide | 385 | 1.6 | 6.9 | 1.8 | 99% |
| 308 | B7 | C18 | 4-[(2-naphthylsulfonyl)amino]-N-(2-phenylethyl)-1H-pyrazole-3-carboxamide | 421 | 2.7 | 10.7 | 1.9 | 98% |
| 309 | B7 | AC | 4-{[(4-fluorophenyl)sulfonyl]amino}-N-(2-phenylethyl)-1H-pyrazole-3-carboxamide | 389 | 4.2 | 18.0 | 1.8 | 96% |
| 310 | B7 | C20 | 4-[(mesitylsulfonyl)amino]-N-(2-phenylethyl)-1H-pyrazole-3-carboxamide | 413 | 0.7 | 2.8 | 1.9 | 97% |
| 311 | B7 | C21 | 4-{[(4-methoxy-2-nitrophenyl)sulfonyl]amino}-N-(2-phenylethyl)-1H-pyrazole-3-carboxamide | 446 | 3.5 | 13.1 | 1.8 | 89% |
| 312 | B7 | C22 | 4-{[(4-chlorophenyl)sulfonyl]amino}-N-(2-phenylethyl)-1H-pyrazole-3-carboxamide | 405 | 3.2 | 13.2 | 1.8 | 98% |
| 313 | B7 | C23 | 4-{[(4-bromophenyl)sulfonyl]amino}-N-(2-phenylethyl)-1H-pyrazole-3-carboxamide | 449 | 4.2 | 15.6 | 1.9 | 97% |
| 314 | B8 | C1 | 4-[(biphenyl-4-ylsulfonyl)amino]-N-(2-furylmethyl)-1H-pyrazole-3-carboxamide | 423 | 5.4 | 21.3 | 1.8 | 98% |

**Table 5-11**

| Ex. No. | 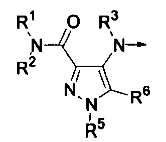 | R⁴ | compound name | MS (M⁺+1) | yield (mg) | yield (%) | retention time (min) | purity |
|---|---|---|---|---|---|---|---|---|
| 315 | B8 | C2 | N-(2-furylmethyl)-4-[(methylsulfonyl)amino]-1H-pyrazole-3-carboxamide | 285 | 0.8 | 4.7 | 1.1 | 95% |
| 316 | B8 | C3 | N-(2-furylmethyl)-4-[{(1R)-2-oxo-bornane-10-sulfonyl}amino]-1H-pyrazole-3-carboxamide | 421 | 3.3 | 13.1 | 1.7 | 98% |
| 317 | B8 | C4 | 4-[(benzylsulfonyl)amino]-N-(2-furylmethyl)-1H-pyrazole-3-carboxamide | 361 | 3.9 | 18.1 | 1.6 | 82% |
| 318 | B8 | C5 | N-(2-furylmethyl)-4-[(phenylsulfonyl)amino]-1H-pyrazole-3-carboxamide | 347 | 3.1 | 14.9 | 1.6 | 96% |
| 319 | B8 | C6 | N-(2-furylmethyl)-4-[(1-naphthylsulfonyl)amino]-1H-pyrazole-3-carboxamide | 397 | 3.8 | 16.0 | 1.7 | 99% |
| 320 | B8 | C7 | N-(2-furylmethyl)-4-{[(4-vinylphenyl)sulfonyl]-amino}-1H-pyrazole-3-carboxamide | 373 | 1.2 | 5.4 | 1.7 | 99% |
| 321 | B8 | C8 | 4-{[(4-ethylphenyl)sulfonyl]amino}-N-(2-furylmethyl)-1H-pyrazole-3-carboxamide | 375 | 1.8 | 8.0 | 1.7 | 98% |
| 322 | B8 | C9 | N-(2-furylmethyl)-4-{[(4-methoxyphenyl)-sulfonyl]amino}-1H-pyrazole-3-carboxamide | 377 | 4.3 | 19.1 | 1.6 | 95% |
| 323 | B8 | C10 | 4-{[(2,5-dichlorophenyl)sulfonyl]amino}-N-(2-furylmethyl)-1H-pyrazole-3-carboxamide | 415 | 3.3 | 13.3 | 1.8 | 95% |
| 324 | B8 | C11 | 4-({[4-(acetylamino)phenyl]sulfonyl}amino)-N-(2-furylmethyl)-1H-pyrazole-3-carboxamid | 404 | 3.0 | 12.4 | 1.4 | 1% |
| 325 | B8 | C12 | N-(2-furylmethyl)-4-[(quinolin-8-ylsulfonyl)amino]-1H-pyrazole-3-carboxamide | 398 | 2.2 | 9.2 | 1.5 | 97% |
| 326 | B8 | C13 | 4-[(butylsulfonyl)amino]-N-(2-furylmethyl)-1H-pyrazole-3-carboxamide | 327 | 4.8 | 24.5 | 1.6 | 98% |
| 327 | B8 | C14 | N-(2-furylmethyl)-4-[(isopropylsulfonyl)amino]-1H-pyrazole-3-carboxamide | 313 | 3.1 | 16.6 | 1.4 | 95% |
| 328 | B8 | C15 | N-(2-furylmethyl)-4-[{(1S)-2-oxo-bornane-10-sulfonyl}amino]-1H-pyrazole-3-carboxamide | 421 | 2.4 | 9.5 | 1.7 | 99% |
| 329 | B8 | C16 | N-(2-furylmethyl)-4-{[(2-phenylethyl)-sulfonyl]amino}-1H-pyrazole-3-carboxamide | 425 | 1.6 | 6.3 | 1.8 | 98% |

**Table 5-12**

| Ex. No. | 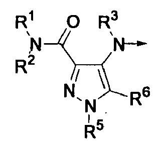 | R⁴ | compound name | MS (M⁺+1) | yield (mg) | yield (%) | retention time (min) | purity |
|---|---|---|---|---|---|---|---|---|
| 330 | B8 | C17 | N-(2-furylmethyl)-4-{[(4-methylphenyl)sulfonyl]-amino}-1H-pyrazole-3-carboxamide | 361 | 1.6 | 7.4 | 1.6 | 91% |
| 331 | B8 | C18 | N-(2-furylmethyl)-4-[(2-naphthylsulfonyl)amino]-1H-pyrazole-3-carboxamide | 397 | 0.1 | 0.4 | 1.7 | 97% |
| 332 | B8 | C19 | 4-{[(4-fluorophenyl)sulfonyl]amino}-N-(2-furylmethyl)-1H-pyrazole-3-carboxamide | 365 | 0.1 | 0.5 | 1.6 | 84% |
| 333 | B8 | C20 | N-(2-furylmethyl)-4-[(mesitylsulfonyl)amino]-1H-pyrazole-3-carboxamide | 389 | 2.5 | 10.7 | 1.8 | 92% |
| 334 | B8 | C21 | N-(2-furylmethyl)-4-{[(4-methoxy-2-nitrophenyl)-sulfonyl]amino}-1H-pyrazole-3-carboxamide | 422 | 3.9 | 15.4 | 1.7 | 95% |
| 335 | B8 | C22 | 4-{[(4-chlorophenyl)sulfonyl]amino}-N-(2-furylmethyl)-1H-pyrazole-3-carboxamide | 381 | 1.3 | 5.7 | 1.7 | 83% |
| 336 | B8 | C23 | 4-{[(4-bromophenyl)sulfonyl]amino}-N-(2-furylmethyl)-1H-pyrazole-3-carboxamide | 425 | 3.8 | 14.9 | 1.7 | 91% |

### Example 337

### N-(3-((benzylamino)carbonyl)-1H-pyrazol-4-yl)pyridine-2-carboxamide

A solution of 4-((pyridin-2-ylcarbonyl)amino)-1H-pyrazole-3-carboxylic acid synthesized in Reference Example 37 (0.30 g, 1.30 mmol), benzylamine (0.14 g, 1.30 mmol), HOBt (0.21 g, 1.60 mmol) and WSC (0.30 g, 1.60 mmol) in DMF (10 mL) was stirred at room temperature overnight. The mixture was diluted with water, and the resulting white precipitate was collected by filtration. The solid was dissolved in ethyl acetate, and washed with saturated brine. The organic layer was dried over anhydrous sodium sulfate, and the solvent was evaporated under reduced pressure. The obtained residue was purified by basic silica gel column chromatography (ethyl acetate) to give the title compound (0.19 g, yield 46%). melting point 204-205°C (ethyl acetate).
¹H-NMR (DMSO-d₆):δ 4.50(2H, d, J=6.3 Hz), 7.13-7.43(5H, m), 7.59-7.74(1H, m), 8.07(1H, t, J=8.1 Hz), 8.11-8.22(1H, m), 8.43(1H, s), 8.74(1H, d, J=5.0 Hz), 9.02(1H, t, J=5.9 Hz), 11.61(1H, br), 13.37(1H, br).

### Example 338

### N-(3-(((2-phenylethyl)amino)carbonyl)-1H-pyrazol-4-yl)pyridine-2-carboxamide

In the same manner as in Example 337, the title compound (0.22 g, yield 49%) was obtained using 4-((pyridin-2-ylcarbonyl)amino)-1H-pyrazole-3-carboxylic acid synthesized in Reference Example 37 and 2-phenylethylamine. melting point 214-215°C (THF-ethyl acetate).
¹H-NMR (DMSO-d₆):δ 2.87(2H, t, J=7.4 Hz), 3.30-3.68 (2H,m), 7.01-7.43(5H, m), 7.54-7.80 (1H, m), 7.99-8.11(1H, m), 8.12-8.22 (1H, m), 8.41(1H, s), 8.46(1H, t, J=5.8 Hz), 8.76(1H, d, J=4.7 Hz), 11.62 (1H,s), 13.54 (1H, s).

### Example 339

### N-(3-(2,3-dihydro-1H-inden-2-ylamino)carbonyl)-1H-pyrazol-4-yl)pyridine-2-carboxamide

In the same manner as in Example 337, the title compound (0.16 g, yield 35%) was obtained using 4-((pyridin-2-ylcarbonyl)amino)-1H-pyrazole-3-carboxylic acid synthesized in Reference Example 37 and 2-aminoindane. melting point 233-234°C (THF-ethyl acetate).
¹H-NMR (DMSO-d₆):δ 3.01-3.25(4H, m), 4.75-4.83(1H, m), 7.14-7.25(4H, m), 7.66-7.70(1H, m), 8.08(1H, t, J=7.8 Hz), 8.17(1H, d, J=7.8 Hz), 8.42(1H, s), 8.60(1H, d, J=7.5 Hz), 8.76(1H, d, J=4.5 Hz), 11.65(1H, s), 13.34(1H,br).

### Example 340

### N-(3-(2,3-dihydro-1H-inden-1-ylamino)carbonyl)-1H-pyrazol-4-yl)pyridine-2-carboxamide

In the same manner as in Example 337, the title compound (0.15 g, yield 34%) was obtained using 4-((pyridin-2-ylcarbonyl)amino)-1H-pyrazole-3-carboxylic acid synthesized in Reference Example 37 and 1-aminoindane. melting point 234-235°C (THF-ethyl acetate).
¹H-NMR (DMSO-d₆):δ 2.02-2.24(1H, m), 2.33-2.48(1H, m), 2.73-2.91(1H, m), 2.94-3.13(1H, m), 5.60(1H, q, J=8.2 Hz), 7.08-7.35(4H, m), 7.60-7.76 (1 m), 8.01-8.13(1H, m), 8.17(1H, d, J=7.7 Hz), 8.43(1H, s), 8.62(1H, d, J=8.5 Hz), 8.76(1H, d, J=4.7 Hz), 11.68(1H, s), 13.35(1H, br).

### Example 341

### N-(3-(anilinocarbonyl)-1H-pyrazol-4-yl)pyridine-2-carboxamide

In the same manner as in Example 337, the title compound (0.14 g, yield 35%) was obtained using 4-((pyridin-2-ylcarbonyl)amino)-1H-pyrazole-3-carboxylic acid synthesized in Reference Example 37 and aniline. melting point 281-282°C (THF-ethyl acetate).
¹H-NMR (DMSO-d₆):δ 7.12(1H, t, J=7.3 Hz), 7.37(2H, t, J=7.8 Hz), 7.60-7.76(1H, m), 7.86(2H, d, J=7.7 Hz), 8.01-8.13(1H, m), 8.18(1H, d, J=8.0 Hz), 8.50(1H, s), 8.79(1H, d, J=4.7 Hz), 10.30(1H, s), 11.57(1H, s), 13.56(1H, br).

### Example 342

### N-(3-(((3-phenylpropyl)amino)carbonyl)-1H-pyrazol-4-yl)pyridine-2-carboxamide

In the same manner as in Example 337, the title compound (0.21 g, yield 47%) was obtained using 4-((pyridin-2-ylcarbonyl)amino)-1H-pyrazole-3-carboxylic acid synthesized in Reference Example 37 and 3-phenylpropylamine. melting point 179-180°C (ethyl acetate).
¹H-NMR (DMSO-d₆):δ 1.75-1.94(2H, m), 2.56-2.69(2H, m), 3.28-3.35(2H, m), 7.10-7.38(5H, m), 7.60-7.75(1H, m), 8.02-8.11(1H, m), 8.13-8.21(1H, m), 8.41(1H, d, J=0.8 Hz), 8.50(1H, t, J=5.9 Hz), 8.65-8.85(1H, m), 11.64(1H, s), 13.32(1H, s).

### Example 343

### N-(3-(((3-(trifluoromethyl)phenyl)amino)carbonyl)-1H-pyrazol-4-yl)pyridine-2-carboxamide

In the same manner as in Example 337, the title compound (0.15 g, yield 31%) was obtained using 4-((pyridin-2-ylcarbonyl)amino)-1H-pyrazole-3-carboxylic acid synthesized in Reference Example 37 and 3-trifluoromethylaniline. melting point 236-237°C (ethyl acetate).
¹H-NMR (DMSO-d₆) :δ 7.47(1H, d, J=8.5 Hz), 7.61(1H, t, J=7.9 Hz), 7.66-7.75 (1H, m), 8.02-8.13(1H, m), 8.13-8.22(2H, m), 8.34(1H, m), 8.53(1H, s), 8.80(1H, d, J=4.8 Hz), 10.70(1H, br), 11.49(1H, s), 13.63(1H, br).

### Example 344

### N-(3-((cyclopentylamino)carbonyl)-1H-pyrazol-4-yl)pyridine-2-carboxamide

In the same manner as in Example 337, the title compound (0.18 g, yield 45%) was obtained using 4-((pyridin-2-ylcarbonyl)amino)-1H-pyrazole-3-carboxylic acid synthesized in Reference Example 37 and cyclopentylamine. melting point 240-241°C (ethyl acetate-hexane).
¹H-NMR (DMSO-d₆):δ 1.40-1.76(6H, m), 1.79-1.97(2H, m), 4.17-4.41(1H, m), 7.68 (1H, dd, J=5.6 Hz and 6.8 Hz), 8.00-8.12 (1H, m), 8.16 (1H, d, J=7.9 Hz), 8.23 (1H, d, J=7.5 Hz), 8.40 (1H, s), 8.75 (1H, d, J=4.8 Hz), 11.66 (1H, s), 13.31 (1H, br).

### Example 345

### N-(3-((isopropylamino)carbonyl)-1H-pyrazol-4-yl)pyridine-2-carboxamide

In the same manner as in Example 337, the title compound (0.14 g, yield 40%) was obtained using 4-((pyridin-2-ylcarbonyl)amino)-1H-pyrazole-3-carboxylic acid synthesized in Reference Example 37 and isopropylamine. melting point 254-255°C (ethyl acetate-hexane).
¹H-NMR (DMSO-d₆):δ 1.18(3H, s), 1.20(3H, s), 4.12-4.24(1H, m), 7.68(1H, ddd, J=1.3 Hz and 4.9 Hz and 7.3 Hz), 8.07(1H, dt, J=1.3 Hz and 7.3 Hz), 8.11-8.17(2H, m), 8.40(1H, s), 8.73-8.77(1H, m), 11.65(1H, s), 13.32(1H, br).

### Example 346

### N-(3-((methylamino)carbonyl)-1H-pyrazol-4-yl)pyridine-2-carboxamide

In the same manner as in Example 337, the title compound (0.13 g, yield 42%) was obtained using 4-((pyridin-2-ylcarbonyl)amino)-1H-pyrazole-3-carboxylic acid synthesized in Reference Example 37 and methylamine. melting point 248-249°C (ethyl acetate-hexane).
¹H-NMR (Methanol-d₄): δ 2.94(3H, s), 7.52-7.67(1H, m), 7.92-8.06 (1H, m), 8.13-8.24 (1H, m), 8.39 (1H, s), 8.62-8.78 (1H, m).

### Example 347

### N-(3-(((2-furylmethyl)amino)carbonyl)-1H-pyrazol-4-yl)pyridine-2-carboxamide

In the same manner as in Example 337, the title compound (0.22 g, yield 69%) was obtained using 4-((pyridin-2-ylcarbonyl)amino)-1H-pyrazole-3-carboxylic acid synthesized in Reference Example 37 and furfurylamine. melting point 198-199°C (THF-ethyl acetate-hexane).
¹H-NMR (DMSO-d₆) : δ 4.47(2H, d, J=6.2 Hz), 6.27(1H, dd, J=0.7 Hz and 3.3 Hz), 6.40(1H, dd, J=1.8 Hz and 3.3 Hz), 7.57(1H, dd, J=0.7 Hz and 1.8 Hz), 7.68(1H, ddd, J=1.4 Hz and 4.8 Hz and 7.5 Hz), 8.07(1H, dt, J=1.4 Hz and 7.5 Hz), 8.12-8.20(1H, m), 8.42(1H, s), 8.71-8.80(1H, m), 8.87(1H, t, J=5.9 Hz), 11.59(1H, s), 13.37 (1H, br).

### Example 348

### N-(3-(((2-(1H-indol-3-yl)ethyl)amino)carbonyl)-1H-pyrazol-4-yl)pyridine-2-carboxamide

In the same manner as in Example 337, the title compound (0.30 g, yield 61%) was obtained using 4-((pyridin-2-ylcarbonyl)amino)-1H-pyrazole-3-carboxylic acid synthesized in Reference Example 37 and triptamine. melting point 250-251°C (THF-ethyl acetate-hexane).
¹H-NMR (DMSO-d₆):δ 2.97(2H, t, J=7.4 Hz), 3.59(2H, q, J=7.4 Hz), 6.94-7.03(1H, m), 7.04-7.12(1H, m), 7.21(1H, d, J=2.2 Hz), 7.34(1H d, J=8.1 Hz), 7.61(1H, d, J=7.7 Hz), 7.68(1H ddd, J=1.0 Hz and 4.8 Hz and 7.5 Hz), 8.08(1H, dt, J=1.5 Hz and 7.5 Hz), 8.16(1H, d, J=7.8 Hz), 8.41(1H, s), 8.50(1H, t, J=6.0 Hz), 8.77(1H, d, J=4.8 Hz), 10.84(1H br), 11.65(1H, s), 13.32(1H, br).

### Example 349

### N-(3-((cyclohexylamino)carbonyl)-1H-pyrazol-4-yl)pyridine-2-carboxamide

In the same manner as in Example 337, the title compound (0.18 g, yield 45%) was obtained using 4-((pyridin-2-ylcarbonyl)amino)-1H-pyrazole-3-carboxylic acid synthesized in Reference Example 37 and cyclohexylamine. melting point 262-263°C (ethyl acetate).
¹H-NMR (DMSO-d₆) :δ 0.93-1.89(10H, m), 3.70-3.94(1H, m), 7.56-7.78(1H, m), 7.94-8.24(3H, m), 8.39(1H, s), 8.74(1H, d, J=4.2 Hz), 11.67(1H, s), 13.31(1H, s).

### Example 350

### N-(3-(((2-((5-cyanopyridin-2-yl)aminoethyl)amino)carbonyl)-1H-pyrazol-4-yl)pyridine-2-carboxamide

In the same manner as in Example 337, the title compound (0.35 g, yield 72%) was obtained using 4-((pyridin-2-ylcarbonyl)amino)-1H-pyrazole-3-carboxylic acid synthesized in Reference Example 37 and 6-((2-aminoethyl)amino)nicotinonitrile synthesized in Reference Example 38. melting point 292-293°C (THF).
¹H-NMR (DMSO-d₆):δ 3.49(4H, br), 6.57(1H, d, J=8.7 Hz), 7.65-7.72(2H, m), 7.76(1H, br), 8.04-8.10(1H, m), 8.15(1H, d, J=7.9 Hz), 8.41(2H, s), 8.56(1H, br), 8.75(1H, d, J=4.0 Hz), 11.61(1H, s), 13.33(1H, br).

### Example 351

### N-(3-(((2-(4-bromophenyl)ethyl)amino)carbonyl)-1H-pyrazol-4-yl)pyridine-2-carboxamide

In the same manner as in Example 337, the title compound (0.35 g, yield 66%) was obtained using 4-((pyridin-2-ylcarbonyl)amino)-1H-pyrazole-3-carboxylic acid synthesized in Reference Example 37 and 4-bromophenylethylamine. melting point 266-270°C (THF-ethyl acetate).
¹H-NMR (DMSO-d₆):δ 2.85(2H, t, J=7.3 Hz), 3.51(2H, q, J=7.3 Hz), 7.23(2H, d, J=8.3 Hz), 7.48(2H, d, J=8.3 Hz), 7.61-7.76(1H, m), 7.99-8.11(1H, m), 8.12-8.20(1H, m), 8.40(1H, s), 8.48(1H, t, J=6.00 Hz), 8.67-8.81(1H, m), 11.60(1H, s), 13.30(1H, br).

### Example 352

### N-(3-(((2-pyridin-3-ylethyl)amino)carbonyl)-1H-pyrazol-4-yl)pyridine-2-carboxamide

In the same manner as in Example 337, the title compound (0.17 g, yield 50%) was obtained using 4-((pyridin-2-ylcarbonyl)amino)-1H-pyrazole-3-carboxylic acid synthesized in Reference Example 37 and 3-(2-aminoethyl)pyridine. melting point 267-268°C (methanol-THF-ethyl acetate).
¹H-NMR (DMSO-d₆) :δ 2.90(2H, t, J=7.2 Hz), 3.55 (2H, q, J=7.2 Hz), 7.32(1H, dd, J=4.8 Hz and J=7.8), 7.61-7.79(2H, m), 8.00-8.11(1H, m), 8.15(1H, d, J=8.0 Hz), 8.35-8.44(2H, m), 8.47(1H, s), 8.54 (1H, t, J=5.5 Hz), 8.75 (1H, d, J=4.4 Hz), 11.59 (1H, s), 13.32 (1H, br).

### Example 353

### N-(3-(((3-isopropoxypropyl)amino)carbonyl)-1H-pyrazol-4-yl)pyridine-2-carboxamide

In the same manner as in Example 337, the title compound (0.26 g, yield 61%) was obtained using 4-((pyridin-2-ylcarbonyl)amino)-1H-pyrazole-3-carboxylic acid synthesized in Reference Example 37 and 3-isopropoxypropylamine. melting point 152-153°C (ethyl acetate-hexane).
¹H-NMR (DMSO-d₆) :δ 1.09(3H, s), 1.11(3H, s), 1.70-1.80(2H, m), 3.31-3.40(2H, m), 3.43(2H, t, J=6.1 Hz), 3.48-3.58(1H, m), 7.65-7.71(1H, m), 8.07(1H, dt, J=1.8 Hz and 7.6 Hz), 8.12-8.18 (1H, m), 8.37-8.45(2H, m), 8.73-8.78(1H, m), 11.64 (1H, s), 13.31(1H, s).

### Example 354

### N-(3-(((biphenyl-4-ylmethyl)amino)carbonyl)-1H-pyrazol-4-yl)pyridine-2-carboxamide

In the same manner as in Example 337, the title compound (0.28 g, yield 56%) was obtained using 4-((pyridin-2-ylcarbonyl)amino)-1H-pyrazole-3-carboxylic acid synthesized in Reference Example 37 and 4-phenylbenzylamine. melting point 227-228°C (ethyl acetate-hexane).
¹H-NMR (DMSO-d₆) :δ 4.53(2H, d, J=6.4 Hz), 7.27-7.38(1H, m), 7.45(4H, t, J=7.9 Hz), 7.56-7.75(5H, m), 7.99-8.10(1H, m), 8.16(1H, d, J=7.7 Hz), 8.43(1H, s), 8.65-8.80(1H, m), 9.07(1H, t, J=6.2 Hz), 11.61(1H, s), 13.37(1H, br).

### Example 355

### N-(3-(((2-phenoxyethyl)amino)carbonyl)-1H-pyrazol-4-yl)pyridine-2-carboxamide

In the same manner as in Example 337, the title compound (0.32 g, yield 71%) was obtained using 4-((pyridin-2-ylcarbonyl)amino)-1H-pyrazole-3-carboxylic acid synthesized in Reference Example 37 and 2-phenoxyethylamine. melting point 182-183°C (ethyl acetate).
¹H-NMR (DMSO-d₆):δ 3. 68 (2H, q, J=6.1 Hz), 4.13 (2H, t, J=6.1 Hz), 6.82-7.11(3H, m), 7.21-7.38(2H, m), 7.61-7.77(1H, m), 8.01-8.12(1H, m), 8.12-8.23(1H, m), 8.41(1H, s), 8.54 (1H, t, J=6.1 Hz), 8.69-8.83(1H, m), 11.61(1H, s), 13.35(1H, br).

### Example 356

### N-(3-(((2-methoxyethyl)amino)carbonyl)-1H-pyrazol-4-yl)pyridine-2-carboxamide

In the same manner as in Example 337, the title compound (0.14 g, yield 36%) was obtained using 4-((pyridin-2-ylcarbonyl)amino)-1H-pyrazole-3-carboxylic acid synthesized in Reference Example 37 and 2-methoxyethylamine. melting point 166-167°C (ethyl acetate).
¹H-NMR (DMSO-d₆):δ 3.27(3H, s), 3.41-3.54(4H, m), 7.60-7.82(1H, m), 8.01-8.12(1H, m), 8.13-8.22(1H, m), 8.25-8.36(1H, m), 8.40(1H, s), 8.67-8.84(1H, m), 11.60(1H, s), 13.32(1H, br).

### Example 357

### N-(3-(((2-isopropoxyethyl)amino)carbonyl)-1H-pyrazol-4-yl)pyridine-2-carboxamide

In the same manner as in Example 337, the title compound (0.23 g, yield 57%) was obtained using 4-((pyridin-2-ylcarbonyl)amino)-1H-pyrazole-3-carboxylic acid synthesized in Reference Example 37 and 2-isopropoxyethylamine. melting point 163-164°C (ethyl acetate).
¹H-NMR (DMSO-d₆):δ 1.08(3H, s), 1.10(3H, s), 3.37-3.46(2H, m), 3.46-3.53(2H, m), 3.54-3.68(1H, m), 7.59-7.78(1H, m), 8.07(1H, dt, J=1.8 Hz and J=7.6 Hz), 8.15(1H, d, J=7.6 Hz), 8.24(1H, t, J=5.7 Hz), 8.40 (1H, s), 8.67-8.82 (1H, m), 11. 60 (1H, s), 13.30(1H, br).

### Example 358

### N-(3-(((2-cyanoethyl)amino)carbonyl)-1H-pyrazol-4-yl)pyridine-2-carboxamide

In the same manner as in Example 337, the title compound (0.18 g, yield 48%) was obtained using 4-((pyridin-2-ylcarbonyl)amino)-1H-pyrazole-3-carboxylic acid synthesized in Reference Example 37 and 2-cyanoethylamine. melting point 223-224°C (ethyl acetate).
¹H-NMR (DMSO-d₆) :δ 2.81(2H, t, J=6.5 Hz), 3.54(2H, q, J=6.5 Hz), 7.61-7.78(1H, m), 8.07(1H, dt, J=1.6 Hz and 7.7 Hz), 8.16(1H, d, J=7.7 Hz), 8.42 (1H, s), 8.67-8.82(2H, m), 11.57(1H, s), 13.39 (1H, br).

### Example 359

### N-(3-(((2-oxo-2-phenylethyl)amino)carbonyl)-1H-pyrazol-4-yl)pyridine-2-carboxamide

In the same manner as in Example 337, the title compound (0.15 g, yield 33%) was obtained using 4-((pyridin-2-ylcarbonyl)amino)-1H-pyrazole-3-carboxylic acid synthesized in Reference Example 37 and 2-oxo-2-phenylethylamine. melting point 270-271°C (ethyl acetate).
¹H-NMR (DMSO-d₆):δ 4.83(2H, d, J=5.4 Hz), 7.58 (2H, t, J=7.7 Hz), 7.63-7.76(2H, m), 7.99-8.11(3H, m), 8.15(1H, d, J=7.9 Hz), 8.44(1H, s), 8.60(1H, t, J=5.8), 8.69(1H, d, J=4.6 Hz), 11.56(1H, s), 13.40(1H, br).

### Example 360

### N-(3-(((2,2,2-trifluoroethyl)amino)carbonyl)-1H-pyrazol-4-yl)pyridine-2-carboxamide

In the same manner as in Example 337, the title compound (0.27 g, yield 66%) was obtained using 4-((pyridin-2-ylcarbonyl)amino)-1H-pyrazole-3-carboxylic acid synthesized in Reference Example 37 and 2,2,2-trifluoroethylamine. melting point 235-236°C (ethyl acetate).
¹H-NMR (DMSO-d₆): δ 3.89-4.29(2H, m), 7.57-7.87(1H, m), 8.08(1H, dt, J=1.5 Hz and 7.6 Hz), 8.16(1H, d, J=7.7 Hz), 8.45(1H, s), 8.75(1H, d, J=4.8 Hz), 9.03(1H, t, J=6.5 Hz), 11.50(1H, s), 13.49(1H, br).

### Example 361

### N-(3-(((2-(phenylthio)ethyl)amino)carbonyl)-1H-pyrazol-4-yl)pyridine-2-carboxamide

In the same manner as in Example 337, the title compound (0.20 g, yield 41%) was obtained using 4-((pyridin-2-ylcarbonyl)amino)-1H-pyrazole-3-carboxylic acid synthesized in Reference Example 37 and 2-(phenylthio)ethylamine. melting point 180-181°C (ethyl acetate).
¹H-NMR (DMSO-d₆): δ 3.09-3.24(2H, m), 3.44-3.60(2H, m), 7.14-7.25(1H, m), 7.29-7.38(2H, m), 7.39-7.47(2H, m), 7.62-7.79(1H, m), 8.02-8.11(1H, m), 8.13-8.22 (1H, m), 8.41(1H, s), 8.58-8.69(1H, m), 8.71-8.82(1H, m), 11.59(1H, s), 13.35(1H, br).

### Example 362

### N-cyclopentyl-4-((2,3-dihydro-1-benzofuran-7-ylcarbonyl)amino)-1H-pyrazole-3-carboxamide

In the same manner as in Example 1, the title compound was obtained using 4-amino-N-cyclopentyl-1H-pyrazole-3-carboxamide obtained in Reference Example 13 and 2,3-dihydro-1-benzofuran-7-carboxylic acid. yield 46%.
¹H-NMR (CDCl₃) :δ 1.44-1.84 (6H, m), 1.99-2.15 (2H, m), 3.29 (2H, t, J=8.8 Hz), 4.38-4.56 (1H, m), 4.89(2H, t, J=8.8 Hz), 6.86 (1H, d, J=7.9 Hz), 6.97 (1H, t, J=7.4 Hz), 7.34 (1H, d, J=7.4 Hz), 7.93 (1H, d, J=7.4 Hz), 8.57(1H, s), 10.17(1H, s), 11.20(1H, s).

### Example 363

### N-(3-((cyclopentylamino)carbonyl)-1H-pyrazol-4-yl)-2-methylnicotinamide

In the same manner as in Example 1, the title compound was obtained using 4-amino-N-cyclopentyl-1H-pyrazole-3-carboxamide obtained in Reference Example 13 and 2-methylnicotinic acid. yield 48%.
¹H-NMR (CDCl₃): δ 1.45-1.84(6H, m), 1.98-2.15(2H, m), 2.80(3H, s), 4.25-4.44 (1H, m), 6.86 (1H, d, J=7.3 Hz), 7.23 (1H, dd, J=4.9, 7.7 Hz), 7.90 (1H, dd, J=1.7, 7.7 Hz), 8.48 (1H, s), 8.61 (1H, dd, J=1.7, 4.9 Hz), 10.21 (1H, s), 10.53 (1H, s).

### Example 364

### N-(3-((cyclopentylamino)carbonyl)-1H-pyrazol-4-yl)-6-methylnicotinamide

In the same manner as in Example 1, the title compound was obtained using 4-amino-N-cyclopentyl-1H-pyrazole-3-carboxamide obtained in Reference Example 13 and 6-methylnicotinic acid. yield 47%.
¹H-NMR (CDCl₃) :δ 1.46-1.6(6H, m), 2.02-2.20(2H, m), 2.64(3H, s), 4.33-4.50 (1H, m), 6.87(1H, d, J=7.5 Hz), 7.26(1H, d, J=8.1 Hz), 8.11(1H, dd, J=2.2, 8.1 Hz), 8.47 (1H, s), 9.15(1H, d, J=2.2 Hz), 10.31(1H, brs), 10.68(1H, s).

### Example 365

### N-(3-((cyclopentylamino)carbonyl)-1H-pyrazol-4-yl)-2-methoxynicotinamide

In the same manner as in Example 1, the title compound was obtained using 4-amino-N-cyclopentyl-1H-pyrazole-3-carboxamide obtained in Reference Example 13 and 2-methoxynicotinic acid. yield 51%.
¹H-NMR (CDCl₃):δ 1.45-1.85(6H, m), 2.00-2.16(2H, m), 4.30(3H, s), 4.35-4.57(1H, m), 6.84(1H, d, J=7.2 Hz), 7.08(1H, dd, J=4..9, 7.5 Hz), 8.32(1H, dd, J=2.0, 4.9 Hz), 8.52(1H, s), 8.54(1H, dd, J=2.0, 7.5 Hz), 10.04 (1H, brs), 11.86 (1H, s).

### Example 366

### N-cyclopentyl-4-((2,6-difluorobenzoyl)amino)-1H-pyrazole-3-carboxamide

In the same manner as in Example 1, the title compound was obtained using 4-amino-N-cyclopentyl-1H-pyrazole-3-carboxamide obtained in Reference Example 13 and 2,6-difluorobenzoic acid. yield 62%.
¹H-NMR (CDCl₃) :δ 1.44-1.84 (6H, m), 1.98-2.12 (2H, m), 4.27-4.46 (1H, m), 6.83(1H, d, J=8.1 Hz), 6.99(2H, t, J=8.3 Hz), 7.32-7.50(1H, m), 8.49(1H, s), 10.09(1H, brs), 10.34(1H, s).

### Example 367

### N-cyclopentyl-4-(cyclopentanecarbonylamino)-1H-pyrazole-3-carboxamide

In the same manner as in Example 1, the title compound was obtained using 4-amino-N-cyclopentyl-1H-pyrazole-3-carboxamide obtained in Reference Example 13 and cyclopentanecarboxylic acid. yield 55%.
¹H-NMR (CDCl₃):δ 1.41-2.16(16H, m), 2.77(1H, septet, J=8.0 Hz), 4.29-4.44 (1H, m), 6.82(1H, d, J=7.0 Hz), 8.33(1H, s), 9.72 (1H, s), 10.08(1H, brs).

### Example 368

### N-cyclopentyl-4-((2-ethylbutanoyl)amino)-1H-pyrazole-3-carboxamide

In the same manner as in Example 1, the title compound was obtained using 4-amino-N-cyclopentyl-1H-pyrazole-3-carboxamide obtained in Reference Example 13 and pentane-3-carboxylic acid. yield 36%.
¹H-NMR (CDCl₃) : δ 0.93(6H, t, J=7.5 Hz), 1.47-1.85 (10H, m), 1.99-2.24(3H, m), 4.30-4.46 (1H, m), 6.83 (1H, d, J=7.7 Hz), 8.37 (1H, s), 9.71 (1H, s), 10.23 (1H, brs).

### Example 369

### 4-((3-(acetylamino)benzoyl)amino)-N-(2-phenoxyethyl)-1H-pyrazole-3-carboxamide

In the same manner as in Example 1, the title compound was obtained using 4-((3-(acetylamino)benzoyl)amino)-1H-pyrazole-3-carboxylic acid p-toluenesulfonate obtained in Reference Example 43 and 2-phenoxyethaneamine. yield 89%.
¹H-NMR (DMSO-d₆):δ 2.08 (3H, s), 3.67(2H, dt, J=5.9, 6.1 Hz), 4.13(2H, t, J=6.1 Hz), 6.86-7.00 (3H, m), 7.21-7.34 (2H, m), 7.44-7.55 (2H, m), 7.79-7.90 (1H,m), 8.12 (1H, s), 8.33 (1H, s), 8.63 (1H, t, J=5.8 Hz), 10.22(1H, s), 10.62(1H, s), 13.35(1H, s).

### Example 370

### 4-((3-(acetylamino)benzoyl)amino)-N-(2-methoxyethyl)-1H-pyrazole-3-carboxamide

In the same manner as in Example 1, the title compound was obtained using 4-((3-(acetylamino)benzoyl)amino)-1H-pyrazole-3-carboxylic acid p-toluenesulfonate obtained in Reference Example 43 and 2-methoxyethaneamine. yield 49%.
¹H-NMR.(DMSO-d₆):δ 2.08(3H, s), 3.27(3H, s), 3.40-3.52(4H, m), 7.50(2H, d, J=5.1 Hz), 7.81-7.90(1H, m), 8.11(1H, s), 8.33(1H, s), 8.35-8.44(1H, m), 10.22 (1H, s), 10.63(1H, s), 13.32(1H, s).

### Example 371

### 4-((3-(acetylamino)benzoyl)amino)-N-(2-isopropoxyethyl)-1H-pyrazole-3-carboxamide

In the same manner as in Example 1, the title compound was obtained using 4-((3-(acetylamino)benzoyl)amino)-1H-pyrazole-3-carboxylic acid p-toluenesulfonate obtained in Reference Example 43 and 2-aminoethylisopropylether. yield 89%.
¹H-NMR (DMSO-d₆):δ 1.09(6H, d, J=6.0 Hz), 2.08(3H, s), 3.36-3.66(5H, m), 7.50(2H, d, J=5.1 Hz), 7. 82-7. 90 (1H, m), 8.12 (1H, s), 8.28-8.39 (1H, m), 8.32(1H, s), 10.22(1H, s), 10.62(1H, s), 13.31(1H, s).

### Example 372

### 4-((3-(acetylamino)benzoyl)amino)-N-(2-cyanoethyl)-1H-pyrazole-3-carboxamide

In the same manner as in Example 1, the title compound was obtained using 4-((3-(acetylamino)benzoyl)amino)-1H-pyrazole-3-carboxylic acid p-toluenesulfonate obtained in Reference Example 43 and 3-aminopropionitrile. yield 76%.
¹H-NMR (DMSO-d₆):δ 2.08(3H, s), 2.81(2H, t, J=6.5 Hz), 3.47-3.59(2H, m), 7.45-7.54(2H, m), 7.81-7.90 (1H, m), 8.12 (1H, s), 8.34 (1H, s), 8.77-8.87 (1H, m), 10.22 (1H, s), 10.55 (1H, s), 13.40 (1H, s).

### Example 373

### 4-((3-(acetylamino)benzoyl)amino)-N-(2-(dimethylamino)ethyl)-1H-pyrazole-3-carboxamide

In the same manner as in Example 1, the title compound was obtained using 4-((3-(acetylamino)benzoyl)amino)-1H-pyrazole-3-carboxylic acid p-toluenesulfonate obtained in Reference Example 43 and N,N-dimethylethylenediamine. yield 34%.
¹H-NMR (DMSO-d₆) :δ 2.08(3H, s), 2.18(6H, s), 2.41(2H, t, J=6.7 Hz), 3.34-3.44(2H, m), 7.44-7.55(2H, m), 7.80-7.90(1H, m), 8.11(1H, s), 8.25(1H, t, J=5.8 Hz), 8.32(1H, s), 10.22(1H, s), 10.63(1H, s), 13.30(1H, s).

### Example 374

### 4-((3-(acetylamino)benzoyl)amino)-N-(2-oxo-2-phenylethyl)-1H-pyrazole-3-carboxamide

In the same manner as in Example 1, the title compound was obtained using 4-((3-(acetylamino)benzoyl)amino)-1H-pyrazole-3-carboxylic acid p-toluenesulfonate obtained in Reference Example 43 and 2-aminoacetophenone. yield 18%.
¹H-NMR (DMSO-d₆):δ 2.06(3H, s), 4.83(2H, d, J=5.8 Hz), 7.44-7.51(2H, m), 7.53-7.63(2H, m), 7.66-7.74 (1H, m), 7.79-7.90 (1H, m), 8.02-8.13(3H, m), 8.37 (1H, s), 8.68 (1H, t, J=5.8 Hz), 10.20 (1H, s), 10.53 (1H, s), 13.41 (1H, s).

### Example 375

### 4-((3-(acetylamino)benzoyl)amino)-N-(2,2,2-trifluoroethyl)-1H-pyrazole-3-carboxamide

In the same manner as in Example 1, the title compound was obtained using 4-((3-(acetylamino)benzoyl)amino)-1H-pyrazole-3-carboxylic acid p-toluenesulfonate obtained in Reference Example 43 and 2,2,2-trifluoroethylamine. yield 76%.
¹H-NMR (DMSO-d₆):δ 2.08(3H, s), 3.94-4.16(2H, m), 7.44-7.54(2H, m), 7.78-7.89(1H, m), 8.12(1H, s), 8.37(1H, s), 9.05-9.17(1H, m), 10.22(1H, s), 10.38(1H, s), 13.49(1H, s).

### Example 376

### 4-((3-(acetylamino)benzoyl)amino)-N-(2-(phenylthio)ethyl)-1H-pyrazole-3-carboxamide

In the same manner as in Example 1, the title compound was obtained using 4-((3-(acetylamino)benzoyl)amino)-1H-pyrazole-3-carboxylic acid p-toluenesulfonate obtained in Reference Example 43 and 2-(phenylthio)ethylamine. yield 81%. ¹H-NMR (DMSO-d₆):δ 2.08(3H, s), 3.13-3.21 (2H, m), 3.43-3.56 (2H, m), 7.16-7.23 (1H, m), 7.30-7.37 (2H, m), 7.38-7.44(2H, m), 7.47-7.55(2H, m), 7.80-7.89 (1H, m), 8.13 (1H, s), 8.33 (1H, s), 8.67-8.80 (1H, m), 10.22 (1H, s), 10.60 (1H, s), 13.35 (1H, s).

### Example 377

### N-(3-((cyclopentylamino)carbonyl)-5-methyl-1H-pyrazol-4-yl)pyrazine-2-carboxamide

To a solution of N-cyclopentyl-5-methyl-4-nitro-1H-pyrazine-3-carboxamide obtained in Reference Example 44 (0.154 g, 0.65 mmol) and ammonium formate (300 mg) in ethanol (5 mL), 10% palladium carbon (containing 50% water, 0.1 g) was added, and the mixture was stirred for 15 hr. The catalyst was removed by filtration, and the filtrate was evaporated under reduced pressure. The residue was dissolved in ethyl acetate (50 mL), washed with saturated aqueous sodium hydrogen carbonate solution, washed with water, dried over anhydrous magnesium sulfate, and evaporated under reduced pressure. The residue was dissolved in DMF (10 mL). Pyrazine-2-carboxylic acid (96.3 mg, 0.776 mmol), WSC (167 mg, 0.97 mmol) and HOBt (149 mg, 0.97 mmol) were added, and the mixture was stirred for 4 hr. The reaction mixture was diluted with water, and extracted with ethyl acetate. The extract was washed with saturated aqueous sodium hydrogen carbonate solution and water, dried over anhydrous magnesium sulfate, and evaporated under reduced pressure. The residue was purified by silica gel column chromatography (dichloromethane-ethyl acetate 1:1) to give the title compound (0.15 g, yield 74%) as crystals. melting point 170-171°C.
¹H-NMR (CDCl₃): δ 1.40-1.80 (6H, m), 2.00-2.20(2H, m), 2.57(3H, s), 4.35-4.50 (1H, m), 6.84 (1H, brs), 8.60-8.70 (1H, m), 8.70-8.80 (1H, m), 9.42 (1H, d, J=1.2 Hz), 10.03 (1H, brs), 10.93 (1H, brs).

### Example 378

### 4-(benzoylylamino)-N-cyclopentyl-5-methyl-1H-pyrazole-3-carboxamide

To a solution of N-cyclopentyl-5-methyl-4-nitro-1H-pyrazine-3-carboxamide obtained in Reference Example 44 (0.103 g, 0.43 mmol) and ammonium formate (200 mg) in ethanol (5 mL), 10% palladium carbon (containing 50% water, 0.05 g) was added, and the mixture was stirred for 5 hr. The catalyst was removed by filtration, and the filtrate was evaporated under reduced pressure. The residue was dissolved in ethyl acetate (50 mL), and the mixture was washed with saturated aqueous sodium hydrogen carbonate solution, washed with water, dried over anhydrous magnesium sulfate, and evaporated under reduced pressure. The residue was dissolved in DMF (10 mL), benzoic acid (63.4 mg, 0.52 mmol), WSC (111 mg, 0.65 mmol) and HOBt (88 mg, 0.65 mmol) were added, and the mixture was stirred for 4 hr. The reaction mixture was diluted with water, and extracted with ethyl acetate. The extract was washed with saturated aqueous sodium hydrogen carbonate solution and water, dried over anhydrous magnesium sulfate, and evaporated under reduced pressure. The residue was purified by silica gel column chromatography (hexane-ethyl acetate 1:1) to give the title compound 70 m g (yield 52%) as non-crystalline powder.
¹H-NMR (CDCl₃):δ 1.45-1.80(6H, m), 1.95-2.15(2H, m), 2.56(3H, s), 4.30-4.45 (1H, m), 6.82 (1H, brs), 7.40-7.60(3H, m), 7.95-8.05(2H, m), 9.90 (1H, s).

### Example 379

### N-(3-((cyclopentylamino)carbonyl)-5-ethyl-1H-pyrazol-4-yl)pyrazine-2-carboxamide

To a solution of N-cyclopentyl-5-ethyl-4-nitro-1H-pyrazole-3-carboxamide obtained in Reference Example 45 (85 mg, 0.34 mmol) and ammonium formate (150 mg) in ethanol (5 mL), 10% palladium carbon (containing 50% water, 50 mg) was added, and the mixture was stirred for 8 hr. The catalyst was removed by filtration, and the filtrate was evaporated under reduced pressure. The residue was dissolved in ethyl acetate (50 mL), and the mixture was washed with saturated aqueous sodium hydrogen carbonate solution, washed with water, dried over anhydrous magnesium sulfate, and evaporated under reduced pressure. The residue was dissolved in DMF (10 mL). Pyrazine-2-carboxylic acid (50 mg, 0.40 mmol), 1-ethyl-3-(3-dimethylaminopropyl)carbodiimide hydrochloride (87 mg, 0.505 mmol) and N-hydroxybenzotriazole (68 mg, 0.505 mmol) were added, and the mixture was stirred for 15 hr. The reaction mixture was diluted with water, and extracted with ethyl acetate. The extract was washed with saturated aqueous sodium hydrogen carbonate solution and water, dried over anhydrous magnesium sulfate, and evaporated under reduced pressure. The residue was purified by silica gel column chromatography (dichloromethane-ethyl acetate 1:1) to give the title compound (86.7 mg, yield 78%) as crystals. melting point 175-176°C.
¹H-NMR (CDCl₃) :δ 1.29 (3H, t, J=7.5 Hz), 1. 40-1. 85 (6H, m), 2.00-2.20(2H, m), 3.06(2H, q, J=7.5 Hz), 4.35-4.50(1H, m), 6.84(1H, brs), 8.60-8.70(1H, m), 8.70-8.80(1H, m), 9.43(1H, d, J=2.1 Hz), 9.87(1H, brs), 10.96(1H, brs).

### Example 380

### 4-((3-(acetylamino)benzoyl)amino)-N-(cyanomethyl)-1H-pyrazole-3-carboxamide

To a solution of 4-((3-(acetylamino)benzoyl)amino)-1H-pyrazole-3-carboxylic acid p-toluenesulfonate obtained in Reference Example 43 (0.46 g, 1.0 mmol), aminoacetonitrile (259 mg, 1.0 mmol) and triethylamine (0.21 mL, 1.2 mmol) in DMF (10 mL), WSC (206 mg, 1.2 mmol) and HOBt (162 mg, 1.2 mmol) were added, and the mixture was stirred at room temperature for 4.5 hr. Water was added to the reaction mixture, and the mixture was extracted with ethyl acetate. The extract was washed with saturated aqueous sodium hydrogen carbonate solution and water, dried over anhydrous magnesium sulfate, and evaporated under reduced pressure. The residue was purified by silica gel column chromatography (ethyl acetate) to give the title compound (0.28 g, yield 86%) as crystals. melting point 255-260°C (decomposition).
¹H-NMR (d₆-DMSO):δ 2.08(3H, s), 4.25-4.40(2H, m), 7.52 (1H, s), 7.50-7.60(1H, m), 7.80-7.90(1H, m), 8.14 (1H, s), 8.30 (1H, s), 9.21 (1H, s), 10.24 (1H, s), 10.35 (1H, s), 13.48 (1H, s).

### Example 381

### 4-((3-(acetylamino)benzoyl)amino)-N-(2-tert-butoxyethyl)-1H-pyrazole-3-carboxamide

In the same manner as in Example 380, the title compound (0.30 g, yield 77%) was obtained using 4-((3-(acetylamino)benzoyl)amino)-1H-pyrazole-3-carboxylic acid p-toluenesulfonate obtained in Reference Example 43 and 2-tert-butoxyethylamine as crystals. melting point 124-126°C.
¹H-NMR (d₆-DMSO):δ 1.14(9H, s), 20.8(3H, s), 3.35-3.50(4H, m), 7.45-7.55(2H, m), 7.80-7.90(1H, m), 8.11(1H, s), 8.22-8.35(1H, m), 8.33(1H, s), 10.23(1H, s), 10.62(1H, s), 13.32(1H, s).

### Example 382

### 4-((3-(acetylamino)benzoyl)amino)-N-(2-(methylsulfonyl)ethyl)-1H-pyrazole-3-carboxamide

In the same manner as in Example 380, the title compound (0.25 g, yield 69%) was obtained using 4-((3-(acetylamino)benzoyl)amino)-1H-pyrazole-3-carboxylic acid p-toluenesulfonate obtained in Reference Example 43 and 2-(methylsulfonyl)ethylamine as crystals. melting point 230-232°C.
¹H-NMR (d₆-DMSO):δ 2.06(3H, s), 3.02(3H, s), 3.38(2H, t, J=6.3 Hz), 3.63-3.80(2H, m), 7.48(2H, d, J=5.1 Hz), 7.80-7.90(1H, m), 8.11(1H, s), 8.32(1H, s), 10.21(1H, s), 10.53(1H, s), 13.38(1H, s).

### Example 383

### 4-((3-(acetylamino)benzoyl)amino)-N-(2-((4-chlorophenyl) sulfonyl)ethyl)-1H-pyrazole-3-carboxamide

In the same manner as in Example 380, the title compound (0.38 g, yield 84%) was obtained using 4-((3-(acetylamino)benzoyl)amino)-1H-pyrazole-3-carboxylic acid p-toluenesulfonate obtained in Reference Example 43 and 2-((4-chlorophenyl)sulfonyl)ethylamine as crystals. melting point 219-220°C.
¹H-NMR (d₆-DMSO): δ 2.07(3H, s), 3.50-3.70(4H, m), 7.47 (1H, s), 7.45-7.55 (1H, m), 7.64(2H, d, J=8.40 Hz), 7.80-7.90 (1H, m), 7.91(2H, d, J=8.4 Hz), 8.12 (1H, s), 8.28 (1H, s), 8.40-8.50 (1H, m), 10.21 (1H, s), 10.42 (1H, s), 13.30 (1H, s).

### Example 384

### 4-((3-(acetylamino)benzoyl)amino)-N-(2-(pyridin-2-ylsulfonyl)ethyl)-1H-pyrazole-3-carboxamide

In the same manner as in Example 380, the title compound (0.38 g, yield 90%) was obtained using 4-((3-(acetylamino)benzoyl)amino)-1H-pyrazole-3-carboxylic acid p-toluenesulfonate obtained in Reference Example 43 and 2-(pyridin-2-ylsulfonyl)ethylamine as crystals. melting point 240-241°C.
¹H-NMR (d₆-DMSO):δ 2.09(3H, s), 3.33(3H, s), 3.60-3.75(2H, m), 3.75-3.85(2H, m), 7.49(1H, s), 7.45-7.60(1H, m), 7.65-7.75(1H, m), 7.80-7.90(1H, m), 8.00-8.20(3H, m), 8.30(1H, s), 8.50-8.60(1H, m), 8.75(1H, d, J=4.0 Hz), 10.23(1H, s), 10.45(1H, s), 13.32(1H, s).

### Example 385

### 4-((3-(acetylamino)benzoyl)amino)-N-(3-isopropoxypropyl)-1H-pyrazole-3-carboxamide

In the same manner as in Example 380, the title compound (0.36 g, yield 93%) was obtained using 4-((3-(acetylamino)benzoyl)amino)-1H-pyrazole-3-carboxylic acid p-toluenesulfonate obtained in Reference Example 43 and 3-isopropoxypropylamine as crystals. melting point 140-142°C.
¹H-NMR (d₆-DMSO) :δ 1.09(6H, d, J=6.0 Hz), 1.70-1.82(2H, m), 2.08(3H, s), 3.30 -3.50(4H, m), 3.50-3.60(1H, m), 7.45-7.55(2H, m), 7.80-7.90(1H, m), 8.11(1H, s), 8.32(1H, s), 8.45-8.55(1H, m), 10.22(1H s), 10.69 (1H, s), 13.32(1H, s).

### Example 386

### N-(3-(((2-cyanoethyl)amino)carbonyl)-1-(tetrahydro-2H-pyran-2-yl)-1H-pyrazol-4-yl)-6-methylpyridine-2-carboxamide

To a solution of 4-(((6-methylpyridin-2-yl)carbonyl)amino)-1-(tetrahydro-2H-pyran-2-yl)-1H-pyrazole-3-carboxylic acid obtained in Reference Example 47 (0.66 g, 2.0 mmol) and 3-aminopropionitrile (0.18 mL, 2.4 mmol) in DMF (15 mL), WSC (0.41 g, 2.4 mmol) and HOBt (0.32 g, 2.4 mmol) were added, and the mixture was stirred at room temperature for 6 hr. Saturated aqueous sodium hydrogen carbonate solution was added to the reaction mixture, and the mixture was extracted with ethyl acetate. The extract was washed with water, dried over anhydrous magnesium sulfate, and evaporated under reduced pressure. The residue was purified by silica gel column chromatography (hexane-ethyl acetate 1:1) to give the title compound (0.75 g, yield 92%) as crystals. melting point 186-187°C.
¹H-NMR (CDCl₃) :δ 1.60-1.80(3H, m), 2.00-2.15 (2H, m), 2.15-2.30 (1H, m), 2.70(3H, s), 2.76(2H, t, J=6.6 Hz), 3.75(2H, t, J=6.6 Hz), 3.65-3.80 (1H, m), 4.00-4.55 (1H, m), 5.36 (1H, d, J=7.2 Hz), 7.20-7.30 (1H, m), 7.32 (1H, d, J=7.2 Hz), 7.75 (1H, t, J=7.7 Hz), 8.03 (1H, d, J=6.9 Hz), 8.58 (1H, s), 11.53 (1H, s).

### Example 387

### N-(3-(((2-cyanoethyl)amino)carbonyl)-1H-pyrazol-4-yl)-6-methylpyridine-2-carboxamide

A mixture of N-[3-(((2-cyanoethyl)amino)carbonyl)-1-(tetrahydro-2H-pyran-2-yl)-1H-pyrazol-4-yl]-6-methylpyridine-2-carboxamide obtained in Example 386 (0.70 g, 1.83 mmol), p-toluenesulfonic acid monohydrate (0.77 g, 4.03 mmol) and ethanol (25 mL) was stirred at 70°C for 2 hr. The reaction mixture was concentrated under reduced pressure. Saturated aqueous sodium hydrogen carbonate solution was added, and the mixture was extracted with ethyl acetate. The extract was washed with water, dried over anhydrous magnesium sulfate, passed through a small amount of silica gel and concentrated under reduced pressure. Diethyl ether was added to the precipitated crystals, and the crystals were collected by filtration to give the title compound (0.53 g, yield 97%). melting point 225-227°C.
¹H-NMR (d₆-DMSO) :δ 2.61(3H, s), 2.81 (2H, t, J=6.2 Hz), 3.55(2H, q, J=6.2 Hz), 7.50-7.60(1H, m), 7.90-8.00(2H, m), 8.42(1H, s), 8.69(1H, t, J=5.8 Hz), 11.52(1H, s), 13.36(1H, s).

### Example 388

### 6-methyl-N-(1-(tetrahydro-2H-pyran-2-yl)-3-(((2,2,2-trifluoroethyl)amino)carbonyl)-1H-pyrazol-4-yl)pyridine-2-carboxamide

To a solution of 4-(((6-methylpyridin-2-yl)carbonyl)amino)-1-(tetrahydro-2H-pyran-2-yl)-1H-pyrazole-3-carboxylic acid obtained in Reference Example 47 (0.66 g, 2.0 mmol) and 2,2,2-trifluoroethylamine (0.19 mL, 2.4 mmol) in DMF (10 mL), WSC (0.41 g, 2.4 mmol) and HOBt (0.32 g, 2.4 mmol) were added, and the mixture was stirred at room temperature for 2.5 hr. Saturated aqueous sodium hydrogen carbonate solution was added to the reaction mixture, and the mixture was extracted with ethyl acetate. The extract was washed with water, dried over anhydrous magnesium sulfate, and evaporated under reduced pressure. The residue was purified by silica gel column chromatography (hexane-ethyl acetate 1:1) to give the title compound (0.77 g, yield 94%) as crystals. melting point 184-185°C.
¹H-NMR (CDCl₃) :δ 1.60-1.80 (4H, m), 2.07(2H, d, J=9.9 Hz), 2.15-2.30(2H, m), 2.69(3H, s), 3.65-3.80(1H, m), 4.05-4.20(3H, m), 5.38(1H, dd, J=9.6, 2.4 Hz), 7.10-7.20(1H, m), 7.32(1H, d, J=7.2 Hz), 7.75(1H, t, J=7.8 Hz), 8.03(1H, d, J=7.2 Hz), 11.51(1H, s).

### Example 389

### 6-methyl-N-(3-(((2,2,2-trifluoroethyl)amino)carbonyl)-1H-pyrazol-4-yl)pyridine-2-carboxamide

A mixture of 6-methyl-N-(1-(tetrahydro-2H-pyran-2-yl)-3-(((2,2,2-trifluoroethyl)amino)carbonyl)-1H-pyrazol-4-yl)pyridine-2-carboxamide obtained in Example 388 (0.72 g, 1.75 mmol), p-toluenesulfonic acid monohydrate (0.67 g, 3.50 mmol) and ethanol (15 mL) was stirred at 60°C for 3.5 hr. The reaction mixture was concentrated under reduced pressure. Saturated aqueous sodium hydrogen carbonate solution was added, and the mixture was extracted with ethyl acetate. The extract was washed with water, dried over anhydrous magnesium sulfate, and evaporated under reduced pressure. The residue was purified by silica gel column chromatography (hexane-ethyl acetate 1:1) to give the title compound (0.45 g, yield 79%). melting point 257-258°C.
¹H-NMR (d₆-DMSO):δ 2. 61 (3H, s), 4.00-4.20(2H, m), 7.50-7.60 (1H, m), 7.90-8.00(2H, m), 8.45(1H, s), 8.90-9.03 (1H, m), 11.44(1H, s), 13.47 (1H, s).

### Example 390

### N-(3-(((3-phenoxypropyl)amino)carbonyl)-1-(tetrahydro-2H-pyran-2-yl)-1H-pyrazol-4-yl)pyridine-2-carboxamide

To a solution of 4-((pyridin-2-ylcarbonyl)amino)-1-(tetrahydro-2H-pyran-2-yl)-1H-pyrazole-3-carboxylic acid obtained in Reference Example 54 (0.64 g, 2.0 mmol) and 3-phenoxypropylamine (0.36 g, 2.4 mmol) in DMF (10 mL), WSC (0.41 g, 2.4 mmol) and HOBt (0.32 g, 2.4 mmol) were added, and the mixture was stirred at room temperature for 7 hr. Saturated aqueous sodium hydrogen carbonate solution was added to the reaction mixture, and the mixture was extracted with ethyl acetate. The extract was washed with water, dried over anhydrous magnesium sulfate, passed through a small amount of silica gel and concentrated under reduced pressure. Diethyl ether was added to the precipitated crystals, and the crystals were collected by filtration to give the title compound (0.65 g, yield 72%) as crystal. melting point 106-107°C.
¹H-NMR (CDCl₃):δ 1.60-1.80(3H, m), 2.15(2H, t, J=6.3 Hz), 2.00-2.35(3H, m), 3.71(2H, q, J=6.3 Hz), 3.65-3.80(1H, m), 4.12(2H, t, J=5.7 Hz), 4.02-4.20(1H, m), 5.37(1H, d, J=9.3 Hz), 6.90-7.02(3H, m), 7.31(2H, d, J=7.2 Hz), 7.45(1H, dd, J=8.1, 4.8 Hz), 7,87(1H, t, J=8.4 Hz), 8.28(1H, d, J=8.1 Hz), 8.58(1H, s), 8.73(1H, d, J=4.5 Hz), 11.73(1H, s).

### Example 391

### N-(3-(((3-phenoxypropyl)amino)carbonyl)-1H-pyrazol-4-yl)pyridine-2-carboxamide

A mixture of N-(3-(((3-phenoxypropyl)amino)carbonyl)-1-(tetrahydro-2H-pyran-2-yl)-1H-pyrazol-4-yl)pyridine-2-carboxamide obtained in Example 390 (0.60 g, 1.33 mmol), p-toluenesulfonic acid monohydrate (0.51 g, 2.67 mmol) and ethanol (15 mL) was stirred at 60°C for 5 hr. The reaction mixture was concentrated under reduced pressure. Saturated aqueous sodium hydrogen carbonate solution was added, and the mixture was extracted with ethyl acetate. The extract was washed with water, dried over anhydrous magnesium sulfate, passed through a small amount of silica gel and concentrated under reduced pressure. Diethyl ether was added to the precipitated crystals, and the crystals were collected by filtration to give the title compound (0.44 g, yield 90%). melting point 182-184°C.
¹H-NMR (d₆-DMSO):δ 1.95-2.10 (2H, m), 3.46(2H, q, J=6.8 Hz), 4.04(2H, t, J=6.8 Hz), 6.90-7.00(3H, m), 7.28 (2H, t, J=7.2 Hz), 7.65-7.75 (1H, m), 8.02-8.15 (1H, m), 8.15 (1H, d, J=7.0 Hz), 8.41 (1H, s), 8.50-8.60 (1H, m), 8.75-8.80 (1H, m), 11.64 (1H, s), 13.33 (1H, s).

### Example 392

### N-(1-(tetrahydro-2H-pyran-2-yl)-3-(((3,3,3-trifluoropropyl)amino)carbonyl)-1H-pyrazol-4-yl)pyridine-2-carboxamide

To a solution of 4-((pyridin-2-ylcarbonyl)amino)-1-(tetrahydro-2H-pyran-2-yl)-1H-pyrazole-3-carboxylic acid obtained in Reference Example 54 (0.50 g, 1.57 mmol) and 3,3,3-trifluoropropylamine hydrochloride (0.235 g, 1.57 mmol) in DMF (10 mL), WSC (0.32 g, 1.89 mmol), HOBt (0.26 g, 1.89 mmol) and triethylamine (0.44 mL, 3.15 mmol) were added, and the mixture was stirred at room temperature for 8 hr. Saturated aqueous sodium hydrogen carbonate solution was added to the reaction mixture, and the mixture was extracted with ethyl acetate. The extract was washed with water, dried over anhydrous magnesium sulfate, passed through a small amount of silica gel and concentrated under reduced pressure. Diethyl ether was added to the precipitated crystals, and the crystals were collected by filtration to give the title compound (0.56 g, yield 87%) as crystals. melting point 144-145°C.
¹H-NMR (CDCl₃) :δ 1.60-1.80 (3H, m), 2.00-2.15 (2H, m), 2.15-2.30 (1H, m), 2.40-2.60(2H, m), 3.75(2H, q, J=6.9 Hz), 3.65-3.80 (1H, m), 4.02-4.15 (1H, m), 5.37 (1H, d, J=9.6 Hz), 7.05-7.20 (1H, m), 7.40-7.50 (1H, m), 7.88 (1H, t, J=7.8 Hz), 8.23 (1H, d, J=8.1 Hz), 8.58 (1H, s), 11.62 (1H, s).

### Example 393

### N-(3-(((3,3,3-trifluoropropyl)amino)carbonyl)-1H-pyrazol-4-yl)pyridine-2-carboxamide

A mixture of N-(1-(tetrahydro-2H-pyran-2-yl)-3-(((3,3,3-trifluoropropyl)amino)carbonyl)-1H-pyrazol-4-yl)pyridine-2-carboxamide obtained in Example 392 (0.51 g, 1.24 mmol), p-toluenesulfonic acid monohydrate (0.47 g, 2.48 mmol) and ethanol (15 mL) was stirred at 60°C for 5 hr. The reaction mixture was concentrated under reduced pressure. Saturated aqueous sodium hydrogen carbonate solution was added, and the mixture was extracted with ethyl acetate. The extract was washed with water, dried over anhydrous magnesium sulfate, passed through a small amount of silica gel and evaporated under reduced pressure. Diethyl ether was added to the precipitated crystals, and the crystals were collected by filtration to give the title compound (0.32 g, yield 79%). melting point 241-243°C.
¹H-NMR (d₆-DMSO) :δ 2.50-2.70 (2H, m), 3.54(2H, q, J=6.7 Hz), 7. 65-7.75(1H, m), 8.00-8.15(1H, m), 8.16(1H, d, J=9.6 Hz), 8.42(1H, s), 8.55-8.70(1H, m), 8.75-8.80(1H, m), 11.58(1H, s), 13.38(1H, s).

### Example 394

### N-(3-(((cyanomethyl)amino)carbonyl)-1-(tetrahydro-2H-pyran-2-yl)-1H-pyrazol-4-yl)pyridine-2-carboxamide

To a solution of 4-((pyridin-2-ylcarbonyl)amino)-1-(tetrahydro-2H-pyran-2-yl)-1H-pyrazole-3-carboxylic acid obtained in Reference Example 54 (0.64 g, 2.0 mmol) and aminoacetonitrile (0.11 g, 2.4 mmol) in DMF (10 mL), WSC (0.41 g, 2.4 mmol), HOBt (0.32 g, 2.4 mmol) and triethylamine (0.33 mL, 2.4 mmol) were added, and the mixture was stirred at room temperature for 6 hr. Saturated aqueous sodium hydrogen carbonate solution was added to the reaction mixture, and the mixture was extracted with ethyl acetate. The extract was washed with water, dried over anhydrous magnesium sulfate, passed through a small amount of silica gel and evaporated under reduced pressure. Diethyl ether was added to the precipitated crystals, and the crystals were collected by filtration to give the title compound (0.54 g, yield 76%) as crystals. melting point 213-214°C.
¹H-NMR (CDCl₃) :δ 1.60-1.80 (3H, m), 2. 00-2.15 (2H, m), 2.15-2.30(1H, m), 3.65-3.80(1H, m), 4.07(2H, d, J=11.4 Hz), 4.42(2H, d, J=6.0 Hz), 5.38 (1H, dd, J=9.0, 3.0 Hz), 7.17-7.30(1H, m), 7.40-7.55(1H, m), 7.80-7.95(1H, m), 8.23(1H, d, J=8.7 Hz), 8.60(1H, s), 8.70-8.80 (1H, m), 11.49 (1H, s).

### Example 395

### N-(3-(((cyanomethyl)amino)carbonyl)-1H-pyrazol-4-yl)pyridine-2-carboxamide

A mixture of N-(3-(((cyanomethyl)amino)carbonyl)-1-(tetrahydro-2H-pyran-2-yl)-1H-pyrazol-4-yl)pyridine-2-carboxamide obtained in Example 394 (0.50 g, 1.41 mmol), p-toluenesulfonic acid monohydrate (0.54 g, 2.82 mmol), ethanol (20 mL) and chloroform (10 mL) was stirred at 60°C for 6 hr. Saturated aqueous sodium hydrogen carbonate solution was added to the reaction mixture, and the mixture was extracted with ethyl acetate. The extract was washed with water, dried over anhydrous magnesium sulfate, passed through a small amount of silica gel and evaporated under reduced pressure. Diethyl ether was added to the precipitated crystals, and the crystals were collected by filtration to give the title compound (0.35 g, yield 92%). melting point not less than 255°C (decomposition).
¹H-NMR (d₆-DMSO) :δ 4.31(2H, d, J=4.5 Hz), 7.65-7.75(1H, m), 8.08(1H, t, J=7.5 Hz), 8.16(1H, d, J=8.1 Hz), 8.45(1H, s), 8.75-9.00(1H, m), 9.10-9.20(1H, m), 11.49(1H, s), 13.47(1H, s).

### Example 396

### N-(3-(((2-(pyridin-2-ylamino)ethyl)amino)carbonyl)-1-(tetrahydro-2H-pyran-2-yl)-1H-pyrazol-4-yl)pyridine-2-carboxamide

To a solution of 4-((pyridin-2-ylcarbonyl)amino)-1-(tetrahydro-2H-pyran-2-yl)-1H-pyrazole-3-carboxylic acid obtained in Reference Example 54 (0.63 g, 2.0 mmol) and 2-pyridylethylenediamine·dihydrochloride (0.50 g, 2.4 mmol) in DMF (10 mL), WSC (0.41 g, 2.4 mmol), HOBt (0.32 g, 2.4 mmol) and triethylamine (0.50 mL, 3.6 mmol) were added, and the mixture was stirred at room temperature for 10 hr. Saturated aqueous sodium hydrogen carbonate solution was added to the reaction mixture, and the mixture was extracted with ethyl acetate. The extract was washed with water, dried over anhydrous magnesium sulfate, passed through a small amount of silica gel and concentrated under reduced pressure. Diethyl ether was added to the precipitated crystals, and the crystals were collected by filtration to give the title compound (0.62 g, yield 71%) as crystals. melting point 178-180°C.
¹H-NMR (CDCl₃) :δ 1.55-1.80(3H, m), 2.00-2.15(2H, m), 2.15-2.30(1H, m), 3.55-3.80(6H, m), 4.00-4.15(1H, m), 4.75-4.85(1H, m), 5.36(1H, dd, J=9.6, 1.8 Hz), 6.44(1H, d, J=8.4 Hz), 6.55-6.62(1H, m), 7.35-7.70(1H, m), 7.85(1H, t, J=8.4 Hz), 8.10-8.20(1H, m), 8.23(1H, d, J=7.8 Hz), 8.70-8.80(1H, m), 11.71(1H, s).

### Example 397

### N-(3-(((2-(pyridin-2-ylamino)ethyl)amino)carbonyl)-1H-pyrazol-4-yl)pyridine-2-carboxamide

A mixture of N-(3-(((2-(pyridin-2-ylamino)ethyl)amino)carbonyl)-1-(tetrahydro-2H-pyran-2-yl)-1H-pyrazol-4-yl)pyridine-2-carboxamide obtained in Example 396 (0.58 g, 1.33 mmol), p-toluenesulfonic acid monohydrate (0.76 g, 4.00 mmol) and ethanol (15 mL) was stirred at 60°C for 4 hr. Saturated aqueous sodium hydrogen carbonate solution (10 mL) and water (20 mL) were added to the reaction mixture, and the precipitated crystals were collected by filtration, washed with water, acetone and diethyl ether to give the title compound (0.39 g, yield 82%). melting point 260-262°C.
¹H-NMR (d₆-DMSO):δ 3.40-3.60(4H, m), 6.40-6.50(2H, m), 6.60-6.70(1H, m), 7.37(1H, t, J=6.8 Hz), 7.60-7.75(1H, m), 7.95-8.03 (1H, m), 8.07 (1H, t, J=6.8 Hz), 8.16(1H, d, J=9.0 Hz), 8.40(1H, s), 8.60-8.70(1H, m), 8.70-8.80(1H, m), 11.64(1H, s), 13.32(1H, s).

### Example 398

### N-(3-(((2-anilinoethyl)amino)carbonyl)-1-(tetrahydro-2H-pyran-2-yl)-1H-pyrazol-4-yl)pyridine-2-carboxamide

To a solution of 4-((pyridin-2-ylcarbonyl)amino)-1-(tetrahydro-2H-pyran-2-yl)-1H-pyrazole-3-carboxylic acid obtained in Reference Example 54 (0.63 g, 2.0 mmol) and N-phenylethylenediamine (0.31 mL, 2.4 mmol) in DMF (10 mL), WSC (0.41 g, 2.4 mmol), HOBt (0.32 g, 2.4 mmol) and triethylamine (0.33 mL, 2.4 mmol) were added, and the mixture was stirred at room temperature for 3 hr. Saturated aqueous sodium hydrogen carbonate solution was added to the reaction mixture, and the mixture was extracted with ethyl acetate. The extract was washed with water, dried over anhydrous magnesium sulfate, passed through a small amount of silica gel and evaporated under reduced pressure. Diethyl ether was added to the precipitated crystals, and the crystals were collected by filtration to give the title compound (0.69 g, yield 79%) as crystals. melting point 182-183°C.
¹H-NMR (CDCl₃):δ 1.55-1.80(3H, m), 1.95-2.15(2H, m), 2.15-2.30(1H, m), 3.35-3.50(2H, m), 3.65-3.80(3H, m), 4.00-4.15(2H, m), 5.35(1H, dd, J=9.6 Hz), 6.66(2H, d, J=8.4 Hz), 6.72(1H, t, J=7.2 Hz), 7.10-7.30(3H, m), 7.40-7.50(1H, m), 7.88(1H, td, J=7.5, 1.5 Hz), 8.24(1H, d, J=7.8 Hz), 8.58(1H, s), 8.75(1H, d, J=4.5 Hz), 11.69(1H, s).

### Example 399

### N-(3-(((2-anilinoethyl)amino)carbonyl)-1H-pyrazol-4-yl)pyridine-2-carboxamide

A mixture of N-(3-(((2-anilinoethyl)amino)carbonyl)-1-(tetrahydro-2H-pyran-2-yl)-1H-pyrazol-4-yl)pyridine-2-carboxamide obtained in Example 398 (0.64 g, 1.47 mmol), p-toluenesulfonic acid monohydrate (0.84 g, 4.42 mmol) and ethanol (15 mL) was stirred at 70°C for 5 hr. Saturated aqueous sodium hydrogen carbonate solution was added to the reaction mixture, and the mixture was extracted with ethyl acetate. The extract was washed with water, dried over anhydrous magnesium sulfate, passed through a small amount of silica gel and concentrated under reduced pressure. Diethyl ether was added to the precipitated crystals, and the crystals were collected by filtration to give the title compound (0.28 g, yield 54%). melting point 184-186°C.
¹H-NMR (d₆-DMSO) :δ 3.15-3.30(2H, m), 3.40-3.60(2H, m), 5.70-5.80(1H, m), 6.50-6. 60(1H, m), 6.62(2H, d, J=7.2 Hz), 7.00-7.15(2H, m), 7.63-7.75(1H, m), 8.00-8.15(1H, m), 8.16(1H, d, J=9.0 Hz), 8.42(1H, s), 8.50-8.60(1H, m), 8.75-8.80 (1H, m), 11.63(1H, s), 13.33(1H, s).

### Example 400

### N-(3-(((2-hydroxyethyl)amino)carbonyl)-1-(tetrahydro-2H-pyran-2-yl)-1H-pyrazol-4-yl)pyridine-2-carboxamide

To a solution of 4-((pyridin-2-ylcarbonyl)amino)-1-(tetrahydro-2H-pyran-2-yl)-1H-pyrazole-3-carboxylic acid obtained in Reference Example 54 (0.63 g, 2.0 mmol) and 2-aminoethanol (0.14 mL, 2.4 mmol) in DMF (10 mL), WSC (0.41 g, 2.4 mmol), HOBt (0.32 g, 2.4 mmol) and triethylamine (0.33 mL, 2.4 mmol) were added, and the mixture was stirred at room temperature for 10 hr. Saturated aqueous sodium hydrogen carbonate solution was added to the reaction mixture, and the mixture was extracted with ethyl acetate. The extract was washed with water, dried over anhydrous magnesium sulfate, passed through a small amount of silica gel and evaporated under reduced pressure. Diethyl ether was added to the precipitated crystals, and the crystals were collected by filtration to give the title compound (0.53 g, yield 73%) as crystals. melting point 214-215°C.
¹H-NMR (CDCl₃) :δ 1.60-1.80(3H, m), 2.00-2.15(2H, m), 2.15-2.30(1H, m), 3.60-3.80(3H, m), 3.80-3.90(2H, m), 4.00-4.15(1H, m), 5.36(1H, d, J=6.9 Hz), 7.20-7.40(1H, m), 7.40-7.50(1H, m), 7.87(1H, t, J=8.0 Hz), 8.22(1H, d, J=7.8 Hz), 8.57(1H, s), 8.72(1H, d, J=5.4 Hz), 11.65(1H, s).

### Example 401

### N-(3-(((2-hydroxyethyl)amino)carbonyl)-1H-pyrazol-4-yl)pyridine-2-carboxamide

A mixture of N-(3-(((2-hydroxyethyl)amino)carbonyl)-1-(tetrahydro-2H-pyran-2-yl)-1H-pyrazol-4-yl)pyridine-2-carboxamide obtained in Example 400 (0.48 g, 1.34 mmol), p-toluenesulfonic acid monohydrate (0.51 g, 2.67 mmol) and ethanol (10 mL) was stirred at 60°C for 3 hr. Saturated aqueous sodium hydrogen carbonate solution was added to the reaction mixture, and the mixture was extracted with ethyl acetate. The extract was washed with water, dried over anhydrous magnesium sulfate, passed through a small amount of silica gel and concentrated under reduced pressure. Diethyl ether was added to the precipitated crystals, and the crystals were collected by filtration to give the title compound (0.32 g, yield 87%). melting point 155-156°C.
¹H-NMR (d₆-DMSO):δ 3.30-3.50(3H, m), 3.50-3.60(2H, m), 4.75-4.85(1H, m), 7.65-7.75(1H, m), 8.08(1H, t, J=6.0 Hz), 8.16(1H, d, J=8.1 Hz), 8.20-8.30(1H, m), 8.41(1H, s), 8.75(1H, d, J=4.5 Hz), 11.63(1H, s), 13.32(1H, s).

### Example 402

### N-(2-cyanoethyl)-4-((cyclopentylcarbonyl)amino)-1-(tetrahydro-2H-pyran-2-yl)-1H-pyrazole-3-carboxamide

To a solution of 4-amino-N-(2-cyanoethyl)-1-(tetrahydro-2H-pyran-2-yl)-1H-pyrazole-3-carboxamide obtained in Reference Example 52 (395 mg, 1.5 mmol) and cyclopentanecarboxylic acid (0.179 mL, 1.65 mmol) in DMF (5 mL), WSC (345 mg, 1.8 mmol) and HOBt (243 mg, 1.8 mmol) were added, and the mixture was stirred at room temperature for 4 hr. The reaction mixture was diluted with water, and extracted with ethyl acetate. The extract was washed with saturated aqueous sodium hydrogen carbonate solution, dried over anhydrous sodium sulfate, and evaporated under reduced pressure. The residue was purified by silica gel column chromatography (ethyl acetate-hexane, 1:1) to give the title compound 444 m g (yield 75%) as an oil.
¹H-NMR (CDCl₃): δ 1.57-2.25(14H, m), 2.67-2.80(3H, m), 3.63-3.75(3H, m), 3.97-4.07(1H, m), 5.27-5.23(1H, m), 7.24(1H, t, J=6.3 Hz), 8.38 (1H, s), 9.39 (1H, s).

### Example 403

### N-(2-cyanoethyl)-4-(2-furoylamino)-1-(tetrahydro-2H-pyran-2-yl)-1H-pyrazole-3-carboxamide

In the same manner as in Example 402, the title compound was obtained using 4-amino-N-(2-cyanoethyl)-1-(tetrahydro-2H-pyran-2-yl)-1H-pyrazole-3-carboxamide obtained in Reference Example 52 and 2-furancarboxylic acid. yield 74%. melting point 157-159°C (ethyl acetate-hexane).
¹H-NMR (CDCl₃) :δ 1.60-1.80(3H, m), 2.01-2.12(2H, m), 2.13-2.25(1H, m), 2.74(2H, t, J=6.6 Hz), 3.65-3.79(3H, m), 4.02-4.13(1H, m), 5.36(1H, dd, J=2.3 Hz, 9.5 Hz), 6.54(1H, dd, J=1.7 Hz, 3.6 Hz), 7.20-7.26(2H, m), 7.55-7.56(1H, m), 8.45(1H, s), 10.27(1H, s).

### Example 404

### N-(2-cyanoethyl)-4-(3-methylbenzoylamino)-1-(tetrahydro-2H-pyran-2-yl)-1H-pyrazole-3-carboxamide

In the same manner as in Example 402, the title compound was obtained using 4-amino-N-(2-cyanoethyl)-1-(tetrahydro-2H-pyran-2-yl)-1H-pyrazole-3-carboxamide obtained in Reference Example 52 and 3-methylbenzoic acid. yield 66%. melting point 113-114°C (ethyl acetate-hexane).
¹H-NMR (CDCl₃) : δ 1.60-1.81(3H, m), 2.02-2.11(1H, m), 2.13-2.48(1H, m), 2.44(3H, s), 2.74(2H, t, J=6.6 Hz), 3.67-3.79(3H, m), 4.03-4.11(1H, m), 5.36(1H, dd, J =2.3 Hz, 9.5 Hz), 7.22-7.30 (1H, m), 7.33-7.40(2H, m), 7.71-7.75 (1H, m), 7.77 (1H, s), 8.54 (1H, s), 10.27 (1H, s).

### Example 405

### N-(2-cyanoethyl)-4-(3-methoxybenzoylamino)-1-(tetrahydro-2H-pyran-2-yl)-1H-pyrazole-3-carboxamide

In the same manner as in Example 402, the title compound was obtained using 4-amino-N-(2-cyanoethyl)-1-(tetrahydro-2H-pyran-2-yl)-1H-pyrazole-3-carboxamide obtained in Reference Example 52 and 3-methoxybenzoic acid. yield 82%. melting point 118-121 (ethyl acetate-hexane).
¹H-NMR (CDCl₃):δ 1.60-1.78(3H, m), 1.99-2.09(2H, m), 2.12-2.43(1H, m), 2.74(2H, t, J=6.6 Hz), 3.67-3.75(3H, m), 3.88(3H, s), 4.02-4.10(1H, m), 5.36(1H, dd, J=2.2 Hz and 9.3 Hz), 7.05-7.12(1H, m), 7.22-7.28(1H, m), 7.39(1H, t, J=7.8 Hz), 7.46-7.50(1H, m), 7.51-7.55(1H, m), 8.53(1H, s).

### Example 406

### N-(2-cyanoethyl)-4-(2,2-dimethylpropanoylamino)-1-(tetrahydro-2H-pyran-2-yl)-1H-pyrazole-3-carboxamide

In the same manner as in Example 402, the title compound was obtained using 4-amino-N-(2-cyanoethyl)-1-(tetrahydro-2H-pyran-2-yl)-1H-pyrazole-3-carboxamide obtained in Reference Example 52 and pivalic acid. yield 82%. yield 87%. oily substance.
¹H-NMR (CDCl₃):δ 1.31(9H, s), 1.61-1.75(3H, m), 1.97-2.05(2H, m), 2.10-2.19(1H, m), 2.72(2H, t, J=6.6 Hz), 3.63-3.75(3H, m), 3.99-4.06(1H, m), 5.31(1H, dd, J=2.5 Hz and 9.3 Hz), 7.20-7.28 (1H, m), 8.40(1H, s), 9.75(1H, brs).

### Example 407

### N-(2-cyanoethyl)-4-(2-thienylcarbonylamino)-1-(tetrahydro-2H-pyran-2-yl)-1H-pyrazole-3-carboxamide

In the same manner as in Example 402, the title compound was obtained using 4-amino-N-(2-cyanoethyl)-1-(tetrahydro-2H-pyran-2-yl)-1H-pyrazole-3-carboxamide obtained in Reference Example 52 and thiophene-2-carboxylic acid. yield 88%. melting point 155-158°C (ethyl acetate-hexane).
¹H-NMR (CDCl₃):δ 1.60-1.78 (3H, m), 1.99-2.09(2H, m), 2.12-2.45 (1H, m), 2.74(2H, t, J=6.6 Hz), 3.68-3.78(3H, m), 3.88(3H, s), 4.03-4.10 (1H, m), 5.36 (1H, dd, J=2.2 Hz and 9.3 Hz), 7.13 (1H, dd, J=3.7 Hz and 5.1 Hz), 7.20-7.28 (1H, m), 7.55 (1H, dd, J=1.2 Hz and 5.1 Hz), 7.67 (1H, dd, J=1.2 Hz and 3.7 Hz), 8.45 (1H, s).

### Example 408

### 4-(3-cyanobenzoylamino)-N-(2-cyanoethyl)-1-(tetrahydro-2H-pyran-2-yl)-1H-pyrazole-3-carboxamide

In the same manner as in Example 402, the title compound was obtained using 4-amino-N-(2-cyanoethyl)-1-(-tetrahydro-2H-pyran-2-yl)-1H-pyrazole-3-carboxamide obtained in Reference Example 52 and 3-cyanobenzoic acid. yield 84%. melting point 161-163°C (ethyl acetate-hexane).
¹H-NMR (CDCl₃) :δ 1.60-1.82(3H, m), 1.99-2.26(3H, m), 2.75(2H, t, J=6.6 Hz), 3.70-3.78(3H, m), 4.04-4.12(1H, m), 5.36(1H, dd, J=2.3 Hz and 9.4 Hz), 7.20-7.28(1H, m), 7.63(1H, t, J=7.8 Hz), 7.81-7.86(1H, m), 8.11-8.17(1H, m), 8.25-8.27(1H, m), 8.51(1H, s), 10.44(1H, s).

### Example 409

### N-(2-cyanoethyl)-4-(4-methylbenzoylamino)-1-(tetrahydro-2H-pyran-2-yl)-1H-pyrazole-3-carboxamide

In the same manner as in Example 402, the title compound was obtained using 4-amino-N-(2-cyanoethyl)-1-(tetrahydro-2H-pyran-2-yl)-1H-pyrazole-3-carboxamide obtained in Reference Example 52 and 4-methylbenzoic acid. yield 74%. melting point 133-135°C (ethyl acetate-hexane).
¹H-NMR (CDCl₃):δ 1.60-1.82 (3H, m), 2.00-2.36(3H, m), 2.43(3H, s), 2.74(2H, t, J=6.6 Hz), 3.70-3.78(3H, m), 4.02-4.12(1H, m), 5.36(1H, dd, J=2.3 Hz and 9.5 Hz), 7.20-7.28(3H, m), 7.85(2H, d, J=8.2 Hz), 8.53(1H, s), 10.28 (1H, s).

### Example 410

### N-(2-cyanoethyl)-4-(4-methoxybenzoylamino)-1-(tetrahydro-2H-pyran-2-yl)-1H-pyrazole-3-carboxamide

In the same manner as in Example 402, the title compound was obtained using 4-amino-N-(2-cyanoethyl)-1-(tetrahydro-2H-pyran-2-yl)-1H-pyrazole-3-carboxamide obtained in Reference Example 52 and 4-methoxybenzoic acid. yield 82%. melting point 162-164°C (ethyl acetate-hexane).
¹H-NMR (CDCl₃) :δ 1.60-1.80(3H, m), 2.01-2.13(2H, s), 2.15-2.36 (1H, m), 2.79(2H, t, J=6.6 Hz), 3.68-3.78(3H, m), 3.87(3H, s), 4.02-4.12(1H, m), 5.36(1H, dd, J=2.5 Hz and 9.3 Hz), 6.98(2H, d, J=8.9 Hz), 7.23-7.28(1H, m), 7.92(2H, d, J=8.9 Hz), 8.52(1H, s), 10.24 (1H, s).

### Example 411

### N-(2-cyanoethyl)-4-((cyclopentylcarbonyl)amino)-1H-pyrazole-3-carboxamide

In the same manner as in Example 401, the title compound was obtained using N-(2-cyanoethyl)-4-((cyclopentylcarbonyl)amino)-1-(tetrahydro-2H-pyran-2-yl)-1H-pyrazole-3-carboxamide obtained in Example 402. yield 75%. melting point 180-181°C (ethyl acetate-hexane).
¹H-NMR (CDCl₃) :δ 1.60-2.07(9H, m), 2.74-2.82(3H, m), 3.69(2H, q, J=6.6 Hz), 7.72(1H, br), 8.25(1H, s), 9.51(1H, s), 12.60(1H, s).

### Example 412

### N-(2-cyanoethyl)-4-(2-furoylamino)-1H-pyrazole-3-carboxamide

In the same manner as in Example 401, the title compound was obtained using N-(2-cyanoethyl)-4-(2-furoylamino)-1-(tetrahydro-2H-pyran-2-yl)-1H-pyrazole-3-carboxamide obtained in Example 403. yield 75%. melting point (decomposition) 260-265°C (ethyl acetate-hexane).
¹H-NMR (DMSO-d₆):δ 2.81(2H, t, J=6.5 Hz), 3.53(2H, q, J=6.5 Hz), 6.71-6.74(1H, m), 7.23(1H, d, J=3.0 Hz), 7.97(1H, s), 8.29(1H, s), 8.81(1H, t, J=5.8 Hz), 10.49(1H, s), 13.37(1H, m).

### Example 413

### N-(2-cyanoethyl)-4-(3-methylbenzoylamino)-1H-pyrazole-3-carboxamide

In the same manner as in Example 401, the title compound was obtained using N-(2-cyanoethyl)-4-(3-methylbenzoylamino)-1-(tetrahydro-2H-pyran-2-yl)-1H-pyrazole-3-carboxamide obtained in Example 404. yield 82%. melting point 149-151°C (ethyl acetate-hexane).
¹H-NMR (CDCl₃) :δ 2.45(3H, s), 2.77 (2H, t, J=6.6 Hz), 3.75(2H, q, J=6.6 Hz), 7.35-7.43(3H, m), 7.70-7.80(2H, m), 8.52(1H, s), 10.33(1H, s), 10.45(1H, brs).

### Example 414

### N-(2-cyanoethyl)-4-(3-methoxybenzoylamino)-1H-pyrazole-3-carboxamide

In the same manner as in Example 401, the title compound was obtained using N-(2-cyanoethyl)-4-(3-methoxybenzoylamino)-1-(tetrahydro-2H-pyran-2-yl)-1H-pyrazole-3-carboxamide obtained in Example 405. yield 55%. melting point 118-121°C (ethyl acetate-hexane).
¹H-NMR (CDCl₃) :δ 2.76(2H, t, J=6.6 Hz), 3.72(2H, q, J=6.6 Hz), 3.88(3H, s), 7.05-7.10(1H, m), 7.36-7.53(3H, m), 7.84(1H, brt, J=5.8 Hz), 8.37(1H, s), 10.44(1H, s), 12.22(1H, s).

### Example 415

### N-(2-cyanoethyl)-4-(2,2-dimethylpropanoylamino)-1H-pyrazole-3-carboxamide

In the same manner as in Example 401, the title compound was obtained using N-(2-cyanoethyl)-4-(2,2-dimethylpropanoylamino)-1-(tetrahydro-2H-pyran-2-yl)-1H-pyrazole-3-carboxamide obtained in Example 406. yield 77%. melting point 165-166°C (ethyl acetate-hexane).
¹H-NMR (CDCl₃):δ 1.33(9H, s), 2.74(2H, t, J=6.3 Hz), 3.72(2H, q, J=6.3 Hz), 7.37(1H, br), 8.35(1H, s), 9.82(1H, s), 10.53(1H, br).

### Example 416

### N-(2-cyanoethyl)-4-(2-thienylcarbonylamino)-1H-pyrazole-3-carboxamide

In the same manner as in Example 401, the title compound was obtained using N-(2-cyanoethyl)-4-(2-thienylcarbonylamino)-1-(tetrahydro-2H-pyran-2-yl)-1H-pyrazole-3-carboxamide obtained in Example 407. yield 77%. melting point 226-227°C (ethyl acetate-hexane).
¹H-NMR (CDCl₃):δ 2.74 (2H, t, J=6.6 Hz), 3.73 (2H, q, J=6.6 Hz), 7.13-7.16(1H, m), 7.54-7.58(1H, m), 7.66-7.69(1H, m), 7.83(1H, br), 8.29 (1H, s), 10.32(1H, s), 12.77(1H, m).

### Example 417

### N-(2-cyanoethyl)-4-(3-cyanobenzoylamino)-1H-pyrazole-3-carboxamide

In the same manner as in Example 401, the title compound was obtained using 4-(3-cyanobenzoylamino)-N-(2-cyanoethyl)-1-(tetrahydro-2H-pyran-2-yl)-1H-pyrazole-3-carboxamide obtained in Example 408. yield 81%. melting point 248-249°C (ethyl acetate).
¹H-NMR (DMSO-d₆) :δ 2.81(2H, t, J=6.6 Hz), 3.53(2H, q, J=6.6 Hz), 7.80(1H, t, J=7.8 Hz), 8.08-8.17(2H, m), 8.25(1H, s), 8.34(1H, s), 8.84(1H, t, J=5.9 Hz), 10.63(1H, s), 13.42(1H, s).

### Example 418

### N-(2-cyanoethyl)-4-(4-methylbenzoylamino)-1H-pyrazole-3-carboxamide

In the same manner as in Example 401, the title compound was obtained using N-(2-cyanoethyl)-4-(4-methylbenzoylamino)-1-(tetrahydro-2H-pyran-2-yl)-1H-pyrazole-3-carboxamide obtained in Example 409. yield 86%. melting point 207-209°C (ethyl acetate-hexane).
¹H-NMR (DMSO-d₆):δ 2.40(3H, s), 2.81(2H, t, J=6.6 Hz), 3.53(2H, q, J=6.6 Hz), 7.39(2H, d, J=8.2 Hz), 7.77(2H, t, J=8.2 Hz), 8.33(1H, d, J=1.1 Hz), 8.83(1H, t, J=6.6 Hz), 10.59(1H, s), 13.35(1H, s).

### Example 419

### 4-(benzoylamino)-N-(2-cyanoethyl)-1-(tetrahydro-2H-pyran-2-yl)-1H-pyrazole-3-carboxamide

In the same manner as in Example 1, the title compound was obtained using 4-(benzoylamino)-1-(tetrahydro-2H-pyran-2-yl)-1H-pyrazole-3-carboxylic acid obtained in Reference Example 49 and 3-aminopropionitrile. yield 90%. melting point 143-144°C (ethyl acetate).
¹H-NMR (CDCl₃) :δ 1.60-1.72(3H, m), 2.00-2.23(3H, m), 2.73(2H, t, J=6.3 Hz), 3.64-3.73(3H, m), 4.05(1H, d, J=11.6 Hz), 5.34(1H, dd, J=2.1 Hz and J=9.3 Hz), 7.48-7.57(3H, m), 7.95(2H, td, J=1.7 Hz and J=6.6 Hz), 8.53(1H, s).

### Example 420

### 4-(benzoylamino)-N-(2-cyanoethyl)-1H-pyrazole-3-carboxamide

In the same manner as in Example 387, the title compound was obtained using 4-(benzoylamino)-N-(2-cyanoethyl)-1-(tetrahydro-2H-pyran-2-yl)-1H-pyrazole-3-carboxamide obtained in Example 419. yield 97%. melting point 186-187°C (ethyl acetate).
¹H-NMR (DMSO-d₆) :δ 2.79(2H, t, J=6.6 Hz), 3. 52 (2H, q, J=6.6 Hz), 7.57-7.62(3H, m), 7.86(2H, d, J=6.3 Hz), 8.23(1H, s), 8.83(1H, brs).

### Example 421

### 4-(benzoylamino)-1-(tetrahydro-2H-pyran-2-yl)-N-(2,2,2-trifluoroethyl)-1H-pyrazole-3-carboxamide

In the same manner as in Example 386, the title compound was obtained using 4-(benzoylamino)-1-(tetrahydro-2H-pyran-2-yl)-1H-pyrazole-3-carboxylic acid obtained in Reference Example 49 and 2,2,2-trifluoroethylamine. yield 69%. melting point 153-154°C (ethyl acetate).
¹H-NMR (CDCl₃) :δ 1.63-1.79(3H, m), 2.04-2.21(3H, m), 4.04-4.15(3H, m), 5.37(1H, dd, J=2.4 Hz and J=9.6 Hz), 7.46-7.55(5H, m), 7.95(2H, dd, J=1.6 Hz and J=8.0 Hz), 8.55(1H, s).

### Example 422

### 4-(benzoylamino)-N-(2,2,2-trifluoroethyl)-1H-pyrazole-3-carboxamide

In the same manner as in Example 387, the title compound was obtained using 4-(benzoylamino)-1-(tetrahydro-2H-pyran-2-yl)-N-(2,2,2-trifluoroethyl)-1H-pyrazole-3-carboxamide obtained in Example 421. yield 70%. melting point 203-206°C (ethyl acetate).
¹H-NMR (DMSO-d₆) :δ 4.08(2H, q, J=9.3 Hz), 7.57-7.67(3H, m), 7.89(2H, d, J=6.3 Hz), 8.37(1H, s), 9.12(1H, brs).

### Example 423

### 4-(benzoylamino)-N-(2-isopropoxyethyl)-1-(tetrahydro-2H-pyran-2-yl)-1H-pyrazole-3-carboxamide

In the same manner as in Example 386, the title compound was obtained using 4-(benzoylamino)-1-(tetrahydro-2H-pyran-2-yl)-1H-pyrazole-3-carboxylic acid obtained in Reference Example 49 and 2-aminoethylisopropylether. yield 91%. melting point 85-87°C (ethyl acetate).
¹H-NMR (CDCl₃):δ 1.18(6H, d, J=7.2 Hz), 1.61-1.78 (4H, m), 2.04,(2H, d, J=9.3 Hz), 3.46(1H, m), 3.58-3.75(5H, m), 4.05(1H, d, J=11.1 Hz), 5.37(1H, dd, J=2.4 Hz and J=9.3 Hz), 7.43-7.55(3H, m), 7.96(2H, d, J=1.6 Hz and J=8.0 Hz), 8.51(1H, s).

### Example 424

### 4-(benzoylamino)-N-(2-isopropoxyethyl)-1H-pyrazole-3-carboxamide

In the same manner as in Example 387, the title compound was obtained using 4-(benzoylamino)-N-(2-isopropoxyethyl)-1-(tetrahydro-2H-pyran-2-yl)-1H-pyrazole-3-carboxamide obtained in Example 423. yield 81%. melting point 121-122°C (ethyl acetate).
¹H-NMR (DMSO-d₆):δ 1.29(6H, d, J=6.0 Hz), 3.63-3.83(5H, m), 7.47-7.58(3H, m), 8.02(2H, d, J=7.4 Hz), 8.49 (1H, s), 9.40 (1H, brs).

### Example 425

### N-(3-(((2-isopropoxyethyl)amino)carbonyl)-1-(tetrahydro-2H-pyran-2-yl)-1H-pyrazol-4-yl)-6-methylpyridine-2-carboxamide

In the same manner as in Example 386, the title compound was obtained using 4-(((6-methylpyridin-2-yl)carbonyl)amino)-1-(tetrahydro-2H-pyran-2-yl)-1H-pyrazole-3-carboxylic acid obtained in Reference Example 47 and 2-aminoethylisopropylether. yield 88%. melting point 134-135°C (ethyl acetate).
¹H-NMR (CDCl₃) :δ 1.12(6H, d, J=6.0 Hz), 1.62(3H, m), 2.06(2H, d, J=9.6 Hz), 2.20-2.24(1H, m), 2.69(3H, s), 3.57-3.76(6H, m), 4.06(1H, d, J=11.6 Hz), 5.38(1H, dd, J=2.7 Hz and J=9.6 Hz), 7.30(1H, d, J=7.7 Hz), 7.74(1H, t, J=7.7 Hz), 8.03(1H, d, J=7.7 Hz), 8.58 (1H, s).

### Example 426

### N-(3-(((2-isopropoxyethyl)amino)carbonyl)-1H-pyrazol-4-yl)-6-methylpyridine-2-carboxamide

In the same manner as in Example 387, the title compound was obtained using N-(3-(((2-isopropoxyethyl)amino)carbonyl)-1-(tetrahydro-2H-pyran-2-yl)-1H-pyrazol-4-yl)-6-methylpyridine-2-carboxamide obtained in Example 425. yield 82%. melting point 150-151°C (ethyl acetate).
¹H-NMR (DMSO-d₆) :δ 1.10(6H, d, J=6.0 Hz), 2.61(3H, s), 3.40-3.63(5H, m), 7.53(1H, dd, J=3.3 Hz and J=5.5 Hz), 7.91-7.97(2H, m), 8.18(1H, t, J=5.5 Hz), 8.40(1H, s).

### Example 427

### 6-methyl-N-(3-(((2-(4-fluorophenyloxy)ethyl)amino)carbonyl-1-(tetrahydro-2H-pyran-2-yl)-1H-pyrazol-4-yl)pyridine-2-carboxamide

In the same manner as in Example 386, the title compound was obtained using 4-(((6-methylpyridin-2-yl)carbonyl)amino)-1-(tetrahydro-2H-pyran-2-yl)-1H-pyrazole-3-carboxylic acid obtained in Reference Example 47 and 2-(4-fluorophenyloxy)ethylamine. yield 20%. melting point 188-190°C (ethyl acetate).
¹H-NMR (CDCl₃):δ 1.64-1.80(3H, m), 2.10(11.2 Hz), 2.25-2.28(1H, m), 2.70(3H, s), 3.74(1H,t, 9.6 Hz), 4.07(1H, d, J=11.2 Hz), 5.00(2H, d, J=4.4 Hz), 5.43(1H, dd, J=2.8 Hz and J=9.6 Hz), 7.31(1H, d, J=7.7 Hz), 7.53(2H, t, J=7.2 Hz), 7.64(1H, t, J=7.2 Hz), 7.75(1H, t, J=7.7 Hz), 8.03-8.06(3H, m), 8.60(1H, s).

### Example 428

### 6-methyl-N-(3-(((2-(4-fluorophenyloxy)ethyl)amino)-1H-pyrazol-4-yl)pyridine-2-carboxamide

In the same manner as in Example 387, the title compound was obtained using 6-methyl-N-(3-(((2-(4-fluorophenyloxy)ethyl)amino)carbonyl)-1-(tetrahydro-2H-pyran-2-yl)-1H-pyrazol-4-yl)pyridine-2-carboxamide obtained in Example 427. yield 71%. melting point 200-201°C.
¹H-NMR (DMSO-d₆):δ 2.55(3H, s), 4.86(2H, d, J=5.7 Hz), 7.50 (1H, d, J=7.2 Hz), 7.61(2H,t, 7.6 Hz), 7.93-7.96(2H, m), 8.08(2H, d, J=7.6 Hz), 8.44 (1H, s).

### Example 429

### N-(3-(((3-isopropoxypropyl)amino)carbonyl)-1-(tetrahydro-2H-pyran-2-yl)-1H-pyrazol-4-yl)-6-methylpyridine-2-carboxamide

In the same manner as in Example 386, the title compound was obtained using 4-(((6-methylpyridin-2-yl)carbonyl)amino)-1-(tetrahydro-2H-pyran-2-yl)-1H-pyrazole-3-carboxylic acid obtained in Reference Example 47 and 2-isopropoxypropylamine. yield 90%. melting point 105-106°C.
¹H-NMR (CDCl₃):δ 1.20(6H, d, J=6.1 Hz), 1.58-1.73(3H, m), 1.80-1.94(2H, m), 2.01-2.04(2H, m), 2.68(3H, s), 3.52-3.68(7H, m), 4.04(1H, d, J=11.8 Hz), 5.33(1H, d, J=9.4 Hz), 7.28(1H, d, J=7.7 Hz), 7.4.5(1H, brs), 7.72(1H, t, J=7.7 Hz), 8.00(1H, d, J=7.7 Hz), 8.55(1H, s).

### Example 430

### N-(3-(((3-isopropoxypropyl)amino)carbonyl)-1H-pyrazol-4-yl)-6-methylpyridine-2-carboxamide

In the same manner as in Example 387, the title compound was obtained using N-(3-(((3-isopropoxypropyl)amino)carbonyl)-1-(tetrahydro-2H-pyran-2-yl)-1H-pyrazol-4-yl)-6-methylpyridine-2-carboxamide obtained in Example 429. yield 79%. melting point 169-170°C (ethyl acetate).
¹H-NMR (DMSO-d₆) :δ 1.20(6H, d, J=6.1 Hz), 1.92(4H, m), 2.65(3H, s), 3.58-3.64(6H, m), 7.28(1H, d, J=7.7 Hz), 7.65(1H, brs), 7.73(1H, t, J=7.7 Hz), 8.02(1H, d, J=7.7 Hz), 8.53(1H, s).

### Example 431

### N-(3-(((2-(ethyl(3-methylphenyl)amino)ethyl)amino)carbonyl)-1-(tetrahydro-2H-pyran-2-yl)-1H-pyrazol-4-yl)-6-methylpyridine-2-carboxamide

In the same manner as in Example 386, the title compound was obtained using 4-(((6-methylpyridin-2-yl)carbonyl)amino)-1-(tetrahydro-2H-pyran-2-yl)-1H-pyrazole-3-carboxylic acid obtained in Reference Example 47 and N-(2-aminoethyl)-N-ethyl-m-toluidine. yield 93%. melting point 125-126°C (ethyl acetate).
¹H-NMR (CDCl₃) :δ 1.17(3H, t, J=7.0 Hz), 1.62-1.78(4H, m), 1.98-2.09(2H, m), 2.32(3H, s), 3.42(2H, q, J=7.0 Hz), 3.53 (2H, t, J=6.5 Hz), 3.61-3.76(3H, m), 4.05(1H, d, J=11.3 Hz), 5.37(1H, dd, J=2.2 Hz and J=11.3 Hz), 6.53(1H, d, J=7.1 Hz), 6.56-6.68(2H, m), 7.04-7.18(2H, m), 7.32(1H, d, J=7.7 Hz), 7.75(1H, t, J=7.7 Hz), 8.04(1H, d, J=7.7 Hz), 8.57(1H, s).

### Example 432

### N-(3-(((2-(ethyl(3-methylphenyl)amino)ethyl)amino)carbonyl)-1H-pyrazol-4-yl)-6-methylpyridine-2-carboxamide

In the same manner as in Example 387, the title compound was obtained using N-(3-(((2-(ethyl(3-methylphenyl)amino)ethyl)amino)carbonyl)-1-(tetrahydro-2H-pyran-2-yl)-1H-pyrazol-4-yl)-6-methylpyridine-2-carboxamide obtained in Example 431. yield 86%. melting point 203-205°C (ethyl acetate).
¹H-NMR (DMSO-d₆):δ 1.08(3H, t, J=7.0 Hz), 2.23(3H, s), 2.80(3H, s), 3.32-3.41(6H, m), 6.39(1H, d, J=7.1 Hz), 6.60-6.66(2H, m), 7.01(1H, t, J=8.0 Hz), 7.50-7.53 (1H, m), 7.92-7.96(2H, m), 8.39(1H, s), 8.51(1H, brs).

### Example 433

### N-((4-(((6-methylpyridin-2-yl)carbonyl)amino)-1-(tetrahydro-2H-pyran-2-yl)-1H-pyrazol-3-yl)carbonyl)-β-alanine ethyl ester

In the same manner as in Example 386, the title compound was obtained using 4-(((6-methylpyridin-2-yl)carbonyl)amino)-1-(tetrahydro-2H-pyran-2-yl)-1H-pyrazole-3-carboxylic acid obtained in Reference Example 47 and β-alanine ethyl ester hydrochloride. yield 97%. melting point 126-127°C (ethyl acetate).
¹H-NMR (CDCl₃):δ 1.28(3H, t, J=7.2 Hz), 1.61-1.74(3H, m), 2.03-2.06(2H, m), 2.18-2.22(1H, m), 2.64-2.69(6H, m), 3.67-3.79(3H, m), 4.06(1H, d, J=11.1 Hz), 4.18(2H, q, J=7.2 Hz), 5.35(1H, dd, J=2.1 Hz and J=11.1 Hz), 7.26(1H, d, J=7.7 Hz), 7.36(1H, brs), 7.73(1H, t, J=7.7 Hz), 8.02(1H, d, J=7.7 Hz), 8.56(1H, s).

### Example 434

### N-((4-(((6-methylpyridin-2-yl)carbonyl)amino)-1H-pyrazol-3-yl)carbonyl)-β-alanine ethyl ester

In the same manner as in Example 387, the title compound was obtained using N-((4-(((6-methylpyridin-2-yl)carbonyl)amino)-1-(tetrahydro-2H-pyran-2-yl)-1H-pyrazol-3-yl)carbonyl)-p-alanine ethyl ester obtained in Example 433. yield 91%. melting point 228-229°C (ethyl acetate).
¹H-NMR (DMSO-d₆):δ 1.19(3H, t, J=7.1 Hz), 2.56-2.69(5H, m), 3.55(2H, q, J=6.8 Hz), 4.08(2H, q, J=7.1 Hz), 7.53 (1H, dd, J=6.0, 2.7 Hz), 7.88-8.03(2H, m), 8.30-8.48(2H, m), 11.56 (1H, brs), 13.31 (1H, brs).

### Example 435

### N-((4-(((6-methylpyridin-2-yl)carbonyl)amino)-1H-pyrazo1-3-yl)carbonyl)-β-alanine

A solution of N-((4-(((6-methylpyridin-2-yl)carbonyl)amino)-1H-pyrazol-3-yl)carbonyl)-β-alanine ethyl ester obtained in Example 434 (207 mg, 0.6 mmol) and 2N aqueous sodium hydroxide solution (0.6 ml) in THF (1 ml)-MeOH (0.5 ml) was stirred at room temperature for 4 hr. The mixture was acidified with 6N hydrochloric acid and extracted with ethyl acetate. The extract was washed with water, and dried over anhydrous sodium hydrogensulfate. The solvent was evaporated under reduced pressure, and the obtained residue was purified by recrystallization (THF-hexane) to give the title compound 140 mg (yield 74%). melting point 246-247°C.
¹H-NMR (DMSO-d₆):δ 2.49-2.56(2H, m), 2.60(3H, s), 3.43-3.54(2H, m), 5.70(1H, dd, J =3.8 Hz and J=5.2 Hz), 7.92-793(1H, m), 8.30(1H, t, J=5.2 Hz), 8.38(1H, s).

### Example 436

### N-((4-(((6-methylpyridin-2-yl)carbonyl)amino)-1-(tetrahydro-2H-pyran-2-yl)-1H-pyrazol-3-yl)carbonyl)glycine methyl ester

In the same manner as in Example 386, the title compound was obtained using 4-(((6-methylpyridin-2-yl)carbonyl)amino)-1-(tetrahydro-2H-pyran-2-yl)-1H-pyrazole-3-carboxylic acid obtained in Reference Example 47 and glycine methyl ester hydrochloride. yield 75%. melting point 115-118°C (ethyl acetate).
¹H-NMR (CDCl₃):δ 1.60-1.75(3H, m), 1.99-2.10 (2H, m), 2.15-2.26 (1H, m), 2.67(3H, s), 3.79(3H, s), 4.05 (1H, d, J=11.5 Hz), 4.27 (2H, d, J=5.5 Hz), 5.37 (1H, d, J=11.5 Hz), 7.29 (1H, d, J=7.7 Hz), 7.36 (1H, brs), 7.73 (1H, t, J=7.7.Hz), 8.01 (1H, d, J=7.7 Hz), 8.57(1H, s), 11.53(1H, brs).

### Example 437

### N-((4-(((6-methylpyridin-2-yl)carbonyl)amino)-1H-pyrazol-3-yl)carbonyl)glycine ethyl ester

In the same manner as in Example 387, the title compound was obtained using N-((4-(((6-methylpyridin-2-yl)carbonyl)amino)-1-(tetrahydro-2H-pyran-2-yl)-1H-pyrazol-3-yl)carbonyl)glycine methyl ester obtained in Example 436. yield 75%. melting point 228-229°C (ethyl acetate).
¹H-NMR (DMSO-d₆) :δ 1.21(3H, t, J=7.2 Hz), 2.58(3H, s), 4.02(2H, d, J=5.8 Hz), 4.12(2H, q, J=7.2 Hz), 7.51(1H, dd, J=3.2 Hz and J=5.9 Hz), 7.93(2H, m), 8.40(1H, s), 8.65(1H, brs).

### Example 438

### N-(3-((2-hydroxyethyl)amino)carbonyl)-1-(tetrahydro-2H-pyran-2-yl)-1H-pyrazol-4-yl)-6-methylpyridine-2-carboxamide

In the same manner as in Example 386, the title compound was obtained using 4-(((6-methylpyridin-2-yl)carbonyl)amino)-1-(tetrahydro-2H-pyran-2-yl)-1H-pyrazole-3-carboxylic acid obtained in Reference Example 47. yield 76%. melting point 137-138°C (ethyl acetate).
¹H-NMR (CDCl₃) δ:1.59-1.69(3H, m), 2.02-2.06(2H, m), 2.21-2.29(1H, m), 2.68(3H, s), 3.60(2H, t, J=5.5 Hz), 3.66-3.75(1H, m), 3.78(2H, d, J=5.5 Hz), 4.03(1H, d, J=11.2 Hz), 5.36(1H, d, J=11.2 Hz), 7.29(1H, d, J=7.7 Hz), 7.73(1H, t, J=7.7 Hz), 8.02(1H, d, J=7.7 Hz), 8.56(1H, s).

### Example 439

### N-(3-(((2-hydroxyethyl)amino)carbonyl)-1H-pyrazol-4-yl)-6-methylpyridine-2-carboxamide

In the same manner as in Example 387, the title compound was obtained using N-(3-((2-hydroxyethyl)amino)carbonyl)-1-(tetrahydro-2H-pyran-2-yl)-1H-pyrazol-4-yl)-6-methylpyridine-2-carboxamide obtained in Example 438. yield 51%. melting point 252-253°C (ethyl acetate).
¹H-NMR (DMSO-d₆):δ 2.59(3H, s), 3.34-3.39(2H, m), 3.51(2H, t, J=5.2 Hz), 4.77(1H, brs), 7.51(1H, dd, J=2.9 Hz and J=5.7 Hz), 7.85-7.98(2H, m), 8.17(1H, t, J=5. 7 Hz), 8.38(1H s), 11.56(1H, brs), 13.27(1H, brs).

### Example 440

### N-(3-(((2-hydroxy-3-phenylpropyl)amino)carbonyl)-1-(tetrahydro-2H-pyran-2-yl)-1H-pyrazol-4-yl)-6-methylpyridine-2-carboxamide

In the same manner as in Example 386, the title compound was obtained using 4-(((6-methylpyridin-2-yl)carbonyl)amino)-1-(tetrahydro-2H-pyran-2-yl)-1H-pyrazole-3-carboxylic acid obtained in Reference Example 47 and 1-amino-3-phenyl-2-propanol. yield 82%. melting point 130-131°C (ethyl acetate).
¹H-NMR (CDCl₃):δ 1.60-1.76(3H, m), 2.00-2.09(2H, m), 2.14-2.24(1H, m), 2.68(3H, s), 2.85(2H, d, J=6.9 Hz), 3.03(1H, brs), 3.36-3.49(1H, m), 3.64-3.77(2H, m), 4.04-4.13(2H, m), 5.35(1H, d, J=8.8 Hz), 7.24-7.34(6H, m), 7.73(1H, t, J=7.7 Hz), 8.02 (1H, d, J=7.7 Hz), 8.57(1H, s), 11.61(1H, brs).

### Example 441

### N-(3-(((2-hydroxy-3-phenylpropyl)amino)carbonyl)-1H-pyrazol-4-yl)-6-methylpyridine-2-carboxamide

In the same manner as in Example 387, the title compound was obtained using N-(3-(((2-hydroxy-3-phenylpropyl)amino)carbonyl)-1-(tetrahydro-2H-pyran-2-yl)-1H-pyrazol-4-yl)-6-methylpyridine-2-carboxamide obtained in Example 440. yield 46%. melting point 184-185°C (ethyl acetate).
¹H-NMR (DMSO-d₆):δ 2.58(3H, s), 2.64(1H, dd, J=7.7 Hz), 2.76(1H, dd, J=7.7 Hz and 13.8 Hz), 3.15-3.27(1H, m), 3.36-3.44(1H, m), 3.67(1H, brs), 5.00(1H, d, J=5.2 Hz), 7.17-7.26(5H, m), 7.50-7.52(1H, m), 7.91-7.93(2H, m), 8.09(1H, brs), 8.39(1H, s), 11.53(1H, brs), 13.30(1H, brs).

### Example 442

### N-(3-(((3-hydroxypropyl)amino)carbonyl)-1-(tetrahydro-2H-pyran-2-yl)-1H-pyrazol-4-yl)-6-methylpyridine-2-carboxamide

In the same manner as in Example 386, the title compound was obtained using 4-(((6-methylpyridin-2-yl)carbonyl)amino)-1-(tetrahydro-2H-pyran-2-yl)-1H-pyrazole-3-carboxylic acid obtained in Reference Example 47 and 1-amino-3-propanol. yield 82%. melting point 145-149°C (ethyl acetate).
¹H-NMR (CDCl₃):δ 1.70-1.82(3H, m), 2.03-2.06(2H, m), 2.15-2.26(1H, m), 2.66(3H, s), 3.49-3.53(1H, m), 3.60-3.75(6H, m), 4.08(1H, dd, J=2.2 Hz and J=10.0 Hz), 5.35(1H, dd, J=2.2 Hz and J=10.0 Hz), 7.15(1H, brs), 7.30(1H, d, J=7.7 Hz), 7.73(1H, t, J=7.7 Hz), 8.01(1H, d, J=7.7 Hz), 8.57(1H, s), 11.67(1H, brs).

### Example 443

### N-(3-(((3-hydroxypropyl)amino)carbonyl)-1H-pyrazol-4-yl)-6-methylpyridine-2-carboxamide

In the same manner as in Example 387, the title compound was obtained using N-(3-(((3-hydroxypropyl)amino)carbonyl)-1-(tetrahydro-2H-pyran-2-yl)-1H-pyrazol-4-yl)-6-methylpyridine-2-carboxamide obtained in Example 442. yield 46%. melting point 196-197°C (ethyl acetate).
¹H-NMR (DMSO-d₆):δ 1.60-1.74(2H, m), 2.59(3H, s), 3.30-3.99(2H, m), 3.47(2H, q, J=6.0 Hz), 4.53(1H, brs), 7.51(1H, dd, J=2.5 Hz and J=5.8 Hz), 7.92(1H, d, J=2.5 Hz), 7.93(1H, brs), 8.33(1H, t, J=5.8 Hz), 8.38(1H, s), 11.57(1H, s).

### Example 444

### N-(3-(((3-hydroxy-2,2-dimethylpropyl)amino)carbonyl)-1-(tetrahydro-2H-pyran-2-yl)-1H-pyrazol-4-yl)-6-methylpyridine-2-carboxamide

In the same manner as in Example 386, the title compound was obtained using 4-(((6-methylpyridin-2-yl)carbonyl)amino)-1-(tetrahydro-2H-pyran-2-yl)-1H-pyrazole-3-carboxylic acid obtained in Reference Example 47 and 1-amino-2,2-dimethyl-3-propanol. yield 82%. melting point 172-173°C (ethyl acetate).
¹H-NMR (CDCl₃):δ 0.96(6H, s), 1.66-1.75(3H, m), 2.04-2.10(2H, m), 2.16-2.26 (1H, m), 2.65(3H, s), 3.24(2H, d, J=7.4 Hz), 3.30(2H, d, J=6.9 Hz), 3.70-3.76 (1H, m), 4.07-4.13 (1H, m), 4.39 (1H, t, J=7.4 Hz), 5.36 (1H, dd, J=2.5 Hz and J=9.6 Hz), 7.20 (1H, t, J=6.9 Hz), 7.30 (1H, t, J=7.7 Hz), 7.73 (1H, t, J=7.7 Hz), 8.01 (1H, d, J=7.7 Hz), 8.57 (1H, s), 11.68 (1H, brs).

### Example 445

### N-(3-(((3-hydroxy-2,2-dimethylpropyl)amino)carbonyl)-1H-pyrazol-4-yl)-6-methylpyridine-2-carboxamide

In the same manner as in Example 387, the title compound was obtained using N-(3-(((3-hydroxy-2,2-dimethylpropyl)amino)carbonyl)-1-(tetrahydro-2H-pyran-2-yl)-1H-pyrazol-4-yl)-6-methylpyridine-2-carboxamide obtained in Example 444. yield 60%. melting point 217-218°C (ethyl acetate).
¹H-NMR (DMSO-d₆) :δ 0.83(6H, s), 2.58(3H, s), 3.14-3.20(4H, m), 4.81(1H, brs), 7.50-7.53(1H, m), 7.92-7.94(2H, m), 8.23(1H, brs), 8.40(1H, s), 11.53(1H, brs), 13.30(1H, brs).

### Example 446

### N-(3-(((2-(4-trifluoromethylphenyl)-2-hydroxyethyl)amino)carbonyl)-1-(tetrahydro-2H-pyran-2-yl)-1H-pyrazol-2-yl)-6-methylpyridine-2-carboxamide

In the same manner as in Example 386, the title compound was obtained using 4-(((6-methylpyridin-2-yl)carbonyl)amino)-1-(tetrahydro-2H-pyran-2-yl)-1H-pyrazole-3-carboxylic-acid obtained in Reference Example 47 and 2-amino-1-(4-trifluoromethylphenyl)ethanol. yield 70%. melting point 138-140°C (ethyl acetate).
¹H-NMR (CDCl₃):δ 1.63-1.73(3H, m), 2.00-2.10(2H, m), 2.12-2.23(1H, m), 3.49-3.61(1H, m), 3.66-3.74(1H, m), 3.89(1H, ddd, J=3.0 Hz and J=6.9 Hz and J=14.3 Hz), 4.06(1H, d, J=11.5 Hz), 4.19(1H, brs), 5.06(1H, d, J=5.8 Hz), 5.34(1H, dd, J=2.5 Hz and J=11.5 Hz), 7.31-7.37(2H, m), 7.57(2H, d, J=5.8 Hz), 7.64(2H, d, J=8.5 Hz), 7.76(1H, d, J=7.7 Hz), 8.03(1H, d, J=7.7 Hz), 8.58(1H, s), 11.60(1H, brs).

### Example 447

### N-(3-(((2-(4-trifluoromethylphenyl)-2-hydroxyethyl)amino)carbonyl)-1H-pyrazol-2-yl)-6-methylpyridine-2-carboxamide

In the same manner as in Example 387, the title compound was obtained using N-(3-(((2-(4-trifluoromethylphenyl)-2-hydroxyethyl)amino)carbonyl)-1-(tetrahydro-2H-pyran-2-yl)-1H-pyrazol-2-yl)-6-methylpyridine-2-carboxamide obtained in Example 446. yield 30%. melting point 250-251°C (ethyl acetate).
¹H-NMR (DMSO-d₆):δ 2.59(3H, s), 3.44-3.56(2H, m), 4.91(1H, brs), 5.82(1H d, J=4.1 Hz), 7.51(1H, t, J=3.6 Hz), 7.60(2H, d, J=8.0 Hz), 7.69(2H, d, J=8.0 Hz), 7.92-7.93(2H, m), 8.16(1H, brs), 8.38(1H, s), 11.49(1H, brs), 13.29(1H, brs).

### Example 448

### N-(3-(((2-fluoroethyl)amino)carbonyl)-1-(tetrahydro-2H-pyran-2-yl)-1H-pyrazol-4-yl)-6-methylpyridine-2-carboxamide

In the same manner as in Example 386, the title compound was obtained using 4-(((6-methylpyridin-2-yl)carbonyl)amino)-1-(tetrahydro-2H-pyran-2-yl)-1H-pyrazole-3-carboxylic acid obtained in Reference Example 47 and 2-fluoroethylamine hydrochloride. yield 84%. melting point 167-168°C (ethyl acetate).
¹H-NMR (CDCl₃):δ 1.56-1.84(3H, m), 1.98-2.31(3H, m), 2.69(3H, s), 3.67-3.80(2H, m), 3.85(1H q, J=5.2 Hz), 4.08(1H, d, J=11.5 Hz), 4.53(1H, t, J=4.9 Hz), 4.69(1H, t, J=4.9 Hz), 5.37(1H, dd, J=9.6, 2.2 Hz), 7.24(1H, brs), 7.31(1H, d, J=7.7 Hz), 7.75(1H, t, J=7.7 Hz), 8.03(1H, d, J=7.7 Hz), 8.58(1H, s), 11.61(1H, brs).

### Example 449

### N-(3-(((2-fluoroethyl)amino)carbonyl)-1H-pyrazol-4-yl)-6-methylpyridine-2-carboxamide

In the same manner as in Example 387, the title compound was obtained using N-(3-(((2-fluoroethyl)amino)carbonyl)-1-(tetrahydro-2H-pyran-2-yl)-1H-pyrazol-4-yl)-6-methylpyridine-2-carboxamide obtained in Example 448. yield 50%. melting point 220-221°C (ethyl acetate).
¹H-NMR (DMSO-d₆):δ 3.33(3H, s), 3.56 (1H, q, J=5.2 Hz), 3.65 (1H, q, J=5.2 Hz), 4.48 (1H, t, J=5.2 Hz), 4.63 (1H, t, J=5.2 Hz), 7.53 (1H, dd, J=5.5, 3.3 Hz), 7.92-7.97(2H, m), 8.41 (1H, s), 8.52 (1H, brs), 11.54 (1H, brs), 13.34 (1H, brs).

### Example 450

### N-(3-(((2-cyano-2-methylpropyl)amino)carbonyl)-1-(tetrahydro-2H-pyran-2-yl)-1H-pyrazol-4-yl)-6-methylpyridine-2-carboxamide

In the same manner as in Example 386, the title compound was obtained using 4-(((6-methylpyridin-2-yl)carbonyl)amino)-1-(tetrahydro-2H-pyran-2-yl)-1H-pyrazole-3-carboxylic acid obtained in Reference Example 47 and 3-amino-2-dimethylpropanenitrile. yield 60%. melting point 159-160°C (ethyl acetate).
¹H-NMR (CDCl₃):δ 1.44(6H, s), 1.61-1.80(3H, m), 2.01-2.30(3H, m), 2.69(3H, s), 3.63(2H, d, J=6.9 Hz), 3.67-3.79(1H, m), 4.04-4.14(1H, m), 5.38(1H, dd, J=9.6, 2.2 Hz), 7.22(1H, brs), 7.30(1H, d, J=7.7 Hz), 7.74(1H, t, J=7.7 Hz), 8.02 (1H, d, J=7.7 Hz), 8.59(1H, s), 11.51(1H, brs).

### Example 451

### N-(3-(((2-cyano-2-methylpropyl)amino)carbonyl)-1H-pyrazol-4-yl)-6-methylpyridine-2-carboxamide

In the same manner as in Example 387, the title compound was obtained using N-(3-(((2-cyano-2-methylpropyl)amino)carbonyl)-1-(tetrahydro-2H-pyran-2-yl)-1H-pyrazol-4-yl)-6-methylpyridine-2-carboxamide obtained in Example 450. yield 50%. melting point 250-251°C (ethyl acetate).
¹H-NMR (DMSO-d₆):δ 1.58-1.80(3H, m), 1.97-2.30(3H, m), 2.49(2H, qt, J=10.7, 6.8 Hz), 2.70(3H, s), 3.65-3.81(1H, m), 3.74(2H, q, J=6.8 Hz), 4.07(1H, dd, J=12.0, 2.6 Hz), 5.36(1H, dd, J=9.5, 2.3 Hz), 7.10(1H, brs, J=6.0, 6.0 Hz), 7.31(1H, d, J=7.7 Hz), 7.74 (1H, t, J=7.7 Hz), 8.02 (1H, d, J=7.7 Hz), 8.57(1H, s), 11.57(1H, brs).

### Example 452

### N-(3-(((2-(pyridin-2-ylthio)ethyl)amino)carbonyl)-1-(tetrahydro-2H-pyran-2-yl)-1H-pyrazol-4-yl)-pyridine-2-carboxamide 1) tert-butyl (2-(pyridin-2-ylthio)ethyl)carbamate

To a suspension of cysteamine hydrochloride (10 g, 88.0 mmol) in 1,4-dioxane (100 ml), sodium hydride (60% in oil, 7.04 g, 176 mmol) was added, and the mixture was stirred for 30 min. Then, 2-chloropyridine (10.5 g, 72.9 mmol) was added, and the mixture was stirred with heating at 110°C for 19 hr. The reaction mixture was diluted with saturated aqueous sodium hydrogen carbonate solution and ethyl acetate. Di-tert-butyldicarbonate (23 g, 106 mmol) was added, and the mixture was stirred for 4.5 hr. The ethyl acetate layer was washed with water, dried over anhydrous magnesium sulfate, and evaporated under reduced pressure. The residue was purified by silica gel column chromatography (hexane:ethyl acetate=5:1-3:1) to give the title compound (12.3 g, yield 55%) as an oil.
¹H-NMR (CDCl₃) :δ 1.43(9H, s), 3.30 (2H, t, J=6.6 Hz), 3.40-3.50(2H, m), 5.34 (1H, brs), 6.99 (1H, dd, J=8.4, 6.0 Hz), 7.21 (1H, d, J=7.8 Hz), 7.45-7.55 (1H, m), 8.40 (1H, d, J=4.8 Hz).

### 2) N-(3-(((2-(pyridin-2-ylthio)ethyl)amino)carbonyl)-1-(tetrahydro-2H-pyran-2-yl)-1H-pyrazol-4-yl)-pyridine-2-carboxamide

tert-Butyl (2-(pyridin-2-ylthio)ethyl)carbamate obtained in 1) (0.61 g, 2.4 mmol) was dissolved in concentrated hydrochloric acid (4 ml), and the mixture was stirred for 30 min. The reaction mixture was dried under reduced pressure to give 2-(pyridin-2-ylthio)ethanamine dihydrochloride. To a mixture of the present compound, 4-((pyridin-2-ylcarbonyl)amino)-1-(tetrahydro-2H-pyran-2-yl)-1H-pyrazole-3-carboxylic acid obtained in Reference Example 54 (0.63 g, 2.0 mmol) and DMF (10 ml), WSC (0.41 g, 2.4 mmol), HOBt (0.32 g, 2.4 mmol) and triethylamine (0.33 ml, 2.4 mmol) were added, and the mixture was stirred at room temperature for 4 hr. Saturated aqueous sodium hydrogen carbonate solution was added to the reaction mixture, and the mixture was extracted with ethyl acetate. The extract was washed with water, dried over anhydrous magnesium sulfate, passed through a small amount of silica gel and evaporated under reduced pressure to give the title compound (0.25 g, yield 28%) as an oil.
¹H-NMR (d₆-DMSO):δ 1.60-1.80(3H, m), 2.00-2.15(2H, m), 2.15-2. 30 (1H, m), 3.45(2H, t, J=6.3 Hz), 3.70-3.80(1H, m), 3.83(2H, q, J=6.0 Hz), 4.05-4.15(1H, m), 5.36(1H, dd, J=9.3, 2.7 Hz), 6.97-7.03(1H, m), 7.23(1H, d, J=8.1 Hz), 7.40-7.55 (2H, m), 7.86 (1H, t, J=7.8 Hz), 8.22(1H, d, J=7.8 Hz), 8.50-8.58(1H, m), 8.56(1H, s), 8.70-8.80(1H, m), 11.74(1H, s).

### Example 453

### N-(3-(((2-(pyridin-2-ylthio)ethyl)amino)carbonyl)-1H-pyrazol-4-yl)pyridine-2-carboxamide

A mixture of N-(3-(((2-(pyridin-2-ylthio)ethyl)amino)carbonyl)-1-(tetrahydro-2H-pyran-2-yl)-1H-pyrazol-4-yl)pyridine-2-carboxamide obtained in Example 452 (0.25 g, 0.55 mmol), p-toluenesulfonic acid monohydrate (0.32 g, 1.66 mmol) and ethanol (10 ml) was stirred at 60°C for 3 hr. Saturated aqueous sodium hydrogen carbonate solution was added to the reaction mixture, and the mixture was extracted with ethyl acetate. The extract was washed with water, dried over anhydrous magnesium sulfate, passed through a small amount of silica gel and evaporated under reduced pressure. Diethyl ether was added to the precipitated crystals, and the crystals were collected by filtration to give the title compound (0.16 g, yield 88%). melting point 162-163°C.
¹H-NMR (d₆-DMSO) :δ 3.37(2H, t, J=7.4 Hz), 3.55-3.65(2H, m), 7.10-7.20(1H, m), 7.36(1H, d, J=7.8 Hz), 7.60-7.75(2H, m), 8.07(1H, t, J=7.4 Hz), 8.15(1H, d, J=7.8 Hz), 8.41(1H, s), 8.42-8.50(1H, m), 8.65-8.80(2H, m), 11.60(1H, s), 13.33(1H, s).

### Example 454

### N-(3-(((3-tert-butoxypropyl)amino)carbonyl)-1-(tetrahydro-2H-pyran-2-yl)-1H-pyrazol-4-yl)pyridine-2-carboxamide

To a solution of 4-((pyridin-2-ylcarbonyl)amino)-1-(tetrahydro-2H-pyran-2-yl)-1H-pyrazole-3-carboxylic acid obtained in Reference Example 54 (0.63 g, 2.0 mmol) and 3-tert-butoxypropylamine hydrochloride (0.40 g, 2.4 mmol) in DMF (10 ml), WSC (0.41 g, 2.4 mmol), HOBt (0.32 g, 2.4 mmol) and triethylamine (0.33 ml, 2.4 mmol) were added, and the mixture was stirred at room temperature for 3 hr. Saturated aqueous sodium hydrogen carbonate solution was added to the reaction mixture, and the mixture was extracted with ethyl acetate. The extract was washed with water, dried over anhydrous magnesium sulfate, passed through a small amount of silica gel and evaporated under reduced pressure. Diethyl ether was added to the precipitated crystals, and the crystals were collected by filtration to give the title compound (0.76 g, yield 87%) as crystals. melting point 101-103°C.
¹H-NMR (CDCl₃):δ 1.25(9H, s), 1.60-1.80(3H, m), 1.80-1.95(2H, m), 2.00-2.15(1H, m), 2.15-2.30(1H, m), 3.53(2H, t, J=5.7 Hz), 3.59(2H, q, J=5.7 Hz), 3.60-3.80(1H, m), 4.05(1H, d, J=11.4 Hz), 5.33(1H, dd, J=9.3, 2.4 Hz), 7.40-7.50(1H, m), 7.50-7.60(1H, m), 7.85(1H, td, J=7.8, 1.8 Hz), 8.21(1H, d, J=7.8 Hz), 8.54(1H, s), 8.70-8.80(1H, m), 11.77 (1H, s).

### Example 455

### N-(3-(((3-tert-butoxypropyl)amino)carbonyl)-1H-pyrazol-4-yl)pyridine-2-carboxamide

A mixture of N-(3-(((3-tert-butoxypropyl)amino)carbonyl)-1-(tetrahydro-2H-pyran-2-yl)-1H-pyrazol-4-yl)pyridine-2-carboxamide obtained in Example 454 (0.71 g, 1.65 mmol), p-toluenesulfonic acid monohydrate (0.31 g, 1.65 mmol) and ethanol (20 ml) was stirred at 50°C for 8 hr. Saturated aqueous sodium hydrogen carbonate solution was added to the reaction mixture, and the mixture was extracted with ethyl acetate. The extract was washed with water, dried over anhydrous magnesium sulfate, and evaporated under reduced pressure. The residue was purified by silica gel column chromatography (hexane:ethyl acetate=1:1-1:2). Diethyl ether was added to the precipitated crystals, and the crystals were collected by filtration to give the title compound (0.42 g, yield 74%). melting point 193-194°C.
¹H-NMR (d₆-DMSO):δ 1.15(9H, s), 1.65-1.80(2H, m), 3.30-3.50(4H, m), 7.60-7.75(1H, m), 8.06(1H, d, J=7.8 Hz), 8.15(1H, d, J=7.8 Hz), 8.30-8.40(1H, m), 8.38(1H, s), 8.74(1H, d, J=6.0 Hz), 11.62 (1H, s), 13.27 (1H, s).

### Example 456

### N-(3-(((cyanopropyl)amino)carbonyl)-1-(tetrahydro-2H-pyran-2-yl)-1H-py,razol-4-yl)pyridine-2-carboxamide

To a solution of 4-((pyridin-2-ylcarbonyl)amino)-1-(tetrahydro-2H-pyran-2-yl)-1H-pyrazole-3-carboxylic acid obtained in Reference Example 54 (0.64 g, 2.0 mmol) and 4-aminobutyronitrile hydrochloride (0.347 g, 2.88 mmol) in DMF (10 ml), WSC (0.41 g, 2.4 mmol), HOBt (0.32 g, 2.4 mmol) and triethylamine (0.54 ml, 3.9 mmol) were added, and the mixture was stirred at room temperature for 5 hr. Saturated aqueous sodium hydrogen carbonate solution was added to the reaction mixture, and the mixture was extracted with ethyl acetate. The extract was washed with water, dried over anhydrous magnesium sulfate, and evaporated under reduced pressure. The residue was purified by silica gel column chromatography (hexane:ethyl acetate=1:1-1:2). Diethyl ether was added to the precipitated crystals, and the crystals were collected by filtration to give the title compound (0.31 g, yield 41%) as crystals. melting point 155-157°C.
¹H-NMR (CDCl₃):δ 1.60-1.80(3H, m), 2.00(2H, q, J=6.9 Hz), 2.00-2.15(2H, m), 2.15-2.30(1H, m), 2.48(2H, t, J=7.2 Hz), 3.59(2H, q, J=6.9 Hz), 3.65-3.80(1H, m), 4.05-4.15(1H, m), 5.37(1H, dd, J=9.6, 1.8 Hz), 7.00-7.10(1H, m), 7.40-7.50(1H, m), 7.87(1H, td, J=7.8, 1.8 Hz), 8.23(1H, d, J=8.1 Hz), 8.58(1H, s), 8.70-8.80(1H, m), 11.61(1H, s).

### Example 457

### N-(3-(((cyanopropyl)amino)carbonyl)-1H-pyrazol-4-yl)pyridine-2-carboxamide

A mixture of N-(3-(((cyanopropyl)amino)carbonyl)-1-(tetrahydro-2H-pyran-2-yl)-1H-pyrazol-4-yl)pyridine-2-carboxamide obtained in Example 456 (0.27 g, 0.71 mmol), p-toluenesulfonic acid monohydrate (0.27 g, 1.41 mmol) and ethanol (15 ml) was stirred at 60°C for 4 hr. Saturated aqueous sodium hydrogen carbonate solution was added to the reaction mixture, and the mixture was extracted with ethyl acetate. The extract was washed with water, dried over anhydrous magnesium sulfate, passed through a small amount of silica gel and evaporated under reduced pressure. Diethyl ether was added to the precipitated crystals, and the crystals were collected by filtration to give the title compound (0.20 g, yield 95%). melting point 231-233°C. ¹H-NMR (d₆-DMSO):δ 1.80-1.95(2H, m), 2.53(2H, q, J=7.5 Hz), 3.37(2H, q, J=6.9 Hz), 7.65-7.75(1H, m), 8.07 (1H, t, J=7.6 Hz), 8.16 (1H, d, J=7.6 Hz), 8.41(1H, s), 8.55-8.75(1H, m), 8.76(1H, d, J=4.5 Hz), 11.61(1H, s), 13.32(1H, s).

### Example 458

### N-(3-(((2-cyano-2-methylpropyl)amino)carbonyl)-1-(tetrahydro-2H-pyran-2-yl)-1H-pyrazol-4-yl)-pyridine-2-carboxamide

To a solution of 4-((pyridin-2-ylcarbonyl)amino)-1-(tetrahydro-2H-pyran-2-yl)-1H-pyrazole-3-carboxylic acid obtained in Reference Example 54 (0.63 g, 2.0 mmol) and 2-cyano-2-methylpropylamine obtained in Reference Example 57 (0.40 g, 4.0 mmol) in DMF (10 ml), WSC (0.41 g, 2.4 mmol) and HOBt (0.32 g, 2.4 mmol) were added, and the mixture was stirred at room temperature for 24 hr. Saturated aqueous sodium hydrogen carbonate solution was added to the reaction mixture, and the mixture was extracted with ethyl acetate. The extract was washed with water, dried over anhydrous magnesium sulfate, passed through a small amount of silica gel and concentrated under reduced pressure. Diethyl ether was added to the precipitated crystals, and the crystals were collected by filtration to give the title compound (0.63 g, yield 79%) as crystals. melting point 138-140°C.
¹H-NMR (CDCl₃) :δ 1.44 (6H, s), 1.60-1.80(3H, m), 2.00-2.15 (2H, m), 2.15-2.30 (1H, m), 3.65(2H, d, J=6.9 Hz), 3.70-3.80 (1H, m), 4.05-4.20 (1H, m), 5.39 (1H, d, J=9.3 Hz), 7.20-7.30 (1H, m), 7.40-7.50 (1H, m), 7.88 (1H, t, J=7.8 Hz), 8.23(1H, d, J=7.8 Hz), 8.61 (1H, s), 8.70-8.80 (1H, m), 11.56(1H, s).

### Example 459

### N-(3-(((2-cyano-2-methylpropyl)amino)carbonyl)-1H-pyrazol-4-yl)pyridine-2-carboxamide

A mixture of N-(3-(((2-cyano-2-methylpropyl)amino)carbonyl)-1-(tetrahydro-2H-pyran-2-yl)-1H-pyrazol-4-yl)-pyridine-2-carboxamide obtained in Example 458 (0.59 g, 1.49 mmol), p-toluenesulfonic acid monohydrate (0.57 g, 2.98 mmol) and ethanol (20 ml) was stirred at 60°C for 12 hr. Saturated aqueous sodium hydrogen carbonate solution was added to the reaction mixture, and the mixture was extracted with ethyl acetate. The extract was washed with water, dried over anhydrous magnesium sulfate, evaporated under reduced pressure, passed through a small amount of silica gel and concentrated under reduced pressure. Diethyl ether was added to the precipitated crystals, and the crystals were collected by filtration to give the title compound (0.29 g, yield 62%). melting point 238-240°C.
¹H-NMR (d₆-DMSO):δ 1.34(6H, s), 3.50(2H, t, J=6.6 Hz), 7.60-7.70(1H, m), 8.07(1H, t, J=8.0 Hz), 8.15(1H, d, J=7.4 Hz), 8.44(1H, s), 8.65-8.75(1H, m), 8.75(1H, d, J=5.0 Hz), 11.54(1H, s), 13.41 (1H, s).

### Example 460

### N-(3-(((2-cyano-2-methyl-3-phenylpropyl)amino)carbonyl)-1-(tetrahydro-2H-pyran-2-yl)-1H-pyrazol-4-yl)pyridine-2-carboxamide

To a solution of 4-((pyridin-2-ylcarbonyl)amino)-1-(tetrahydro-2H-pyran-2-yl)-1H-pyrazole-3-carboxylic acid obtained in Reference Example 54 (0.63 g, 2.0 mmol) and 3-amino-2-benzyl-2-methylpropanenitrile (0.52 g, 3.0 mmol) in DMF (10 ml), WSC (0.41 g, 2.4 mmol) and HOBt (0.32 g, 2.4 mmol) were added, and the mixture was stirred at room temperature for 3 hr. Saturated aqueous sodium hydrogen carbonate solution was added to the reaction mixture, and the mixture was extracted with ethyl acetate. The extract was washed with water, dried over anhydrous magnesium sulfate, passed through a small amount of silica gel and concentrated under reduced pressure. Diethyl ether was added to the precipitated crystals, and the crystals were collected by filtration to give the title compound (0.90 g, yield 95%) as crystals. melting point 187-190°C.
¹H-NMR (CDCl₃):δ 1.25(3H, s), 1.60-1.80(3H, m), 2.00-2.15(2H, m), 2.15-2.30(1H, m), 2.83(1H, d, J=13.5 Hz), 3.05(1H, d, J=13.5 Hz), 3.60-3.85(3H, m), 4.05-4.20(1H, m), 5.39(1H, d, J=7.2 Hz), 7.25-7.40(5H, m), 7.40-7.50(1H, m), 7.87(1H, t, J=7.7 Hz), 8.27 (1H, d, J=8.1 Hz), 8.59(1H, s), 8.72(1H, d, J=3.9 Hz), 11.55(1H, s).

### Example 461

### N-(3-(((2-cyano-2-methyl-3-phenylpropyl)amino)carbonyl)-1H-pyrazol-4-yl)pyridine-2-carboxamide

To a solution of N-(3-(((2-cyano-2-methyl-3-phenylpropyl)amino)carbonyl)-1-(tetrahydro-2H-pyran-2-yl)-1H-pyrazol-4-yl)pyridine-2-carboxamide obtained in Example 460 (0.86 g, 1.82 mmol) in ethanol-chloroform (15-15 ml), p-toluenesulfonic acid monohydrate (0.69 g, 3.64 mmol) was added, and the mixture was stirred at 60°C for 7 hr. The reaction mixture was diluted with saturated aqueous sodium hydrogen carbonate solution, and the mixture was extracted with ethyl acetate. The extract was washed with water, dried over anhydrous magnesium sulfate, passed through a small amount of silica gel and concentrated under reduced pressure. Diethyl ether was added to the precipitated crystals, and the crystals were collected by filtration to give the title compound (0.54 g, yield 76%) as crystals. melting point 196-197°C.
¹H-NMR (CDCl₃) :δ 1.26(3H, s), 2.84(1H d, J=12.5 Hz), 3.03(1H, d, J=12.5 Hz), 3.50-3.60(1H, m), 3.60-3.75(1H, m), 7.25-7.40(5H, m), 7.60-7.70(1H, m), 8.05-8.15(1H, m), 8.15(1H, d, J=7.2 Hz), 8.45(1H, s), 8.70-8.80(2H, m), 11.54(1H, s), 13.43(1H, s).

### Example 462

### Ethyl 4-(((4-((pyridin-2-ylcarbonyl)amino)-1-(tetrahydro-2H-pyran-2-yl)-1H-pyrazol-3-yl)carbonyl)amino)butanoate

To a solution of 4-((pyridin-2-ylcarbonyl)amino)-1-(tetrahydro-2H-pyran-2-yl)-1H-pyrazole-3-carboxylic acid obtained in Reference Example 54 (1.27 g, 4.0 mmol) and ethyl 4-aminobutanoate.hydrochloride (0.74 g, 4.4 mmol) in DMF (20 ml), WSC (0.825 g, 4.8 mmol) and HOBt (0.65 g, 4.8 mmol) were added, and the mixture was stirred at room temperature for 24 hr. Saturated aqueous sodium hydrogen carbonate solution was added to the reaction mixture, and the mixture was extracted with ethyl acetate. The extract was washed with water, dried over anhydrous magnesium sulfate, and evaporated under reduced pressure. The residue was purified by silica gel column chromatography (hexane:ethyl acetate=1:1) to give the title compound (0.88 g, yield 46%) as crystals. melting point 114-115°C.
¹H-NMR (CDCl₃):δ 1.25(3H, t, J=7.2 Hz), 1.60-1.80(3H, m), 1.95-2.05(2H, m), 2.05-2.15(2H, m), 2.15-2.30(1H, m), 2.42(2H, t, J=7.2 Hz), 3.52(2H, d, J=6.6 Hz), 3.65-3.80(1H, m), 4.13(2H, q, J=7.2 Hz), 4.00-4.15(1H, m), 5.35-5.40 (1H, m), 6.95-7.05(1H, m), 7.40-7.50(1H, m), 7.86(1H, td, J=7.5, 1.8 Hz), 8.21(1H, t, J=7.8 Hz), 8.56(1H, s), 8.71(1H, d, J=4.0 Hz), 11.69(1H, s).

### Example 463

### Ethyl 4-(((4-((pyridin-2-ylcarbonyl)amino)-1H-pyrazol-3-yl)carbonyl)amino)butanoate

A mixture of ethyl 4-(((4-((pyridin-2-ylcarbonyl)amino)-1-(tetrahydro-2H-pyran-2-yl)-1H-pyrazol-3-yl)carbonyl)amino)butanoate obtained in Example 462 (0.84 g, 1.96 mmol), p-toluenesulfonic acid monohydrate (0.74 g, 3.91 mmol) and ethanol (20 ml) was stirred at 60°C for 4 hr. Saturated aqueous sodium hydrogen carbonate solution was added to the reaction mixture, and the mixture was extracted with ethyl acetate. The extract was washed with water, dried over anhydrous magnesium sulfate, and evaporated under reduced pressure. The residue was purified by silica gel column chromatography (hexane:ethyl acetate=1:1-1:2). Diethyl ether was added to the precipitated crystals, and the crystals were collected by filtration to give the title compound (0.55 g, yield 81%). melting point 179-180°C.
¹H-NMR (d₆-DMSO):δ 1.17(3H, t, J=7.2 Hz), 1.75-1.90(2H, m), 2.34(2H, t, J=7.2 Hz), 3.29(2H, t, J=6.9 Hz), 4.04(2H, q, J=7.2 Hz), 7.60-7.70(1H, m), 8.06(1H, t, J=7.7 Hz), 8.15(1H, d, J=7.2 Hz), 8.40(1H, s), 8.40-8.55(1H, m), 8.75(1H, d, J=3.6 Hz), 11. 62 (1H, s), 13.30(1H, s).

### Example 464

### 4-(((4-((pyridin-2-ylcarbonyl)amino)-1H-pyrazol-3-yl)carbonyl)amino)butanoic acid

To a mixture of ethyl 4-(((4-((pyridin-2-ylcarbonyl)amino)-1H-pyrazol-3-yl)carbonyl)amino)butanoate obtained in Example 463 (0.40 g, 1.16 mmol), ethanol (5 ml) and tetrahydrofuran (3 ml), 1N aqueous sodium hydroxide solution (4.63 ml, 4.63 mmol) was added, and the mixture was stirred at room temperature for 4.5 hr. The reaction mixture was neutralized with 1N hydrochloric acid (4.63 ml), and concentrated under reduced pressure. Water was added to the precipitated crystals, and the crystals were collected by filtration to give the title compound (0.36 g, yield 98%). melting point 227-228°C.
¹H-NMR (d₆-DMSO) :δ 1.70-1.90(2H, m), 2.27(2H, t, J=7.4 Hz), 3.29(2H, t, J=6.6 Hz), 7.67(1H, t, J=6.0 Hz), 8.06(1H, t, J=7.5 Hz), 8.15(1H, d, J=7.5 Hz), 8.39(1H, s), 8.40-8.55(1H, m), 8.75(1H, d, J=4.5 Hz), 11.62(1H, s), 12.06(1H, s), 13.30(1H, s).

### Example 465

### N-(3-(((2-hydroxy-1,1-dimethylethyl)amino)carbonyl)-1-(tetrahydro-2H-pyran-2-yl)-1H-pyrazol-4-yl)pyridine-2-carboxamide

To a solution of 4-((pyridin-2-ylcarbonyl)amino)-1-(tetrahydro-2H-pyran-2-yl)-1H-pyrazole-3-carboxylic acid obtained in Reference Example 54 (0.63 g, 2.0 mmol) and 2-amino-2-methylpropanol (0.21 g, 2.4 mmol) in DMF (10 ml), WSC (0.41 g, 2.4 mmol) and HOBt (0.32 g, 2.4 mmol) were added, and the mixture was stirred at room temperature for 17 hr. Saturated aqueous sodium hydrogen carbonate solution was added to the reaction mixture, and the mixture was extracted with ethyl acetate. The extract was washed with water, dried over anhydrous magnesium sulfate, passed through a small amount of silica gel and evaporated under reduced pressure. Diethyl ether was added to the precipitated crystals, and the crystals were collected by filtration to give the title compound (0.53 g, yield 75%) as crystals. melting point 194-195°C.
¹H-NMR (CDCl₃) : δ 1.43(6H, s), 1.60-1.80(3H, m), 2.00-2.15(2H, m), 2.15-2.30 (1H, m), 3.73(2H, d, J=6.3 Hz), 3.65-3.80(1H, m), 4.10(1H, d, J=10.5 Hz), 4.95(1H, t, J=6.5 Hz), 5.35(1H, dd, J=9.9, 1.6 Hz), 6.96(1H, s), 7.40-7.50(1H, m), 7.87(1H, td, J=7.8, 1.5 Hz), 8.22(1H, d, J=9.0 Hz), 8.59(1H, s), 8.70-8.80(1H, m), 11.54(1H, s).

### Example 466

### N-(3(((2-hydroxy-1,1-dimethylethyl)amino)carbonyl)-1H-pyrazol-4-yl)pyridine-2-carboxamide

A mixture of N-3-(((2-hydroxy-1,1-dimethylethyl)amino)carbonyl)-1-(tetrahydro-2H-pyran-2-yl)-1H-pyrazol-4-yl)pyridine-2-carboxamide obtained in Example 465 (0.54 g, 1.39 mmol), p-toluenesulfonic acid monohydrate (0.53 g, 2.79 mmol) and ethanol (10 ml) was stirred at 60°C for 5 hr. Saturated aqueous sodium hydrogen carbonate solution was added to the reaction mixture, and the mixture was extracted with ethyl acetate. The extract was washed with water, dried over anhydrous magnesium sulfate, and evaporated under reduced pressure. The residue was purified by silica gel column chromatography (hexane:ethyl acetate=1:2-1:4) to give the title compound (0.27 g, yield 64%). melting point 193-195°C.
¹H-NMR (d₆-DMSO):δ 1.37(6H, s), 3.45(2H, d, J=5.1 Hz), 5.10-5.22(1H, m), 7.36 (1H, s), 7.60-7.75 (1H, m), 8.07(1H, t, J=7.8 Hz), 8.16 (1H, d, J=7.8 Hz), 8.41 (1H, s), 8.78 (1H, d, J=4.5 Hz), 11.50 (1H, s), 13.27 (1H, s).

### Example 467

### N-(3-(((3-hydroxypropyl)amino)carbonyl)-1-(tetrahydro-2H-pyran-2-yl)-1H-pyrazol-4-yl)pyridine-2-carboxamide

To a solution of 4-((pyridin-2-ylcarbonyl)amino)-1-(tetrahydro-2H-pyran-2-yl)-1H-pyrazole-3-carboxylic acid obtained in Reference Example 54 (0.63 g, 2.0 mmol) and 3-hydroxypropylamine (0.15 ml, 2.4 mmol) in DMF (10 ml), WSC (0.41 g, 2.4 mmol) and HOBt (0.32 g, 2.4 mmol) were added, and the mixture was stirred at room temperature for 24 hr. Saturated aqueous sodium hydrogen carbonate solution was added to the reaction mixture, and the mixture was extracted with ethyl acetate. The extract was washed with water, dried over anhydrous magnesium sulfate, passed through a small amount of silica gel and evaporated under reduced pressure. Diethyl ether was added to the precipitated crystals, and the crystals were collected by filtration to give the title compound (0.74 g, yield 99%) as crystals. melting point 139-140°C.
¹H-NMR (CDCl₃) :δ 1.50-1.90 (5H,m), 2.00-2.15 (2H, m), 2.15-2.30 (1H, m), 3.10-3.20 (1H, m), 3. 64 (2H, q, J=6.0 Hz), 3. 65-3. 80 (2H, m), 4.00-4.20 (1H, m), 5.36 (1H, d, J=9.3 Hz), 7.10-7.25 (1H, m), 7.45 (1H, dd, J=11.1, 6.3 Hz), 7.86 (1H, t, J=8.8 Hz), 8.21 (1H, d, J=7.5 Hz), 8.58 (1H, s), 8.71 (1H, d, J=3.9 Hz), 11.62 (1H, s).

### Example 468

### N-(3-(((3-hydroxypropyl)amino)carbonyl)-1H-pyrazol-4-yl)pyridine-2-carboxamide

A mixture of N-(3-(((3-hydroxypropyl)amino)carbonyl)-1-(tetrahydro-2H-pyran-2-yl)-1H-pyrazol-4-yl)pyridine-2-carboxamide obtained in Example 467 (0.70 g, 1.87 mmol), p-toluenesulfonic acid monohydrate (0.71 g, 3.75 mmol) and ethanol (15 ml) was stirred at 60°C for 5 hr. Saturated aqueous sodium hydrogen carbonate solution was added to the reaction mixture, and the mixture was extracted with ethyl acetate. The extract was washed with water, dried over anhydrous magnesium sulfate, passed through a small amount of silica gel and concentrated under reduced pressure. The precipitated crystals were recrystallized from ethyl acetate to give the title compound (0.42 g, yield 77%). melting point 209-211°C.
¹H-NMR (d₆-DMSO):δ 1.60-1.80(2H, m), 3.30-3.40(2H, m), 3.40-3.55(2H, m), 4.50-4.60(1H, m), 7.60-7.70(1H, m), 8.06(1H, td, J=7.8, 1.2 Hz), 8.15(1H, d, J=7.2 Hz), 8.39(1H, s), 8.35-8.50(1H, m), 8.74 (1H, d, J=8.4 Hz), 11. 63 (1H, s), 13.28 (1H, s).

### Example 469

### N-(3-(((3-hydroxy-2,2-dimethylpropyl)amino)carbonyl)-1-(tetrahydro-2H-pyran-2-yl)-1H-pyrazol-4-yl)pyridine-2-carboxamide

To a solution of 4-((pyridin-2-ylcarbonyl)amino)-1-(tetrahydro-2H-pyran-2-yl)-1H-pyrazole-3-carboxylic acid obtained in Reference Example 54 (0.63 g, 2.0 mmol) and 3-amino-2,2-dimethylpropanol (0.25 g, 2.4 mmol) in DMF (10 ml), WSC (0.41 g, 2.4 mmol) and HOBt (0.32 g, 2.4 mmol) were added, and the mixture was stirred at room temperature for 21 hr. Saturated aqueous sodium hydrogen carbonate solution was added to the reaction mixture, and the mixture was extracted with ethyl acetate. The extract was washed with water, dried over anhydrous magnesium sulfate, passed through a small amount of silica gel and evaporated under reduced pressure. Diethyl ether was added to the precipitated crystals, and the crystals were collected by filtration to give the title compound (0.69 g, yield 86%) as crystals. melting point 189-190°C.
¹H-NMR (CDCl₃):δ 0.96(6H, s), 1.60-1.80(3H, m), 2.00-2.15(2H, m), 2.15-2.30(1H, m), 3.25 (2H, d, J=7.2 Hz), 3.31(2H, d, J=7.2 Hz), 3.65-3.80(1H, m), 4.00-4.15(2H, m), 5.37(1H, dd, J=9.3, 2.4 Hz), 7.20-7.30(1H, m), 7.40-7.50(1H, m), 7.86(1H, td, J=7.8, 1.8 Hz), 8.21(1H, d, J=8.1 Hz), 8.59(1H, s), 8.65-8.75(1H, m), 11.57(1H, s).

### Example 470

### N-(3-(((3-hydroxy-2,2-dimethylpropyl)amino)carbonyl)-1H-pyrazol-4-yl)pyridine-2-carboxamide

A mixture of N-(3-(((3-hydroxy-2,2-dimethylpropyl)amino)carbonyl)-1-(tetrahydro-2H-pyran-2-yl)-1H-pyrazol-4-yl)pyridine-2-carboxamide obtained in Example 469 (0.64 g, 1.59 mmol), p-toluenesulfonic acid monohydrate (0.61 g, 3.19 mmol) and ethanol (20 ml) was stirred at 60°C for 5 hr. Saturated aqueous sodium hydrogen carbonate solution was added to the reaction mixture, and the mixture was extracted with ethyl acetate. The extract was washed with water, dried over anhydrous magnesium sulfate, and evaporated under reduced pressure. The residue was purified by silica gel column chromatography (hexane:ethyl acetate=1:1-1:3). Diethyl ether was added to the precipitated crystals, and the crystals were collected by filtration to give the title compound (0.36 g, yield 71%). melting point 217-218°C.
¹H-NMR (d₆-DMSO):δ 0.84(6H, s), 3.10-3.30(4H, m), 4.77 (1H, t, J=5.3 Hz), 7.67 (1H,t, J=6.0 Hz), 8.06 (1H,d, J=7.8 Hz), 8.15 (1H, d, J=7.8 Hz), 8.41 (1H, s), 8.75 (1H, d, J=4.2 Hz), 11.57 (1H, s), 13.31 (1H, s).

### Example 471

### N-(3-(((2-hydroxybutyl)amino)carbonyl)-1-(tetrahydro-2H-pyran-2-yl)-1H-pyrazol-4-yl)pyridine-2-carboxamide

To a solution of 4-((pyridin-2-ylcarbonyl)amino)-1-(tetrahydro-2H-pyran-2-yl)-1H-pyrazole-3-carboxylic acid obtained in Reference Example 54 (0.63 g, 2.0 mmol) and 2-hydroxybutylamine (0.23 ml, 2.4 mmol) in DMF (10 ml), WSC (0.41 g, 2.4 mmol) and HOBt (0.32 g, 2.4 mmol) were added, and the mixture was stirred at room temperature for 24 hr. Saturated aqueous sodium hydrogen carbonate solution was added to the reaction mixture, and the mixture was extracted with ethyl acetate. The extract was washed with water, dried over anhydrous magnesium sulfate, passed through a small amount of silica gel and evaporated under reduced pressure. Diethyl ether was added to the precipitated crystals, and the crystals were collected by filtration to give the title compound (0.76 g, yield 98%) as crystals. melting point 160-161°C.
¹H-NMR (CDCl₃) :δ 1.01(3H, t, J=7.5 Hz), 1.50-1.80(5H, m), 2.00-2.15(2H, m), 2.15-2.30(1H, m), 2.55-2.65(1H, m), 3.30-3.40(1H, m), 3.60-3.85(3H, m), 5.36 (1H, d, J=9.6 Hz), 7.20-7.35(1H, m), 7.44(1H, dd, J=7.5, 4.8 Hz), 7.87(1H, td, J=7.8, 0.9 Hz), 8.22(1H d, J=7.8 Hz), 8.57(1H, d, J=4.8 Hz), 11.65(1H, s).

### Example 472

### N-(3-(((2-hydroxybutyl)amino)carbonyl)-1H-pyrazol-4-yl)pyridine-2-carboxamide

A mixture of N-(3-(((2-hydroxybutyl)amino)carbonyl)-1-(tetrahydro-2H-pyran-2-yl)-1H-pyrazol-4-yl)pyridine-2-carboxamide obtained in Example 471 (0.72 g, 1.86 mmol), p-toluenesulfonic acid monohydrate (0.71 g, 3.69 mmol) and ethanol (15 ml) was stirred at 60°C for 3.5 hr. Saturated aqueous sodium hydrogen carbonate solution was added to the reaction mixture, and the mixture was extracted with ethyl acetate. The extract was washed with water, dried over anhydrous magnesium sulfate, passed through a small amount of silica gel and concentrated under reduced pressure. The precipitated crystals were recrystallized from ethyl acetate to give the title compound (0.41 g, yield 73%). melting point 202-203°C.
¹H-NMR (d₆-DMSO):δ 0.91(3H, t, J=6.9 Hz), 2.15-2.55(2H, m), 3.30-3.45(2H, m), 3.50-3.65(1H, m), 4.79(1H, d, J=4.8 Hz), 7.60-7.70 (1H, m), 8.00-8.20(3H, m), 8.41 (1H, s), 8.74 (1H, d, J=5.7 Hz), 11.60 (1H, s), 13.31(1H, s).

### Example 473

### N-(3-(((2-hydroxy-3-phenylpropyl)amino)carbonyl)-1-(tetrahydro-2H-pyran-2-yl)-1H-pyrazol-4-yl)pyridine-2-carboxamide

To a solution of 4-((pyridin-2-ylcarbonyl)amino)-1-(tetrahydro-2H-pyran-2-yl)-1H-pyrazole-3-carboxylic acid obtained in Reference Example 54 (0.63 g, 2.0 mmol) and 2-hydroxy-3-phenylpropylamine (0.36 g, 2.4 mmol) in DMF (10 ml), WSC (0.41 g, 2.4 mmol) and HOBt (0.32 g, 2.4 mmol) were added, and the mixture was stirred at room temperature for 5 hr. Saturated aqueous sodium hydrogen carbonate solution was added to the reaction mixture, and the mixture was extracted with ethyl acetate. The extract was washed with water, dried over anhydrous magnesium sulfate, passed through a small amount of silica gel and evaporated under reduced pressure. Diethyl ether was added to the precipitated crystals, and the crystals were collected by filtration to give the title compound (0.88 g, yield 98%) as crystals. melting point 132-134°C.
¹H-NMR (CDCl₃) :δ 1.60-1.80 (3H, m), 2.00-2.15 (2H, m), 2.15-2.30(1H, m), 2.70(1H, brs), 2.81(1H, dd, J=13.5, 8.1 Hz), 2.90(1H, dd, J=13.5, 5.4 Hz), 3.35-3.50(1H, m), 3.65-3.85(2H, m), 4.00-4.20(2H, m), 5.35 (1H, d, J=9.6 Hz), 7.20-7.40(5H, m), 7.40-7.50(1H, m), 7.86(1H, td, J=7.7, 1.8 Hz), 8.22(1H, d, J=8.1 Hz), 8.57 (1H, s), 8.71(1H, d, J=4.2 Hz), 11.64 (1H, s).

### Example 474

### N-(3-(((2-hydroxy-3-phenylpropyl)amino)carbonyl)-1H-pyrazol-4-yl)pyridine-2-carboxamide

A mixture of N-(3-(((2-hydroxy-3-phenylpropyl)amino)carbonyl)-1-(tetrahydro-2H-pyran-2-yl)-1H-pyrazol-4-yl)pyridine-2-carboxamide obtained in Example 473 (0.83 g, 1.85 mmol), p-toluenesulfonic acid monohydrate (0.70 g, 3.69 mmol) and ethanol (20 ml) was stirred at 60°C for 3 hr. Saturated aqueous sodium hydrogen carbonate solution was added to the reaction mixture, and the mixture was extracted with ethyl acetate. The extract was washed with water, dried over anhydrous magnesium sulfate, passed through a small amount of silica gel and concentrated under reduced pressure. Diethyl ether was added to the precipitated crystals, and the crystals were collected by filtration to give the title compound (0.55 g, yield 82%). melting point 180-181°C.
¹H-NMR (d₆-DMSO):δ 2.60-2.70(1H, m), 2.70-2.80(1H, m), 3.20-3.30(1H, m), 3.35-3.50(1H, m), 3.80-4.00(1H, m), 4.99(1H, d, J=4.5 Hz), 7.15-7.40(5H, m), 7.60-7.70(1H, m), 8.06(1H, t, J=6.3 Hz), 8.15-8.20(2H, m), 8.41(1H, s), 8.70-8.80 (1H, m), 11.59(1H, s), 13.32 (1H, s).

### Example 475

### N-(3-(((3-hydroxy-3-methylbutyl)amino)carbonyl)-1-(tetrahydro-2H-pyran-2-yl)-1H-pyrazol-4-yl)pyridine-2-carboxamide

To a solution of 4-((pyridin-2-ylcarbonyl)amino)-1-(tetrahydro-2H-pyran-2-yl)-1H-pyrazole-3-carboxylic acid obtained in Reference Example 54 (0.63 g, 2.0 mmol) and 4-amino-2-methylbutan-2-ol (0.31 g, 3.0 mmol) in DMF (10 ml), WSC (0.41 g, 2.4 mmol) and HOBt (0.32 g, 2.4 mmol) were added, and the mixture was stirred at room temperature for 16 hr. Saturated aqueous sodium hydrogen carbonate solution was added to the reaction mixture, and the mixture was extracted with ethyl acetate. The extract was washed with water, dried over anhydrous magnesium sulfate, and purified by silica gel column chromatography (hexane:ethyl acetate=1:1-1:5) to give the title compound (0.65 g, yield 81%) as crystals. melting point 138-139°C.
¹H-NMR (CDCl₃):δ 1.31(6H, s), 1.60-1.80(3H, m), 1.82(2H, t, J=6.9 Hz), 2.00-2.15(1H, m), 3.64(2H, q, J=6.4 Hz), 3.70-3.80(1H, m), 4.00-4.15(1H, m), 5.35(1H, d, J=7.5 Hz), 7.40-7.50(2H, m), 7.86(1H, t, J=7.7 Hz), 8.21(1H, d, J=7.5 Hz), 8.55(1H, s), 8.71(1H, d, J=3.9 Hz), 11.73(1H, s).

### Example 476

### N-(3-(((3-hydroxy-3-methylbutyl)amino)carbonyl)-1H-pyrazol-4-yl)pyridine-2-carboxamide

A mixture of N-(3-(((3-hydroxy-3-methylbutyl)amino)carbonyl)-1-(tetrahydro-2H-pyran-2-yl)-1H-pyrazol-4-yl)pyridine-2-carboxamide obtained in Example 475 (0.60 g, 1.49 mmol), p-toluenesulfonic acid monohydrate (0.57 g, 2.99 mmol) and ethanol (10 ml) was stirred at 60°C for 6 hr. Saturated aqueous sodium hydrogen carbonate solution was added to the reaction mixture, and the mixture was extracted with ethyl acetate. The extract was washed with water, dried over anhydrous magnesium sulfate, passed through a small amount of silica gel and concentrated under reduced pressure. The precipitated crystals were recrystallized from ethyl acetate to give the title compound (0.37 g, yield 80%). melting point 193-194°C.
¹H-NMR (d₆-DMSO):δ 1.15(6H, s), 1.65(2H, t, J=7.7 Hz), 3.30-3.50(2H, m), 4.44(1H, s), 7.65-7.75(1H, m), 8.06(1H, t, J=7.5 Hz), 8.15 (1H, d, J=8.1 Hz), 8.39(1H, s), 8.35-8.45(1H, m), 8.74(1H, d, J=5.1 Hz), 11.63(1H, s), 13.27(1H, s).

### Example 477

### N-(3-(((2-isopropoxyethyl)amino)carbonyl)-1-(tetrahydro-2H-pyran-2-yl)-1H-pyrazol-4-yl)-6-methylnicotinamide

To a solution of 4-(((6-methylpyridin-3-yl)carbonyl)amino)-1-(tetrahydro-2H-pyran-2-yl)-1H-pyrazole-3-carboxylic acid obtained in Reference Example 56 (0.66 g, 2.0 mmol) and 2-(2-isopropyl)ethylamine (0.29 ml, 2.4 mmol) in DMF (10 ml), WSC (0.41 g, 2.4 mmol) and HOBt (0.32 g, 2.4 mmol) were added, and the mixture was stirred at room temperature for 24 hr. Saturated aqueous sodium hydrogen carbonate solution was added to the reaction mixture, and the mixture was extracted with ethyl acetate. The extract was washed with water, dried over anhydrous magnesium sulfate, passed through a small amount of silica gel and evaporated under reduced pressure to give the title compound (0.80 g, yield 96%) as an oil.
¹H-NMR (CDCl₃):δ 1.20(6H, d, J=6.0 Hz), 1.55-1.80(3H, m), 2.00-2.15(2H, m), 2.15-2.30 (1H, m), 3.60(3H, s), 3.65-3.80 (1H, m), 4.06 (1H, d, J=10.5 Hz), 5.37 (1H, dd, J=9.0 Hz), 7.25 (1H, d, J=7.8 Hz), 8.08 (1H, dd, J=7.8, 2.4 Hz), 8.49 (1H, s), 9.11 (1H, s), 10.58 (1H, s).

### Example 478

### N-(3-(((2-isopropoxyethyl)amino)carbonyl)-1H-pyrazol-4-yl)-6-methylnicotinamide

A mixture of N-[3-(((2-isopropoxyethyl)amino)carbonyl)-1-(tetrahydro-2H-pyran-2-yl)-1H-pyrazol-4-yl]-6-methylnicotinamide obtained in Example 477 (0.80 g, 1.93 mmol), p-toluenesulfonic acid monohydrate (0.73 g, 3.85 mmol) and ethanol (15 ml) was stirred at 60°C for 3 hr. The reaction mixture was concentrated under reduced pressure. Saturated aqueous sodium hydrogen carbonate solution was added, and the mixture was extracted with ethyl acetate. The extract was washed with water, dried over anhydrous magnesium sulfate, and evaporated under reduced pressure. The residue was purified by silica gel column chromatography (hexane:ethyl acetate=1:3-ethyl acetate) to give the title compound (0.48 g, yield 75%). melting point 168-169°C.
¹H-NMR (d₆-DMSO) :δ 1.08(6H, d, J=7.5 Hz), 2.56(3H, s), 3.40-3.50(2H, m), 3.50-3.55(2H, m), 3.55-3.65(1H, m), 7.45(1H, d, J=8.1 Hz), 8.05-8.15(1H, m), 8.30(1H, s), 8.30-8.40(1H, m), 8.92(1H s), 10.67(1H s), 13.31 (1H, s).

### Example 479

### 6-methyl-N-(1-(tetrahydro-2H-pyran-2-yl)-3-(((2,2,2-trifluoroethyl)amino)carbonyl)-1H-pyrazol-4-yl)pyridine-3-carboxamide

To the solution 4-(((6-methylpyridin-3-yl)carbonyl)amino)-1-(tetrahydro-2H-pyran-2-yl)-1H-pyrazole-3-carboxylic acid obtained in Reference Example 56 (0.66 g, 2.0 mmol) and 2,2,2-trifluoroethylamine (0.19 ml, 2.4 mmol) in DMF (10 ml), WSC (0.41 g, 2.4 mmol) and HOBt (0.32 g, 2.4 mmol) were added, and the mixture was stirred at room temperature for 2.5 hr. Saturated aqueous sodium hydrogen carbonate solution was added to the reaction mixture, and the mixture was extracted with ethyl acetate. The extract was washed with water, dried over anhydrous magnesium sulfate, passed through a small amount of silica gel and evaporated under reduced pressure. Diethyl ether was added to the precipitated crystals, and the crystals were collected by filtration to give the title compound (0.51 g, yield 62%) as crystals. melting point 132-133°C.
¹H-NMR (CDCl₃):δ 1.60-1.80(3H, m), 2.00-2.15(1H, m), 2.15-2.30(1H, m), 2.64(3H, s), 3.65-3.80(1H, m), 4.00-4.20(2H, m), 5.37(1H, dd, J=9.6, 2.7 Hz), 7.10-7.20(1H, m), 7.26(1H, d, J=6.3 Hz), 8.07(1H, dd, J=8.4, 2.7 Hz), 8.52(1H, s), 9.09(1H, d, J=2.7 Hz), 10.27(1H, s).

### Example 480

### 6-methyl-N-(3-(((2,2,2-trifluoroethyl)amino)carbonyl)-1H-pyrazol-4-yl)pyridine-3-carboxamide

A mixture of 6-methyl-N-(1-(tetrahydro-2H-pyran-2-yl)-3-(((2,2,2-trifluoroethyl)amino)carbonyl)-1H-pyrazol-4-yl)pyridine-3-carboxamide obtained in Example 479 (0.48 g, 1.17 mmol), p-toluenesulfonic acid monohydrate (0.44 g, 2.33 mmol) and ethanol (20 ml) was stirred at 60°C for 5 hr. The reaction mixture was concentrated under reduced pressure. Saturated aqueous sodium hydrogen carbonate solution was added, and the mixture was extracted with ethyl acetate. The extract was washed with water, dried over anhydrous magnesium sulfate, and evaporated under reduced pressure. The residue was purified by silica gel column chromatography (hexane:ethyl acetate=1:1-1:4) to give the title compound (0.30 g, yield 79%). melting point 218-219°C.
¹H-NMR (d₆-DMSO) :δ 2.57 (3H, s), 4.00-4.20(2H, m), 7.46 (1H, d, J=7.8 Hz), 8.09(1H, d, J=7.8 Hz), 8.34(1H, s), 8.92 (1H, s), 9.00-9.20 (1H, m), 10.41 (1H, s), 13.34 (1H, s).

### Example 481

### 6-methyl-N-(1-(tetrahydro-2H-pyran-2-yl)-3-(((3,3,3-trifluoropropyl)amino)carbonyl)-1H-pyrazol-4-yl)nicotinamide

To a solution of 4-(((6-methylpyridin-3-yl)carbonyl)amino)-1-(tetrahydro-2H-pyran-2-yl)-1H-pyrazole-3-carboxylic acid obtained in Reference Example 56 (0.50 g, 1.5 mmol) and 3,3,3-trifluoropropylamine hydrochloride (0.22 g, 1.5 mmol) in DMF (10 ml), WSC (0.31 g, 1.8 mmol), HOBt (0.24 g, 1.8 mmol) and triethylamine (0.42 ml, 3.0 mmol) were added, and the mixture was stirred at room temperature for 24 hr. Saturated aqueous sodium hydrogen carbonate solution was added to the reaction mixture, and the mixture was extracted with ethyl acetate. The extract was washed with water, dried over anhydrous magnesium sulfate, passed through a small amount of silica gel and evaporated under reduced pressure. Hexane-diethyl ether was added to the precipitated crystals, and the crystals were collected by filtration to give the title compound (0.45 g, yield 71%) as crystals. melting point 147-148°C.
¹H-NMR (CDCl₃):δ 1.60-1.80(3H, m), 2.00-2.15(2H, m), 2.15-2.30(1H, m), 2.40-2.60(2H, m), 2.64(3H, s), 3.70(2H, q, J=6.3 Hz), 4.00-4.15(1H, m), 5.30-5.40(1H, m), 7.05-7.20(1H, m), 7.26(1H, d, J=8.1 Hz), 8.07(1H, dd, J=8.1, 2.3 Hz), 8.50(1H, s), 9.10(1H, s), 10.42(1H, s).

### Example 482

### 6-methyl-N-(3-(((3,3,3-trifluoropropyl)amino)carbonyl)-1H-pyrazol-4-yl)nicotinamide

A mixture of 6-methyl-N-(1-(tetrahydro-2H-pyran-2-yl)-3-(((3,3,3-trifluoropropyl)amino)carbonyl)-1H-pyrazol-4-yl)nicotinamide obtained in Example 481 (0.41 g, 0.96 mmol), p-toluenesulfonic acid monohydrate (0.37 g, 1.93 mmol) and ethanol (20 ml) was stirred at 60°C for 5 hr. Saturated aqueous sodium hydrogen carbonate solution was added to the reaction mixture, and the mixture was extracted with ethyl acetate. The extract was washed with water, dried over anhydrous magnesium sulfate, passed through a small amount of silica gel and concentrated under reduced pressure. Diethyl ether was added to the precipitated crystals, and the crystals were collected by filtration to give the title compound (0.30 g, yield 93%). melting point 196-197°C.
¹H-NMR (d₆-DMSO):δ 2.56(3H, s), 2.50-2.70(2H, m), 3.53(2H, q, J=5.4 Hz), 7.46(1H, d, J=7.8 Hz), 8.09(1H, d, J=7.5 Hz), 8.31(1H, s), 8.70-8.80(1H, m), 8.92(1H, s), 10.61(1H, s), 13.37(1H, s).

### Example 483

### N-(3-(((2-cyanoethyl)amino)carbonyl)-1-(tetrahydro-2H-pyran-2-yl)-1H-pyrazol-4-yl)-6-methylpyridine-3-carboxamide

To a solution of 4-(((6-methylpyridin-3-yl)carbonyl)amino)-1-(tetrahydro-2H-pyran-2-yl)-1H-pyrazole-3-carboxylic acid obtained in Reference Example 56 (0.66 g, 2.0 mmol) and 3-aminopropionitrile (0.18 ml, 2.4 mmol) in DMF (15 ml), WSC (0.41 g, 2.4 mmol) and HOBt (0.32 g, 2.4 mmol) were added, and the mixture was stirred at room temperature for 24 hr. Saturated aqueous sodium hydrogen carbonate solution was added to the reaction mixture, and the mixture was extracted with ethyl acetate. The extract was washed with water, dried over anhydrous magnesium sulfate, passed through a small amount of silica gel and evaporated under reduced pressure. Diethyl ether was added to the precipitated crystals, and the crystals were collected by filtration to give the title compound (0.60 g, yield 86%) as crystals. melting point 118-120°C.
¹H-NMR (CDCl₃):δ 1.55-1.80(3H, m), 2.00-2.15(2H, m), 2.15-2.30(1H, m), 2.64(3H, s), 2.74(2H, t, J=6.6 Hz), 3.65-3.80(3H, m), 4.00-4.15(1H, m), 5.36(1H, dd, J=9.3, 2.1 Hz), 7.26(1H, d, J=7.5 Hz), 7.20-7.30(1H, m), 8.07(1H, dd, J=8.4, 2.7 Hz), 8.50(1H, s), 9.09(1H, d, J=2.7 Hz), 10.34(1H, s).

### Example 484

### N-(3-(((2-cyanoethyl)amino)carbonyl)-1H-pyrazol-4-yl)-6-methylpyridine-3-carboxamide

A mixture of N-(3-(((2-cyanoethyl)amino)carbonyl)-1-(tetrahydro-2H-pyran-2-yl)-1H-pyrazol-4-yl)-6-methylpyridine-3-carboxamide obtained in Example 483 (0.62 g, 1.62 mmol), p-toluenesulfonic acid monohydrate (0.62 g, 3.24 mmol) and ethanol (20 ml) was stirred at 60°C for 8 hr. The reaction mixture was concentrated under reduced pressure. Saturated aqueous sodium hydrogen carbonate solution was added, and the mixture was extracted with ethyl acetate. The extract was washed with water, dried over anhydrous magnesium sulfate, and evaporated under reduced pressure. The residue was purified by silica gel column chromatography (hexane:ethyl acetate=1:5-ethyl acetate) to give the title compound (0.34 g, yield 70%). melting point 201-203°C.
¹H-NMR (d₆-DMSO):δ 2.56(3H, s), 2.81(2H, t, J=6. 6 Hz), 3.53(2H, q, J=6.1 Hz), 7.46(1H, d, J=7.8 Hz), 8.05-8.15(1H, m), 8.32(1H, s), 8.78-8.90(1H, m), 8.92(1H, s), 10.58(1H, s), 13.37(1H, s).

### Example 485

### N-(3-(((2-hydroxyethyl)amino)carbonyl)-1-(tetrahydro-2H-pyran-2-yl)-1H-pyrazol-4-yl)-6-methylnicotinamide

To a solution of 4-(((6-methylpyridin-3-yl)carbonyl)amino)-1-(tetrahydro-2H-pyran-2-yl)-1H-pyrazole-3-carboxylic acid obtained in Reference Example 56 (0.66 g, 2.0 mmol) and 2-aminoethanol (0.14 ml, 2.4 mmol) in DMF (10 ml), WSC (0.41 g, 2.4 mmol) and HOBt (0.32 g, 2.4 mmol) were added, and the mixture was stirred at room temperature for 5 hr. Saturated aqueous sodium hydrogen carbonate solution was added to the reaction mixture, and the mixture was extracted with ethyl acetate. The extract was washed with water, dried over anhydrous magnesium sulfate, passed through a small amount of silica gel and evaporated under reduced pressure. Diethyl ether was added to the precipitated crystals, and the crystals were collected by filtration to give the title compound (0.70 g, yield 94%) as crystals. melting point 140-140°C.
¹H-NMR (CDCl₃) :δ 1.60-1.80(3H, m), 2.00-2.15(2H, m), 2.15-2.30(1H, m), 2.64(3H, s), 3.62(2H, q, J=5.1 Hz), 3.65-4.80(1H, m), 3.80-3.90(1H, m), 4.00-4.10(1H, m), 5.35(1H, d, J=9.3 Hz), 7.20-7.40(2H, m), 8.00-8.10(1H, m), 8.49(1H, s), 9.09(1H, s), 10.48(1H, s).

### Example 486

### N-(3-(((2-hydroxyethyl)amino)carbonyl)-1H-pyrazol-4-yl)-6-methylnicotinamide

A mixture of N-(3-(((2-hydroxyethyl)amino)carbonyl)-1-(tetrahydro-2H-pyran-2-yl)-1H-pyrazol-4-yl)-6-methylnicotinamide obtained in Example 485 (0.65 g, 1.74 mmol), p-toluenesulfonic acid monohydrate (0.66 g, 3.48 mmol) and ethanol (20 ml) was stirred at 60°C for 6 hr. Saturated aqueous sodium hydrogen carbonate solution was added to the reaction mixture, and the mixture was extracted with ethyl acetate. The extract was washed with water, dried over anhydrous magnesium sulfate, passed through a small amount of silica gel and concentrated under reduced pressure. The precipitated crystals were recrystallized from ethyl acetate to give the title compound (0.39 g, yield 77%). melting point 233-235°C.
¹H-NMR (d₆-DMSO) :δ 2.56(3H, s), 3.36(2H, q, J=6.0 Hz), 3.52(2H, q, J=5.4 Hz), 4.75-4.85(1H, m), 7.46(1H, d, J=7.8 Hz), 8.09(1H, d, J=8.1 Hz), 8.30(1H, s), 8.30-8.40(1H, m), 8.91(1H, s), 10.71(1H, s), 13.31 (1H, s).

### Example 487

### 4-chloro-N-(3-(((2-cyanoethyl)amino)carbonyl)-1-(tetrahydro-2H-pyran-2-yl)-1H-pyrazol-4-yl)-pyridine-2-carboxamide

To a solution of 4-(((4-chloropyridin-2-yl)carbonyl)amino)-1-(tetrahydro-2H-pyran-2-yl)-1H-pyrazole-3-carboxylic acid obtained in Reference Example 59 (0.70 g, 2.0 mmol) and 3-aminopropionitrile (0.18 ml, 2.4 mmol) in DMF (10 ml), WSC (0.41 g, 2.4 mmol) and HOBt (0.32 g, 2.4 mmol) were added, and the mixture was stirred at room temperature for 24 hr. Saturated aqueous sodium hydrogen carbonate solution was added to the reaction mixture, and the mixture was extracted with ethyl acetate. The extract was washed with water, dried over anhydrous magnesium sulfate, evaporated under reduced pressure, passed through a small amount of silica gel and evaporated under reduced pressure to give the title compound (0.58 g, yield 72%) as crystals. melting point 218-219°C.
¹H-NMR (CDCl₃) :δ 1.60-1.95(3H, m), 2.00-2.15(2H, m), 2.15-2.30(1H, m), 2.75(2H, t, J=6.5 Hz), 3.75(2H, q, J=6.6 Hz), 3.70-3.80(1H, m), 4.00-4.15(1H, m), 5.35-5.40(1H, m), 7.20-7.35(1H, m), 7.40-7.50(1H, m), 8.22(1H, s), 8.55(1H, s), 8.62(1H, d, J=5.1 Hz), 11.51(1H, s).

### Example 488

### 4-chloro-N-(3-(((2-cyanoethyl)amino)carbonyl)-1H-pyrazol-4-yl)pyridine-2-carboxamide

A mixture of 4-chloro-N-(3-(((2-cyanoethyl)amino)carbonyl)-1-(tetrahydro-2H-pyran-2-yl)-1H-pyrazol-4-yl)-pyridine-2-carboxamide obtained in Example 487 (0.55 g, 1.37 mmol), p-toluenesulfonic acid monohydrate (0.52 g, 2.74 mmol) and ethanol (20 ml) was stirred at 60°C for 8 hr. The reaction mixture was concentrated under reduced pressure. Saturated aqueous sodium hydrogen carbonate solution was added, and the mixture was extracted with ethyl acetate. The extract was washed with water, dried over anhydrous magnesium sulfate, passed through a small amount of silica gel and evaporated under reduced pressure. The precipitated crystals were recrystallized from ethanol to give the title compound (0.40 g, yield 92%). melting point 261-263°C.
¹H-NMR (d₆-DMSO) :δ 2.81(2H, t, J=7.2 Hz), 3.53(2H, q, J=7.2 Hz), 7.85 (1H, d, J=5.1 Hz), 8.14 (1H, s), 8.42 (1H, s), 8.74 (1H, t, J=6.6 Hz), 8.75-8.85(1H, m), 11.56(1H, s), 13.40(1H, s).

### Example 489

### N-(2-cyanoethyl)-4-(4-methoxybenzoylamino)-1H-pyrazole-3-carboxamide

In the same manner as in Example 401, the title compound was obtained using N-(2-cyanoethyl)-4-(4-methoxybenzoylamino)-1-(tetrahydro-2H-pyran-2-yl)-1H-pyrazole-3-carboxamide obtained in Example 410. yield 75%. melting point 199-200°C (ethyl acetate-hexane).
¹H-NMR (CDCl₃):δ 2.81(2H, t, J=6.6 Hz), 3.54(2H, q, J=6.6 Hz), 3.85(3H, s), 7.12(2H, d, J=9.1 Hz), 7.84(2H, d, J=9.1 Hz), 8.31(1H, s), 8.83(1H, t, J=6.0 Hz), 10.54(1H, s), 13.36(1H, s).

### Example 490

### N-(2-cyanoethyl)-1-(tetrahydro-2H-pyran-2-yl)-4-((4-(trifluoromethyl)benzoyl)amino)-1H-pyrazole-3-carboxamide

In the same manner as in Example 402, the title compound was obtained using 4-amino-N-(2-cyanoethyl)-1-(tetrahydro-2H-pyran-2-yl)-1H-pyrazole-3-carboxamide obtained in Reference Example 52 and 4-trifluoromethylbenzoic acid. yield 83%.
¹H-NMR (CDCl₃):δ 1.62-1.81(3H, m), 2.02-2.15(2H, m), 2.15-2.25(1H, m), 2.74(2H, t, J=6.6 Hz), 3.72-3.81(3H, m), 4.04-4.13(1H, m), 5.38(1H, dd, J=2.5 Hz and 9.3 Hz), 7.24-7.31(1H, m), 7.76(2H, d, J=8.1 Hz), 8.06(1H, d, J=8.1 Hz), 8.53(1H, s), 10.44(1H, s).

### Example 491

### N-(2-cyanoethyl)-1-(tetrahydro-2H-pyran-2-yl)-4-((3-(trifluoromethyl)benzoyl)amino)-1H-pyrazole-3-carboxamide

In the same manner as in Example 402, the title compound was obtained using 4-amino-N-(2-cyanoethyl)-1-(tetrahydro-2H-pyran-2-yl)-1H-pyrazole-3-carboxamide obtained in Reference Example 52 and 3-trifluoromethylbenzoic acid. yield 67%.
¹H-NMR (CDCl₃) :δ 1.62-1.81(3H, m), 2.02-2.15 (2H, m), 2.15-2.25(1H, m), 2.74(2H, t, J=6.6 Hz), 3.72-3.81(3H, m), 4.04-4.13 (1H, m), 5.38(1H, dd, J=2.5 Hz and 9.3 Hz), 7.24-7.31 (1H, m), 7.76(2H, d, J=8.1 Hz), 8.06(1H, d, J=8.1 Hz), 8.53(1H, s), 10.44(1H, s).

### Example 492

### N-(2-cyanoethyl)-4-((4-(trifluoromethyl)benzoyl)amino)-1H-pyrazole-3-carboxamide

In the same manner as in Example 401, the title compound was obtained using N-(2-cyanoethyl)-1-(tetrahydro-2H-pyran-2-yl)-4-((4-(trifluoromethyl)benzoyl)amino)-1H-pyrazole-3-carboxamide obtained in Example 490. yield 82%. melting point 245-246°C (ethyl acetate).
¹H-NMR (CDCl₃): δ 2.82(2H, t, J=6.6 Hz), 3.54 (2H, t, J=6.6 Hz), 7.98(2H, d, J=8.2 Hz), 8.09(2H, d, J=8.2 Hz), 8.37(1H, s), 8.82(1H, t, J=5.9 Hz), 10.74(1H, s), 13.45(1H, s).

### Example 493

### N-(2-cyanoethyl)-4-((3-(trifluoromethyl)benzoyl)amino)-1H-pyrazole-3-carboxamide

In the same manner as in Example 401, the title compound was obtained using N-(2-cyanoethyl)-1-(tetrahydro-2H-pyran-2-yl)-4-((3-(trifluoromethyl)benzoyl)amino)-1H-pyrazole-3-carboxamide obtained in Example 491. yield 83%. melting point 188-190°C (ethyl acetate).
¹H-NMR (CDCl₃):δ 2.81(2H, t, J=6.6 Hz), 3.54(2H, t, J=6.6 Hz), 7.86(1H, t, J=8.0 Hz), 8.03(1H, d, J=8.0 Hz), 8.13-8.20(2H, m), 8.36(1H, s), 8.86(1H, t, J=5.9 Hz), 10.71(1H, s), 13.44(1H, s).

### Example 494

### 4-((5-bromo-2-furoyl)amino)-N-(2-cyanoethyl)-1-(tetrahydro-2H-pyran-2-yl)-1H-pyrazole-3-carboxamide

In the same manner as in Example 402, the title compound was obtained using 4-amino-N-(2-cyanoethyl)-1-(tetrahydro-2H-pyran-2-yl)-1H-pyrazole-3-carboxamide obtained in Reference Example 52 and 5-bromofuran-2-carboxylic acid. yield 87%.
¹H-NMR (CDCl₃):δ 1.61-1.76(3H, m), 1.98-2.11(2H, m), 2.11-2.25(1H, m), 2.75(2H, t, J=6.6 Hz), 3.66-3.78(3H, m), 4.03-4.11(1H, m), 5.35(1H, dd, J=2.5 Hz, 9.4 Hz), 6.48(1H, d, J=3.6 Hz), 7.15(1H, d, J=3.6 Hz), 7.19-7.28(1H, m), 8.44(1H, s), 8.47(1H, s), 10.15(1H, s).

### Example 495

### N-(2-cyanoethyl)-4-((5-methyl-2-furoyl)amino)-1-(tetrahydro-2H-pyran-2-yl)-1H-pyrazole-3-carboxamide

In the same manner as in Example 402, the title compound was obtained using 4-amino-N-(2-cyanoethyl)-1-(tetrahydro-2H-pyran-2-yl)-1H-pyrazole-3-carboxamide obtained in Reference Example 52 and 5-methylfuran-2-carboxylic acid. yield 83%. melting point 195-196°C (ethyl acetate-hexane).
¹H-NMR (CDCl₃) :δ 1.54-1.82(3H, m), 1.97-2.10(2H, m), 2.11-2.30(1H, m), 2.74(2H, t, J=6.6 Hz), 3.64-3.81(3H, m), 4.02-4.11(1H, m), 5.34(1H, dd, J=2.3 Hz, 9.5 Hz), 6.13(1H, d, J=3.3 Hz), 7.10(1H, d, J=3.3 Hz), 7.22-7.32(1H, m), 8.46(1H, s), 8.47(1H, s), 10.07(1H, s).

### Example 496

### 4-((5-bromo-2-furoyl)amino)-N-(2-cyanoethyl)-1H-pyrazole-3-carboxamide

In the same manner as in Example 401, the title compound was obtained using 4-((5-bromo-2-furoyl)amino)-N-(2-cyanoethyl)-1-(tetrahydro-2H-pyran-2-yl)-1H-pyrazole-3-carboxamide obtained in Example 494. yield 66%. melting point 220-222°C (ethyl acetate).
¹H-NMR (CDCl₃) :δ 2.81(2H, t, J=6.5 Hz), 3.54(2H, q, J=6.5 Hz), 6.87(1H, d, J=3.6 Hz), 7.27(1H, d, J=3.6 Hz), 8.28(1H, d, J=1.4 Hz), 8.83(1H t, J=5.9 Hz), 10.49(1H, s), 10.43 (1H, s), 13.37(1H, s).

### Example 497

### N-(2-cyanoethyl)-4-((5-methyl-2-furoyl)amino)-1H-pyrazole-3-carboxamide

In the same manner as in Example 401, the title compound was obtained using N-(2-cyanoethyl)-4-((5-methyl-2-furoyl)amino)-1-(tetrahydro-2H-pyran-2-yl)-1H-pyrazole-3-carboxamide obtained in Example 495. yield 74%. melting point 135-137°C (ethyl acetate-hexane).
¹H-NMR (CDCl₃) :δ 1.60-1.80 (3H,m), 1.98-2.10 (2H,m), 2.11-2.25 (1H, m), 2.73(2H, t, J=6.6 Hz), 3.66-3.77(3H, m), 4.03-4.11 (1H, m), 5.35 (1H,dd, J=2.5 Hz, 9.3 Hz), 6.54 (1H,dd, J=1.7 Hz, 3.6 Hz), 7.21 (1H, dd, J=0.8 Hz, 3.6 Hz), 7.24-7.32 (1H,m), 7.55 (1H, dd, J=0.8 Hz, 1.7 Hz), 8.47 (1H, s), 10.28 (1H, s).

### Example 498

### 4-((3-acetylbenzoyl)amino)-N-(2-cyanoethyl)-1-(tetrahydro-2H-pyran-2-yl)-1H-pyrazole-3-carboxamide

In the same manner as in Example 402, the title compound was obtained using 4-amino-N-(2-cyanoethyl)-1-(tetrahydro-2H-pyran-2-yl)-1H-pyrazole-3-carboxamide obtained in Reference Example 52 and 3-acetylbenzoic acid. yield 82%. melting point 192-194°C (ethyl acetate-hexane).
¹H-NMR (CDCl₃) :δ 1.60-1.80(3H, m), 2.00-2.30(3H, m), 2.69(3H, s), 2.75(2H, t, J=6.6 Hz), 3.67-3.80(3H, m), 4.03-4.11(1H, m), 5.38(1H, dd, J=2.3 Hz, 9.4 Hz), 7.22-7.30(1H, m), 7.61(1H, t, J=7.7 Hz), 8.07-8.18(2H, m), 8.53(1H, s), 8.56(1H, t, J=1.7 Hz), 10.45(1H, s).

### Example 499

### N-(2-cyanoethyl)-4-((2,3-dihydro-1-benzofuran-5-ylcarbonyl)amino)-1-(tetrahydro-2H-pyran-2-yl)-1H-pyrazole-3-carboxamide

In the same manner as in Example 402, the title compound was obtained using 4-amino-N-(2-cyanoethyl)-1-(tetrahydro-2H-pyran-2-yl)-1H-pyrazole-3-carboxamide obtained in Reference Example 52 and 2,3-dihydro-1-benzofuran-5-carboxylic acid. yield 73%. melting point 176-177°C (ethyl acetate-hexane).
¹H-NMR (CDCl₃) :δ 1.61-1.79 (3H, m), 1.99-2.12(2H, m), 2.12-2.30 (1H, m), 2.74(2H, t, J=6.6 Hz), 3.28(2H, t, J=8.8 Hz), 3.65-3.80(3H, m), 4.02-4.11(1H, m), 4.66(2H, t, J=8.8 Hz), 5.36(1H, dd, J=2.2, 9.5 Hz), 6.85(1H, d, J=8.5 Hz), 7.22-7.23(1H, m), 7.76(1H, dd, J=2.2, 8.5 Hz), 8.51(1H, s), 10.17(1H, brs).

### Example 500

### N-(2-cyanoethyl)-4-(3-furoylamino)-1-(tetrahydro-2H-pyran-2-yl)-1H-pyrazole-3-carboxamide

In the same manner as in Example 402, the title compound was obtained using 4-amino-N-(2-cyanoethyl)-1-(tetrahydro-2H-pyran-2-yl)-1H-pyrazole-3-carboxamide obtained in Reference Example 52 and furan-3-carboxylic acid. yield 74%. melting point 135-137°C (ethyl acetate-hexane).
¹H-NMR (CDCl₃): δ 1.60-1.80(3H, m), 1.98-2.10(2H, m), 2.11-2.25 (1H, m), 2.73(2H, t, J=6.6 Hz), 3.66-3.77(3H, m), 4.03-4.11 (1H, m), 5.35 (1H, dd, J=2.5 Hz, 9.3 Hz), 6.54 (1H, dd, J=1.7 Hz, 3.6 Hz), 7.21 (1H, dd, J=0.8 Hz, 3.6 Hz), 7.24-7.32 (1H, m), 7.55 (1H, dd, J=0.8 Hz, 1.7 Hz), 8.47 (1H, s), 10.28 (1H, s).

### Example 501

### 4-((3-acetylbenzoyl)amino)-N-(2-cyanoethyl)-1H-pyrazole-3-carboxamide

In the same manner as in Example 401, the title compound was obtained using 4-((3-acetylbenzoyl)amino)-N-(2-cyanoethyl)-1-(tetrahydro-2H-pyran-2-yl)-1H-pyrazole-3-carboxamide obtained in Example 498. yield 79%. melting point 205-208°C (ethyl acetate).
¹H-NMR (CDCl₃) :δ 2.66(3H, s), 2.81(2H, t, J=6.5 Hz), 3.53(2H, q, J=6.5 Hz), 6.73(1H, m), 7.23(1H, d, J=3.0 Hz), 7.97(1H, s), 8.29 (1H, s), 8.81(1H, t, J=5.8 Hz), 10.49(1H, s), 13.37(1H, s).

### Example 502

### N-(2-cyanoethyl)-4-((2,3-dihydro-1-benzofuran-5-ylcarbonyl)amino)-1H-pyrazole-3-carboxamide

In the same manner as in Example 401, the title compound was obtained using N-(2-cyanoethyl)-4-((2,3-dihydro-1-benzofuran-5-ylcarbonyl)amino)-1-(tetrahydro-2H-pyran-2-yl)-1H-pyrazole-3-carboxamide obtained in Example 499. yield 32%. melting point 206-207°C (ethyl acetate-hexane).
¹H-NMR (CDCl₃) :δ 2.83(2H, t, J=6.6 Hz), 3.28(2H, t, J=8.8 Hz), 3.53(2H, q, J=6.3 Hz), 4.64(2H, t, J=8.8 Hz), 6.93(1H, d, J=8.4 Hz), 7.66(1H, d, J=8.4 Hz), 7.73(1H, s), 8.82(1H, t, J=5.8 Hz), 10.47(1H, s), 13.34(1H, s).

### Example 503

### N-(2-cyanoethyl)-4-(3-furoylamino)-1H-pyrazole-3-carboxamide

In the same manner as in Example 401, the title compound was obtained using N-(2-cyanoethyl)-4-(3-furoylamino)-1-(tetrahydro-2H-pyran-2-yl)-1H-pyrazole-3-carboxamide obtained in Example 500. yield 84%. melting point 250-252°C (ethyl acetate-hexane).
¹H-NMR (CDCl₃):δ 2.80(2H, t, J=6.6 Hz), 3.52(2H, q, J=6.3 Hz), 6.77-6.79 (1H, m), 7.83-7.85 (1H, m), 8.25 (1H, s), 8.34 (1H, s), 8.79 (1H, t, J=5.9 Hz), 10.10 (1H, s), 13.34 (1H, s).

### Example 504

### N-(2-cyanoethyl)-4-((5-phenyl-2-furoyl)amino)-1-(tetrahydro-2H-pyran-2-yl)-1H-pyrazole-3-carboxamide

In the same manner as in Example 402, the title compound was obtained using 4-amino-N-(2-cyanoethyl)-1-(tetrahydro-2H-pyran-2-yl)-1H-pyrazole-3-carboxamide obtained in Reference Example 52 and 5-phenyl-2-furancarboxylic acid. yield 82%. melting point 200-202°C (ethyl acetate-hexane).
¹H-NMR (CDCl₃):δ 1.59-1.80(3H, m), 1.99-2.12(2H, m), 2.12-2.32(1H, m), 2.76(2H, t, J=6.6 Hz), 4.03-4.12 (1H, m), 5.36(1H, dd, J=2.3 Hz, 9.5 Hz), 6.78 (1H, d, J=3.6 Hz), 7.19-7.25 (1H, m), 7.28 (1H, d, J=3.6 Hz), 7.32-7.40(1H, m), 7.41-7.52(2H, m), 7.77-7.85(2H, m), 8.48(1H, s), 10.37(1H, s).

### Example 505

### 4-((1-benzofuran-2-ylcarbonyl)amino)-N-(2-cyanoethyl)-1-(tetrahydro-2H-pyran-2-yl)-1H-pyrazole-3-carboxamide

In the same manner as in Example 402, the title compound was obtained using 4-amino-N-(2-cyanoethyl)-1-(tetrahydro-2H-pyran-2-yl)-1H-pyrazole-3-carboxamide obtained in Reference Example 52 and 1-benzofuran-2-carboxylic acid. yield 89%. melting point 192-194°C (ethyl acetate-hexane).
¹H-NMR (CDCl₃):δ 1.58-1.82(3H, m), 1.98-2.13(2H, m), 2.12-2.31(1H, m), 2.77(2H, t, J=6.5 Hz), 3.66-3.83(3H, m), 4.02-4.12(2H, m), 5.37(1H, dd, J=2.3 Hz, 9.5 Hz), 7.23-7.35(2H, m), 7.44(1H, ddd, J=1.1 Hz, 7.15 Hz, 8.25 Hz), 7.56(1H, s), 7.63(1H, dd, J=0.8 Hz, 8.25 Hz), 7.66-7.71(1H, m), 8.53(1H, s), 10.47(1H, s).

### Example 506

### 4-((1-benzofuran-2-ylcarbonyl)amino)-N-(2-cyanoethyl)-1H-pyrazole-3-carboxamide

In the same manner as in Example 401, the title compound was obtained using 4-((1-benzofuran-2-ylcarbonyl)amino)-N-(2-cyanoethyl)-1-(tetrahydro-2H-pyran-2-yl)-1H-pyrazole-3-carboxamide obtained in Example 505. yield 85%. melting point 231-233°C (ethyl acetate-hexane).
¹H-NMR (CDCl₃):δ 2.83(2H, t, J=6.5 Hz), 3.57(2H, q, J=6.3 Hz), 7.38(1H, t, J=7.6 Hz), 7.53(1H, t, J=7.4 Hz), 7.70(1H, s), 7.80(2H, dd, J=12.1 Hz, 8.3 Hz), 8.38(1H s), 8.87(1H,t, J=5.8 Hz), 10.74 (1H, s), 13.47 (1H, s).

### Example 507

### N-(2-cyanoethyl)-4-((5-phenyl-2-furoyl)amino)-1H-pyrazole-3-carboxamide

In the same manner as in Example 401, the title compound was obtained using N-(2-cyanoethyl)-4-((5-phenyl-2-furoyl)amino)-1-(tetrahydro-2H-pyran-2-yl)-1H-pyrazole-3-carboxamide obtained in Example 504. yield 73%. melting point 228-230°C (ethyl acetate).
¹H-NMR (CDCl₃):δ 2.84(2H, t, J=6.5 Hz), 3.58 (2H,q, J=6.4 Hz), 7. 23 (1H, d, J=3.6 Hz), 7.35 (1H, d, J=3.6 Hz), 7. 43 (1H, t, J=7.4 Hz), 7.54(2H, t, J=7.6 Hz), 8.32(1H, s), 8.82(1H, t, J=5.8 Hz), 10.64(1H, s), 13.42(1H, s).

The compounds obtained in Examples 337 - 507 are shown in the following Table 6-1 to Table 6-13. In the Tables, THP means tetrahydropyranyl.

**Table 6-1**

| | | | | | | | |
|---|---|---|---|---|---|---|---|
| 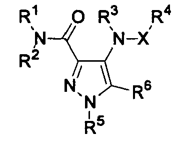 | | | | | | | |

| Ex. | R1 | R2 | R3 | X | R4 | R5 | R6 |
|---|---|---|---|---|---|---|---|
| 337 | PhCH2 | H | H | CO | 2-Py | H | H |
| 338 | PhCH2CH2 | H | H | CO | 2-Py | H | H |
| 339 | 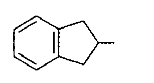 | H | H | CO | 2-Py | H | H |
| 340 | 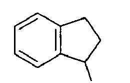 | H | H | CO | 2-Py | H | H |
| 341 | Ph | H | H | CO | 2-Py | H | H |
| 342 | PhCH2CH2CH2 | H | H | CO | 2-Py | H | H |
| 343 | 3-CF3-Ph | H | H | CO | 2-Py | H | H |
| 344 | 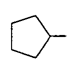 | H | H | CO | 2-Py | H | H |
| 345 | i-Pr | H | H | CO | 2-Py | H | H |
| 346 | Me | H | H | CO | 2-Py | H | H |
| 347 | 2-FurylCH2 | H | H | CO | 2-Py | H | H |
| 348 | 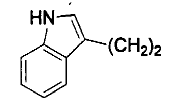 | H | H | CO | 2-Py | H | H |
| 349 | Cyclohexyl | H | H | CO | 2-Py | H | H |

**Table 6-2**

| Ex. | R1 | R2 | R3 | X | R4 | R5 | R6 |
|---|---|---|---|---|---|---|---|
| 350 | 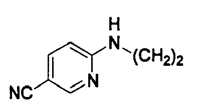 | H | H | CO | 2-Py | H | H |
| 351 | 4-Br-PhCH2CH2 | H | H | CO | 2-Py | H | H |
| 352 | 3-PyCH2CH2 | H | H | CO | 2-Py | H | H |
| 353 | iPrO(CH2)3 | H | H | CO | 2-Py | H | H |
| 354 | 4-Ph-PhCH2 | H | H | CO | 2-Py | H | H |
| 355 | PhO (CH2)2 | H | H | CO | 2-Py | H | H |
| 356 | MeO (CH2) 2 | H | H | CO | 2-Py | H | H |
| 357 | iPrO(CH2)2 | H | H | CO | 2-Py | H | H |
| 358 | NC(CH2)2 | H | H | CO | 2-Py | H | H |
| 359 | PhCOCH2 | H | H | CO | 2-Py | H | H |
| 360 | CF3CH2 | H | H | CO | 2-Py | H | H |
| 361 | PhS(CH2)2 | H | H | CO | 2-Py | H | H |
| 362 | 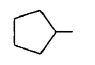 | H | H | CO | 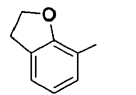 | H | H |
| 363 | 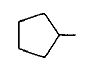 | H | H | CO | 2-Me-3-Py | H | H |

**Table 6-3**

| Ex. | R1 | R2 | R3 | X | R4 | R5 | R6 |
|---|---|---|---|---|---|---|---|
| 364 | 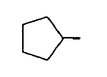 | H | H | CO | 5-Me-3-Py | H | H |
| 365 | 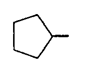 | H | H | CO | 2-MeO-3-Py | H | H |
| 366 | 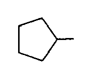 | H | H | CO | 2,5-diF-Ph | H | H |
| 367 | 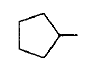 | H | H | CO | 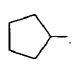 | H | H |
| 368 | 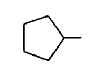 | H | H | CO | 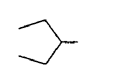 | H | H |
| 369 | PhO(CH2)2 | H | H | CO | 3-AcNH-Ph | H | H |
| 370 | MeO(CH2)2 | H | H | CO | 3-AcNH-Ph | H | H |
| 371 | iPrO(CH2)2 | H | H | CO | 3-AcNH-Ph | H | H |
| 372 | NC(CH2)2 | H | H | CO | 3-AcNH-Ph | H | H |
| 373 | Me2N(CH2)2 | H | H | CO | 3-AcNH-Ph | H | H |
| 374 | PhCOCH2 | H | H | CO | 3-AcNH-Ph | H | H |
| 375 | CF3CH2 | H | H | CO | 3-AcNH-Ph | H | H |
| 376 | PhS (CH2) 2 | H | H | CO | 3-AcNH-Ph | H | H |
| 377 | 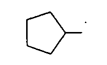 | H | H | CO | Pyrazinyl | H | Me |

**Table 6-4**

| Ex. | R1 | R2 | R3 | X | R4 | R5 | R6 |
|---|---|---|---|---|---|---|---|
| 378 | 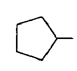 | H | H | CO | Ph | H | Me |
| 379 | 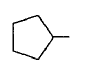 | H | H | CO | Pyrazinyl | H | Et |
| 380 | NCCH2 | H | H | CO | 3-AcNH-Ph | H | H |
| 381 | tBuO(CH2)2 | H | H | CO | 3-AcNH-Ph | H | H |
| 382 | MeSO2(CH2)2 | H | H | CO | 3-AcNH-Ph | H | H |
| 383 | 4-Cl-PhSO2(CH2)2 | H | H | CO | 3-AcNH-Ph | H | H |
| 384 | 2-PySO2(CH2)2 | H | H | CO | 3-AcNH-Ph | H | H |
| 385 | iPrO(CH2)3 | H | H | CO | 3-AcNH-Ph | H | H |
| 386 | NC(CH2)2 | H | H | CO | 6-MePy | THP | H |
| 387 | NC(CH2)2 | H | H | CO | 6-MePy | H | H |
| 388 | CF3CH2 | H | H | CO | 6-MePy | THP | H |
| 389 | CF3CH2 | H | H | CO | 6-MePy | H | H |
| 390 | PhO(CH2)2 | H | H | CO | 2-Py | THP | H |
| 391 | PhO(CH2)2 | H | H | CO | 2-Py | H | H |

**Table 6-5**

| Ex. | R1 | R2 | R3 | X | R4 | R5 | R6 |
|---|---|---|---|---|---|---|---|
| 392 | CF3CH2CH2 | H | H | CO | 2-Py | THP | H |
| 393 | CF3CH2CH2 | H | H | CO | 2-Py | H | H |
| 394 | NCCH2 | H | H | CO | 2-Py | THP | H |
| 395 | NCCH2 | H | H | CO | 2-Py | H | H |
| 396 | 2-Py-NH(CH2)2 | H | H | CO | 2-Py | THP | H |
| 397 | 2-Py-NH(CH2)2 | H | H | CO | 2-Py | H | H |
| 398 | Ph-NH(CH2)2 | H | H | CO | 2-Py | THP | H |
| 399 | Ph-NH(CH2)2 | H | H | CO | 2-Py | H | H |
| 400 | HO(CH2)2 | H | H | CO | 2-Py | THP | H |
| 401 | HO(CH2)2 | H | H | CO | 2-Py | H | H |
| 402 | NC(CH2)2 | H | H | CO | 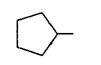 | THP | H |
| 403 | NC(CH2)2 | H | H | CO | 2-Furyl | THP | H |
| 404 | NC(CH2)2 | H | H | CO | 3-Me-Ph | THP | H |
| 405 | NC(CH2)2 | H | H | CO | 3-MeO-Ph | THP | H |

**Table 6-6**

| Ex. | R1 | R2 | R3 | X | R4 | R5 | R6 |
|---|---|---|---|---|---|---|---|
| 406 | NC (CH2) 2 | H | H | CO | tBu | THP | H |
| 407 | NC(CH2)2 | H | H | CO | 2-Thienyl | THP | H |
| 408 | NC (CH2) 2 | H | H | CO | 3-CN-Ph | THP | H |
| 409 | NC (CH2) 2 | H | H | CO | 4-Me-Ph | THP | H |
| 410 | NC (CH2) 2 | H | H | CO | 4-Me-Ph | THP | H |
| 411 | NC (CH2) 2 | H | H | CO | 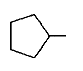 | H | H |
| 412 | NC (CH2) 2 | H | H | CO | 2-Furyl | H | H |
| 413 | NC (CH2) 2 | H | H | CO | 3-Me-Ph | H | H |
| 414 | NC (CH2) 2 | H | H | CO | 3-MeO-Ph | H | H |
| 415 | NC (CH2) 2 | H | H | CO | tBu | H | H |
| 416 | NC (CH2) 2 | H | H | CO | 2-Thienyl | H | H |
| 417 | NC(CH2)2 | H | H | CO | 3-CN-Ph | H | H |
| 418 | NC (CH2) 2 | H | H | CO | 4-Me-Ph | H | H |
| 419 | NC (CH2) 2 | H | H | CO | Ph | THP | H |

**Table 6-7**

| Ex. | R1 | R2 | R3 | X | R4 | R5 | R6 |
|---|---|---|---|---|---|---|---|
| 420 | NC(CH2)2 | H | H | CO | Ph | H | H |
| 421 | CF3CH2 | H | H | CO | Ph | THP | H |
| 422 | CF3CH2 | H | H | CO | Ph | H | H |
| 423 | iPrO (CH2) 2 | H | H | CO | Ph | THP | H |
| 424 | iPrO(CH2)2 | H | H | CO | Ph | H | H |
| 425 | iPrO(CH2)2 | H | H | CO | 6-Me-2-Py | THP | H |
| 426 | iPrO(CH2)2 | H | H | CO | 6-Me-2-Py | H | H |
| 427 | 4-F-PhO(CH2)2 | H | H | CO | 6-Me-2-Py | THP | H |
| 428 | 4-F-PhO(CH2)2 | H | H | CO | 6-Me-2-Py | H | H |
| 429 | iPrO (CH2) 3 | H | H | CO | 6-Me-2-Py | THP | H |
| 430 | iPrO (CH2) 3 | H | H | CO | 6-Me-2-Py | H | H |
| 431 | (3-Me-Ph)EtN(CH2)2 | H | H | CO | 6-Me-2-Py | THP | H |
| 432 | (3-Me-Ph)EtN(CH2)2 | H | H | CO | 6-Me-2-Py | H | H |
| 433 | EtOOC(CH2)2 | H | H | CO | 6-Me-2-Py | THP | H |

**Table 6-8**

| Ex. | R1 | R2 | R3 | X | R4 | R5 | R6 |
|---|---|---|---|---|---|---|---|
| 434 | EtOOC(CH2)2 | H | H | CO | 6-Me-2-Py | H | H |
| 435 | HOOC(CH2)2 | H | H | CO | 6-Me-2-Py | H | H |
| 436 | MeOOCCH2 | H | H | CO | 6-Me-2-Py | THP | H |
| 437 | MeOOCCH2 | H | H | CO | 6-Me-2-Py | H | H |
| 438 | HO(CH2)2 | H | H | CO | 6-Me-2-Py | THP | H |
| 439 | HO(CH2)2 | H | H | CO | 6-Me-2-Py | H | H |
| 440 | PhCH2(OH)CHCH2 | H | H | CO | 6-Me-2-Py | THP | H |
| 441 | PhCH2(OH)CHCH2 | H | H | CO | 6-Me-2-Py | H | H |
| 442 | HO(CH2)3 | H | H | CO | 6-Me-2-Py | THP | H |
| 443 | HO(CH2)3 | H | H | CO | 6-Me-2-Py | H | H |
| 444 | HOCH2(CH3)2CCH2 | H | H | CO | 6-Me-2-Py | THP | H |
| 445 | HOCH2 (CH3) 2CCH2 | H | H | CO | 6-Me-2-Py | H | H |
| 446 | 4-CF3-Ph(OH)CHCH2 | H | H | CO | 6-Me-2-Py | THP | H |
| 447 | 4-CF3-Ph(OH)CHCH2 | H | H | CO | 6-Me-2-Py | H | H |

**Table 6-9**

| Ex. | R1 | R2 | R3 | X | R4 | R5 | R6 |
|---|---|---|---|---|---|---|---|
| 448 | F(CH2)2 | H | H | CO | 6-Me-2-Py | THP | H |
| 449 | F(CH2)2 | H | H | CO | 6-Me-2-Py | H | H |
| 450 | NC(CH3)2CCH2 | H | H | CO | 6-Me-2-Py | THP | H |
| 451 | NC(CH3)2CCH2 | H | H | CO | 6-Me-2-Py | H | H |
| 452 | 2-Py-S(CH2)2 | H | H | CO | 2-Py | THP | H |
| 453 | 2-Py-S(CH2)2 | H | H | CO | 2-Py | H | H |
| 454 | tBuO(CH2)3 | H | H | CO | 2-Py | THP | H |
| 455 | tBuO(CH2)3 | H | H | CO | 2-Py | H | H |
| 456 | NC(CH2)3 | H | H | CO | 2-Py | THP | H |
| 457 | NC(CH2)3 | H | H | CO | 2-Py | H | H |
| 458 | CH3C(CN)(CH3)CH2 | H | H | CO | 2-Py | THP | H |
| 459 | CH3C(CN)(CH3)CH2 | H | H | CO | 2-Py | H | H |
| 460 | PhCH2C(CH3)(CN)CH2 | H | H | CO | 2-Py | THP | H |
| 461 | PhCH2C(CH3)(CN)CH2 | H | H | CO | 2-Py | H | H |

**Table 6-10**

| Ex. | R1 | R2 | R3 | X | R4 | R5 | R6 |
|---|---|---|---|---|---|---|---|
| 462 | EtOOC(CH2)3 | H | H | CO | 2-Py | THP | H |
| 463 | EtOOC(CH2)3 | H | H | CO | 2-Py | H | H |
| 464 | HOOC(CH2)3 | H | H | CO | 2-Py | H | H |
| 465 | HOCH2C(CH3)2 | H | H | CO | 2-Py | THP | H |
| 466 | HOCH2C(CH3)2 | H | H | CO | 2-Py | H | H |
| 467 | HO(CH2)3 | H | H | CO | 2-Py | THP | H |
| 468 | HO(CH2)3 | H | H | CO | 2-Py | H | H |
| 469 | HOCH2C(CH3)2CH2 | H | H | CO | 2-Py | THP | H |
| 470 | HOCH2C(CH3)2CH2 | H | H | CO | 2-Py | H | H |
| 471 | CH3CH2CH(OH)CH2 | H | H | CO | 2-Py | THP | H |
| 472 | CH3CH2CH(OH)CH2 | H | H | CO | 2-Py | H | H |
| 473 | PhCH2CH(OH)CH2 | H | H | CO | 2-Py | THP | H |
| 474 | PhCH2CH(OH)CH2 | H | H | CO | 2-Py | H | H |
| 475 | CH3CH(OH) (CH3)CH2CH2 | H | H | CO | 2-Py | THP | H |

**Table 6-11**

| Ex. | R1 | R2 | R3 | X | R4 | R5 | R6 |
|---|---|---|---|---|---|---|---|
| 476 | CH3CH(OH) (CH3)CH2CH2 | H | H | CO | 2-Py | H | H |
| 477 | iPrO(CH2)2 | H | H | CO | 6-Me-3-Py | THP | H |
| 478 | iPrO(CH2)2 | H | H | CO | 6-Me-3-Py | H | H |
| 479 | CF3CH2 | H | H | CO | 6-Me-3-Py | THP | H |
| 480 | CF3CH2 | H | H | CO | 6-Me-3-Py | H | H |
| 481 | CF3CH2CH2 | H | H | CO | 6-Me-3-Py | THP | H |
| 482 | CF3CH2CH2 | H | H | CO | 6-Me-3-Py | H | H |
| 483 | NCCH2CH2 | H | H | CO | 6-Me-3-Py | THP | H |
| 484 | NCCH2CH2 | H | H | CO | 6-Me-3-Py | H | H |
| 485 | HOCH2CH2 | H | H | CO | 6-Me-3-Py | THP | H |
| 486 | HOCH2CH2 | H | H | CO | 6-Me-3-Py | H | H |
| 487 | NCCH2CH2 | H | H | CO | 4-Cl-2-Py | THP | H |
| 488 | NCCH2CH2 | H | H | CO | 4-Cl-2-Py | H | H |
| 489 | NCCH2CH2 | H | H | CO | 4-MeOPh | H | H |

**Table 6-12**

| Ex. | R1 | R2 | R3 | X | R4 | R5 | R6 |
|---|---|---|---|---|---|---|---|
| 490 | NCCH2CH2 | H | H | CO | 4-CF3Ph | THP | H |
| 491 | NCCH2CH2 | H | H | CO | 3-CF3Ph | THP | H |
| 492 | NCCH2CH2 | H | H | CO | 4-CF3Ph | H | H |
| 493 | NCCH2CH2 | H | H | CO | 3-CF3Ph | H | H |
| 494 | NCCH2CH2 | H | H | CO | 5-Br-2-furyl | THP | H |
| 495 | NCCH2CH2 | H | H | CO | 5-Me-2-furyl | THP | H |
| 496 | NCCH2CH2 | H | H | CO | 5-Br-2-furyl | H | H |
| 497 | NCCH2CH2 | H | H | CO | 5-Me-2-furyl | H | H |
| 498 | NCCH2CH2 | H | H | CO | 3-AcPh | THP | H |
| 499 | NCCH2CH2 | H | H | CO | 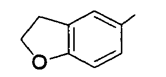 | THP | H |
| 500 | NCCH2CH2 | H | H | CO | 3-furyl | THP | H |
| 501 | NCCH2CH2 | H | H | CO | 3-AcPh | H | H |
| 502 | NCCH2CH2 | H | H | CO | 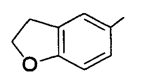 | H | H |
| 503 | NCCH2CH2 | H | H | CO | 3-furyl | H | H |

**Table 6-13**

| Ex. | R1 | R2 | R3 | X | R4 | R5 | R6 |
|---|---|---|---|---|---|---|---|
| 504 | NCCH2CH2 | H | H | CO | 5-Ph-2-furyl | THP | H |
| 505 | NCCH2CH2 | H | H | CO | 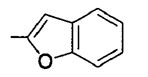 | THP | H |
| 506 | NCCH2CH2 | H | H | CO | 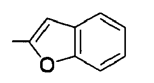 | H | H |
| 507 | NCCH2CH2 | H | H | CO | 5-Ph-2-furyl | H | H |

### Experimental Example 1

### a) test material

### Cloning of human GSK3β gene and preparation of recombinant Baculovirus

Human GSK3β gene was cloned by a PCR method using a primer set:
GSK3b-U: 5'-AAAGAATTCACCATGGACTACAAGGACGACGATGACAAGTCAGGGCGGCCCAGAACCACCTCCT
T-3' (SEQ ID NO: 1) and GSK3b-L: 5'-AAAAGTCGACTCAGGTGGAGTTGGAAGCTGATGCAGAAG-3' (SEQ ID NO: 2) prepared using human brain cDNA (Clontech; trade name: QUICK-Clone cDNA) as a template and in reference to the base sequence of GSK3β gene registered in GenBank under Accession Number NM_002093.

PCR was done according to the protocol attached to KOD plus DNA polymerase (Toyo Boseki Kabushiki Kaisha). The obtained PCR product was electrophoresed on agarose gel (1%), and 1.2kb of DNA fragment including GSK3β gene was collected from the gel and then digested with restriction enzymes EcoR I and Sal I. The restriction enzyme-treated DNA was electrophoresed on agarose gel (1%). The obtained DNA fragment was collected, and ligated to the plasmid pFASTBAC1 (Invitrogen), which had been digested with restriction enzyme EcoR I and Sal I, to produce expression plasmid pFB-GSK3B. The base sequence of insertion fragment was confirmed to find that it corresponded to the objective sequence. In addition, virus stock BAC-GSK3B of recombinant Baculovirus was prepared using BAC-TO-BAC Baculovirus Expression System (Invitrogen).

### Preparation of recombinant GSK3β enzyme

Sf-21 cells (Invitrogen) were seeded on Sf-900 II SFM medium (Invitrogen)(150 ml) containing 10% fetal calf serum at 1x10⁶cells/ml, and cultured at 27°C for 24 hr. The virus stock BAC-IKKB obtained above of recombinant Baculovirus was added by 150 µl to the obtained culture medium, and the cells were cultured for another 60 hr. The culture medium was centrifuged (3000 rpm, 10 min) to separate the cells, and the cells were washed once with PBS. The cells were suspended in 10 ml of cell lysis buffer (25 mM HEPES (pH 7.5), 1% Triton X, 130 mM sodium chloride, 1 mM EDTA, 1 mM Dithiothreitol, 25 mM β-glycerophosphate, Protease inhibitor Complete (Boehringer), 1 mM Sodium orthovanadate), treated 4 times using a homogenizer (POLYTRON) at 20000 rpm for 30 sec to disrupt the cells. The disrupted solution of cells was centrifuged (40000 rpm, 45 min.), and GSK3β was purified from the obtained supernatant using Anti-FLAG M2 Affinity Gel (Sigma Ltd.).

### b) Experimental method

### Measurement of GSK3β inhibitory activity

To a reaction solution (25 mM HEPES (pH 7.5), 10 mM magnesium acetate, 1 mM DTT, 0.01% bovine serum albumin (Wako Pure Chemical Industries, Ltd.)) (37.5 µl) containing recombinant GSK3β enzyme (100 ng) obtained above and a matrix peptide from glycogen synthase (YRRAAVPPSPSLSRHSSPHQpSEDEEE (SEQ ID NO: 3), pS shows phosphorylated serine) (100 ng) was added a test compound (2.5 µl) dissolved in DMSO, and the mixture was incubated at room temperature for 5 min. 10 µl of ATP solution (2.5 µM ATP) was added to the obtained mixture, and the mixture was reacted at room temperature for 30 min. Kinase Glo Reagent (Promega) (50 µl) was added to the reaction solution to stop the reaction. After reaction at room temperature for 10 min, the luminescent intensity was measured using ARVO multilabel counter (PerkinElmer Life Sciences). The concentrations of the test compounds needed for 50% inhibition of luminescent intensity (IC₅₀ value) were calculated by PRISM 3.0 (GraphPad Software). The results are shown in Table 7.

**Table 7**

| Ex. No. | IC50 (nM) | Ex. No. | IC50 (nM) | Ex. No. | IC50 (nM) | Ex. No. | IC50 (nM) |
|---|---|---|---|---|---|---|---|
| 001 | 267 | 081 | 106 | 148 | 388 | 364 | <100 |
| 003 | <100 | 083 | <100 | 149 | 334 | 365 | <100 |
| 004 | <100 | 084 | <100 | 152 | <100 | 366 | <100 |
| 005 | 307 | 085 | <100 | 154 | 271 | 367 | <100 |
| 017 | <100 | 087 | 115 | 155 | <100 | 368 | <100 |
| 019 | <100 | 089 | 379 | 156 | <100 | 369 | <100 |
| 020 | <100 | 097 | <100 | 157 | 140 | 370 | <100 |
| 021 | <100 | 098 | <100 | 159 | <100 | 371 | <100 |
| 022 | 233 | 099 | 102 | 160 | 194 | 372 | <100 |
| 023 | <100 | 101 | <100 | 161 | <100 | 373 | <100 |
| 024 | <100 | 102 | <100 | 162 | <100 | 374 | <100 |
| 025 | 245 | 103 | <100 | 197 | 109 | 375 | <100 |
| 026 | 169 | 105 | 273 | 227 | 274 | 376 | <100 |
| 027 | <100 | 106 | 242 | 240 | 208 | 378 | 1000 |
| 028 | <100 | 109 | <100 | 305 | <100 | 380 | <100 |
| 030 | <100 | 112 | <100 | 337 | <100 | 381 | 140 |
| 031 | <100 | 113 | <100 | 338 | <100 | 382 | <100 |
| 032 | <100 | 114 | 394 | 339 | <100 | 383 | <100 |
| 033 | 171 | 116 | <100 | 340 | 300 | 384 | <100 |
| 034 | <100 | 117 | 157 | 341 | <100 | 385 | <100 |
| 035 | <100 | 119 | <100 | 342 | <100 | 387 | <100 |
| 036 | <100 | 120 | <100 | 343 | <100 | 389 | <100 |
| 037 | <100 | 121 | <100 | 344 | <100 | 391 | <100 |
| 045 | 269 | 123 | <100 | 345 | <100 | 393 | <100 |
| 059 | <100 | 124 | <100 | 346 | <100 | 395 | <100 |
| 062 | <100 | 125 | <100 | 347 | <100 | 397 | <100 |
| 063 | <100 | 127 | <100 | 348 | <100 | 399 | <100 |
| 064 | <100 | 128 | <100 | 349 | <100 | 401 | <100 |
| 065 | 234 | 129 | 157 | 350 | <100 | 411 | <100 |
| 067 | <100 | 130 | 128 | 351 | <100 | 412 | <100 |
| 068 | <100 | 131 | <100 | 352 | <100 | 413 | <100 |
| 069 | <100 | 132 | 101 | 353 | <100 | 414 | <100 |
| 070 | <100 | 133 | <100 | 354 | <100 | 415 | <100 |
| 071 | <100 | 134 | <100 | 355 | <100 | 416 | <100 |
| 072 | <100 | 135 | <100 | 356 | <100 | 417 | <100 |
| 073 | <100 | 136 | 485 | 357 | <100 | 418 | <100 |
| 074 | 299 | 137 | <100 | 358 | <100 | 420 | <100 |
| 075 | <100 | 138 | <100 | 359 | <100 | 422 | <100 |
| 076 | <100 | 141 | <100 | 360 | <100 | 424 | <100 |
| 077 | <100 | 143 | 155 | 361 | <100 | 426 | <100 |
| 078 | <100 | 145 | <100 | 362 | <100 | 428 | <100 |
| 079 | <100 | 147 | <100 | 363 | <100 | | |

### Experimental Example 2

### Measurement of nerve cell death suppressive activity

The cerebral cortex was taken from fetus of TP-14 pregnant SD rat (CLEA Japan, Inc.), and nerve cell was prepared using nerve cell culture system (SUMITOMO BAKELITE). The cerebral cortex was placed in enzymatic solution of cell dispersion solution kit (SUMITOMO BAKELITE), and incubated at 37°C for 30 min. The cells were suspended by pipetting, and collected by centrifugation at 1000 rpm for 5 min. The supernatant was removed, and cell dispersion solution (SUMITOMO BAKELITE) was added. The cells were suspended by pipetting. The cell suspension was placed on removing solution (SUMITOMO BAKELITE), and the cells were collected by centrifugation at 1000 rpm for 5 min. The supernatant was removed, and the cells were suspended in nerve cell culture medium (SUMITOMO BAKELITE). 100 µl of the suspension was seeded on a poly D lysine laminin 96 well plate (Becton, Dickinson and Company) at 1x10⁵ cells/well. Then, the plate was cultured in a carbon dioxide gas incubator at 37°C for 4 days. A test compound (50 µl) was added on the 96 well plate, and 50 µL/well of LY294002 was added at 50 µM of the final concentration. Then, the plate was cultured in a carbon dioxide gas incubator for 24 hr. The medium (100 µl) was sampled, and 100 µl of Cell Titer Glo solution (Promega) was added to count the viable cell number. The concentration of the test compound needed for 50% inhibition of nerve cell death (IC₅₀ value) was calculated by PRISM 3.0 (GraphPad Software). The results are shown in Table 8.

**[Table 8]**

| Example number | IC50 value (nM) |
|---|---|
| 79 | 70 |
| 98 | 440 |

### Experimental Example 3

### Measurement of sugar release suppressive action using cultured hepatocyte

H4IIE-C3 cells (from rat liver) were seeded on 24 well or 96 well collagen coated plate (manufactured by Falcon), and cultured in Dulbecco's Modified Eagle Medium (DMEM: manufactured by GIBCO) containing 10% FBS, 25 mM Glucose until the cells became sub-confluent. Then, the cells were starvation treated with DMEM containing 0% FBS and 5.6 mM glucose. The medium was exchanged to DMEM containing test compound adjusted to 10 µM and sugar production stimulant (Dexamethasone (500 nM), 8CPT-cAMP (0.1 mM)), and the cells were pretreated for 5 hr. After washing with PBS, the cells were cultured in Phenol Red-free, Glucose-free DMEM containing the test compound and sugar production stimulant for 3 hr, and the glucose content released in the medium was measured. A glucose measurement kit (A-22189) manufactured by Molecular Probes was used for the glucose measurement. The obtained results of sugar release suppression activity are shown in the following. The sugar release suppressive activity is shown in a relative value when the released glucose content of a GSK3β inhibitor free group is 100%.
Example 387 69%
Example 412 74%
Example 413 64%
Example 416 62%
Example 420 62%
Example 426 55%
Example 430 76%
Example 455 79%
Example 470 75%
Example 480 69%

### Industrial Applicability

The pyrazole compound of the present invention is useful as an agent for the prophylaxis or treatment of GSK-3β related pathology or disease.

This application is based on application Nos. 2005-033356 and 2005-370962 filed in Japan, the contents of which are incorporated hereinto by reference.

## Claims

1. A compound represented by the formula 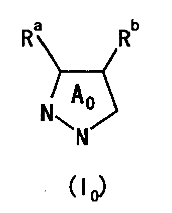 wherein
ring A₀ is a pyrazole ring optionally further having 1 or 2 substituents;
R^{a} is a substituted carbamoyl group; and
R^{b} is an optionally substituted acylamino group]
or a salt thereof, excluding the following compounds;
1) N-methyl-2,5-dimethyl-4-{2-[4-(4-nitrophenoxy)phenyl]acetylamino}-2H-pyrazole-3-carboxamide,
2) N-methyl-4-{2-[4-(4-chlorophenoxy)phenyl]acetylamino}-2,5-dimethyl-2H-pyrazole-3-carboxamide,
3) N-(9-methyl-4-oxo-1-phenyl-3,4,6,7-tetrahydro-[1,4]diazepino[6,7,1-hi]indol-3-yl)-4-acetylamino-2,5-dimethyl-2H-pyrazole-3-carboxamide,
4) N-phenyl-4-benzoylamino-2-ethyl-5-methyl-2H-pyrazole-3-carboxamide,
5) N-phenyl-2-ethyl-5-methyl-4-(2-nitrobenzoylamino)-2H-pyrazole-3-carboxamide,
6) N-(4-chlorophenyl)-2-ethyl-5-methyl-4-(4-nitrobenzoylamino)-2H-pyrazole-3-carboxamide,
7) N-phenyl-2-ethyl-5-methyl-4-(4-methylbenzoylamino)-2H-pyrazole-3-carboxamide,
8) N-methyl-N-(2-phenyl-2H-pyrazol-3-yl)-4-acetylamino-2-ethyl-5-methyl-2H-pyrazole-3-carboxamide,
9) N-methyl-N-(5-methyl-2-phenyl-2H-pyrazol-3-yl)-4-acetylamino-2-ethyl-5-methyl-2H-pyrazole-3-carboxamide,
10) N-methyl-N-(2-methyl-2H-pyrazol-3-yl)-4-acetylamino-2-ethyl-5-methyl-2H-pyrazole-3-carboxamide,
11) N-(2,5-dimethyl-2H-pyrazol-3-yl)-N-methyl-4-acetylamino-2-ethyl-5-methyl-2H-pyrazole-3-carboxamide,
12) N-(4-tert-butylbenzyl)-4-acetylamino-2,5-dimethyl-2H-pyrazole-3-carboxamide,
13) N-(4-tert-butylbenzyl)-4-(2-chloroacetylamino)-2,5-dimethyl-2H-pyrazole-3-carboxamide,
14) N-(4-tert-butylbenzyl)-4-benzoylamino-2,5-dimethyl-2H-pyrazole-3-carboxamide,
15) N,N-dimethyl-4-methanesulfonylamino-1-methyl-5-[1-methyl-2,5-dioxo-4-(1-phenylpropylamino)-2,5-dihydro-1H-pyrrol-3-ylamino]-1H-pyrazole-3-carboxamide,
16) N,N-dimethyl-5-[2,5-dioxo-4-(1-phenylpropylamino)-2,5-dihydro-1H-pyrrol-3-ylamino]-4-methanesulfonylamino-1-methyl-1H-pyrazole-3-carboxamide,
17) N,N-dimethyl-5-(4-chloro-1-methyl-2,5-dioxo-2,5-dihydro-1H-pyrrol-3-ylamino)-4-methanesulfonylamino-1-methyl-1H-pyrazole-3-carboxamide,
18) N,N-dimethyl-5-(4-chloro-1-methyl-2,5-dioxo-2,5-dihydro-1H-pyrrol-3-ylamino)-4-methanesulfonylamino-1-methyl-1H-pyrazole-3-carboxamide.

2. The compound of claim 1, wherein the compound represented by the formula (I₀) is a compound represented by the formula: 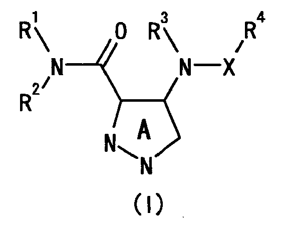 wherein
R¹ is an optionally substituted hydrocarbon group or an optionally substituted heterocyclic group,
R² is a hydrogen atom or an optionally substituted hydrocarbon group, or
R¹ and R² form, together with the adjacent nitrogen atom, an optionally substituted nitrogen-containing heterocycle;
R³ is a hydrogen atom or an optionally substituted hydrocarbon group;
R⁴ is an optionally substituted hydrocarbon group, an optionally substituted heterocyclic group, an optionally substituted hydroxy group, or an optionally substituted amino group;
ring A is a pyrazole ring represented by the formula: 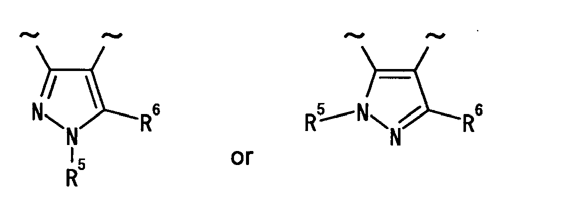 wherein R⁵ is a hydrogen atom, an optionally substituted hydrocarbon group or an optionally substituted acyl group; and R⁶ is a hydrogen atom or an optionally substituted hydrocarbon group; and
X is a carbonyl group or a sulfonyl group.

3. The compound of claim 2, wherein R² is a hydrogen atom.

4. The compound of claim 2, wherein R¹ is a C₁₋₆ alkyl group, a C₂₋₆ alkenyl group or a C₂₋₆ alkynyl group, which is optionally substituted by 1 to 5 substituents selected from halogen atom, optionally substituted hydroxy group, optionally substituted thio group, optionally substituted sulfinyl group, optionally substituted sulfonyl group, optionally substituted amino group, nitro group, cyano group, and optionally substituted carbonyl group.

5. The compound of claim 2, wherein R¹ is a C₁₋₆ alkyl group, a C₂₋₆ alkenyl group, or a C₂₋₆ alkynyl group optionally having, at substitutable positions, 1 to 5 substituents selected from
(1) halogen atom;
(2) hydroxy group;
(3) amino group;
(4) nitro group;
(5) cyano group;
(7) mono- or di-C₁₋₆ alkyl-amino group;
(8) mono- or di-C₆₋₁₄ aryl-amino group;
(9) mono- or di-C₇₋₁₆ aralkyl-amino group;
(10) N-C₁₋₆ alkyl-N-C₆₋₁₄ aryl-amino group;
(11) N-C₁₋₆ alkyl-N-(C₁₋₆ alkyl-C₆₋₁₄ aryl)-amino group;
(12) N-C₁₋₆ alkyl-N-C₇₋₁₆ aralkyl-amino group;
(14) optionally halogenated C₁₋₆ alkoxy group;
(15) C₁₋₆ alkylthio group optionally substituted by C₁₋₆ alkoxy group;
(18) optionally esterified carboxyl group;
(19) carbamoyl group;
(20) thiocarbamoyl group;
(21) mono- or di-C₁₋₆ alkyl-carbamoyl group;
(22) mono- or di-C₆₋₁₄ aryl-carbamoyl group;
(23) mono- or di-5- to 7-membered heterocycle-carbamoyl group;
(24) C₁₋₆ alkyl-carbonylamino group optionally substituted by carboxyl group;
(25) C₆₋₁₄ aryloxy group optionally substituted by 1 to 3 substituents selected from halogen atom, hydroxy group, amino group, nitro group, cyano group, optionally halogenated C₁₋₆ alkyl group, mono- or di-C₁₋₆ alkyl-amino group, C₆₋₁₄ aryl group, mono- or di-C₆₋₁₄ aryl-amino group, C₃₋₈ cycloalkyl group, C₁₋₆ alkoxy group, C₁₋₆ alkoxy-C₁₋₆ alkoxy group, C₁₋₆ alkylthio group, C₁₋₆ alkylsulfinyl group, C₁₋₆ alkylsulfonyl group, an optionally esterified carboxyl group, carbamoyl group, thiocarbamoyl group, mono- or di-C₁₋₆ alkyl-carbamoyl group, mono- or di-C₆₋₁₄ aryl-carbamoyl group, sulfamoyl group, mono- or di-C₁₋₆ alkyl-sulfamoyl group and mono- or di-C₆₋₁₄ aryl-sulfamoyl group;
(27) heterocyclic oxy group optionally substituted by 1 to 3 substituents selected from halogen atom, hydroxy group, amino group, nitro group, cyano group, optionally halogenated C₁₋₆ alkyl group, mono- or di-C₁₋₆ alkyl-amino group, C₆₋₁₄ aryl group, mono- or di-C₆₋₁₄ aryl-amino group, C₃₋₈ cycloalkyl group, C₁₋₆ alkoxy group, C₁₋₆ alkoxy-C₁₋₆ alkoxy group, C₁₋₆ alkylthio group, C₁₋₆ alkylsulfinyl group, C₁₋₆ alkylsulfonyl group, an optionally esterified carboxyl group, carbamoyl group, thiocarbamoyl group, mono- or di-C₁₋₆ alkyl-carbamoyl group, mono- or di-C₆₋₁₄ aryl-carbamoyl group, sulfamoyl group, mono- or di-C₁₋₆ alkyl-sulfamoyl group and mono- or di-C₆₋₁₄ aryl-sulfamoyl group;
(31) a C₇₋₁₆ aralkyloxy group optionally substituted by 1 to 3 substituents selected from halogen atom, hydroxy group, amino group, nitro group, cyano group, optionally halogenated C₁₋₆ alkyl group, mono- or di-C₁₋₆ alkyl-amino group, C₆₋₁₄ aryl group, mono- or di-C₆₋₁₄ aryl-amino group, C₃₋₈ cycloalkyl group, C₁₋₆ alkoxy group, C₁₋₆ alkoxy-C₁₋₆ alkoxy group, C₁₋₆ alkylthio group, C₁₋₆ alkylsulfinyl group, C₁₋₆ alkylsulfonyl group, an optionally esterified carboxyl group, carbamoyl group, thiocarbamoyl group, mono- or di-C₁₋₆ alkyl-carbamoyl group, mono- or di-C₆₋₁₄ aryl-carbamoyl group, sulfamoyl group, mono- or di-C₁₋₆ alkyl-sulfamoyl group and mono- or di-C₆₋₁₄ aryl-sulfamoyl group;
(32) C₁₋₆ alkylsulfonyloxy group;
(33) tri-C₁₋₆ alkyl-silyloxy group;
(34) nitrogen-containing heterocycle-carbonyl group;
(35) C₆₋₁₄ aryl-carbonyl group;
(35) C₆₋₁₄ aryl-thio group;
(37) optionally halogenated C₆₋₁₄ aryl-sulfonyl group;
(38) nitrogen-containing heterocycle-sulfonyl group; and
(39) nitrogen-containing heterocycle-amino group optionally substituted by cyano group.

6. The compound of claim 2, wherein R¹ is a C₁₋₆ alkyl group optionally substituted by 1 to 5 substituents selected from halogen atom, optionally substituted hydroxy group, optionally substituted thio group, optionally substituted amino group, nitro group, cyano group, and optionally substituted carbonyl group.

7. The compound of claim 2 wherein R¹ is a C₁₋₆ alkyl group optionally having, at substitutable positions, 1 to 5 substituents selected from
(1) halogen atom;
(2) hydroxy group;
(3) amino group;
(4) nitro group;
(5) cyano group;
(7) mono- or di-C₁₋₆ alkyl-amino group;
(8) mono- or di-C₆₋₁₄ aryl-amino group;
(9) mono- or di-C₇₋₁₆ aralkyl-amino group;
(10) N-C₁₋₆ alkyl-N-C₆₋₁₄ aryl-amino group;
(11) N-C₁₋₆ alkyl-N-(C₁₋₆ alkyl-C₆₋₁₄ aryl)-amino group;
(12) N-C₁₋₆ alkyl-N-C₇₋₁₆ aralkyl-amino group;
(14) optionally halogenated C₁₋₆ alkoxy group;
(15) C₁₋₆ alkylthio group optionally substituted by C₁₋₆ alkoxy group;
(18) optionally esterified carboxyl group;
(19) carbamoyl group;
(20) thiocarbamoyl group;
(21) mono- or di-C₁₋₆ alkyl-carbamoyl group;
(22) mono- or di-C₆₋₁₄ aryl-carbamoyl group;
(23) mono- or di-5- to 7-membered heterocycle-carbamoyl group;
(24) C₁₋₆ alkyl-carbonylamino group optionally substituted by carboxyl group;
(25) C₆₋₁₄ aryloxy group optionally substituted by 1 to 3 substituents selected from halogen atom, hydroxy group, amino group, nitro group, cyano group, optionally halogenated C₁₋₆ alkyl group, mono- or di-C₁₋₆ alkyl-amino group, C₆₋₁₄ aryl group, mono- or di-C₆₋₁₄ aryl-amino group, C₃₋₈ cycloalkyl group, C₁₋₆ alkoxy group, C₁₋₆ alkoxy-C₁₋₆ alkoxy group, C₁₋₆ alkylthio group, C₁₋₆ alkylsulfinyl group, C₁₋₆ alkylsulfonyl group, an optionally esterified carboxyl group, carbamoyl group, thiocarbamoyl group, mono- or di-C₁₋₆ alkyl-carbamoyl group, mono- or di-C₆₋₁₄ aryl-carbamoyl group, sulfamoyl group, mono- or di-C₁₋₆ alkyl-sulfamoyl group and mono- or di-C₆₋₁₄ aryl-sulfamoyl group;
(27) heterocyclic oxy group optionally substituted by 1 to 3 substituents selected from halogen atom, hydroxy group, amino group, nitro group, cyano group, optionally halogenated C₁₋₆ alkyl group, mono- or di-C₁₋₆ alkyl-amino group, C₆₋₁₄ aryl group, mono- or di-C₆₋₁₄ aryl-amino group, C₃₋₈ cycloalkyl group, C₁₋₆ alkoxy group, C₁₋₆ alkoxy-C₁₋₆ alkoxy group, C₁₋₆ alkylthio group, C₁₋₆ alkylsulfinyl group, C₁₋₆ alkylsulfonyl group, an optionally esterified carboxyl group, carbamoyl group, thiocarbamoyl group, mono- or di-C₁₋₆ alkyl-carbamoyl group, mono- or di-C₆₋₁₄ aryl-carbamoyl group, sulfamoyl group, mono- or di-C₁₋₆ alkyl-sulfamoyl group and mono- or di-C₆₋₁₄ aryl-sulfamoyl group;
(31) C₇₋₁₆ aralkyloxy group optionally substituted by 1 to 3 substituents selected from halogen atom, hydroxy group, amino group, nitro group, cyano group, optionally halogenated C₁₋₆ alkyl group, mono- or di-C₁₋₆ alkyl-amino group, C₆₋₁₄ aryl group, mono- or di-C₆₋₁₄ aryl-amino group, C₃₋₈ cycloalkyl group, C₁₋₆ alkoxy group, C₁₋₆ alkoxy-C₁₋₆ alkoxy group, C₁₋₆ alkylthio group, C₁₋₆ alkylsulfinyl group, C₁₋₆ alkylsulfonyl group, an optionally esterified carboxyl group, carbamoyl group, thiocarbamoyl group, mono- or di-C₁₋₆ alkyl-carbamoyl group, mono- or di-C₆₋₁₄ aryl-carbamoyl group, sulfamoyl group, mono- or di-C₁₋₆ alkyl-sulfamoyl group and mono- or di-C₆₋₁₄ aryl-sulfamoyl group;
(32) C₁₋₆ alkylsulfonyloxy group;
(33) tri-C₁₋₆ alkyl-silyloxy group;
(34) nitrogen-containing heterocycle-carbonyl group;
(35) C₆₋₁₄ aryl-carbonyl group;
(35) C₆₋₁₄ aryl-thio group;
(37) optionally halogenated C₆₋₁₄ aryl-sulfonyl group;
(38) nitrogen-containing heterocycle-sulfonyl group; and
(39) nitrogen-containing heterocycle-amino group optionally substituted by cyano group.

8. The compound of claim 2, wherein R³ is a hydrogen atom.

9. The compound of claim 2, wherein R⁴ is an optionally substituted C₆₋₁₄ aryl group or an optionally substituted heterocyclic group.

10. The compound of claim 2, wherein R⁵ is a hydrogen atom.

11. The compound of claim 2, wherein R⁶ is a hydrogen atom.

12. The compound of claim 2, wherein R⁵ and R⁶ are hydrogen atoms.

13. The compound of claim 2, wherein X is a carbonyl group.

14. The compound of claim 2, which is selected from the following:
N-(3-(((2-cyanoethyl)amino)carbonyl)-1H-pyrazol-4-yl)pyridine-2-carboxamide;
N-(3-(((2-cyanoethyl)amino)carbonyl)-1H-pyrazol-4-yl)-6-methylpyridine-2-carboxamide;
N-(3-(((3,3,3-trifluoropropyl)amino)carbonyl)-1H-pyrazol-4-yl)pyridine-2-carboxamide;
4-(benzoylamino)-N-(2-cyanoethyl)-1H-pyrazole-3-carboxamide;
N-(3-(((3-isopropoxypropyl)amino)carbonyl)-1H-pyrazol-4-yl)-6-methylpyridine-2-carboxamide;
N-(2-cyanoethyl)-4-(2-furoylamino)-1H-pyrazole-3-carboxamide:
N-(3-(((3-hydroxypropyl)amino)carbonyl)-1H-pyrazol-4-yl)pyridine-2-carboxamide;
N-(3-(((2-isopropoxyethyl)amino)carbonyl)-1H-pyrazol-4-yl)-6-methylnicotinamide;
6-methyl-N-(3-(((2,2,2-trifluoroethyl)amino)carbonyl)-1H-pyrazol-4-yl)pyridine-3-carboxamide; and
N-(3-(((2-cyanoethyl)amino)carbonyl)-1H-pyrazol-4-yl)-6-methylpyridine-3-carboxamide.

15. A prodrug of the compound of claim 1.

16. A pharmaceutical agent comprising the compound of claim 1 or the prodrug of claim 15.

17. A GSK-3β inhibitor comprising the compound of claim 1 or the prodrug of claim 15.

18. A neural stem cell differentiation promoter comprising the GSK-3β inhibitor of claim 17.

19. An agent for the prophylaxis or treatment of neurodegenerative disease or diabetes, which comprises the compound of claim 1 or the prodrug of claim 15.

20. A hypoglycemic agent comprising the compound of claim 1 or the prodrug of claim 15.

21. A GSK-3β inhibitor comprising a compound represented by the formula: 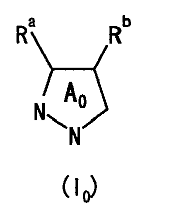 wherein
ring A₀ is a pyrazole ring optionally further having 1 or 2 substituents;
R^{a} is substituted carbamoyl; and
R^{b} is optionally substituted acylamino,
or a salt thereof or a prodrug thereof.

22. A neural stem cell differentiation promoter comprising the GSK-3β inhibitor of claim 21.

23. An agent for the prophylaxis or treatment of neurodegenerative disease or diabetes comprising the GSK-3β inhibitor of claim 21.

24. A hypoglycemic agent comprising the GSK-3β inhibitor of claim 21.

25. A method of inhibiting GSK-3β comprising administering a compound represented by the formula: 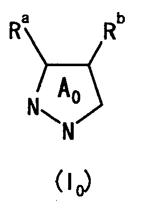 wherein
ring A₀ is a pyrazole ring optionally further having 1 or 2 substituents;
R^{a} is substituted carbamoyl; and
R^{b} is optionally substituted acylamino,
or a salt thereof or a prodrug thereof to a subject.

26. A method of promoting neural stem cell differentiation comprising administering a compound represented by the formula: 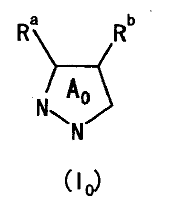 wherein
ring A₀ is a pyrazole ring optionally further having 1 or 2 substituents;
R^{a} is substituted carbamoyl; and
R^{b} is optionally substituted acylamino,
or a salt thereof or a prodrug thereof to a subject.

27. A method of preventing or treating a neurodegenerative disease or diabetes comprising administering a compound represented by the formula: 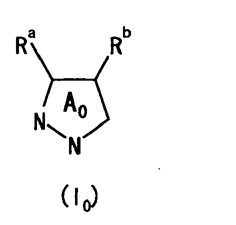 wherein
ring A₀ is a pyrazole ring optionally further having 1 or 2 substituents;
R^{a} is substituted carbamoyl; and
R^{b} is optionally substituted acylamino,
or a salt thereof or a prodrug thereof to a subject.

28. A method of lowering blood glucose comprising administering a compound represented by the formula: 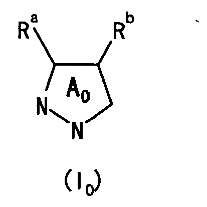 wherein
ring A₀ is a pyrazole ring optionally further having 1 or 2 substituents;
R^{a} is substituted carbamoyl; and
R^{b} is optionally substituted acylamino,
or a salt thereof or a prodrug thereof to a subject.

29. Use of a compound represented by the formula: 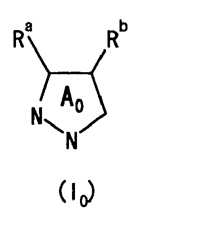 wherein
ring A₀ is a pyrazole ring optionally further having 1 or 2 substituents;
R^{a} is substituted carbamoyl; and
R^{b} is optionally substituted acylamino,
or a salt thereof or a prodrug thereof for the production of a GSK-3β inhibitor.

30. Use of a compound represented by the formula: 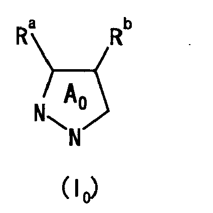 wherein
ring A₀ is a pyrazole ring optionally further having 1 or 2 substituents;
R^{a} is substituted carbamoyl; and
R^{b} is optionally substituted acylamino,
or a salt thereof or a prodrug thereof for the production of a neural stem cell differentiation promoter.

31. Use of a compound represented by the formula: 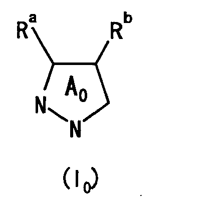 wherein
ring A₀ is a pyrazole ring optionally further having 1 or 2 substituents;
R^{a} is substituted carbamoyl; and
R^{b} is optionally substituted acylamino,
or a salt thereof or a prodrug thereof for the production of an agent for the prophylaxis or treatment of a neurodegenerative disease or diabetes.

32. Use of a compound represented by the formula: 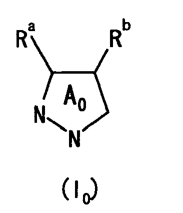 wherein
ring A₀ is a pyrazole ring optionally further having 1 or 2 substituents;
R^{a} is substituted carbamoyl; and
R^{b} is optionally substituted acylamino,
or a salt thereof or a prodrug thereof for the production of a hypoglycemic agent.


SEQUENCE LISTING

<110>  Takeda Pharmaceutical Company Limited

<120>  Pyrazole compound

<130>  09872

<150>  JP 2005-033356
<151>  2005-02-09

<150>  JP 2005-370962
<151>  2005-12-22

<160>  3

<170>  PatentIn version 3.2

<210>  1
<211>  65
<212>  DNA
<213>  Artificial

<220>
<223>  Primer for cloning human GSK3beta gene

<400>  1
aaagaattca ccatggacta caaggacgac gatgacaagt cagggcggcc cagaaccacc      60

tcctt                                                                  65


<210>  2
<211>  39
<212>  DNA
<213>  Artificial

<220>
<223>  Primer for cloning human GSK3beta gene

<400>  2
aaaagtcgac tcaggtggag ttggaagctg atgcagaag                             39


<210>  3
<211>  26
<212>  PRT
<213>  Artificial

<220>
<223>  Substrate peptide


<220>
<221>  MOD_RES
<222>  (21)..(21)
<223>  PHOSPHORYLATION

<400>  3

Tyr Arg Arg Ala Ala Val Pro Pro Ser Pro Ser Leu Ser Arg His Ser
1               5                   10                  15


Ser Pro His Gln Ser Glu Asp Glu Glu Glu
            20                  25